# EUROPEAN PATENT SPECIFICATION

(11) **EP 4 384 523 B1**
(45) Date of publication and mention of the grant of the patent: **08.04.2026**
(21) Application number: 22765441.5
(22) Date of filing: 10.08.2022
(51) Int. Cl.: C07D 491/056, C07D 471/04, A01N 43/90, A01P 5/00, A01P 7/02, A01P 7/04, A01P 9/00

(54) **2,2-DIFLUORO-5H-[1,3]DIOXOLO[4,5-F]ISOINDOL-7-ONE DERIVATIVES AS PESTICIDES**
2,2-DIFLUORO-5H-[1,3]DIOXOLO[4,5-F]ISOINDOL-7-ON-DERIVATE ALS PESTIZIDE
DÉRIVÉS DE 2,2-DIFLUORO-5H-[1,3]DIOXOLO[4,5-F]ISOINDOL-7-ONE EN TANT QUE PESTICIDES

(30) Priority: 10.08.2021 IN 202111036124
(43) Date of publication of application: 19.06.2024
(73) Proprietor: Syngenta Crop Protection AG, 4058 Basel (CH)
(72) Inventor: SIKERVAR, Vikas, Bracknell Berkshire RG42 6EY (GB); MUEHLEBACH, Michel, 4332 Stein (CH); HUETER, Ottmar Franz, 4332 Stein (CH); STOLLER, André, 4332 Stein (CH); EMERY, Daniel, 4332 Stein (CH); SASMAL, Swarnendu, Ilhas Goa 403110 (IN)
(86) International application number: PCT/EP2022/072481
(87) International publication number: WO 2023/017094

(56) References cited:
- WO-A1-2021/140122

## Description

The present invention relates to pesticidally active, in particular insecticidally active heterocyclic derivatives containing sulfur substituents, to processes for their preparation, to compositions comprising those compounds, and to their use in a method for controlling animal pests, including arthropods and in particular insects or representatives of the order Acarina, with the exception of a method for treatment of the human or animal body by surgery or therapy and diagnostic methods practised on the human or animal body.

Heterocyclic benzannulated dihydropyrrolone and phtalimide derivatives with sulfur-containing substituents have been described in the literature, for example in J. Org. Chem. 2003, 62, 8240 and Bull. Chem Soc. Chim. Belg. 1997, 106, 151. However, none of these references have described to have a pesticidal effect. Structurally different pesticidally active heterocyclic derivatives with sulfur-containing substituents have been described, for example in WO2012/012086848, WO2013/018928, WO2019/131575 and WO2020/013147.

It has now surprisingly been found that certain novel pesticidally active derivatives with sulfur containing substitutents have favorable properties as pesticides.

The present invention therefore provides compounds of formula I, 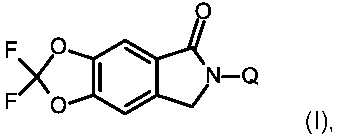 wherein
Q is a radical selected from the group consisting of formula Qa, Qb and Qc, 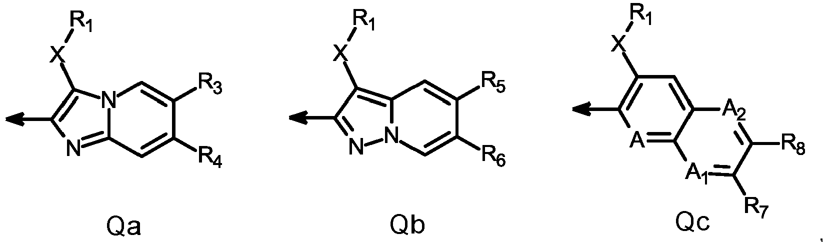
wherein the arrow denotes the point of attachment to the nitrogen atom of the tricyclic ring; and wherein
A, A₁ and A₂ are, independently from each other, CH or N;
X is S, SO, or SO₂;
R₁ is C₁-C₄alkyl or C₃-C₆cycloalkyl-C₁-C₄alkyl;
R₃, R₄, R₅ and R₆ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, -N(R₉R₁₀), or -N(R₉)C(=O)R₁₀;
R₉ and R₁₀ are, independently from each other, hydrogen, C₁-C₄alkyl, C₁-C₆haloalkyl, or C₃-C₆cycloalkyl;
R₇ and R₈ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy or -N(R₁₁)C(=O)R₁₂; and
R₁₁ and R₁₂ are, independently from each other, hydrogen, C₁-C₄alkyl, C₁-C₆haloalkyl, or C₃-C₆cycloalkyl.

The present invention also provides agrochemically acceptable salts, stereoisomers, enantiomers, tautomers and N-oxides of the compounds of formula I.

Compounds of formula I which have at least one basic centre can form, for example, acid addition salts, for example with strong inorganic acids such as mineral acids, for example perchloric acid, sulfuric acid, nitric acid, nitrous acid, a phosphorus acid or a hydrohalic acid, with strong organic carboxylic acids, such as C₁-C₄alkanecarboxylic acids which are unsubstituted or substituted, for example by halogen, for example acetic acid, such as saturated or unsaturated dicarboxylic acids, for example oxalic acid, malonic acid, succinic acid, maleic acid, fumaric acid or phthalic acid, such as hydroxycarboxylic acids, for example ascorbic acid, lactic acid, malic acid, tartaric acid or citric acid, or such as benzoic acid, or with organic sulfonic acids, such as C₁-C₄alkane- or arylsulfonic acids which are unsubstituted or substituted, for example by halogen, for example methane- or p-toluenesulfonic acid. Compounds of formula I which have at least one acidic group can form, for example, salts with bases, for example mineral salts such as alkali metal or alkaline earth metal salts, for example sodium, potassium or magnesium salts, or salts with ammonia or an organic amine, such as morpholine, piperidine, pyrrolidine, a mono-, di- or tri-lower-alkylamine, for example ethyl-, diethyl-, triethyl- or dimethylpropylamine, or a mono-, di- or trihydroxy-lower-alkylamine, for example mono-, di- or triethanolamine.

In each case, the compounds of formula (I) according to the invention are in free form, in oxidized form as a N-oxide or in salt form, e.g. an agronomically usable salt form.

N-oxides are oxidized forms of tertiary amines or oxidized forms of nitrogen containing heteroaromatic compounds. They are described for instance in the book "Heterocyclic N-oxides" by A. Albini and S. Pietra, CRC Press, Boca Raton 1991.

The compounds of formula I according to the invention also include hydrates which may be formed during the salt formation.

Where substituents are indicated as being itself further substituted, this means that they carry one or more identical or different substituents, e.g. one to four substituents. Normally not more than three such optional substituents are present at the same time. Preferably not more than two such substituents are present at the same time (i.e. the group is substituted by one or two of the substituents indicated). Where the additional substituent group is a larger group, such as cycloalkyl or phenyl, it is most preferred that only one such optional substituent is present. Where a group is indicated as being substituted, e.g. alkyl, this includes those groups that are part of other groups, e.g. the alkyl in alkylthio.

The term "C₁-Cₙalkyl" as used herein refers to a saturated straight-chain or branched hydrocarbon radical attached via any of the carbon atoms having 1 to n carbon atoms, for example, any one of the radicals methyl, ethyl, n-propyl, 1-methylbutyl, 2-methylbutyl, 3-methylbutyl, 2, 2-dimethylpropyl, 1-ethylpropyl, n-hexyl, n-pentyl, 1, 1-dimethylpropyl, 1, 2-dimethylpropyl, 1- methylpentyl, 2-methylpentyl, 3-methylpentyl, 4-methylpentyl, 1, 1-dimethylbutyl, 1,2- dimethylbutyl, 1, 3-dimethylbutyl, 2, 2-dimethylbutyl, 2, 3-dimethylbutyl, 3, 3-dimethylbutyl, 1-ethylbutyl, 2-ethylbutyl, 1,1, 2-trimethylpropyl, 1,2, 2-trimethylpropyl, 1-ethyl-1- methylpropyl, or 1-ethyl-2-methylpropyl.

The term "C₁-Cₙhaloalkyl" as used herein refers to a straight-chain or branched saturated alkyl radical attached via any of the carbon atoms having 1 to n carbon atoms (as mentioned above), where some or all of the hydrogen atoms in these radicals may be replaced by fluorine, chlorine, bromine and/or iodine, i.e., for example, any one of chloromethyl, dichloromethyl, trichloromethyl, fluoromethyl, difluoromethyl, trifluoromethyl, chlorofluoromethyl, dichlorofluoromethyl, chlorodifluoromethyl, 2-fluoroethyl, 2-chloroethyl, 2-bromoethyl, 2-iodoethyl, 2, 2-difluoroethyl, 2,2, 2-trifluoroethyl, 2-chloro-2-fluoroethyl, 2-chloro-2, 2-difluoroethyl, 2, 2-dichloro-2-fluoroethyl, 2,2, 2-trichloroethyl, pentafluoroethyl, 2-fluoropropyl, 3-fluoropropyl, 2,2- difluoropropyl, 2, 3-difluoropropyl, 2-chloropropyl, 3-chloropropyl, 2, 3-dichloropropyl, 2- bromopropyl, 3-bromopropyl, 3,3, 3-trifluoropropyl, 3,3, 3-trichloropropyl, 2,2, 3,3, 3- pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl or nonafluorobutyl. According a term "C₁-C₂-fluoroalkyl" would refer to a C₁-C₂-alkyl radical which carries 1,2, 3,4, or 5 fluorine atoms, for example, any one of difluoromethyl, trifluoromethyl, 1-fluoroethyl, 2-fluoroethyl, 2, 2-difluoroethyl, 2,2, 2-trifluoroethyl, 1,1, 2, 2-tetrafluoroethyl or penta- fluoroethyl.

The term "C₁-Cₙalkoxy" as used herein refers to a straight-chain or branched saturated alkyl radical having 1 to n carbon atoms (as mentioned above) which is attached via an oxygen atom, i.e., for example, any one of methoxy, ethoxy, n-propoxy, 1-methylethoxy, n-butoxy, 1-methylpropoxy, 2-methylpropoxy or 1, 1-dimethylethoxy.

The term "C₁-Cₙhaloalkoxy" as used herein refers to a C₁-Cₙalkoxy radical as mentioned above which is partially or fully substituted by fluorine, chlorine, bromine and/or iodine, i.e., for example, any one of chloromethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 2-fluoroethoxy, 2-chloroethoxy, 2-bromoethoxy, 2-iodoethoxy, 2, 2-difluoroethoxy, 2,2, 2-trifluoroethoxy, 2-chloro-2-fluoroethoxy, 2-chloro-2, 2-difluoroethoxy, 2, 2-dichloro-2-fluoroethoxy, 2,2, 2-trichloroethoxy, pentafluoroeth- oxy, 2-fluoropropoxy, 3-fluoropropoxy, 2, 2-difluoropropoxy, 2, 3-difluoropropoxy, 2- chloropropoxy, 3-chloropropoxy, 2, 3-dichloropropoxy, 2-bromopropoxy, 3-bromopropoxy, 3,3, 3-trifluoropropoxy, 3,3, 3-trichloropropoxy, 2,2, 3,3, 3- pentafluoropropoxy, heptafluoropropoxy, 1- (fluoromethyl)-2-fluoroethoxy, 1- (chloromethyl)-2-chloroethoxy, 1-(bromomethyl)-2-bromoethoxy, 4-fluorobutoxy, 4- chlorobutoxy, or 4-bromobutoxy.

The term "C₁-Cₙcyanoalkyl" as used herein refers to a straight chain or branched saturated alkyl radicals having 1 to n carbon atoms (as mentioned above) which is substituted by a cyano group, for example cyanomethylene, cyanoethylene, 1,1-dimethylcyanomethyl, cyanomethyl, cyanoethyl, cyanoisopropyl and 1-dimethylcyanomethyl.

The term "C₁-Cₙcyanoalkoxy" refers to a straight-chain or branched saturated cyanoalkyl radical having 1 to n carbon atoms (as mentioned above) but which is attached *via* an oxygen atom.

The term "C₃-C₆cycloalkyl" as used herein refers to 3-6 membered cycloylkyl groups such as cyclopropane, cyclobutane, cyclopropane, cyclopentane and cyclohexane.

The suffix "-C₁-Cₙalkyl" after terms such as "C₃-Cₙcycloalkyl", wherein n is an integer from 1-6, as used herein refers to a straight chain or branched saturated alkyl radicals which is substituted by C₃-Cₙcycloalkyl. An example of C₃-Cₙcycloalkyl-C₁-Cₙalkyl is for example, cyclopropylmethyl.

The term "C₃-C₆cycloalkyl" monosubstituted by cyano as used herein refers to refers to 3-6 membered cycloylkyl groups (as mentioned above) which is substituted by a cyano group. An example of C₃-C₆cycloalkyl monosubstituted by cyano is 1-cyanocyclopropyl.

Halogen is generally fluorine, chlorine, bromine or iodine. This also applies, correspondingly, to halogen in combination with other meanings, such as haloalkyl.

Certain embodiments according to the invention are provided as set out below.

Embodiment 1 provides compounds of formula I, or an agrochemically acceptable salt, stereoisomer, enantiomer, tautomer or N-oxide thereof, as defined above.

Embodiment 2 provides compounds, or an agrochemically acceptable salt, stereoisomer, enantiomer, tautomer or N-oxide thereof, according to embodiment 1 wherein Q is Qa and having preferred values of X, R₁, R₃, R₄, R₉ and R₁₀ as set out below.

Embodiment 3 provides compounds, or an agrochemically acceptable salt, stereoisomer, enantiomer, tautomer or N-oxide thereof, according to embodiment 1 wherein Q is Qb and having preferred values of X, R₁, R₅, R₆, R₉ and R₁₀ as set out below.

Embodiment 4 provides compounds, or an agrochemically acceptable salt, stereoisomer, enantiomer, tautomer or N-oxide thereof, according to embodiment 1 wherein Q is Qc and having preferred values of A, A₁, A₂, X, R₁, R₇, R₈, R₁₁ and R₁₂ as set out below.

With respect to embodiments 1 - 4, preferred values of X, R₁, A, A₁, A₂, R₃, R₄, R₅, R₆, R₇, R₈, R₉, R₁₀, R₁₁ and R₁₂ are, in any combination thereof, as set out below:
Preferably X is S or SO₂.
Most preferably X is SO₂.
Preferably R₁ is C₁-C₄alkyl or cyclopropyl-C₁-C₄alkyl.
More preferably R₁ is ethyl or cyclopropylmethyl.
Most preferably R₁ is ethyl.
Preferably R₃ and R₄ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, or -N(R₉)C(=O)R₁₀.
More preferably R₃ and R₄ are, independently from each other, hydrogen, halogen, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.
Even more preferably R₄ is hydrogen and R₃ is hydrogen, halogen, trifluoromethyl, 1,1-difluoroethyl, - OCHF₂, -OCH₂CHF₂, -OCH₂CF₃, cyclopropyl, cyanocyclopropyl, cyanoisopropyl, trifluoromethoxy, - CHF₂, -OC(CH₃)₂CN, -NHC(O)CH₃ or -NCH₃C(O)CH₃; or
R₃ is hydrogen and R₄ is halogen, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.
Most preferably R₄ is hydrogen and R₃ is hydrogen, halogen, trifluoromethyl, 1,1-difluoroethyl, - OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or -NCH₃C(O)CH₃; or
R₃ is hydrogen and R₄ is trifluoromethyl, halogen, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, - NHC(O)CH₃ or -NCH₃C(O)CH₃.

Preferably R₅ and R₆ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, or -N(R₉)C(=O)R₁₀.

More preferably R₅ and R₆ are, independently from each other, hydrogen, halogen, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.

Even more preferably R₆ is hydrogen and R₅ is hydrogen, halogen, trifluoromethyl, 1,1-difluoroethyl, - OCHF₂, -OCH₂CHF₂, -OCH₂CF₃, cyclopropyl, cyanocyclopropyl, cyanoisopropyl, trifluoromethoxy, - CHF₂, -OC(CH₃)₂CN, -NHC(O)CH₃ or -NCH₃C(O)CH₃; or

R₅ is hydrogen and R₆ is halogen, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.

Most preferably R₆ is hydrogen and R₅ is hydrogen, halogen, trifluoromethyl, 1,1-difluoroethyl, - OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or -NCH₃C(O)CH₃; or

R₅ is hydrogen and R₆ is halogen, trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, - NHC(O)CH₃ or -NCH₃C(O)CH₃.

Preferably R₉ and R₁₀ are, independently from each other, hydrogen or C₁-C₄alkyl.

More preferably R₉ and R₁₀ are, independently from each other, hydrogen or methyl.

Most preferably R₉ is hydrogen or methyl, and R₁₀ is methyl.

Preferably A is CH or N.

Most preferably A is N.

Preferably A₁ is CH or N.

Preferably A₂ is CH or N.

Also preferred is when A₁ is CH and A₂ is N

Also preferred is when A₁ is N and A₂ is CH

Further preferred is when A₁ and A₂ are both N

Most preferably A₁ and A₂ are both CH

Preferably R₇ and R₈ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, or -N(R₁₁)C(=O)R₁₂.

More preferably R₇ and R₈ are, independently from each other, hydrogen, halogen, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.

Even more preferably R₈ is hydrogen and R₇ is hydrogen, halogen, trifluoromethyl, 1,1-difluoroethyl, - OCHF₂, -OCH₂CHF₂, -OCH₂CF₃, cyclopropyl, cyanocyclopropyl, cyanoisopropyl, trifluoromethoxy, - CHF₂, -OC(CH₃)₂CN, -NHC(O)CH₃ or -NCH₃C(O)CH₃; or

R₇ is hydrogen and R₈ is trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.

Most preferably R₈ is hydrogen and R₇ is hydrogen, trifluoromethyl, 1,1-difluoroethyl, -OCHF₂, - OCH₂CHF₂, -OCH₂CF₃, cyclopropyl, 1-cyanocyclopropyl, 1-cyano-1-methyl-ethyl, trifluoromethoxy, - CHF₂, -OC(CH₃)₂CN, -NHC(O)CH₃ or -NCH₃C(O)CH₃; or

R₇ is hydrogen and R₈ is trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, - NHC(O)CH₃ or -NCH₃C(O)CH₃.

Preferably R₁₁ and R₁₂ are, independently from each other, hydrogen or C₁-C₄alkyl.

More preferably R₁₁ and R₁₂ are, independently from each other, hydrogen or methyl.

Most preferably R₁₁ is hydrogen or methyl, and R₁₂ is methyl.

Further embodiments according to the invention are provided as set forth below.

A preferred group of compounds of formula I is represented by the compounds of formula I-1 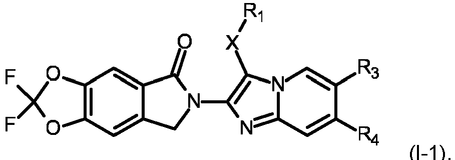 wherein X, R₁, R₃, R₄, R₉ and R₁₀ are as defined under formula I above, or an agrochemically acceptable salt, stereoisomer, enantiomer, tautomer or N-oxide of a compound of formula I-1.

In one preferred group of compounds of formula I-1, R₁ is C₁-C₄alkyl or C₃-C₆cycloalkyl-C₁-C₄alkyl; X is S or SO₂; R₃ and R₄ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, or -N(R₉)C(=O)R₁₀; and R₉ and R₁₀ are, independently from each other, hydrogen or C₁-C₄alkyl; preferably R₉ and R₁₀ are, independently from each other, hydrogen or methyl; more preferably R₉ is hydrogen or methyl, and R₁₀ is methyl.

In another preferred group of compounds of formula I-1, R₁ is ethyl or cyclopropylmethyl, preferably R₁ is ethyl; X is S or SO₂, preferably X is SO₂; and R₃ and R₄ are, independently from each other, hydrogen, fluoro, chloro, bromo, iodo, trifluoromethyl, 1,1-difluoroethyl, -OCHF₂, -OCH₂CHF₂, - OCH₂CF₃, cyclopropyl, cyanocyclopropyl, cyanoisopropyl, trifluoromethoxy, -CHF₂, -OC(CH₃)₂CN, - NHC(O)CH₃ or -NCH₃C(O)CH₃.

In the compounds of formula I-1 and all of the preferred embodiments of compounds of formula I-1 mentioned herein, unless otherwise specified, X, R₁, R₃, R₄, R₉ and R₁₀ are as defined under formula I above.

Another preferred embodiment of the compounds of formula I-1 are those wherein R₁ is ethyl; and X is SO₂.

Also preferred are the compounds of formula I-1 wherein R₄ is hydrogen and R₃ is hydrogen, fluoro, chloro, bromo, iodo, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or - NCH₃C(O)CH₃; or the compounds of formula I-1 wherein R₃ is hydrogen and R₄ is trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.

Further preferred are the compounds of formula I-1 wherein R₄ is hydrogen and R₃ is hydrogen, fluoro, chloro, bromo, iodo, trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or - NCH₃C(O)CH₃; or the compounds of formula I-1 wherein R₃ is hydrogen and R₄ is fluoro, chloro, bromo, iodo, trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or - NCH₃C(O)CH₃.

Another preferred group of compounds of formula I is represented by the compounds of formula I-2 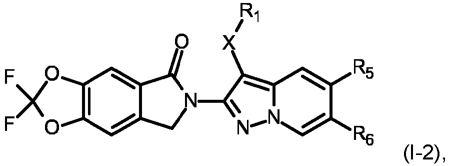 wherein X, R₁, R₅, R₆, R₉ and R₁₀ are as defined under formula I above, or an agrochemically acceptable salt, stereoisomer, enantiomer, tautomer or N-oxide of a compound of formula I-2.

In one preferred group of compounds of formula I-2, R₁ is C₁-C₄alkyl or C₃-C₆cycloalkyl-C₁-C₄alkyl; X is S or SO₂; R₅ and R₆ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, or -N(R₉)C(=O)R₁₀; and R₉ and R₁₀ are, independently from each other, hydrogen or C₁-C₄alkyl; preferably R₉ and R₁₀ are, independently from each other, hydrogen or methyl, more preferably R₉ is hydrogen or methyl, and R₁₀ is methyl.

In another preferred group of compounds of formula I-2, R₁ is ethyl or cyclopropylmethyl, preferably R₁ is ethyl; X is S or SO₂, preferably X is SO₂; R₅ and R₆ are, independently from each other, hydrogen, fluoro, chloro, bromo, iodo, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.

In the compounds of formula I-2 and all of the preferred embodiments of compounds of formula I-2 mentioned herein, unless otherwise specified, X, R₁, R₅, R₆, R₉ and R₁₀ are as defined under formula I above.

Another preferred embodiment of the compounds of formula I-2 are those wherein R₁ is ethyl; and X is SO₂.

Also preferred are the compounds of formula I-2 wherein R₆ is hydrogen and R₅ is hydrogen, fluoro, chloro, bromo, iodo, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or - NCH₃C(O)CH₃; or the compounds of formula I-2 wherein R₅ is hydrogen and R₆ is trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.

Further preferred are the compounds of formula I-2 wherein R₆ is hydrogen and R₅ is hydrogen, fluoro, chloro, bromo, iodo, trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or - NCH₃C(O)CH₃; or the compounds of formula I-2 wherein R₅ is hydrogen and R₆ is trifluoromethyl, 1,1-difluoroethyl, -OCHF₂, -OCH₂CHF₂, -OCH₂CF₃, cyclopropyl, 1-cyanocyclopropyl, 1-cyano-1-methylethyl, trifluoromethoxy, -CHF₂, -OC(CH₃)₂CN, -NHC(O)CH₃ or -NCH₃C(O)CH₃.

Another preferred group of compounds of formula I is represented by the compounds of formula I-3 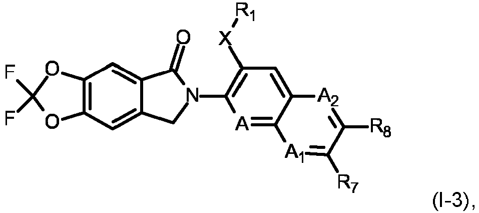 wherein A, A₁, A₂, X, R₁, R₇, R₈, R₁₁ and R₁₂ are as defined under formula I above, or an agrochemically acceptable salt, stereoisomer, enantiomer, tautomer or N-oxide of a compound of formula I-3.

In one preferred group of compounds of formula I-3, A is CH or N; A₁ is CH or N; A₂ is CH or N; R₁ is C₁-C₄alkyl or C₃-C₆cycloalkyl-C₁-C₄alkyl; X is S or SO₂; R₇ and R₈ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, or -N(R₁₁)C(=O)R₁₂; and R₁₁ and R₁₂ are, independently from each other, hydrogen or C₁-C₄alkyl; preferably R₁₁ and R₁₂ are, independently from each other, hydrogen or methyl; more preferably R₁₁ is hydrogen or methyl, and R₁₂ is methyl.

In another preferred group of compounds of formula I-3, A is CH or N, preferably A is N; A₁ is CH or N; A₂ is CH or N; R₁ is ethyl or cyclopropylmethyl, preferably R₁ is ethyl; X is S or SO₂, preferably X is SO₂; R₇ and R₈ are, independently from each other, hydrogen, trifluoromethyl, 1,1-difluoroethyl, - OCHF₂, -OCH₂CHF₂, -OCH₂CF₃, cyclopropyl, cyanocyclopropyl, cyanoisopropyl, trifluoromethoxy, - CHF₂, -OC(CH₃)₂CN, -NHC(O)CH₃ or -NCH₃C(O)CH₃.

In the compounds of formula I-3 and all of the preferred embodiments of compounds of formula I-3 mentioned herein, unless otherwise specified, A, A₁, A₂, X, R₁, R₇, R₈, R₁₁ and R₁₂ are as defined under formula I above.

Another preferred embodiment of the compounds of formula I-3 are those wherein R₁ is ethyl; and X is SO₂.

Also preferred are the compounds of formula I-3 wherein R₈ is hydrogen and R₇ is hydrogen, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃; or the compounds of formula I-3 wherein R₇ is hydrogen and R₈ is trifluoromethyl, 1,1-difluoroethyl, - OCHF₂, -OCH₂CHF₂, -OCH₂CF₃, cyclopropyl, cyanocyclopropyl, cyanoisopropyl, trifluoromethoxy, - CHF₂, -OC(CH₃)₂CN, -NHC(O)CH₃ or -NCH₃C(O)CH₃.

Further preferred are the compounds of formula I-3 wherein R₈ is hydrogen and R₇ is hydrogen, trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or -NCH₃C(O)CH₃; or the compounds of formula I-3 wherein R₇ is hydrogen and R₈ is trifluoromethyl, 1,1-difluoroethyl, - OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or -NCH₃C(O)CH₃.

One further preferred group of compounds according to this embodiment are compounds of formula (I-3a), which are compounds of formula I-3, or of any of the preferred embodiments of the compounds of formula I-3, wherein A is CH.

Another preferred group of compounds according to this embodiment are compounds of formula (I-3b), which are compounds of formula I-3, or of any of the preferred embodiments of the compounds of formula I-3, wherein A is N.

Yet another preferred group of compounds according to this embodiment are compounds of formula (I-3c), which are compounds of formula I-3, or of any of the preferred embodiments of the compounds of formula I-3, wherein A₁ is CH and A₂ is N.

A further preferred group of compounds according to this embodiment are compounds of formula (I-3d), which are compounds of formula I-3, or of any of the preferred embodiments of the compounds of formula I-3, wherein A₁ is N and A₂ is CH.

A further preferred group of compounds according to this embodiment are compounds of formula (I-3e), which are compounds of formula I-3, or of any of the preferred embodiments of the compounds of formula I-3, wherein A₁ and A₂ are both N.

Most preferred are the compounds of formula (I-3f) according to this embodiment, which are compounds of formula I-3, or of any of the preferred embodiments of the compounds of formula I-3, wherein A₁ and A₂ are both CH.

Another preferred group of compounds of formula I is represented by the compounds of formula I-4 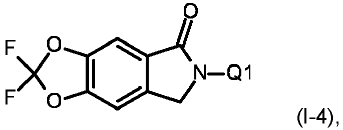 wherein
Q1 is a radical selected from the group consisting of formula Q1a, Q1b and Q1c, 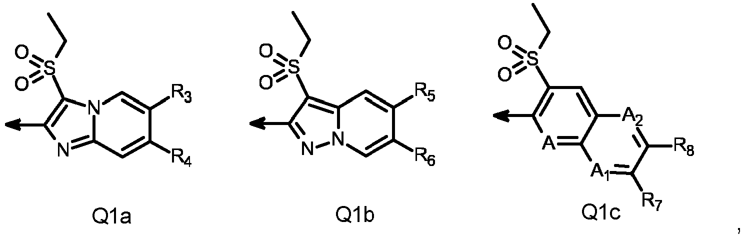
wherein the arrow denotes the point of attachment to the nitrogen atom of the tricyclic ring;
and wherein A, A₁, A₂, R₃, R₄, R₅, R₆, R₇, R₈, R₉, R₁₀, R₁₁ and R₁₂ are as defined under formula I above, or an agrochemically acceptable salt, stereoisomer, enantiomer, tautomer or N-oxide of a compound of formula I-4.

In the compounds of formula I-4 and all of the preferred embodiments of compounds of formula I-4 mentioned herein, unless otherwise specified, A, A₁, A₂, R₃, R₄, R₅, R₆, R₇, R₈, R₉, R₁₀, R₁₁ and R₁₂ are as defined under formula I above.

One preferred group of compounds of formula I-4 are the compounds of formula (I-4a), which are compounds of formula I-4, or of any of the preferred embodiments of the compounds of formula I-4, wherein Q is Q1a, R₄ is hydrogen and R₃ hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, -N(R₉R₁₀), or -N(R₉)C(=O)R₁₀; and R₉ and R₁₀ are, independently from each other, hydrogen, C₁-C₄alkyl, C₁-C₆haloalkyl, or C₃-C₆cycloalkyl; or
wherein Q is Q1a, R₃ is hydrogen and R₄ hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, -N(R₉R₁₀), or -N(R₉)C(=O)R₁₀; and R₉ and R₁₀ are, independently from each other, hydrogen, C₁-C₄alkyl, C₁-C₆haloalkyl, or C₃-C₆cycloalkyl.

Further preferred are the compounds of formula I-4a wherein R₄ is hydrogen and R₃ is hydrogen, halogen, trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or - NCH₃C(O)CH₃; or the compounds of formula I-4a wherein R₃ is hydrogen and R₄ is trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or -NCH₃C(O)CH₃.

Another preferred group of compounds of formula I-4 are the compounds of formula (I-4b), which are compounds of formula I-4, or of any of the preferred embodiments of the compounds of formula I-4, wherein Q is Q1b, R₆ is hydrogen and R₅ hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, -N(R₉R₁₀), or -N(R₉)C(=O)R₁₀; and R₉ and R₁₀ are, independently from each other, hydrogen, C₁-C₄alkyl, C₁-C₆haloalkyl, or C₃-C₆cycloalkyl; or
wherein Q is Q1b, R₅ is hydrogen and R₆ hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, -N(R₉R₁₀), or -N(R₉)C(=O)R₁₀; and R₉ and R₁₀ are, independently from each other, hydrogen, C₁-C₄alkyl, C₁-C₆haloalkyl, or C₃-C₆cycloalkyl.

Further preferred are the compounds of formula I-4b wherein R₆ is hydrogen and R₅ is hydrogen, halogen, trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or - NCH₃C(O)CH₃; or the compounds of formula I-4b wherein R₅ is hydrogen and R₆ is trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or -NCH₃C(O)CH₃.

Another preferred group of compounds of formula I-4 are the compounds of formula (I-4c), which are compounds of formula I-4, or of any of the preferred embodiments of the compounds of formula I-4, wherein Q is Q1c, R₈ is hydrogen and R₇ is hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy or -N(R₁₁)C(=O)R₁₂; and R₁₁ and R₁₂ are, independently from each other, hydrogen, C₁-C₄alkyl, C₁-C₆haloalkyl, or C₃-C₆cycloalkyl; or
wherein Q is Q1c, R₇ is hydrogen and R₈ is hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy or -N(R₁₁)C(=O)R₁₂; and R₁₁ and R₁₂ are, independently from each other, hydrogen, C₁-C₄alkyl, C₁-C₆haloalkyl, or C₃-C₆cycloalkyl.

Further preferred are the compounds of formula I-4c wherein R₈ is hydrogen and R₇ is hydrogen, trifluoromethyl, 1,1-difluoroethyl, -OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or -NCH₃C(O)CH₃; or the compounds of formula I-4c wherein R₇ is hydrogen and R₈ is trifluoromethyl, 1,1-difluoroethyl, - OCHF₂, -OCH₂CHF₂, -OCH₂CF₃, cyclopropyl, 1-cyanocyclopropyl, 1-cyano-1-methyl-ethyl, trifluoromethoxy, -CHF₂, -OC(CH₃)₂CN, -NHC(O)CH₃ or -NCH₃C(O)CH₃.

One further preferred group of compounds according to this embodiment are compounds of formula (I-4c-1), which are compounds of formula I-4c, or of any of the preferred embodiments of the compounds of formula I-4c, wherein A is CH.

Another preferred group of compounds according to this embodiment are compounds of formula (I-4c-2), which are compounds of formula I-4c, or of any of the preferred embodiments of the compounds of formula I-4c, wherein A is N.

Yet another preferred group of compounds according to this embodiment are compounds of formula (I-4c-3), which are compounds of formula I-4c, or of any of the preferred embodiments of the compounds of formula I-4c, wherein A₁ is CH and A₂ is N.

A further preferred group of compounds according to this embodiment are compounds of formula (I-4c-4), which are compounds of formula I-4c, or of any of the preferred embodiments of the compounds of formula I-4c, wherein A₁ is N and A₂ is CH.

A further preferred group of compounds according to this embodiment are compounds of formula (I-4c-5), which are compounds of formula I-4c, or of any of the preferred embodiments of the compounds of formula I-4c, wherein A₁ and A₂ are both N.

Another preferred group of compounds according to this embodiment are the compounds of formula (I-4c-6), which are compounds of formula I-4c, or of any of the preferred embodiments of the compounds of formula I-4c, wherein A₁ and A₂ are both CH.

Compounds according to the invention may possess any number of benefits including, inter alia, advantageous levels of biological activity for protecting plants against insects or superior properties for use as agrochemical active ingredients (for example, greater biological activity, an advantageous spectrum of activity, an increased safety profile, improved physico-chemical properties, or increased biodegradability or environmental profile). In particular, it has been surprisingly found that certain compounds of formula (I) may show an advantageous safety profile with respect to non-target arthropods, in particular pollinators such as honey bees, solitary bees, and bumble bees. Most particularly, Apis mellifera.

In another aspect the present invention provides a composition comprising an insecticidally, acaricidally, nematicidally or molluscicidally effective amount of a compound of formula (I), or an agrochemically acceptable salt, stereoisomer, enantiomer, tautomer or N-oxide thereof, as defined in any of the embodiments under compounds of formula (I), (I-1), (I-2), (I-3) and (I-4) (above), and, optionally, an auxiliary or diluent.

In a further aspect the present invention provides a method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound of formula (I), or an agrochemically acceptable salt, stereoisomer, enantiomer, tautomer or N-oxide thereof, as defined in any of the embodiments under compounds of formula (I), (I), (I-1), (I-2), (I-3) and (I-4) (above) or a composition as defined above, with the exception of a method for treatment of the human or animal body by surgery or therapy and diagnostic methods practised on the human or animal body.

In a yet further aspect, the present invention provides a method for the protection of plant propagation material from the attack by insects, acarines, nematodes or molluscs, which comprises treating the propagation material or the site, where the propagation material is planted, with a composition as defined above.

The process according to the invention for preparing compounds of formula I is carried out in principle by methods known to those skilled in the art. More specifically, the subgroup of compounds of formula I, wherein X is SO (sulfoxide) and/or SO₂ (sulfone), may be obtained by means of an oxidation reaction of the corresponding sulfide compounds of formula I, wherein X is S, involving reagents such 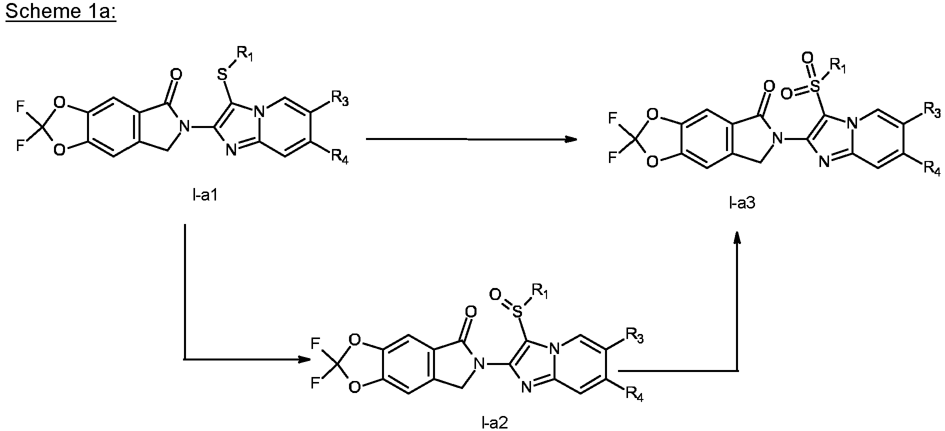 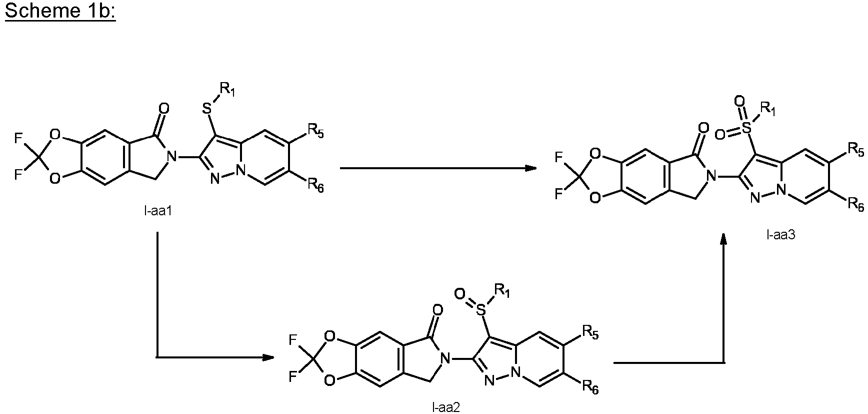 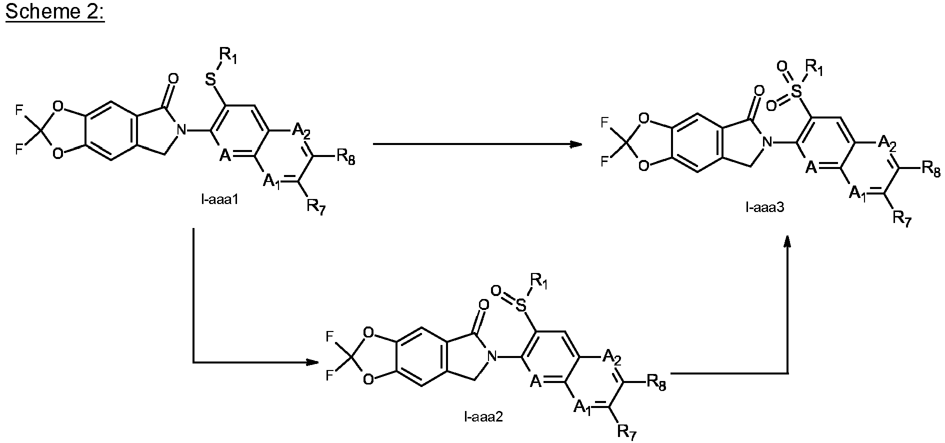 as, for example, m-chloroperoxybenzoic acid (mCPBA), hydrogen peroxide, oxone, sodium periodate, sodium hypochlorite or tert-butyl hypochlorite amongst other oxidants (scheme 1a, 1b and 2). The oxidation reaction is generally conducted in the presence of a solvent. Examples of the solvent to be used in the reaction include aliphatic halogenated hydrocarbons such as dichloromethane and chloroform; alcohols such as methanol and ethanol; acetic acid; water; and mixtures thereof. The amount of the oxidant to be used in the reaction is generally 1 to 3 moles, preferably 1 to 1.2 moles, relative to 1 mole of the sulfide compounds I to produce the sulfoxide compounds I, and preferably 2 to 2.2 moles of oxidant, relative to 1 mole of of the sulfide compounds I to produce the sulfone compounds I. Such oxidation reactions are disclosed, for example, in WO 2013/018928.

Compounds of formula I wherein Q is defined as under formula I above may be prepared (scheme 3) by reacting compounds of formula VII, and compounds of formula VIII, wherein Q is as defined in formula I above and in which LG₃ is a halogen (or a pseudo-halogen leaving group, such as a triflate), in the presence of a base, such as sodium carbonate, potassium carbonate or cesium carbonate, or sodium hydride, in an appropriate solvent such as for example tetrahydrofuran, dioxane, N,N-dimethylformamide, N,N-dimethylacetamide or acetonitrile, at temperatures between 0 and 150°C, optionally under microwave irradiation. Alternatively compounds of formula I wherein Q is defined as under formula I above may be prepared by reacting compounds of formula VII, and compounds of formula VIII, wherein Q is as defined in formula I above and in which LG₃ is a halogen (or a pseudo-halogen leaving group, such as a triflate), preferably bromo or iodo in the presence of a base, such as sodium carbonate, potassium carbonate or cesium carbonate, or potassium tert-butoxide in the presence of a metal catalyst either copper catalyst for example copper(I) iodide, optionally in the presence of a ligand for example diamine ligands (e.g. N,N'-dimethylethylenediamine or *trans-*cyclohexyldiamine) or dibenzylideneacetone (dba), or 1,10-phenanthroline, at temperatures between 30-180°C, optionally under microwave irradiation or palladium catalyst for example palladium(II)acetate, bis(dibenzylideneacetone)palladium(0) (Pd(dba)₂) or tris(dibenzylideneacetone)dipalladium(0) (Pd₂(dba)₃, optionally in form of a chloroform adduct), or a palladium pre-catalyst such as for example *tert*-BuBrettPhos Pd G3 [(2-Di-*tert*-butylphosphino-3,6-dimethoxy-2',4',6'-triisopropyl-1,1'-biphenyl)-2-(2'-amino-1,1'-biphenyl)]palladium(II) methanesulfonate or BrettPhos Pd G3 [(2-di-cyclohexylphosphino-3,6-dimethoxy-2',4',6'- triisopropyl-1,1'-biphenyl)-2-(2'-amino-1,1'-biphenyl)]palladium(II) methanesulfonate, and optionally in the presence of a ligand, for example SPhos, *t*-BuBrettPhos or Xantphos, at temperatures between 60-120°C, optionally under microwave irradiation. The above reaction may be carried out in the presence of solvent such as toluene, dimethylformamide DMF, N-methyl pyrrolidine NMP, dimethyl sulfoxide DMSO, dioxane, 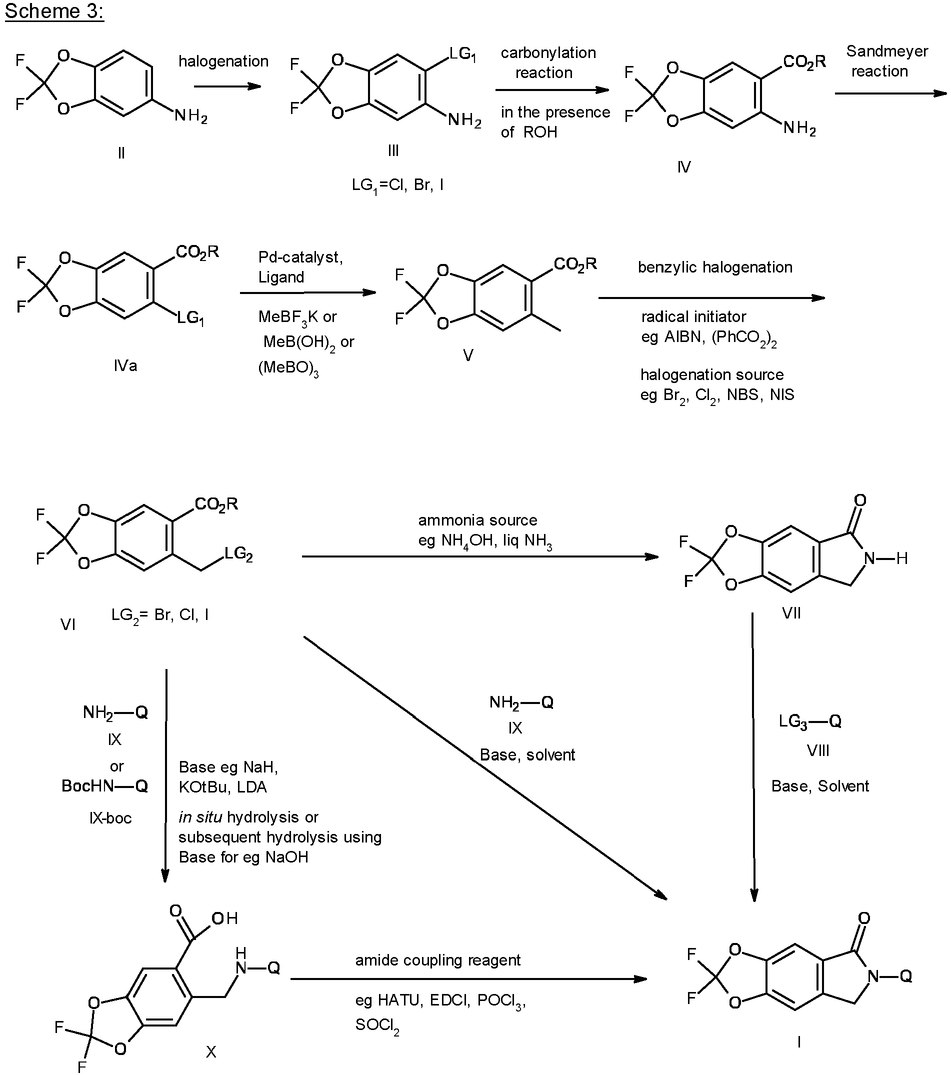 tetrahydrofuran THF and are described in literature for example in WO2012031004, WO2009042907 and Synthetic Communications, 41: 67-72, 2011.

Alternatively compounds of formula I wherein Q is defined as under formula I above may be prepared by reacting compounds of formula VI, wherein LG₂ is a leaving group for example Br, Cl or I, preferably bromo and R is C₁-C₆alkyl, benzyl or phenyl group and compounds of formula IX, wherein Q is as defined in formula I above in the presence of base such as such as sodium carbonate, potassium carbonate or cesium carbonate, or sodium hydride, N,N-Diisopropylethylamine or KOtBu and in the presence of solvent such as ethanol, methanol, dioxane, toluene, DMF, DMA, DMSO, THF at temperatures between 0 and 150°C, optionally under microwave irradiation. Such reactions proceeds *via* nucleophilic substitution and subsequent cyclization and are also reported in literature for example in WO2009042907.

Alternatively compounds of formula I wherein Q is defined as under formula I above can be prepared by cyclizing compounds of formula X, wherein Q is as defined in formula I, for example in the presence of phosphorus oxychloride, optionally in the presence of a solvent or diluent, such as toluene or xylene, at temperatures between 0 and 180°C, preferably between 20 and 120°C.

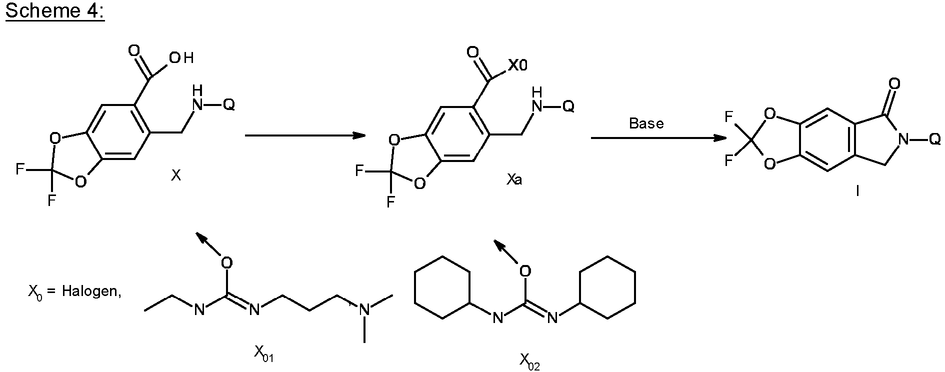

Compounds of formula I, wherein Q is as defined as under formula I above, can also be prepared by cyclization of the formula Xa (scheme 4) wherein Q is defined as under formula I above and in which X₀ is halogen, preferably chlorine, or X₀ is either X₀₁ or X₀₂, in the presence of a base, such as triethylamine, N,N-diisopropyl-ethylamine or pyridine, optionally in the presence of a catalyst (such as 4-dimethylaminopyridine DMAP), in an inert solvents such as dichloromethane, tetrahydrofuran, dioxane, N,N-dimethyl-formamide, N,N-dimethylacetamide, acetonitrile, ethyl acetate or toluene, at temperatures between 0 and 50°C. Certain bases, such as pyridine and triethylamine, may be employed successfully as both base and solvent.

Compounds of formula Xa, wherein Q is defined as under formula I above and in which X₀ is halogen, preferably chlorine, or X₀ is either X₀₁ or X₀₂, can be prepared by activation of compound of formula X, wherein Q is defined as under formula I above, by methods known to those skilled in the art and described in, for example, Tetrahedron, 2005, 61 (46), 10827-10852. Preferred is the formation of an activated species Xa, wherein Q is defined as under formula I above and in which X₀ is halogen, preferably chlorine. For example, compounds Xa where X₀ is halogen, preferably chlorine, are formed by treatment of X with, for example, oxalyl chloride (COCl)₂ or thionyl chloride SOCl₂ in the presence of catalytic quantities of N,N-dimethylformamide DMF in inert solvents such as methylene chloride CH₂Cl₂ or tetrahydrofuran THF at temperatures between 20 to 100°C, preferably 25°C. Alternatively, treatment of compounds of formula X with, for example, 1-ethyl-3-(3-dimethylaminopropyl)carbodiimide EDC or dicyclohexyl carbodiimide DCC will generate an activated species Xa, wherein X₀ is X₀₁ or X₀₂ respectively, in an inert solvent, such as pyridine or tetrahydrofuran THF, optionally in the presence of a base, such as triethylamine, at temperatures between 50-180°C.

Compounds of formula VII, can be prepared by reacting compounds of formula VI, wherein LG₂ is a leaving group for example Br, Cl or I, preferably bromo and R is C₁-C₆alkyl, benzyl or phenyl group with ammonia or surrogates of ammonia for example NH₄OH in the presence of solvent such as ethanol, methanol, dioxane, toluene, DMF, DMA, DMSO, THF at temperatures between 0 and 150°C, optionally under microwave irradiation.

Compounds of formula X, wherein Q is defined as under formula I above, can be prepared by nucleophilic substitution reaction of compound of formula VI, and LG₂ is a leaving group for example Br, Cl or I, preferably bromo and R is C₁-C₆alkyl, benzyl or phenyl group, with amino compound of formula IX (or compounds of formula IX-boc and then deprotect the BOC functional group after reaction), wherein Q is as defined in formula I above, followed by *in situ* hydrolysis of the intermediate ester of formula XVII, wherein Q is defined as under formula I above, and in which R is C₁-C₆alkyl, benzyl or phenyl group.

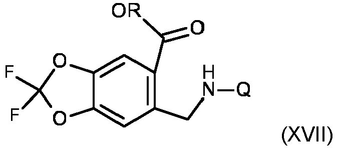

The *in situ* generated unhydrolyzed ester compound of formula XVII may be isolated and can also be converted *via* saponification reaction in the presence of suitable base for example NaOH, LiOH, Ba(OH)₂ to form the carboxylic acid of formula X. The conversion of compound of formula VI to compound of formula X can be carried out in the presence of base such as sodium hydride, KOtBu, butyllithium, lithium diisopropylamide amongst others and in the presence of solvent such as dioxane, DMF, DMA, DMSO, THF at temperatures between -30 and 150°C. Compounds of formula VI, wherein LG₂ is a leaving group for example Br, Cl or I, preferably bromo and R is C₁-C₆alkyl, benzyl or phenyl group can be prepared by radical induced benzylic halogenation of compounds of formula V, wherein R is C₁-C₆alkyl, benzyl or phenyl group. Such reaction are well known to those skilled in the art and may be carried out in the presence of electrophilic halogenating reagents such as Br₂, NBS, Cl₂, NIS amongst others and in the presence of radical initiator for example AIBN (Azobisisobutyronitrile), benzoyl peroxide or under photochemical conditions and at temperatures ranging from 20 °C to the boiling point of solvent and in the presence of solvent such as toluene, xylene, acetonitrile, hexane, dichloroethane, or carbon tetrachloride. Such reactions are known by the name of Wohl-Ziegler bromination and are reported in literature for example in Synthesis, 2015, 47, 1280-1290 and J. Am. Chem. Soc., 1963, 85 (3), pp 354-355.

Compounds of formula V, wherein R is C₁-C₆alkyl, benzyl or phenyl group, may be prepared by a Suzuki reaction, which involves for example, reacting compounds of formula IVa, wherein LG₁ is halogen Br, Cl, I preferably Cl and R is C₁-C₆alkyl, benzyl or phenyl group with trimethylboroxine or potassium methyltrifluoroborate amongst other methyl boronic acid equivalent. The reaction may be catalyzed by a palladium based catalyst, for example tetrakis(triphenyl-phosphine)palladium(0), (1,1'bis(diphenylphosphino)ferrocene)dichloro-palladium-dichloromethane (1:1 complex) or chloro(2-dicyclohexylphosphino-2',4',6'-triisopropyl-1,1'-biphenyl)[2-(2'-amino-1,1'-biphenyl)]palladium(II) (XPhos palladacycle), in presence of a base, like sodium carbonate, tripotassium phosphate or cesium fluoride, in a solvent or a solvent mixture, like, for example dioxane, acetonitrile, N,N-dimethylformamide, a mixture of 1,2-dimethoxyethane and water or of dioxane/water, or of toluene/water, preferably under inert atmosphere. The reaction temperature can preferentially range from room temperature to the boiling point of the reaction mixture, or the reaction may be performed under microwave irradiation. Such Suzuki reactions are well known to those skilled in the art and have been reviewed, for example, in J. Organomet. Chem. 576, 1999, 147-168.

Alternatively compounds of formula V, wherein R is C₁-C₆alkyl, benzyl or phenyl group can be prepared following scheme 3a.

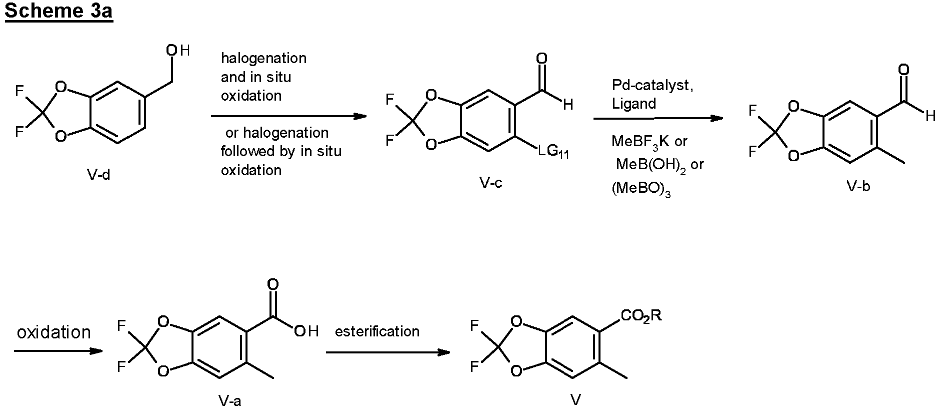

In scheme 3a compounds of formula V, wherein R is C₁-C₆alkyl, benzyl or phenyl group can be prepared from compounds of formula V-a via esterification reactions which involves reacting compounds of formula V-a with R-OH, wherein R is C₁-C₆alkyl, benzyl or phenyl group in the presence of acid catalyst for example sulfuric acid or Lewis acid for example Sc(OTf)₃ or FeCl₃. Such reactions are well known to those skilled in the state of art and known by the name of Fischer Esterification reaction and are reported in literature for example in J. Org. Chem., 2006, 71, 3332-3334, Chem. Commun., 1997, 351-352 and Synthesis, 2008, 3407-3410. Such esterification reaction can also be carried out by reacting compounds of formula III with TMSCHN₂ to form compounds of formula V, wherein R is methyl and are reported in Angew. Chem. Int. Ed. 2007, 46, 7075. Compounds of formula V-a can be prepared by the oxidation reaction of compounds of formula V-b. Such reactions are are well known to person skilled in the art. Examples of the reagent which facilitates such transformation includes Oxone, KMnO₄, NaClO₂ (known by the name of Pinnick oxidation), AgNO₃ in the presence of metal hydroxide or Ag₂O (known by the name of Tollen's reagent). Such reactions are known in the literatrure and described for example in Acta Chem. Scand. 1973, 27: 888-890; Tetrahedron 1981, 37 (11): 2091-2096; Ber. Deut. Chem. Gessel., 15 (1882), pp. 1635-1639; Org. Synth. 1953, 33, 94. Compounds of formula V-b can be prepared from compounds of formula V-c via analogous procedure as described in scheme 3 for the conversion of compounds of formula IVa to compounds of formula V. Compounds of formula V-c can be prepared from compounds of formula V-d via halogenation and in situ oxidation reaction using halogenating reagent such as iodine, bromine, chlorine, N-chlorosuccinimide, N-bromosuccinimide amongst others. Alternatively, the halogenation can be performed and in a subsequent step the oxidation reaction can be carried out using oxidating reagent to form the compounds of formula V-c from compounds of formula V-d in two steps. Compound of formula V-d is known in the literature with cas registery number 72768-97-9.

Compounds of formula IVa, wherein, LG₁ is halogen Br, Cl, I preferably Cl and R is C₁-C₆alkyl, benzyl or phenyl group can be prepared (scheme 3) by reacting compounds of formula IV, wherein R is C₁-C₆alkyl, benzyl or phenyl, with a nitrite, such as tert-butyl nitrite t-BuONO, isoamyl nitrite, or sodium nitrite in the presence of a hydrohalic acid H-LG₁ and a copper salt Cu-LG₁, wherein LG₁ is halogen, for example Br, Cl or I (preferably CI) under Sandmeyer-type reaction conditions. This transformation is preferably performed in an inert solvent, such as acetonitrile or a halogenated solvent like 1,2-dichloroethane, or water at temperatures between 0-150°C, preferably at temperatures ranging from room temperature to the boiling point of the reaction mixture. Compounds of formula IV, wherein R is C₁-C₆alkyl, benzyl or phenyl, can be prepared from compounds of formula III, wherein LG₁ is halogen, preferably Br, Cl or I, by methods found in, for example, WO 2016/020286 involving a carbonylation reaction, in which compounds of formula III are reacted with carbon monoxide CO (usually under pressure), in presence of metal catalyst such as a palladium catalyst (for example: palladium(II) acetate), in an alcohol ROH solvent (optionally in presence of a co-solvent), wherein R is C₁-C₆alkyl, benzyl or phenyl, and optionally in presence of a phosphine ligand, and optionally in presence of a base, at temperatures between 0-180°C. Compounds of formula III, wherein LG₁ is halogen, preferably Br, Cl or I, can be prepared by a halogenation reaction, which involves for example, reacting compounds of formula II, with halogenating reagents such as N-chlorosuccinimide (NCS), N-bromosuccinimide (NBS) or N-iodosuccinimide (NIS), or alternatively chlorine, bromine or iodine. Such halogenation reactions are carried out in an inert solvent, such as chloroform, carbon tetrachloride, 1,2-dichloroethane, acetic acid, ethers, acetonitrile or N,N-dimethylformamide, at temperatures between 20-200°C, preferably room temperature to 100°C.

The compounds of formula VI 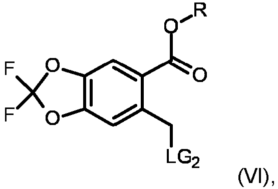 wherein
LG₂ is a leaving group for example Br, Cl or I, and R is C₁-C₆alkyl, benzyl or phenyl are novel, especially developed for the preparation of the compounds of formula I according to the invention and therefore represent a further object of the invention. The preferences and preferred embodiments of the substituents of the compounds of formula I are also valid for the compounds of formula VI. Preferably, LG₂ is bromo or chloro; even more preferably LG₂ is bromo. Preferably R is C₁-C₆alkyl; even more preferably R is methyl or ethyl.

Alternatively compounds of formula I, wherein Q is defined as under formula I above can be prepared following scheme 5.

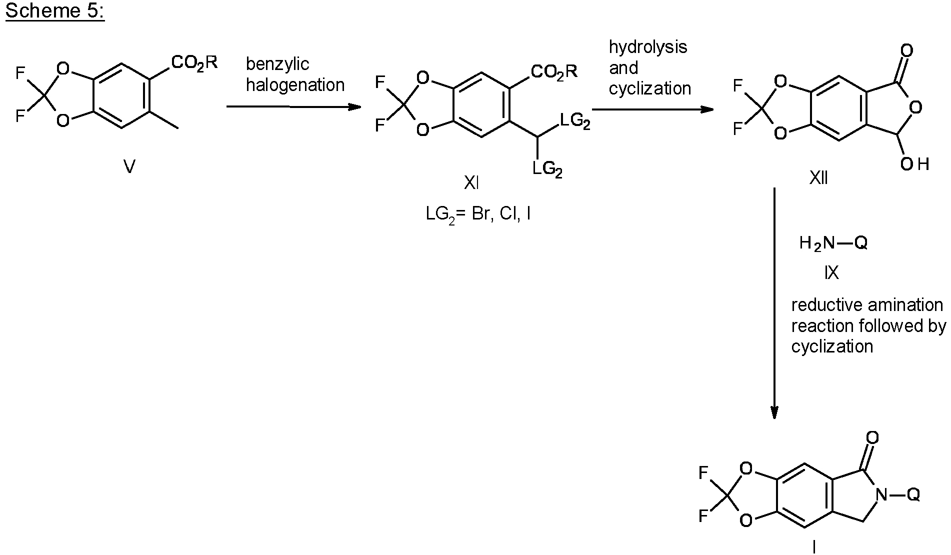

In scheme 5, compounds of formula I, wherein Q is defined as under formula I above can be prepared from compounds of formula X, wherein Q is defined as under formula I above, by method as described in scheme 4 above (scheme 6). Compounds of formula X can be prepared by reacting compounds of formula XII, with compounds of formula IX, wherein Q is as defined in formula I above under reductive amination conditions (scheme 6). The reaction can be carried out in the presence of reducing agent for example sodium cyanoborohydride, sodium triacetoxyborohydride, amongst others and optionally in the presence of acid such as trifluoroacetic acid, formic acid, acetic acid and like others and at temperatures ranging from 0 °C to the boiling point of solvent. The reaction can be carried out in the presence of inert solvents such as ethanol, methanol, dioxane or tetrahydrofuran. Such reactions involving two step conversion from compounds of formula XII to compounds of formula I have been described in literature for example in Bioorganic & Medicinal Chemistry Letters 26 (2016) 5947-5950. Compounds of formula XII, can be prepared from compound of formula XI, wherein LG₂ is chloro, bromo or iodo preferably bromo and R is C₁-C₆alkyl, benzyl or phenyl group by the hydrolysis and subsequent intramolecular cyclization reaction. The reaction can be carried out either using metal hydroxide under basic conditions for example using aqueous sodium hydroxide in the presence of solvent such as dioxane, tetrahydrofuran or water and at temperature ranging from 20 to 150 °C as reported in Synlett 1992, (6), 531-533, or under aqueous acidic conditions for example using acetic acid, hydrochloric acid or sulfuric acid in the presence of solvent such as water, dioxane, halogenate solvents such as dichloroethane as reported in Tetrahedron 62 (2006) 9589-9602. Compounds of formula XI, wherein LG₂ is chloro, bromo or iodo preferably bromo and R is C₁-C₆alkyl, benzyl or phenyl group can be prepared from compounds of formula V, R is C₁-C₆alkyl, benzyl or phenyl group by method similar to as described in scheme 3 for the conversion of compound of formula V to compound of formula VI.

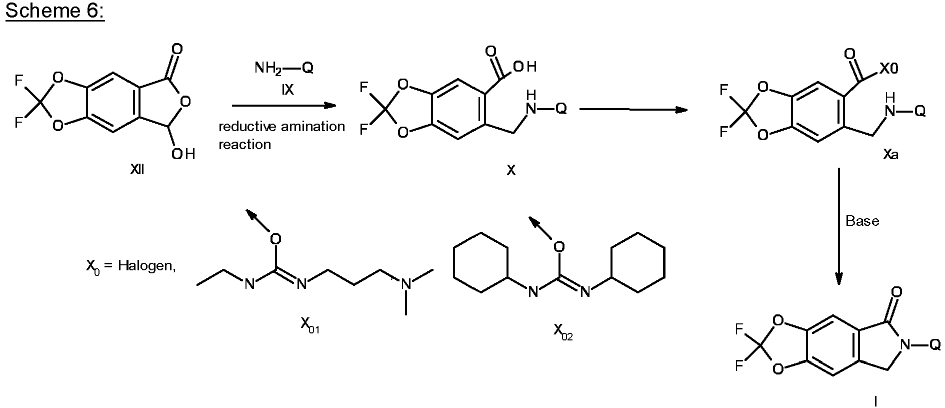

Alternatively compounds of formula I, wherein Q is defined as under formula I, above can be prepared from compounds of formula XV, wherein Q is defined as under formula I, above *via* selective reduction of the carbonyl functional group (Scheme 7).

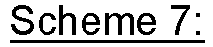

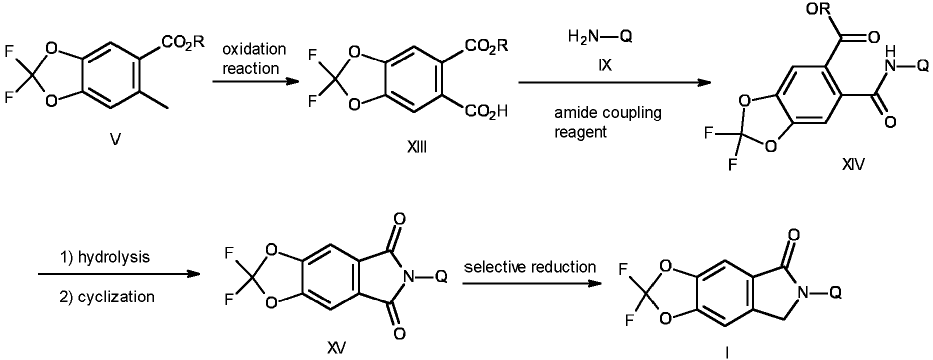

The reaction can be carried out in the presence of reducing agent for example NaBH₄, LiAlH₄, palladium on carbon in the presence of hydrogen or a combination of two reducing agent for example NaBH₄ followed by triethylsilane. Such reactions have been described for example in US20100160303A1. Compounds of formula XV, wherein Q is defined as under formula I, above can be prepared from compounds of formula XIV, wherein Q is defined as under formula I, above by hydrolysis reaction and subsequent cyclization reaction as described in scheme 4 for the conversion of compounds of formula X to compounds of formula I. Compounds of formula XIV, wherein Q is defined as under formula I, above and R is C₁-C₆alkyl, benzyl or phenyl can be prepared by reacting compounds of formula XIII, wherein R is C₁-C₆alkyl, benzyl or phenyl with compounds of formula IX, wherein Q is as defined in formula I above by amidation reaction as also described in scheme 4. Compounds of formula XIII, wherein R is C₁-C₆alkyl, benzyl or phenyl can be prepared by benzylic oxidation of compounds of formula V, wherein R is C₁-C₆alkyl, benzyl or phenyl. The reaction can be carried out in the presence of oxidative reagents such as KMNO₄, *n*Bu₄MnO₄, K₂S₂O₃ in the presence of oxygen, or under photochemical conditions in the presence of oxygen and at temperature ranging from 20 °C to the boiling point of solvent. The reaction is carried out in the presence of inert solvent such as acetonitrile, ethyl acetate, DMSO, dichloroethane. Such reactions are known in the literature for example in Synthesis, 2017, 49, 4007-4016, Synthesis, 2006, 1757-1759 and IOSR Journal of Applied Chemistry, 2014, 7, 16-27.

Alternatively, compounds of formula I, wherein Q is as defined in formula I, above can be prepared by cyclization reaction of compounds of formula XVII, wherein Q is as defined in formula I, above and R is C₁-C₆alkyl, benzyl or phenyl (Scheme 8):

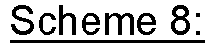

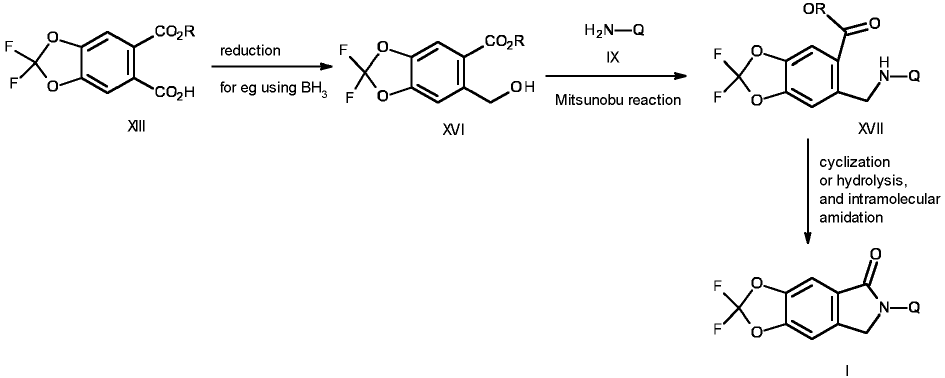

Reaction can be carried out in the presence of base such as potassium tert-butoxide, lithium diisopropylamide, sodium hydride, and similar others and at temperature ranging from -20 °C to the boiling point of solvent and in the presence of inert solvent such as tetrahydrofuran, dioxane, DMF. Such reactions are reported in Synlett 2006(4): 591-594. Compounds of formula XVII, wherein Q is as defined in formula I, above and R is C₁-C₆alkyl, benzyl or phenyl can be prepared by reacting compounds of formula XVI, wherein R is C₁-C₆alkyl, benzyl or phenyl with compounds of formula IX, wherein Q is as defined in formula I above under Mitsunobu conditions. Such reactions are well known to those skilled in the state of art and can be carried out in the presence of phosphine reagent such as triphenylphosphine, tributylphosphine, or polymer supported triphenyl phosphine amongst others and in the presence of an azodicarboxylate reagent such as diethyl azodicarboxylate, diisopropyl azodicarboxylate and at temperature ranging from 0 °C and 100 °C and in the presence of inert solvent such as acetonitrile, dichloromethane, tetrahydrofuran, toluene. Such reactions are reported for example in Synthesis, 1981(1), 1-28. Compounds of formula XVI, wherein R is C₁-C₆alkyl, benzyl or phenyl can be prepared by reacting compounds of formula XIII, wherein R is C₁-C₆alkyl, benzyl or phenyl with reducing agents. Reaction can be performed using reducing reagents for example using metal hydrides such as lithium aluminumhydride, DIBAL-H, or boranes such as diborane, borane tetrahydrofuran amongst others at temperatures ranging from 0 °C and 150 °C and in the presence of inert solvent such as tetrahydrofuran, dioxane. Such reactions have been reported in Tetrahedron Letters, 1982, 23, 2475-2478.

The compounds of formula XVII-a 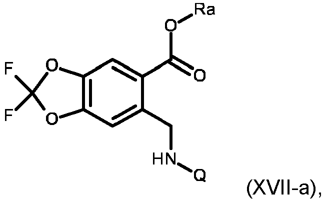 wherein

Q is as defined under formula I above, and Rₐ is hydrogen, C₁-C₆alkyl, benzyl or phenyl are novel, especially developed for the preparation of the compounds of formula I according to the invention and therefore represent a further object of the invention. The preferences and preferred embodiments of the substituents of the compounds of formula I are also valid for the compounds of formula XVII-a. Preferably, Ra is hydrogen or C₁-C₆alkyl; even more preferably, Ra is hydrogen, methyl or ethyl.

Compounds of formula IX, wherein Q is as defined in formula I above, may be prepared by the deprotection reaction of tert-butyl group of compounds of formula XIX, wherein Q is as defined in formula I above (Scheme 9).

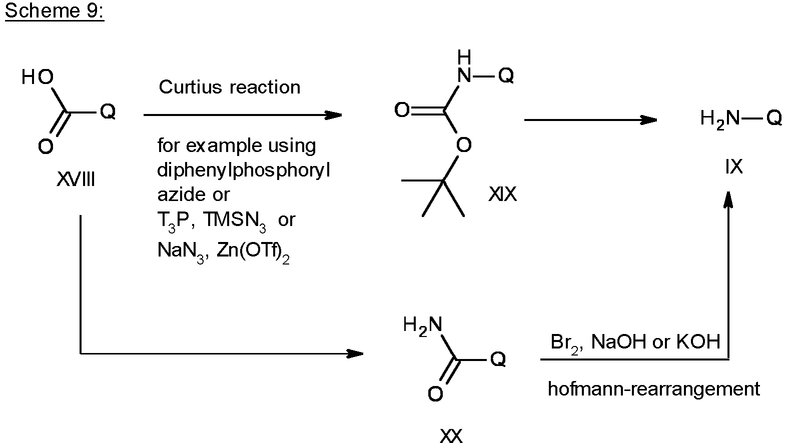

The reaction can be carried out in the presence of acid catalyst such as trifluoroacetic acid, hydrochloric acid or sulfuric acid and like others. Compounds of formula IX, wherein Q is as defined in formula I above, may be prepared by the reaction of compounds of formula XVIII, wherein Q is as defined in formula I above, with an organo-azide in the presence of a suitable base, *t*-BuOH and in the presence of a coupling agent optionally in the presence of Lewis acid and in the presence of an inert solvent at temperatures between 50 °C and boiling point of solvent. The reaction can be carried out in the presence of coupling agent such as T₃P or *via* activation of carboxylic acid with SOCl₂ or oxalyl chloride or other coupling agent as described in scheme 6 for the conversion of compounds of formula X to the compounds of formula Xa. Examples of organo-azide include TMSN₃, sodium azide, or tosyl azide and suitable solvent may be toluene, xylene, THF or acetonitrile. Example of suitable Lewis acid may include Zn(OTf)₂, Sc(OTf)₂, or Cu(OTf)₂ amongst others. Compounds of formula XIX can also be prepared by reacting compounds of formula XVIII with diphenylphosphorylazide in the presence of an organic base such as triethyl amine, diisopropylethylamine and similar others, and in the presence of *tert*-butanol and an inert solvent for example halogenated solvent such as dichloromethane, dichloroethane or cyclic ethers such as tetrahydrofuran amongst others and at temperatures ranging from 50 °C to the boiling point of solvent. Such reactions of converting carboxylic acids to amines are well known to those skilled in the state of art by the name of Curtius reaction and are reported in Org. Lett., 2005, 7, 4107-4110; Journal of Medicinal Chemistry, 49(12), 3614-3627; 2006, J. Am. Chem. Soc., 1972, 94 (17), pp 6203-6205. Compounds of formula IX, wherein Q is as defined in formula I above, may also be prepared from compounds of formula XX, wherein Q is as defined in formula I above by Hofmann-rearrangement reaction. The reaction can be carried out in the presence of base for example metal hydroxides such as aqueous sodium hydroxide or potassium hydroxide or organic bases such as DBU (1,8-Diazabicyclo(5.4.0)undec-7-ene) and in the presence of electrophilic halogenating reagents such as chlorine, bromine or N-bromo-succinimide and at temperatures ranging from 20 °C to the boiling point of solvent. Such reactions are known by the name of Hofmann-rearrangement and are reported in literature for example in Chem. Ber. 1881, 14, 2725. Compounds of formula XX, wherein Q is as defined in formula I above can be prepared by the reaction of compounds of formula XVIII with ammonia for example NH₄OH, NH₃, or other ammonia surrogates in the presence of carboxylic acid activating agent as described in scheme 4.

The subgroup of compounds of formula XVIII, wherein Q is Qa, in which R₃, R₄, X and R₁ are as defined in formula I, can be defined as compounds of formula XVIII-a (scheme 8). Such compounds of formula XVIII-a are either known in the literature, or they can be prepared by following scheme 8 using analogous methods and conditions as described in, for example, WO2017/061497 and WO2018/052136.

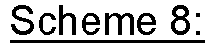

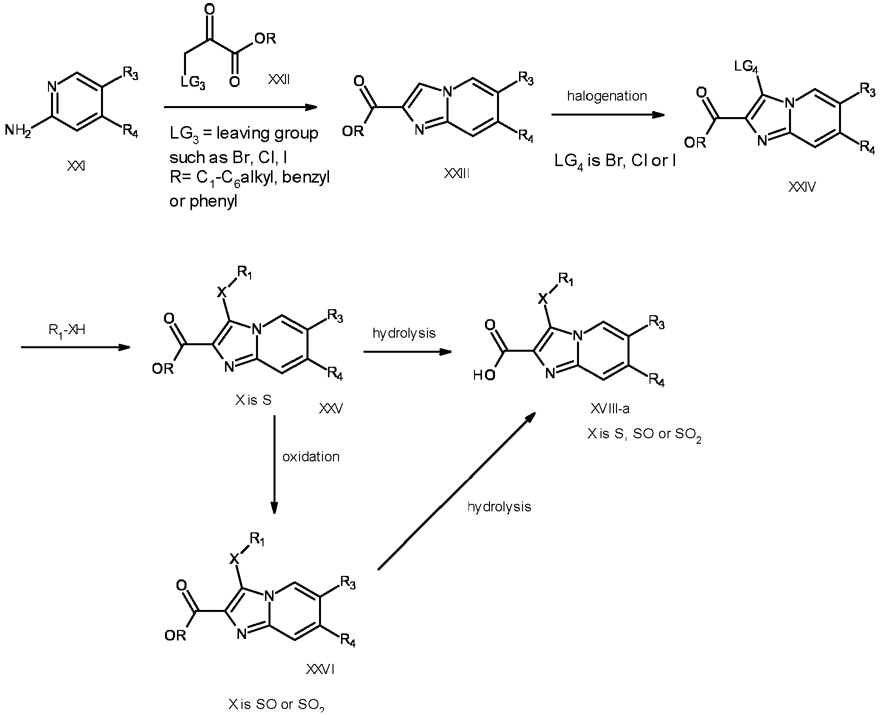

The subgroup of compounds of formula XVIII, wherein Q is Qb, in which R₅, R₆, X and R₁ are as defined in formula I, can be defined as compounds of formula XVIII-b (scheme 9a). Such compounds of formula XVIlI-b are either known in the literature, or they can be prepared by following scheme 9a using analogous methods and conditions as described in, for example, WO2019162174 A1.

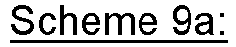

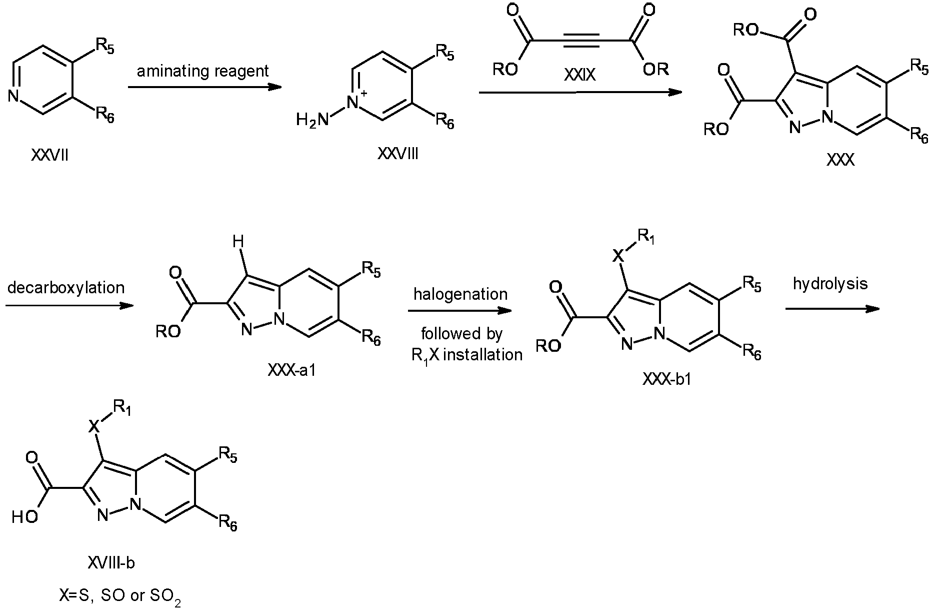

Alternatively compounds of formula XVIII-b, wherein R₁, X, R₅, and R₆ are as defined in formula I, can be prepared by following scheme 9b using analogous methods and conditions as described in literature, for example, WO2009095253 A1.

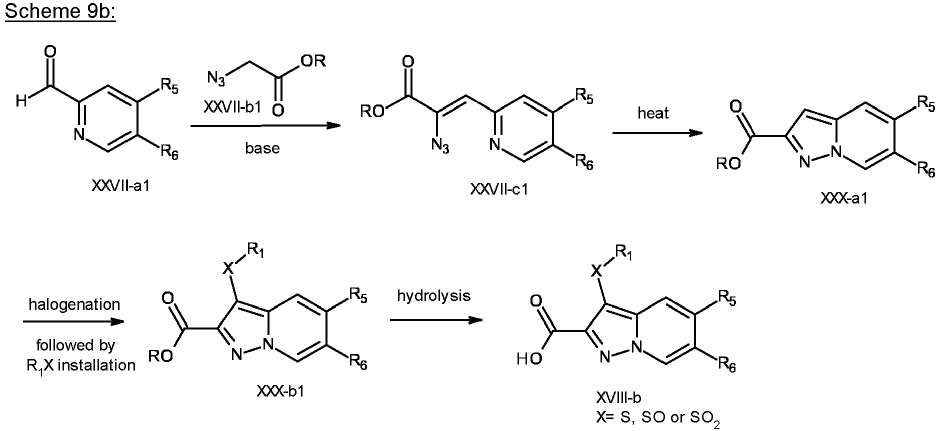

The subgroup of compounds of formula IX, wherein Q is Qb, in which R₅, R₆, and R₁ are as defined in formula I, and X is SO₂ can be defined as compounds of formula IX-b, wherein R₁, R₅ and R₆ are as defined in formula I and X is SO₂ (scheme 9c). Such compounds of formula IX-b, wherein R₁, R₅ and R₆ are as defined in formula I and X is SO₂ can be prepared following scheme 9c.

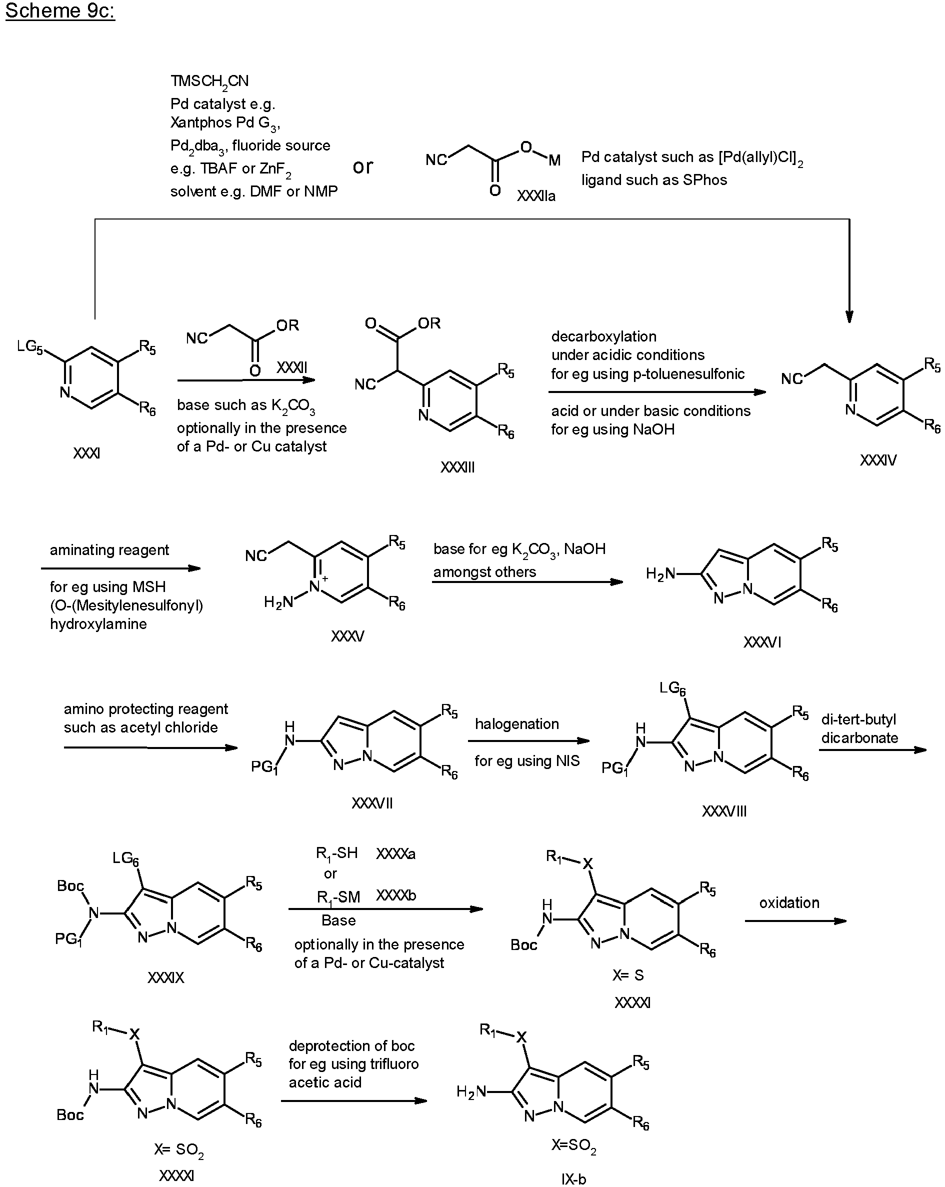

In scheme 9c compounds of formula IX-b, wherein R₅, R₆, and R₁ are as defined in formula I, and X is SO₂ can be prepared from compounds of formula XXXXI, wherein R₅, R₆, and R₁ are as defined in formula I, and X is SO₂ *via* deprotection of *tert*-butoxycarbonyl group. Such reactions can be carried out in the presence of acid catalyst such as trifluoroacetic acid, hydrocholoric acid amongst others acid catalyst and optionally in the presence of a solvent such as dichloromethane, toluene, trifluorotoluene amongst others. Compounds of formula XXXXI, wherein R₅, R₆, and R₁ are as defined in formula I, and X is SO₂ can be prepared via oxidation of compounds of formula XXXXI, wherein R₅, R₆, and R₁ are as defined in formula I, and X is S by following procedure analogous to as described in scheme 1a. Compounds of formula XXXXI, wherein R₅, R₆, and R₁ are as defined in formula I, and X is S can be prepared by the substitution reaction or by cross-coupling reaction of compounds of formula XXXIX, wherein R₅, and R₆, are as defined in formula I, PG₁ is an amino protecting group for example acetyl, benzyl, benzoyl and LG₆ is a leaving group preferably Cl, Br or I with a reagent of the formula XXXXa

R₁-SH (XXXXa),

or a salt thereof, wherein R₁ is as defined in formula I, optionally in the presence of a suitable base, such as alkali metal carbonates, for example sodium carbonate and potassium carbonate, or alkali metal hydrides such as sodium hydride, or alkali metal hydroxides such as sodium hydroxide and potassium hydroxide, or sodium or potassium tert-butoxide, in an inert solvent at temperatures preferably between 25-120°C. Examples of solvent to be used include ethers such as tetrahydrofuran THF, ethylene glycol dimethyl ether, tert-butylmethyl ether, and 1,4-dioxane, aromatic hydrocarbons such as toluene and xylene, nitriles such as acetonitrile or polar aprotic solvents such as N,N-dimethylformamide, N,N-dimethylacetamide, N-methyl-2-pyrrolidone NMP or dimethyl sulfoxide. Examples of salts of the compound of formula XXXXa include compounds of the formula

   R₁-S-M (XXXXb),
wherein R₁ is as defined above and wherein M is, for example, sodium or potassium. Such a process to prepare compounds of formula XXXXb from compounds of formula XXXXa can be found, for example, in WO16/091731.

Alternatively, this reaction to form compounds of formula XXXXI from compounds of formula XXXIX using R₁-SH (XXXXa) or R₁-SM (XXXXb) can be carried out in the presence of a palladium catalyst, such as tris(dibenzylideneacetone)dipalladium(0), in the presence of a phosphine ligand, such as xanthphos, in the presence of a base such as N,N-diisopropylethylamine, and in the presence of an inert solvent, for example, xylene at temperatures between 100-160°C, preferably 140°C, as described in Tetrahedron 2005, 61, 5253-5259. During the conversion of compounds of formula XXXIX to compounds of formula XXXXI, amino protecting group PG₁ is either cleaved under the reaction conditions described above or can be subsequently cleaved using suitable reagent well known to those skilled in the state of art for eg acetyl protecting group can be cleaved under basic conditions using NaOH, KOH, Cs₂CO₃, K₂CO₃ amongst other bases.

Compounds of formula XXXIX, wherein R₅, and R₆, are as defined in formula I, PG₁ is an amino protecting group for example acetyl, benzyl, benzoyl and LG₆ is a leaving group preferably Cl, Br or I can be prepared by the reaction of compounds of formula XXXVIII, wherein R₅, and R₆, are as defined in formula I, PG₁ is an amino protecting group for example acetyl, benzyl, benzoyl and LG₆ is a leaving group preferably Cl, Br or I and di-tert-butyl decarbonate optionally in the presence of a base such as triethyl amine, 4-dimethylaminopyridine amongst others and in the presence of a solvent such as dichloromethane, acetonitrile, toluene, tetrahydrofuran amongst others. Compounds of formula XXXVIII, wherein R₅, and R₆, are as defined in formula I, PG₁ is an amino protecting group for example acetyl, benzyl, benzoyl and LG₆ is a leaving group preferably Cl, Br or I can be prepared by reacting compounds of formula XXXVII, wherein R₅, and R₆, are as defined in formula I, and PG₁ is an amino protecting group for example acetyl, benzyl, benzoyl with a suitable halogenating reagent such as N-Chlorosuccinimide, N-Bromosuccinimide, N-lodosuccinimide amongst others in the presence of solvent such as dichloromethane, acetonitrile, tetrahydrofuran, DMF amongst others. Such reactions are well known to those skilled in the state of art. Compounds of formula XXXVII, wherein R₅, and R₆, are as defined in formula I, and PG₁ is an amino protecting group for example acetyl, benzyl, benzoyl can be prepared by reacting compounds of formula XXXVI, wherein R₅, and R₆, are as defined in formula I with a suitable amino protecting group reagent for example using acetyl chloride in the presence of pyridine.

Compounds of formula XXXVI, wherein R₅, and R₆, are as defined in formula I can be prepared in two steps from compounds of formula XXXIV, wherein R₅, and R₆, are as defined in formula I, which involves N-amination reaction of compounds of formula XXXIV with aminating reagent such as hydroxylamine-O-sulfonic acid, O-(mesitylsulfonyl)hydroxylamine amongst others to form compounds of formula XXXV, wherein R₅, and R₆, are as defined in formula I, followed by intramolecular cyclization of compounds of formula XXXV, wherein R₅, and R₆, are as defined in formula I, in the presence of a base such as sodium hydride, KOH, NaOH, potassium carbonate, cesium carbonate amongst others and in the presence of a solvent such as dichloromethane, dichloroethane, methanol, tetrahydrofuran, dimethylformamide amongst others. Such two step reactions are reported in literature for example as described in Tetrahedron Letters (2014), 55(43), 5963-5966.

Compounds of formula XXXIV, wherein R₅, and R₆, are as defined in formula I, can be prepared from compounds of formula XXXI, wherein R₅, and R₆, are as defined in formula I, and LG₅ is a halogen (or a pseudo-halogen leaving group, such as a triflate), on reaction with a acetonitrile anion equivalents in the presence of metal catalysts. A variety of acetonitrile anion equivalents can be used in such reactions. Examples of such are tri-nbutylstannylacetonitrile, which can be coupled to compounds of formula (XXXI) under Stille reaction conditions as described by Mitiga ef. al. (Chem. Lett. 1984, 15 11) , or trimethylsilylacetonitrile in the presence of a palladium catalyst, such as tris(dibenzylideneacetone)dipalladium(0), XantPhos Pd G3 ([(4,5-Bis(diphenylphosphino)-9,9-dimethylxanthene)-2-(2'-amino-1,1'-biphenyl)]palladium(II) methanesulfonate) and a ligand, for example Xantphos or P(i-Bu)3, a fluoride source, for example ZnF2 ,in an dipolar aprotic solvent such as DMF, at temperatures between 80-1 20 °C. Such reactions are well precedented in the literarure, for example see Hartwig ef. al. (J. Am. Chem. Soc. 2002, 124, 9330, and J. Am. Chem. Soc. 2005, 727, 15824) (scheme 9c). Metal cyanoacetate such as potassium cyanoacetate or sodium cyanoacetate can also be used as an acetonitrile anion equivalent and undergo coupling reaction in the presence of palladium catalyst such as [Pd₂(dba)₃] (Tris(dibenzylideneacetone)dipalladium(0)), [Pd(allyl)Cl]₂ (Allylpalladium(II) chloride dimer) amongst others in the presence of a ligand such as SPhos, Xantphos or P(i-Bu)₃ or P(*tert*-butyl)₃ amongst others. Such reactions are known in the literature and described for example in Angew. Chem. Int. Ed. 2011, 50, 4470 -4474.

Yet another method to prepare compounds of formula XXXIV from compounds of formula XXXI is shown below (scheme 9c-1).

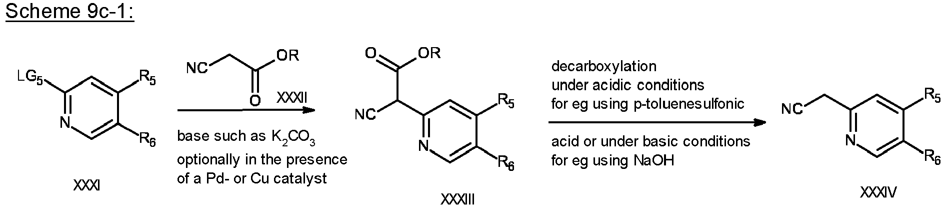

Reaction of compounds of formula XXXI, wherein wherein R₅, and R₆, are as defined in formula I, and LG₅ is a halogen (or a pseudo-halogen leaving group, such as a triflate), with reagents of the formula XXXII, wherein R is C₁-C₆alkyl, in the presence of a base, such as sodium carbonate, potassium carbonate or cesium carbonate, or sodium hydride, sodium methoxide or ethoxide, potassium tert-butoxide, optionally under palladium (for example involving Pd(PPh₃)₂Cl₂) or copper (for example involving Cul) catalysis, in a appropriate solvent such as for example toluene, dioxane, tetrahydrofuran, acetonitrile, N,N-dimethylformamide, N,N-dimethylacetamide, N-methyl-2-pyrrolidone NMP or dimethylsulfoxide DMSO, optionally in presence of a phase transfer catalyst PTC, such as for example tetrabutyl ammonium bromide or triethyl benzyl ammonium chloride TEBAC, at temperatures between room temperature and 180°C, may lead to compounds of formula XXXIII, wherein R₅ and R₆ are as described under formula I above, and in which R is C₁-C₆alkyl. Similar chemistry has been described in, for example, Synthesis 2010, No. 19, 3332-3338.

Compounds of formula XXXIV, wherein R₅, and R₆ are as described under formula I above, may be prepared by saponification/decarboxylation of the compounds of formula XXXIII, wherein R₅ and R₆ are as described under formula I above, and in which R is C₁-C₆alkyl, under conditions known to a person skilled in the art (using for example conditions such as: aqueous sodium, potassium or lithium hydroxide in methanol, ethanol, tetrahydrofuran ordioxane at room temperature, or up to refluxing conditions; followed by acification of the reaction mixture under standard aqueous acid conditions or for example under acidic conditions in the presence of HCl or para-toluene sulfonic acid). Alternatively, treating compounds of formula XXXIII with halide anions, preferably chloride anions, originating from, for example, lithium chloride or sodium chloride, in solvents such as N,N-dimethylformamide, N,N-dimethylacetamide, N-methyl-2-pyrrolidone or dimethylsulfoxide DMSO, optionally in presence of additional water, may also generate the compounds of formula XXXIV. The reaction temperature for such a transformation (Krapcho O-dealkylation/decarboxylation) range preferentially from 20°C to the boiling point of the reaction mixture, or the reaction may be performed under microwave irradiation. Similar chemistry has been described in, for example, Synthesis 2010, No. 19, 3332-3338.

Alternatively compounds of formula IX-b, wherein R₅, R₆, and R₁ are as defined in formula I, and X is SO₂ can be prepared following scheme 9d. In scheme 9d, compounds of formula IX-b, wherein R₅, R₆, and R₁ are as defined in formula I, and X is SO₂ can be prepared from compounds of formula XXXXIII, wherein R₅ and R₆ are as defined in formula I, following procedure analgous to as described in scheme 9c for the conversion of compounds of formula XXXVII to compounds of formula IX-b. 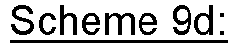 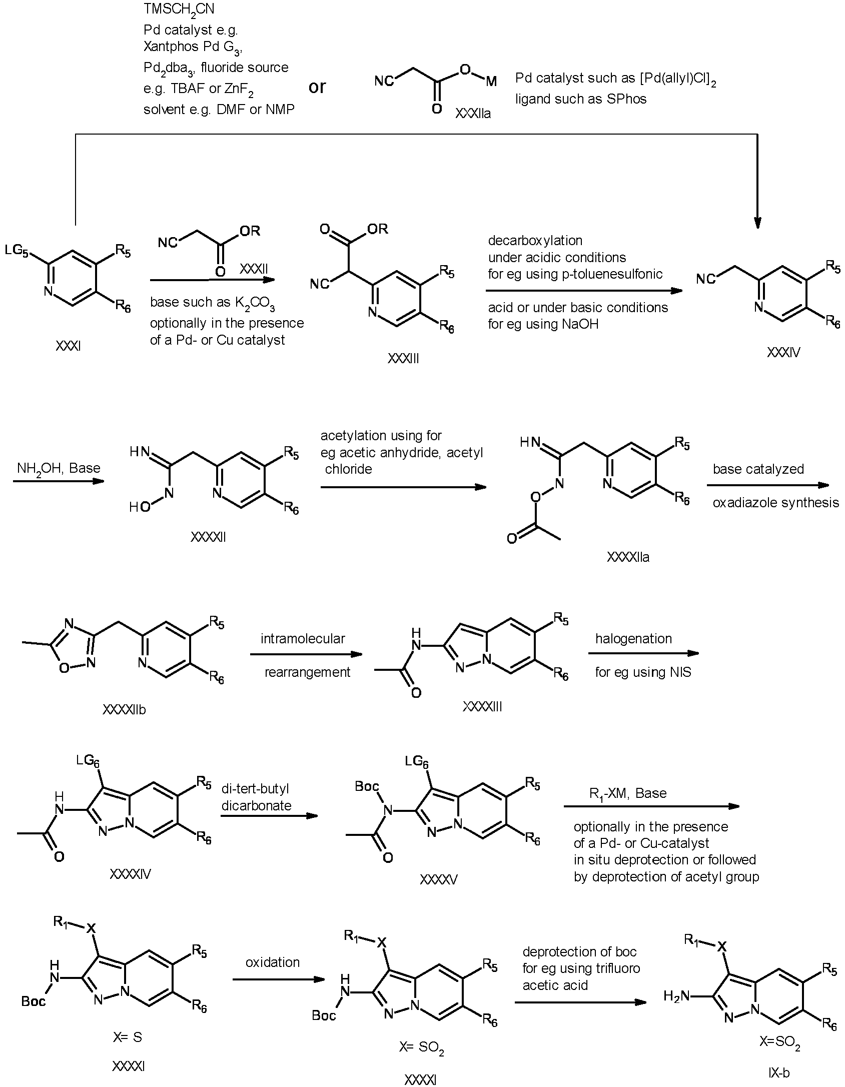 Compounds of formula XXXXIII, wherein R₅ and R₆ are as defined in formula I, can be prepared from compounds of formula XXXIV, wherein R₅ and R₆ are as defined in formula I, via four step procedure which involves reaction with hydroxylamine to form compounds of formula XXXXII, acetylation reaction to form compounds of formula XXXXlla, base catalyzed oxadiazole synthesis to form compounds of formula XXXXIIb and finally intramolecular cyclization/rearrangement to form compounds of formula XXXXIII. Such reactions have been reported in the literature for example described in WO2012146657, WO2012146659 or Tetrahedron Letters (2017), 58(3), 202-205. Compounds of formula XXXIV can be prepared from compounds of formula XXXI as described in scheme 9c.

The subgroup of compounds of formula IX, wherein Q is Qc, in which R₇, R₈, X, R₁, A, A₁ and A₂ are as defined in formula I, can be defined as compounds of formula IX-c, wherein R₇, R₈, X, R₁, A, A₁ and A₂ are as defined in formula I. Compounds of formula IX-c can be prepared from compounds of formula XVIII-c which are the subgroup of compounds of formula XVIII, wherein Q is Qc, in which R₇, R₈, X, R₁, A, A₁ and A₂ are as defined in formula I following scheme 9. Compounds of formula XVIII-c are either known in the literature, or they can be prepared by following analogous methods as described in, for example, WO2016/142326 A1 and WO2016091731 A1.

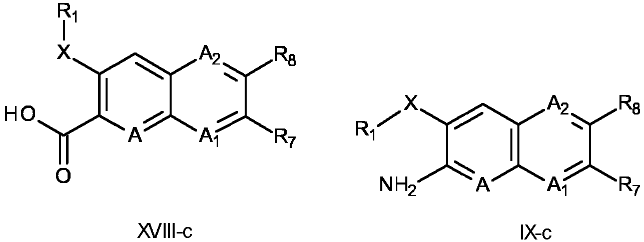

The subgroup of compounds of formula IX, wherein Q is Qc, in which R₇, R₈, X, R₁, A₁ and A₂ are as defined in formula I, and A is N can be defined as compounds of formula IX-c-1, wherein R₇, R₈, X, R₁, A₁ and A₂ are as defined in formula I. Compounds of formula IX-c-1 can be prepared following scheme 9e.

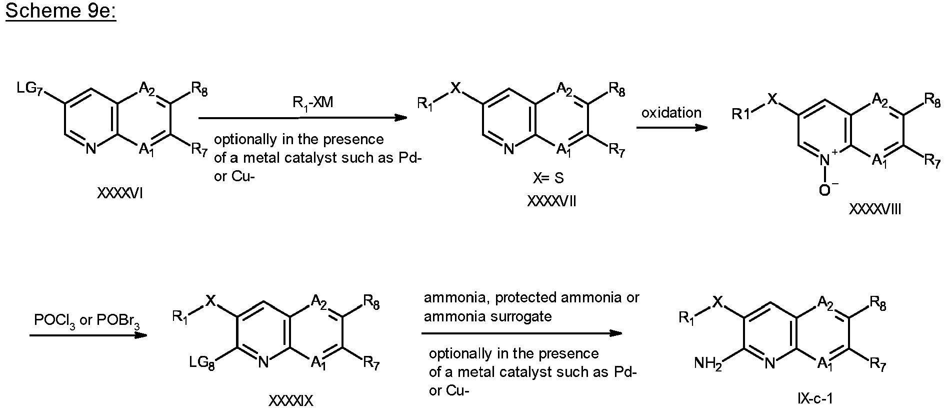

Compounds of formula IX-c-1, wherein R₇, R₈, X, R₁, A₁ and A₂ are as defined in formula I can be prepared by reacting compounds of formula XXXXIX, wherein R₇, R₈, X, R₁, A₁ and A₂ are as defined in formula I and in which LG₈ is a halogen preferably bromo or chloro with ammonia, aquous ammonia or surrogates of ammonia for example NH₄OH (or by using protected ammonia like tert-Butyl Carbamate and then deprotect it after the product formation) optionally in the presence of a base, such as sodium carbonate, potassium carbonate or cesium carbonate, or potassium *tert*-butoxide and optionally in the presence of a metal catalyst either copper catalyst for example copper(I) iodide, copper powder, copper sulfate and optionally in the presence of a ligand for example diamine ligands (e.g. N,N'-dimethylethylenediamine or *trans*-cyclohexyldiamine) or dibenzylideneacetone (dba), or 1,10-phenanthroline, at temperatures between 30-180°C, optionally under microwave irradiation, sealed condition or palladium catalyst for example palladium(II)acetate, bis(dibenzylideneacetone)palladium(0) (Pd(dba)2) or tris(dibenzylideneacetone)dipalladium(0) (Pd2(dba)3, optionally in form of a chloroform adduct), or a palladium pre-catalyst such as for example tert-BuBrettPhos Pd G3 [(2-Di-tert-butylphosphino-3,6-dimethoxy-2',4',6'-triisopropyl-1,1'-biphenyl)-2-(2'-amino-1,1'-biphenyl)]palladium(II) methanesulfonate or BrettPhos Pd G3 [(2-di-cyclohexylphosphino-3,6-dimethoxy-2',4',6'- triisopropyl-1,1'-biphenyl)-2-(2'-amino-1,1'-biphenyl)]palladium(II) methanesulfonate, and optionally in the presence of a ligand, for example SPhos, *t*-BuBrettPhos or Xantphos, at temperatures between 60-120°C, optionally under microwave irradiation. The above reaction may be carried out in the presence of solvent such as toluene, dimethylformamide DMF, N-methyl pyrrolidine NMP, dimethyl sulfoxide DMSO, dioxane, tetrahydrofuran THF and are described in literature for example in Tetrahedron (1987), 43(21), 4931-46, Organic Letters (2013), 15(14), 3734-3737, Organic Letters (2015), 17(23), 5934-5937, and WO2004035549 A1.

Compounds of formula XXXXIX, wherein R₇, R₈, X, R₁, A₁ and A₂ are as defined in formula I and in which LG₃ is a halogen preferably bromo or chloro can be prepared by reacting compounds of formula XXXXVIII, wherein R₇, R₈, X, R₁, A₁ and A₂ are as defined in formula I with halogenating reagent such as POCl₃, POBr₃ or trichloroacetonitrile in the presence of triphenylphosphine amongst other halogenating reagent capable of transforming heteroaryl N-oxides to the halo-functionalized derivatives. Such reactions are well known in the literature and described for example in Tetrahedron Letters (2014), 55(51), 7130-7132, Tetrahedron (2005), 61(38), 9042-9051 and European Journal of Organic Chemistry (2016), 2016(8), 1606-1611. Compounds of formula XXXXVIII, wherein R₇, R₈, X, R₁, A₁ and A₂ are as defined in formula I can be prepared by reacting compounds of formula XXXXVII, wherein R₇, R₈, X, R₁, A₁ and A₂ are as defined in formula I with oxidizing reagent such as Urea-H₂O₂, m-CPBA amongst other oxidizing reagent and in the presence of solvent such as acetonitrile, tetrahydrofuran, dichloromethane, dichloroethane amongst others. Such reactions are well known in the literature and described for example in Organic Letters (2018), 20(8), 2346-2350, Dalton Transactions (2014), 43(21), 8054-8061 and Chemische Berichte (1992), 125(8), 1965-6. Compounds of formula XXXXVII, wherein R₇, R₈, X, R₁, A₁ and A₂ are as defined in formula I can be prepared from compounds of formula XXXXVI, wherein R₇, R₈, X, R₁, A₁ and A₂ are as defined in formula I and and in which LG₇ is a halogen (or a pseudo-halogen leaving group, such as a triflate) following procedure as described in scheme 9c for the conversion of compounds of formula XXXIX to compounds of formula XXXXI.

Compounds of formula I, wherein Q is Qa and wherein R₁, X, R₃, and R₄ are as defined in formula I above, can be defined as compounds of formula I-a. Such compounds of formula I-a can be prepared following scheme 10.

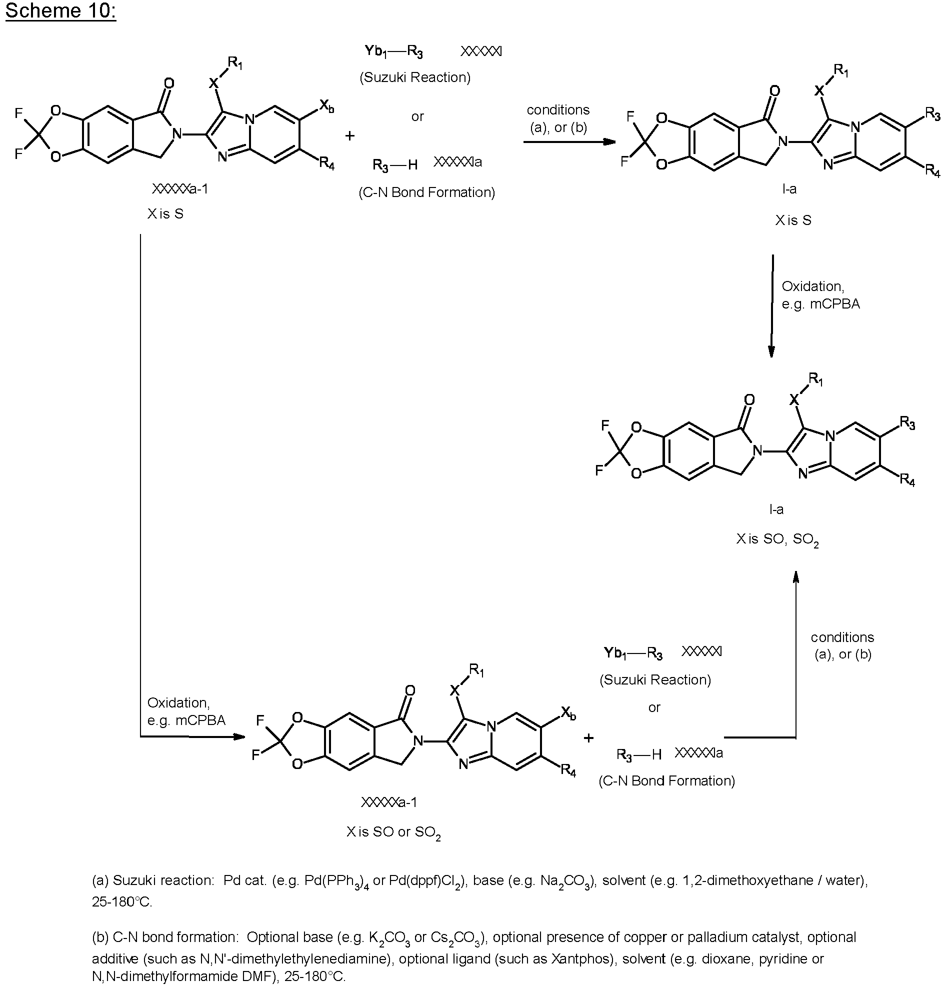

In the particular situation within scheme 10 when R₃ is, -N(R₉)C(=O)R_{10,} wherein R₉ and R₁₀ are as defined in formula I, then compounds of formula I-a, wherein R₁, R₃, and R₄ are as defined in formula I, and X is SO or SO₂, may be prepared from compounds of formula XXXXXa-1, wherein R₁, and R₄ are as defined in formula I, and in which X is SO or SO₂, and wherein X_{b} is a leaving group like, for example, chlorine, bromine or iodine (preferably chlorine or bromine), or an aryl- or alkylsulfonate such as trifluoromethanesulfonate, by reaction (C-N bond formation) with a reagent R₃-H (XXXXXla) equivalent to HN(R₉)C(=O)R₁₀, wherein R₉ and R₁₀ are as defined in formula I. Such a reaction is performed in the presence of a base, such as potassium carbonate, cesium carbonate, sodium hydroxide, in an inert solvent, such as toluene, dimethylformamide DMF, N-methyl pyrrolidine NMP, dimethyl sulfoxide DMSO, dioxane, tetrahydrofuran THF, and the like, optionally in the presence of a catalyst, for example palladium(II)acetate, bis(dibenzylideneacetone)palladium(0) (Pd(dba)₂) or tris(dibenzylideneacetone)dipalladium(0) (Pd₂(dba)₃, optionally in form of a chloroform adduct), or a palladium pre-catalyst such as for example *tert*-BuBrettPhos Pd G3 [(2-Di-*tert*-butylphosphino-3,6-dimethoxy-2',4',6'-triisopropyl-1,1'-biphenyl)-2-(2'-amino-1,1'-biphenyl)]palladium(II) methanesulfonate or BrettPhos Pd G3 [(2-di-cyclohexylphosphino-3,6-dimethoxy-2',4',6'- triisopropyl-1,1'-biphenyl)-2-(2'-amino-1,1'-biphenyl)]palladium(II) methanesulfonate, and optionally in the presence of a ligand, for example SPhos, *t*-BuBrettPhos or Xantphos, at temperatures between 60-120 °C, optionally under microwave irradiation.

In the particular situation within scheme 10 when R₃ is -N(R₉R₁₀), wherein R₉ and R₁₀ are as defined in formula I, then compounds of formula I-a, wherein R₁, R₃, and R₄ are as defined in formula I, and X is SO or SO₂, may be prepared from compounds of formula XXXXXa-1, wherein R₁, and R₄ are as defined in formula I, and in which X is SO or SO₂, and wherein X_{b} is a leaving group like, for example, chlorine, bromine or iodine (preferably chlorine or bromine), or an aryl- or alkylsulfonate such as trifluoromethanesulfonate, by reaction (C-N bond formation) with a reagent R₃-H (XXXXXIa) equivalent to HN(R₉R₁₀), or a salt thereof (such as a hydrohalide salt, preferably a hydrochloride or a hydrobromide salt, or a trifluoroacetic acid salt, or any other equivalent salt), wherein R₉ and R₁₀ are as defined in formula I. Such a reaction is commonly performed in an inert solvent such as alcohols, amides, esters, ethers, nitriles and water, particularly preferred are methanol, ethanol, 2,2,2-trifluoroethanol, propanol, isopropanol, N,N-dimethylformamide, N,N-dimethylacetamide, dioxane, tetrahydrofuran, dimethoxyethane, acetonitrile, ethyl acetate, toluene, water or mixtures thereof, at temperatures between 0-150 °C, optionally under microwave irradiation or pressurized conditions using an autoclave, optionally in the presence of a copper catalyst, such as copper powder, copper(I) iodide or copper sulfate (optionally in form of a hydrate), or mixtures thereof, optionaly in presence a ligand, for example diamine ligands (e.g. N,N'-dimethylethylenediamine or *trans*-cyclohexyldiamine) or dibenzylideneacetone (dba), or 1,10-phenanthroline, and optionally in presence of a base such as potassium phosphate.

Reagents HN(R₉R₁₀) or HN(R₉)COR₁₀, wherein R₉ and R₁₀ are as defined in formula I, are either known, commercially available or may be prepared by methods known to a person skilled in the art.

Alternatively, compounds of formula I-a, wherein R₁, R₃ and R₄ are as defined in formula I, and X is SO or SO₂, may be prepared by a Suzuki reaction, which involves for example, reacting compounds of formula XXXXXa-1, wherein R₁, and R₄ are as defined in formula I, and in which X is SO or SO₂, and wherein X_{b} is a leaving group like, for example, chlorine, bromine or iodine (preferably chlorine or bromine), or an aryl- or alkylsulfonate such as trifluoromethanesulfonate, with compounds of formula (XXXXXI), wherein R₃ is as defined in formula I, and wherein Y_{b1} can be a boron-derived functional group, such as for example B(OH)₂ or B(OR_{b1})₂ wherein R_{b1} can be a C₁-C₄alkyl group or the two groups OR_{b1} can form together with the boron atom a five membered ring, as for example a pinacol boronic ester. The reaction may be catalyzed by a palladium based catalyst, for example tetrakis(triphenyl-phosphine)palladium(0), (1,1'bis(diphenylphosphino)ferrocene)dichloro-palladium-dichloromethane (1:1 complex) or chloro(2-dicyclohexylphosphino-2',4',6'-triisopropyl-1,1'-biphenyl)[2-(2'-amino-1,1'-biphenyl)]palladium(II) (XPhos palladacycle), in presence of a base, like sodium carbonate, tripotassium phosphate or cesium fluoride, in a solvent or a solvent mixture, like, for example dioxane, acetonitrile, N,N-dimethyl-formamide, a mixture of 1,2-dimethoxyethane and water or of dioxane/water, or of toluene/water, preferably under inert atmosphere. The reaction temperature can preferentially range from room temperature to the boiling point of the reaction mixture, or the reaction may be performed under microwave irradiation. Such Suzuki reactions are well known to those skilled in the art and have been reviewed, for example, in J.Organomet. Chem. 576, 1999, 147-168.

Oxidation of compounds of formula XXXXXa-1, wherein R₁, and R₄ are as defined in formula I, and in which X is S, and wherein X_{b} is a leaving group like, for example, chlorine, bromine or iodine (preferably chlorine or bromine), or an aryl- or alkylsulfonate such as trifluoromethanesulfonate, with a suitable oxidizing agent, into compounds of formula XXXXXa-1, wherein X is SO or SO₂ may be achieved under conditions already described above in scheme 1a.

A large number of compounds of the formula (XXXXXI), and (XXXXXIa) are commercially available or can be prepared by those skilled in the art.

Alternatively, compounds of formula I, wherein X is SO or SO₂, may be prepared from compounds of formula XXXXXa-1, wherein X is S (sulfide) by involving the same chemistry as described above, but by changing the order of the steps (i.e. by running the sequence XXXXXa-1 (X is S) to I-a (X is S) via Suzuki, or C-N bond formation, followed by an oxidation step to form I-a (X is SO or SO₂).

Alternatively compounds of formula I-a, wherein R₁, X, R₃, and R₄ are as defined in formula I above, may be prepared following scheme 11.

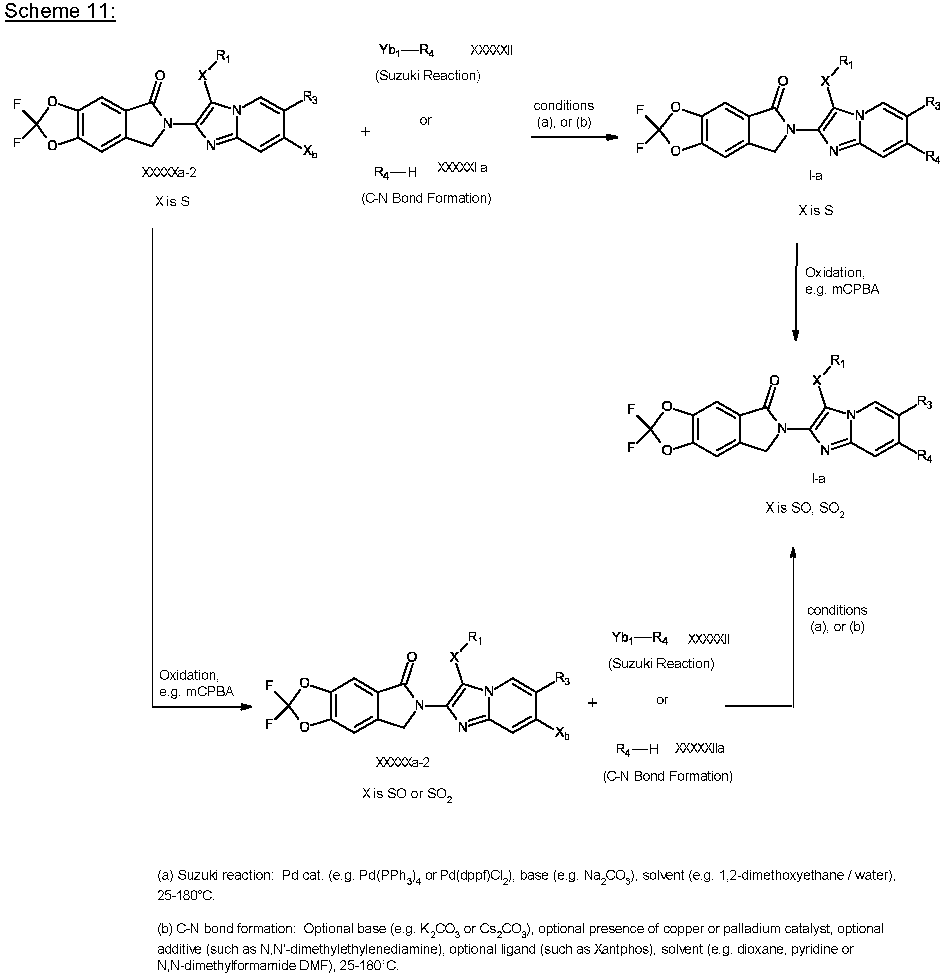

The chemistry described previously in scheme 10 to access compounds of formula I-a from compounds of formula XXXXXa-1, can be applied analogously (scheme 11) for the preparation of compounds of formula I-a from compounds of formula XXXXXa-2, wherein all substituent definitions mentioned previously remain valid.

Compounds of formula I, wherein Q is Qb and wherein R₁, X, R₅, and R₆ are as defined in formula I above, can be defined as compounds of formula I-aa. Such compounds of formula I-aa can be prepared following scheme 12 and 13. The chemistry described in scheme 10 and scheme 11 for the preparation of compounds of formula I-a can be applied analogously for the preparation of compounds of formula I-aa in scheme 12 and scheme 13. 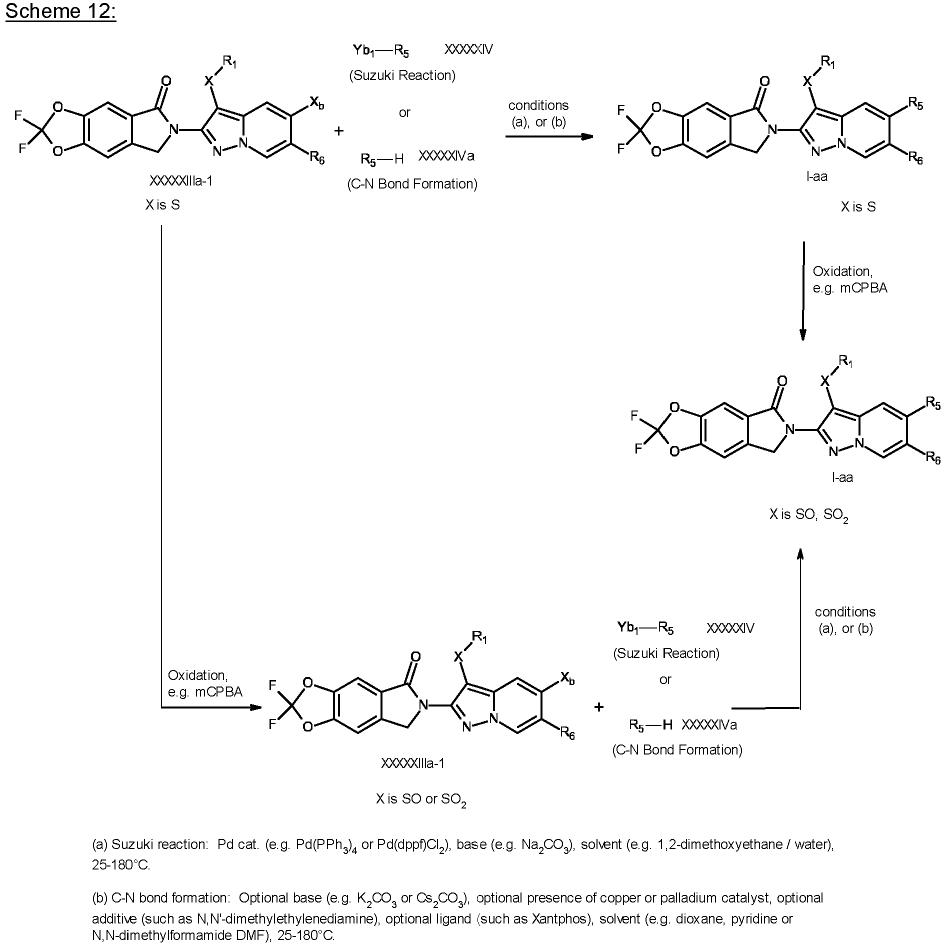 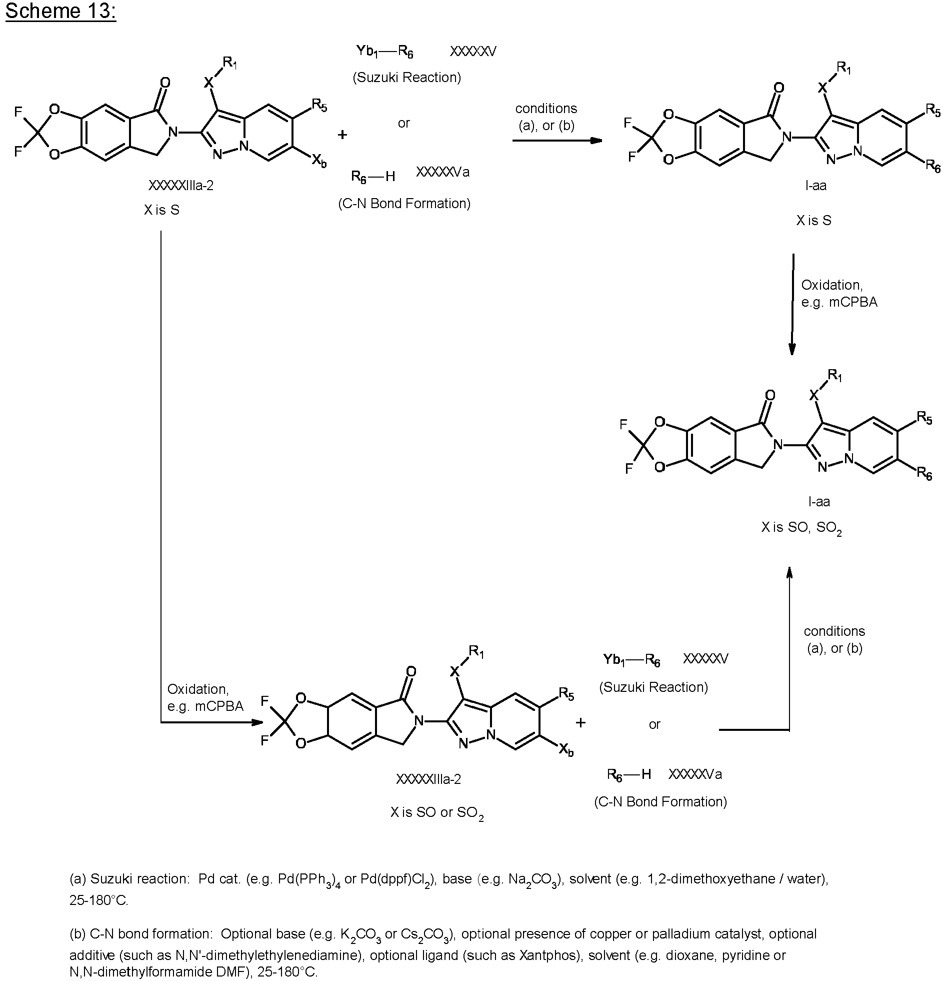 Compounds of formula I, wherein Q is Qc and wherein R₁, X, A, A₁, A₂, R₇, and R₈ are as defined in formula I above, can be defined as compounds of formula I-aaa. Such compounds of formula I-aaa can be prepared following scheme 14 and 15. The chemistry described in scheme 10 and scheme 11 for the preparation of compounds of formula I-a can be applied analogously for the preparation of compounds of formula I-aaa in scheme 14 and scheme 15.

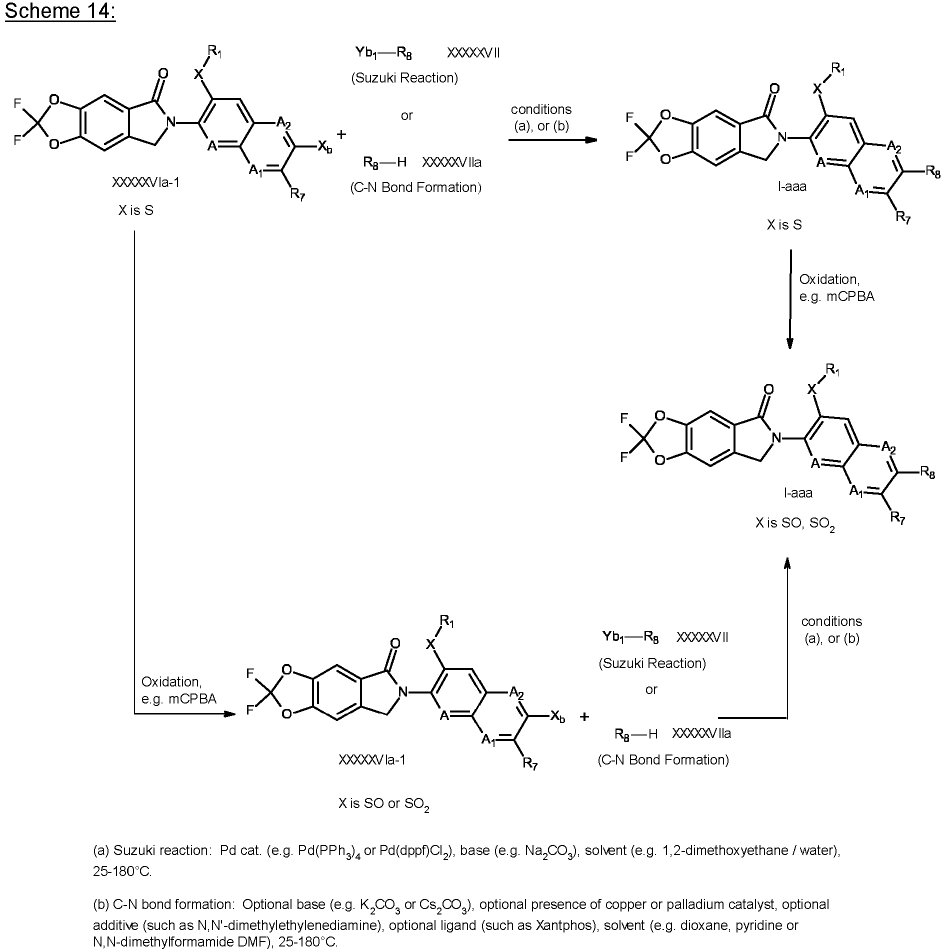

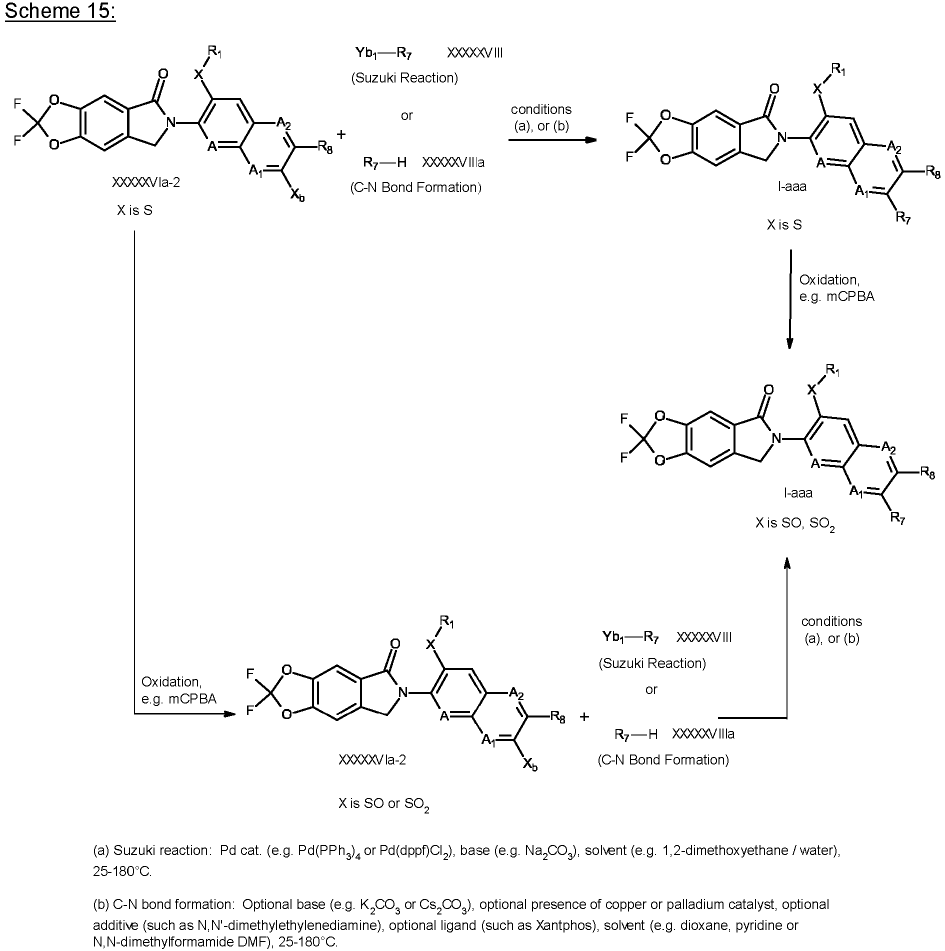

The reactants can be reacted in the presence of a base. Examples of suitable bases are alkali metal or alkaline earth metal hydroxides, alkali metal or alkaline earth metal hydrides, alkali metal or alkaline earth metal amides, alkali metal or alkaline earth metal alkoxides, alkali metal or alkaline earth metal acetates, alkali metal or alkaline earth metal carbonates, alkali metal or alkaline earth metal dialkylamides or alkali metal or alkaline earth metal alkylsilylamides, alkylamines, alkylenediamines, free or N-alkylated saturated or unsaturated cycloalkylamines, basic heterocycles, ammonium hydroxides and carbocyclic amines. Examples which may be mentioned are sodium hydroxide, sodium hydride, sodium amide, sodium methoxide, sodium acetate, sodium carbonate, potassium tert-butoxide, potassium hydroxide, potassium carbonate, potassium hydride, lithium diisopropylamide, potassium bis(trimethylsilyl)amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide and 1,8-diazabicyclo[5.4.0]undec-7-ene (DBU).

The reactants can be reacted with each other as such, i.e. without adding a solvent or diluent. In most cases, however, it is advantageous to add an inert solvent or diluent or a mixture of these. If the reaction is carried out in the presence of a base, bases which are employed in excess, such as triethylamine, pyridine, N-methylmorpholine or N,N-diethylaniline, may also act as solvents or diluents.

The reactions are advantageously carried out in a temperature range from approximately -80°C to approximately +140°C, preferably from approximately -30°C to approximately +100°C, in many cases in the range between ambient temperature and approximately +80°C.

A compound of formula I can be converted in a manner known per se into another compound of formula I by replacing one or more substituents of the starting compound of formula I in the customary manner by (an)other substituent(s) according to the invention, and by post modification of compounds of with reactions such as oxidation, alkylation, reduction, acylation and other methods known by those skilled in the art.

Depending on the choice of the reaction conditions and starting materials which are suitable in each case, it is possible, for example, in one reaction step only to replace one substituent by another substituent according to the invention, or a plurality of substituents can be replaced by other substituents according to the invention in the same reaction step.

Salts of compounds of formula I can be prepared in a manner known per se. Thus, for example, acid addition salts of compounds of formula I are obtained by treatment with a suitable acid or a suitable ion exchanger reagent and salts with bases are obtained by treatment with a suitable base or with a suitable ion exchanger reagent.

Salts of compounds of formula I can be converted in the customary manner into the free compounds I, acid addition salts, for example, by treatment with a suitable basic compound or with a suitable ion exchanger reagent and salts with bases, for example, by treatment with a suitable acid or with a suitable ion exchanger reagent.

Salts of compounds of formula I can be converted in a manner known per se into other salts of compounds of formula I, acid addition salts, for example, into other acid addition salts, for example by treatment of a salt of inorganic acid such as hydrochloride with a suitable metal salt such as a sodium, barium or silver salt, of an acid, for example with silver acetate, in a suitable solvent in which an inorganic salt which forms, for example silver chloride, is insoluble and thus precipitates from the reaction mixture.

Depending on the procedure or the reaction conditions, the compounds of formula I, which have salt-forming properties can be obtained in free form or in the form of salts.

The compounds of formula I and, where appropriate, the tautomers thereof, in each case in free form or in salt form, can be present in the form of one of the isomers which are possible or as a mixture of these, for example in the form of pure isomers, such as antipodes and/or diastereomers, or as isomer mixtures, such as enantiomer mixtures, for example racemates, diastereomer mixtures or racemate mixtures, depending on the number, absolute and relative configuration of asymmetric carbon atoms which occur in the molecule and/or depending on the configuration of non-aromatic double bonds which occur in the molecule; the invention relates to the pure isomers and also to all isomer mixtures which are possible and is to be understood in each case in this sense hereinabove and hereinbelow, even when stereochemical details are not mentioned specifically in each case.

Diastereomer mixtures or racemate mixtures of compounds of formula I, in free form or in salt form, which can be obtained depending on which starting materials and procedures have been chosen can be separated in a known manner into the pure diasteromers or racemates on the basis of the physicochemical differences of the components, for example by fractional crystallization, distillation and/or chromatography.

Enantiomer mixtures, such as racemates, which can be obtained in a similar manner can be resolved into the optical antipodes by known methods, for example by recrystallization from an optically active solvent, by chromatography on chiral adsorbents, for example high-performance liquid chromatography (HPLC) on acetyl celulose, with the aid of suitable microorganisms, by cleavage with specific, immobilized enzymes, via the formation of inclusion compounds, for example using chiral crown ethers, where only one enantiomer is complexed, or by conversion into diastereomeric salts, for example by reacting a basic end-product racemate with an optically active acid, such as a carboxylic acid, for example camphor, tartaric or malic acid, or sulfonic acid, for example camphorsulfonic acid, and separating the diastereomer mixture which can be obtained in this manner, for example by fractional crystallization based on their differing solubilities, to give the diastereomers, from which the desired enantiomer can be set free by the action of suitable agents, for example basic agents.

Pure diastereomers or enantiomers can be obtained according to the invention not only by separating suitable isomer mixtures, but also by generally known methods of diastereoselective or enantioselective synthesis, for example by carrying out the process according to the invention with starting materials of a suitable stereochemistry.

N-oxides can be prepared by reacting a compound of the formula I with a suitable oxidizing agent, for example the H₂O₂/urea adduct in the presence of an acid anhydride, e.g. trifluoroacetic anhydride. Such oxidations are known from the literature, for example from J. Med. Chem., 32 (12), 2561-73, 1989 or WO 2000/15615.

It is advantageous to isolate or synthesize in each case the biologically more effective isomer, for example enantiomer or diastereomer, or isomer mixture, for example enantiomer mixture or diastereomer mixture, if the individual components have a different biological activity.

The compounds of formula I and, where appropriate, the tautomers thereof, in each case in free form or in salt form, can, if appropriate, also be obtained in the form of hydrates and/or include other solvents, for example those which may have been used for the crystallization of compounds which are present in solid form.

The compounds of formula I according to the following Tables A-1 to A-3, B-1 to B-3, C-1 to C-3, D-1 to D-3, E-1 to E-3, and F-1 to F-3 can be prepared according to the methods described above. The examples which follow are intended to illustrate the invention and show preferred compounds of formula I, in the form of a compound of formula la-Qa to Ib-Qc.

The tables below illustrate specific compounds of the invention.

The tables A-1 to A-3 below illustrate specific compound of the invention.

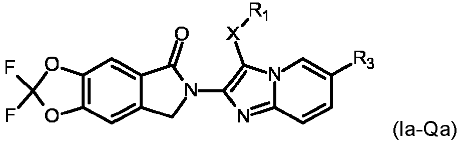

Table A-1 provides 14 compounds A-1.001 to A-1.014 of formula la-Qa wherein X is S, R₁ is ethyl and R₃ are as defined in table M.

**Table M: Substituent definitions of R₃**

| Index | R₃ |
|---|---|
| 1 | H |
| 2 | CF₂CH₃ |
| 3 | CF3 |
| 4 | OCHF₂ |
| 5 | -N(CH₃)COCH₃ |
| 6 | -NHCOCH₃ |
| 7 | -OCH₂CHF₂ |
| 8 | -OCH₂CF₃ |
| 9 | 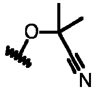 |
| 10 | 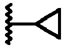 |
| 11 | 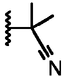 |
| 12 | 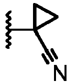 |
| 13 | OCF₃ |
| 14 | CHF₂ |

Table A-2 provides 14 compounds A-2.001 to A-2.014 of formula la-Qa wherein X is SO, R₁ is ethyl and R₃ are as defined in table M.

Table A-3 provides 14 compounds A-3.001 to A-3.014 of formula la-Qa wherein X is SO₂, R₁ is ethyl and R₃ are as defined in table M.

The tables B-1 to B-3 below illustrate further specific compound of the invention.

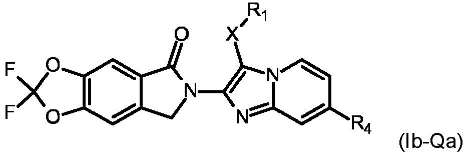

Table B-1 provides 14 compounds B-1.001 to B-1.014 of formula Ib-Qa wherein X is S, R₁ is ethyl and R₄ are as defined in table N.

**Table N: Substituent definitions of R₄**

| Index | R4 |
|---|---|
| 1 | H |
| 2 | CF₂CH₃ |
| 3 | CF₃ |
| 4 | OCHF₂ |
| 5 | -N(CH₃)COCH₃ |
| 6 | -NHCOCH₃ |
| 7 | -OCH₂CHF₂ |
| 8 | -OCH₂CF₃ |
| 9 | 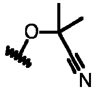 |
| 10 | 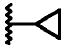 |
| 11 | 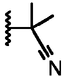 |
| 12 | 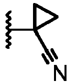 |
| 13 | OCF₃ |
| 14 | CHF₂ |

Table B-2 provides 14 compounds B-2.001 to B-2.014 of formula Ib-Qa wherein X is SO, R₁ is ethyl and R₄ are as defined in table N.

Table B-3 provides 14 compounds B-3.001 to B-3.014 of formula Ib-Qa wherein X is SO₂, R₁ is ethyl and R₄ are as defined in table N.

The tables C-1 to C-3 below illustrate further specific compound of the invention.

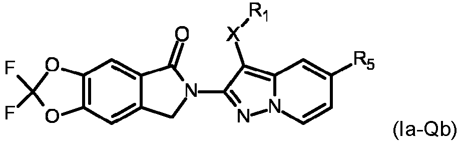

Table C-1 provides 14 compounds C-1.001 to C-1.014 of formula la-Qb wherein X is S, R₁ is ethyl and R₅ are as defined in table O.

**Table O: Substituent definitions of Rs**

| Index | R₅ |
|---|---|
| 1 | H |
| 2 | CF₂CH₃ |
| 3 | CF₃ |
| 4 | OCHF₂ |
| 5 | -N(CH₃)COCH₃ |
| 6 | -NHCOCH₃ |
| 7 | -OCH₂CHF₂ |
| 8 | -OCH₂CF₃ |
| 9 | 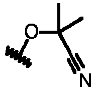 |
| 10 | 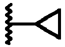 |
| 11 | 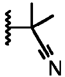 |
| 12 | 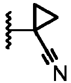 |
| 13 | OCF₃ |
| 14 | CHF₂ |
| 15 | F |
| 16 | Cl |
| 17 | Br |
| 18 | I |

Table C-2 provides 18 compounds C-2.001 to C-2.018 of formula la-Qb wherein X is SO, R₁ is ethyl and R₅ are as defined in table O.

Table C-3 provides 18 compounds C-3.001 to C-3.018 of formula la-Qb wherein X is SO₂, R₁ is ethyl and R₅ are as defined in table O.

The tables D-1 to D-3 below illustrate further specific compound of the invention.

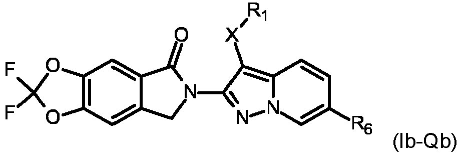

Table D-1 provides 14 compounds D-1.001 to D-1.014 of formula Ib-Qb wherein X is S, R₁ is ethyl and R₆ are as defined in table P1.

**Table P1: Substituent definitions of R₆**

| Index | R₆ |
|---|---|
| 1 | H |
| 2 | CF₂CH₃ |
| 3 | CF₃ |
| 4 | OCHF₂ |
| 5 | -N(CH₃)COCH₃ |
| 6 | -NHCOCH₃ |
| 7 | -OCH₂CHF₂ |
| 8 | -OCH₂CF₃ |
| 9 | 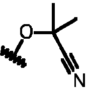 |
| 10 | 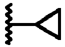 |
| 11 | 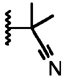 |
| 12 | 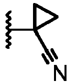 |
| 13 | OCF₃ |
| 14 | CHF₂ |
| 15 | F |
| 16 | Cl |
| 17 | Br |
| 18 | I |

Table D-2 provides 18 compounds D-2.001 to D-2.018 of formula Ib-Qb wherein X is SO, R₁ is ethyl and R₆ are as defined in table P1.

Table D-3 provides 18 compounds D-3.001 to D-3.018 of formula Ib-Qb wherein X is SO₂, R₁ is ethyl and R₆ are as defined in table P1.

The tables E-1 to E-3 below illustrate further specific compound of the invention.

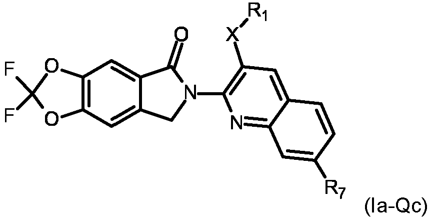

Table E-1 provides 14 compounds E-1.001 to E-1.014 of formula la-Qc wherein X is S, R₁ is ethyl and R₇ are as defined in table Q.

**Table Q: Substituent definitions of R₇**

| Index | R₇ |
|---|---|
| 1 | H |
| 2 | CF₂CH₃ |
| 3 | CF₃ |
| 4 | OCHF₂ |
| 5 | -N(CH₃)COCH₃ |
| 6 | -NHCOCH₃ |
| 7 | -OCH₂CHF₂ |
| 8 | -OCH₂CF₃ |
| 9 | 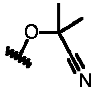 |
| 10 | 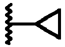 |
| 11 | 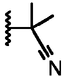 |
| 12 | 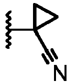 |
| 13 | OCF₃ |
| 14 | CHF₂ |

Table E-2 provides 14 compounds E-2.001 to E-2.014 of formula la-Qc wherein X is SO, R₁ is ethyl and R₇ are as defined in table Q.

Table E-3 provides 14 compounds E-3.001 to E-3.014 of formula la-Qc wherein X is SO₂, R₁ is ethyl and R₇ are as defined in table Q.

The tables F-1 to F-3 below illustrate further specific compound of the invention.

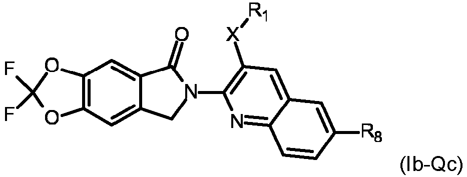

Table F-1 provides 14 compounds F-1.001 to F-1.014 of formula Ib-Qc wherein X is S, R₁ is ethyl and R₈ are as defined in table R.

**Table R: Substituent definitions of R₈**

| Index | R₈ |
|---|---|
| 1 | H |
| 2 | CF₂CH₃ |
| 3 | CF₃ |
| 4 | OCHF₂ |
| 5 | -N(CH₃)COCH₃ |
| 6 | -NHCOCH₃ |
| 7 | -OCH₂CHF₂ |
| 8 | -OCH₂CF₃ |
| 9 | 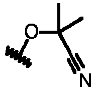 |
| 10 | 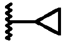 |
| 11 | 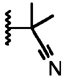 |
| 12 | 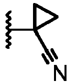 |
| 13 | OCF₃ |
| 14 | CHF₂ |

Table F-2 provides 14 compounds F-2.001 to F-2.014 of formula Ib-Qc wherein X is SO, R₁ is ethyl and R₈ are as defined in table R.

Table F-3 provides 14 compounds F-3.001 to F-3.014 of formula Ib-Qc wherein X is SO₂, R₁ is ethyl and R₈ are as defined in table R.

The compounds of formula I according to the invention are preventively and/or curatively valuable active ingredients in the field of pest control, even at low rates of application, which have a very favorable biocidal spectrum and are well tolerated by warm-blooded species, fish and plants. The active ingredients according to the invention act against all or individual developmental stages of normally sensitive, but also resistant, animal pests, such as insects or representatives of the order Acarina. The insecticidal or acaricidal activity of the active ingredients according to the invention can manifest itself directly, i. e. in destruction of the pests, which takes place either immediately or only after some time has elapsed, for example during ecdysis, or indirectly, for example in a reduced oviposition and/or hatching rate, a good activity corresponding to a destruction rate (mortality) of at least 50 to 60%.

Examples of the above-mentioned animal pests are:
from the order *Acarina,* for example,
Acalitus spp, Aculus spp, Acaricalus spp, Aceria spp, Acarus siro, Amblyomma spp., Argas spp., Boophilus spp., Brevipalpus spp., Bryobia spp, Calipitrimerus spp., Chorioptes spp., Dermanyssus gallinae, Dermatophagoides spp, Eotetranychus spp, Eriophyes spp., Hemitarsonemus spp, Hyalomma spp., Ixodes spp., Olygonychus spp, Ornithodoros spp., Polyphagotarsone latus, Panonychus spp., Phyllocoptruta oleivora, Phytonemus spp, Polyphagotarsonemus spp, Psoroptes spp., Rhipicephalus spp., Rhizoglyphus spp., Sarcoptes spp., Steneotarsonemus spp, Tarsonemus spp. and Tetranychus spp.;
from the order *Anoplura,* for example,
Haematopinus spp., Linognathus spp., Pediculus spp., Pemphigus spp. and Phylloxera spp.;
from the order *Coleoptera,* for example,
Agriotes spp., Amphimallon majale, Anomala orientalis, Anthonomus spp., Aphodius spp, Astylus atromaculatus, Ataenius spp, Atomaria linearis, Chaetocnema tibialis, Cerotoma spp, Conoderus spp, Cosmopolites spp., Cotinis nitida, Curculio spp., Cyclocephala spp, Dermestes spp., Diabrotica spp., Diloboderus abderus, Epilachna spp., Eremnus spp., Heteronychus arator, Hypothenemus hampei, Lagria vilosa, Leptinotarsa decemLineata, Lissorhoptrus spp., Liogenys spp, Maecolaspis spp, Maladera castanea, Megascelis spp, Melighetes aeneus, Melolontha spp., Myochrous armatus, Orycaephilus spp., Otiorhynchus spp., Phyllophaga spp, Phlyctinus spp., Popillia spp., Psylliodes spp., Rhyssomatus aubtilis, Rhizopertha spp., Scarabeidae, Sitophilus spp., Sitotroga spp., Somaticus spp, Sphenophorus spp, Sternechus subsignatus, Tenebrio spp., Tribolium spp. and Trogoderma spp.; from the order *Diptera,* for example,
Aedes spp., Anopheles spp, Antherigona soccata,Bactrocea oleae, Bibio hortulanus, Bradysia spp, Calliphora erythrocephala, Ceratitis spp., Chrysomyia spp., Culex spp., Cuterebra spp., Dacus spp., Delia spp, Drosophila melanogaster, Fannia spp., Gastrophilus spp., Geomyza tripunctata, Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis spp, Rivelia quadrifasciata, Scatella spp, Sciara spp., Stomoxys spp., Tabanus spp., Tannia spp. and Tipula spp.;
from the order *Hemiptera,* for example,
Acanthocoris scabrator, Acrosternum spp, Adelphocoris lineolatus, Amblypelta nitida, Bathycoelia thalassina, Blissus spp, Cimex spp., Clavigralla tomentosicollis, Creontiades spp, Distantiella theobroma, Dichelops furcatus, Dysdercus spp., Edessa spp, Euschistus spp., Eurydema pulchrum, Eurygaster spp., Halyomorpha halys, Horcias nobilellus, Leptocorisa spp., Lygus spp, Margarodes spp, Murgantia histrionic, Neomegalotomus spp, Nesidiocoris tenuis, Nezara spp., Nysius simulans, Oebalus insularis, Piesma spp., Piezodorus spp, Rhodnius spp., Sahlbergella singularis, Scaptocoris castanea, Scotinophara spp. , Thyanta spp , Triatoma spp., Vatiga illudens;
Acyrthosium pisum, Adalges spp, Agalliana ensigera, Agonoscena targionii, Aleurodicus spp, Aleurocanthus spp, Aleurolobus barodensis, Aleurothrixus floccosus, Aleyrodes brassicae, Amarasca biguttula, Amritodus atkinsoni, Aonidiella spp., Aphididae, Aphis spp., Aspidiotus spp., Aulacorthum solani, Bactericera cockerelli, Bemisia spp, Brachycaudus spp, Brevicoryne brassicae, Cacopsylla spp, Cavariella aegopodii Scop., Ceroplaster spp., Chrysomphalus aonidium, Chrysomphalus dictyospermi, Cicadella spp, Cofana spectra, Cryptomyzus spp, Cicadulina spp, Coccus hesperidum, Dalbulus maidis, Dialeurodes spp, Diaphorina citri, Diuraphis noxia, Dysaphis spp, Empoasca spp., Eriosoma larigerum, Erythroneura spp., Gascardia spp., Glycaspis brimblecombei, Hyadaphis pseudobrassicae, Hyalopterus spp, Hyperomyzus pallidus, Idioscopus clypealis, Jacobiasca lybica, Laodelphax spp., Lecanium corni, Lepidosaphes spp., Lopaphis erysimi, Lyogenys maidis, Macrosiphum spp., Mahanarva spp, Metcalfa pruinosa, Metopolophium dirhodum, Myndus crudus, Myzus spp., Neotoxoptera sp, Nephotettix spp., Nilaparvata spp., Nippolachnus piri Mats, Odonaspis ruthae, Oregma lanigera Zehnter, Parabemisia myricae, Paratrioza cockerelli, Parlatoria spp., Pemphigus spp., Peregrinus maidis, Perkinsiella spp, Phorodon humuli, Phylloxera spp, Planococcus spp., Pseudaulacaspis spp., Pseudococcus spp., Pseudatomoscelis seriatus, Psylla spp., Pulvinaria aethiopica, Quadraspidiotus spp., Quesada gigas, Recilia dorsalis, Rhopalosiphum spp., Saissetia spp., Scaphoideus spp., Schizaphis spp., Sitobion spp., Sogatella furcifera, Spissistilus festinus, Tarophagus Proserpina, Toxoptera spp, Trialeurodes spp, Tridiscus sporoboli, Trionymus spp, Trioza erytreae , Unaspis citri, Zygina flammigera, Zyginidia scutellaris, ;
from the order *Hymenoptera,* for example,
Acromyrmex, Arge spp, Atta spp., Cephus spp., Diprion spp., Diprionidae, Gilpinia polytoma, Hoplo-campa spp., Lasius spp., Monomorium pharaonis, Neodiprion spp., Pogonomyrmex spp, Slenopsis invicta, Solenopsis spp. and Vespa spp.;
from the order *Isoptera,* for example,
Coptotermes spp, Corniternes cumulans, Incisitermes spp, Macrotermes spp, Mastotermes spp, Microtermes spp, Reticulitermes spp.; Solenopsis geminate
from the order *Lepidoptera,* for example,
Acleris spp., Adoxophyes spp., Aegeria spp., Agrotis spp., Alabama argillaceae, Amylois spp., Anticarsia gemmatalis, Archips spp., Argyresthia spp, Argyrotaenia spp., Autographa spp., Bucculatrix thurberiella, Busseola fusca, Cadra cautella, Carposina nipponensis, Chilo spp., Choristoneura spp., Chrysoteuchia topiaria, Clysia ambiguella, Cnaphalocrocis spp., Cnephasia spp., Cochylis spp., Coleophora spp., Colias lesbia, Cosmophila flava, Crambus spp, Crocidolomia binotalis, Cryptophlebia leucotreta, Cydalima perspectalis, Cydia spp., Diaphania perspectalis, Diatraea spp., Diparopsis castanea, Earias spp., Eldana saccharina, Ephestia spp., Epinotia spp, Estigmene acrea, Etiella zinckinella, Eucosma spp., Eupoecilia ambiguella, Euproctis spp., Euxoa spp., Feltia jaculiferia, Grapholita spp., Hedya nubiferana, Heliothis spp., Hellula undalis, Herpetogramma spp, Hyphantria cunea, Keiferia lycopersicella, Lasmopalpus lignosellus, Leucoptera scitella, Lithocollethis spp., Lobesia botrana, Loxostege bifidalis, Lymantria spp., Lyonetia spp., Malacosoma spp., Mamestra brassicae, Manduca sexta, Mythimna spp, Noctua spp, Operophtera spp., Orniodes indica, Ostrinia nubilalis, Pammene spp., Pandemis spp., Panolis flammea, Papaipema nebris, Pectinophora gossypi-ela, Perileucoptera coffeella, Pseudaletia unipuncta, Phthorimaea operculella, Pieris rapae, Pieris spp., Plutella xylostella, Prays spp., Pseudoplusia spp, Rachiplusia nu, Richia albicosta, Scirpophaga spp., Sesamia spp., Sparganothis spp., Spodoptera spp., Sylepta derogate, Synanthedon spp., Thaumetopoea spp., Tortrix spp., Trichoplusia ni, Tuta absoluta, and Yponomeuta spp.;
from the order *Mallophaga,* for example,
Damalinea spp. and Trichodectes spp.;
from the order *Orthoptera,* for example,
Blatta spp., Blattella spp., Gryllotalpa spp., Leucophaea maderae, Locusta spp., Neocurtilla hexadactyla, Periplaneta spp. , Scapteriscus spp, and Schistocerca spp.;
from the order *Psocoptera,* for example,
Liposcelis spp.;
from the order *Siphonaptera,* for example,
Ceratophyllus spp., Ctenocephalides spp. and Xenopsylla cheopis;
from the order *Thysanoptera,* for example,
Calliothrips phaseoli, Frankliniella spp., Heliothrips spp, Hercinothrips spp., Parthenothrips spp, Scirtothrips aurantii, Sericothrips variabilis, Taeniothrips spp., Thrips spp;
from the order *Thysanura,* for example, Lepisma saccharina.

The active ingredients according to the invention can be used for controlling, i. e. containing or destroying, pests of the abovementioned type which occur in particular on plants, especially on useful plants and ornamentals in agriculture, in horticulture and in forests, or on organs, such as fruits, flowers, foliage, stalks, tubers or roots, of such plants, and in some cases even plant organs which are formed at a later point in time remain protected against these pests.

Suitable target crops are, in particular, cereals, such as wheat, barley, rye, oats, rice, maize or sorghum; beet, such as sugar or fodder beet; fruit, for example pomaceous fruit, stone fruit or soft fruit, such as apples, pears, plums, peaches, almonds, cherries or berries, for example strawberries, raspberries or blackberries; leguminous crops, such as beans, lentils, peas or soya; oil crops, such as oilseed rape, mustard, poppies, olives, sunflowers, coconut, castor, cocoa or ground nuts; cucurbits, such as pumpkins, cucumbers or melons; fibre plants, such as cotton, flax, hemp or jute; citrus fruit, such as oranges, lemons, grapefruit or tangerines; vegetables, such as spinach, lettuce, asparagus, cabbages, carrots, onions, tomatoes, potatoes or bell peppers; Lauraceae, such as avocado, Cinnamonium or camphor; and also tobacco, nuts, coffee, eggplants, sugarcane, tea, pepper, grapevines, hops, the plantain family and latex plants.

The compositions and/or methods of the present invention may be also used on any ornamental and/or vegetable crops, including flowers, shrubs, broad-leaved trees and evergreens.

For example the invention may be used on any of the following ornamental species: *Ageratum* spp., *Alonsoa* spp., *Anemone* spp., *Anisodontea capsenisis, Anthemis* spp., *Antirrhinum* spp., *Aster* spp., *Begonia* spp. (e.g. B. *elatior, B. semperflorens, B. tubéreux), Bougainvillea* spp., *Brachycome* spp., *Brassica* spp. (ornamental), *Calceolaria* spp., *Capsicum annuum, Catharanthus roseus, Canna* spp., *Centaurea* spp., *Chrysanthemum* spp., *Cineraria* spp. *(C. maritime), Coreopsis* spp., *Crassula coccinea, Cuphea ignea, Dahlia* spp., *Delphinium* spp., *Dicentra spectabilis, Dorotheantus* spp., *Eustoma grandiflorum, Forsythia* spp., *Fuchsia* spp., *Geranium gnaphalium, Gerbera* spp., *Gomphrena globosa, Heliotropium* spp., *Helianthus* spp., *Hibiscus* spp., *Hortensia* spp., *Hydrangea* spp., *Hypoestes phyllostachya, Impatiens* spp. (*I. Walleriana), Iresines* spp., *Kalanchoe* spp., *Lantana camara, Lavatera trimestris, Leonotis leonurus, Lilium* spp., *Mesembryanthemum* spp., *Mimulus* spp., *Monarda* spp., *Nemesia* spp., *Tagetes* spp., *Dianthus* spp. (carnation), *Canna* spp., *Oxalis* spp., *Bellis* spp., *Pelargonium* spp. *(P. peltatum, P. Zonale), Viola* spp. (pansy), *Petunia* spp., *Phlox* spp., *Plecthranthus* spp., *Poinsettia* spp., *Parthenocissus* spp. *(P. quinquefolia, P. tricuspidata), Primula* spp., *Ranunculus* spp., *Rhododendron* spp., *Rosa* spp. (rose), *Rudbeckia* spp., *Saintpaulia* spp., *Salvia* spp., *Scaevola aemola, Schizanthus wisetonensis, Sedum* spp., *Solanum* spp., *Surfinia* spp., *Tagetes* spp., *Nicotinia* spp., *Verbena* spp., *Zinnia* spp. and other bedding plants.

For example the invention may be used on any of the following vegetable species: *Allium* spp. (A. *sativum, A.. cepa, A. oschaninii, A. Porrum, A. ascalonicum, A. fistulosum), Anthriscus cerefolium, Apium graveolus, Asparagus officinalis, Beta vulgarus, Brassica* spp. *(B. Oleracea, B. Pekinensis, B. rapa), Capsicum annuum, Cicer arietinum, Cichorium endivia, Cichorum* spp. *(C. intybus, C. endivia), Citrillus lanatus, Cucumis* spp. *(C. sativus, C. melo), Cucurbita* spp. *(C. pepo, C. maxima), Cyanara* spp. *(C. scolymus, C. cardunculus), Daucus carota, Foeniculum vulgare, Hypericum* spp., *Lactuca sativa, Lycopersicon* spp. *(L. esculentum, L. lycopersicum), Mentha* spp., *Ocimum basilicum, Petroselinum crispum, Phaseolus* spp. *(P. vulgaris, P. coccineus), Pisum sativum, Raphanus sativus, Rheum rhaponticum, Rosemarinus* spp., *Salvia* spp., *Scorzonera hispanica, Solanum melongena, Spinacea oleracea, Valerianella* spp. *(V. locusta, V. eriocarpa)* and *Vicia faba.*

Preferred ornamental species include African violet, *Begonia, Dahlia, Gerbera, Hydrangea, Verbena, Rosa, Kalanchoe, Poinsettia, Aster, Centaurea, Coreopsis, Delphinium, Monarda, Phlox, Rudbeckia, Sedum, Petunia, Viola, Impatiens, Geranium, Chrysanthemum, Ranunculus, Fuchsia, Salvia, Hortensia,* rosemary, sage, St. Johnswort, mint, sweet pepper, tomato and cucumber.

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca(preferably in vegetables, vineyards), Leptinotarsa (preferably in potatos) and Chilo supressalis (preferably in rice).

The active ingredients according to the invention are especially suitable for controlling Aphis craccivora, Diabrotica balteata, Heliothis virescens, Myzus persicae, Plutella xylostella and Spodoptera littoralis in cotton, vegetable, maize, rice and soya crops. The active ingredients according to the invention are further especially suitable for controlling Mamestra (preferably in vegetables), Cydia pomonella (preferably in apples), Empoasca(preferably in vegetables, vineyards), Leptinotarsa (preferably in potatos) and Chilo supressalis (preferably in rice).

In a further aspect, the invention may also relate to a method of controlling damage to plant and parts thereof by plant parasitic nematodes (Endoparasitic-, Semiendoparasitic- and Ectoparasitic nematodes), especially plant parasitic nematodes such as root knot nematodes, Meloidogyne hapla, Meloidogyne incognita, Meloidogyne javanica, Meloidogyne arenaria and other Meloidogyne species; cyst-forming nematodes, Globodera rostochiensis and other Globodera species; Heterodera avenae, Heterodera glycines, Heterodera schachtii, Heterodera trifolii, and other Heterodera species; Seed gall nematodes, Anguina species; Stem and foliar nematodes, Aphelenchoides species; Sting nematodes, Belonolaimus longicaudatus and other Belonolaimus species; Pine nematodes, Bursaphelenchus xylophilus and other Bursaphelenchus species; Ring nematodes, Criconema species, Criconemella species, Criconemoides species, Mesocriconema species; Stem and bulb nematodes, Ditylenchus destructor, Ditylenchus dipsaci and other Ditylenchus species; Awl nematodes, Dolichodorus species; Spiral nematodes, Heliocotylenchus multicinctus and other Helicotylenchus species; Sheath and sheathoid nematodes, Hemicycliophora species and Hemicriconemoides species; Hirshmanniella species; Lance nematodes, Hoploaimus species; false rootknot nematodes, Nacobbus species; Needle nematodes, Longidorus elongatus and other Longidorus species; Pin nematodes, Pratylenchus species; Lesion nematodes, Pratylenchus neglectus, Pratylenchus penetrans, Pratylenchus curvitatus, Pratylenchus goodeyi and other Pratylenchus species; Burrowing nematodes, Radopholus similis and other Radopholus species; Reniform nematodes, Rotylenchus robustus, Rotylenchus reniformis and other Rotylenchus species; Scutellonema species; Stubby root nematodes, Trichodorus primitivus and other Trichodorus species, Paratrichodorus species; Stunt nematodes, Tylenchorhynchus claytoni, Tylenchorhynchus dubius and other Tylenchorhynchus species; Citrus nematodes, Tylenchulus species; Dagger nematodes, Xiphinema species; and other plant parasitic nematode species, such as Subanguina spp., Hypsoperine spp., Macroposthonia spp., Melinius spp., Punctodera spp., and Quinisulcius spp..

The compounds of the invention may also have activity against the molluscs. Examples of which include, for example, Ampullariidae; Arion (A. ater, A. circumscriptus, A. hortensis, A. rufus); Bradybaenidae (Bradybaena fruticum); Cepaea (C. hortensis, C. Nemoralis); ochlodina; Deroceras (D. agrestis, D. empiricorum, D. laeve, D. reticulatum); Discus (D. rotundatus); Euomphalia; Galba (G. trunculata); Helicelia (H. itala, H. obvia); Helicidae Helicigona arbustorum); Helicodiscus; Helix (H. aperta); Limax (L. cinereoniger, L. flavus, L. marginatus, L. maximus, L. tenellus); Lymnaea; Milax (M. gagates, M. marginatus, M. sowerbyi); Opeas; Pomacea (P. canaticulata); Vallonia and Zanitoides.

The term "crops" is to be understood as including also crop plants which have been so transformed by the use of recombinant DNA techniques that they are capable of synthesising one or more selectively acting toxins, such as are known, for example, from toxin-producing bacteria, especially those of the genus Bacillus.

Toxins that can be expressed by such transgenic plants include, for example, insecticidal proteins, for example insecticidal proteins from Bacillus cereus or Bacillus popilliae; or insecticidal proteins from Bacillus thuringiensis, such as δ-endotoxins, e.g. Cry1Ab, Cry1Ac, Cry1F, Cry1Fa2, Cry2Ab, Cry3A, Cry3Bb1 or Cry9C, or vegetative insecticidal proteins (Vip), e.g. Vip1, Vip2, Vip3 or Vip3A; or insecticidal proteins of bacteria colonising nematodes, for example Photorhabdus spp. or Xenorhabdus spp., such as Photorhabdus luminescens, Xenorhabdus nematophilus; toxins produced by animals, such as scorpion toxins, arachnid toxins, wasp toxins and other insect-specific neurotoxins; toxins produced by fungi, such as Streptomycetes toxins, plant lectins, such as pea lectins, barley lectins or snowdrop lectins; agglutinins; proteinase inhibitors, such as trypsin inhibitors, serine protease inhibitors, patatin, cystatin, papain inhibitors; ribosome-inactivating proteins (RIP), such as ricin, maize-RIP, abrin, luffin, saporin or bryodin; steroid metabolism enzymes, such as 3-hydroxysteroidoxidase, ecdysteroid-UDP-glycosyl-transferase, cholesterol oxidases, ecdysone inhibitors, HMG-COA-reductase, ion channel blockers, such as blockers of sodium or calcium channels, juvenile hormone esterase, diuretic hormone receptors, stilbene synthase, bibenzyl synthase, chitinases and glucanases.

In the context of the present invention there are to be understood by δ-endotoxins, for example Cry1Ab, Cry1Ac, Cry1F, Cry1Fa2, Cry2Ab, Cry3A, Cry3Bb1 or Cry9C, or vegetative insecticidal proteins (Vip), for example Vip1, Vip2, Vip3 or Vip3A, expressly also hybrid toxins, truncated toxins and modified toxins. Hybrid toxins are produced recombinantly by a new combination of different domains of those proteins (see, for example, WO 02/15701). Truncated toxins, for example a truncated Cry1Ab, are known. In the case of modified toxins, one or more amino acids of the naturally occurring toxin are replaced. In such amino acid replacements, preferably non-naturally present protease recognition sequences are inserted into the toxin, such as, for example, in the case of Cry3A055, a cathepsin-G-recognition sequence is inserted into a Cry3A toxin (see WO 03/018810). Examples of such toxins or transgenic plants capable of synthesising such toxins are disclosed, for example, in EP-A-0 374 753, WO 93/07278, WO 95/34656, EP-A-0 427 529, EP-A-451 878 and WO 03/052073.

The processes for the preparation of such transgenic plants are generally known to the person skilled in the art and are described, for example, in the publications mentioned above. Cryl-type deoxyribonucleic acids and their preparation are known, for example, from WO 95/34656, EP-A-0 367 474, EP-A-0 401 979 and WO 90/13651.

The toxin contained in the transgenic plants imparts to the plants tolerance to harmful insects. Such insects can occur in any taxonomic group of insects, but are especially commonly found in the beetles (Coleoptera), two-winged insects (Diptera) and moths (Lepidoptera).

Transgenic plants containing one or more genes that code for an insecticidal resistance and express one or more toxins are known and some of them are commercially available. Examples of such plants are: YieldGard^{®} (maize variety that expresses a Cry1Ab toxin); YieldGard Rootworm^{®} (maize variety that expresses a Cry3Bb1 toxin); YieldGard Plus^{®} (maize variety that expresses a Cry1Ab and a Cry3Bb1 toxin); Starlink^{®} (maize variety that expresses a Cry9C toxin); Herculex I^{®} (maize variety that expresses a Cry1Fa2 toxin and the enzyme phosphinothricine N-acetyltransferase (PAT) to achieve tolerance to the herbicide glufosinate ammonium); NuCOTN 33B^{®} (cotton variety that expresses a Cry1Ac toxin); Bollgard I^{®} (cotton variety that expresses a Cry1Ac toxin); Bollgard II^{®} (cotton variety that expresses a Cry1Ac and a Cry2Ab toxin); VipCot^{®} (cotton variety that expresses a Vip3A and a Cry1Ab toxin); NewLeaf^{®} (potato variety that expresses a Cry3A toxin); NatureGard^{®}, Agrisure^{®} GT Advantage (GA21 glyphosate-tolerant trait), Agrisure^{®} CB Advantage (Bt11 corn borer (CB) trait) and Protecta^{®}.

Further examples of such transgenic crops are:
1. **Bt11 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer *(Ostrinia nubilalis* and *Sesamia nonagrioides*) by transgenic expression of a truncated Cry1Ab toxin. Bt11 maize also transgenically expresses the enzyme PAT to achieve tolerance to the herbicide glufosinate ammonium.
2. **Bt176 Maize** from Syngenta Seeds SAS, Chemin de l'Hobit 27, F-31 790 St. Sauveur, France, registration number C/FR/96/05/10. Genetically modified *Zea mays* which has been rendered resistant to attack by the European corn borer *(Ostrinia nubilalis* and *Sesamia nonagrioides*) by transgenic expression of a Cry1Ab toxin. Bt176 maize also transgenically expresses the enzyme PAT to achieve tolerance to the herbicide glufosinate ammonium.
3. **MIR604 Maize** from Syngenta Seeds SAS, Chemin de l'Hobit 27, F-31 790 St. Sauveur, France, registration number C/FR/96/05/10. Maize which has been rendered insect-resistant by transgenic expression of a modified Cry3A toxin. This toxin is Cry3A055 modified by insertion of a cathepsin-G-protease recognition sequence. The preparation of such transgenic maize plants is described in WO 03/018810.
4. **MON 863 Maize** from Monsanto Europe S.A. 270-272 Avenue de Tervuren, B-1150 Brussels, Belgium, registration number C/DE/02/9. MON 863 expresses a Cry3Bb1 toxin and has resistance to certain Coleoptera insects.
5. **IPC 531 Cotton** from Monsanto Europe S.A. 270-272 Avenue de Tervuren, B-1150 Brussels, Belgium, registration number C/ES/96/02.
**6. 1507 Maize** from Pioneer Overseas Corporation, Avenue Tedesco, 7 B-1160 Brussels, Belgium, registration number C/NL/00/10. Genetically modified maize for the expression of the protein Cry1F for achieving resistance to certain Lepidoptera insects and of the PAT protein for achieving tolerance to the herbicide glufosinate ammonium.
7. **NK603 × MON 810 Maize** from Monsanto Europe S.A. 270-272 Avenue de Tervuren, B-1150 Brussels, Belgium, registration number C/GB/02/M3/03. Consists of conventionally bred hybrid maize varieties by crossing the genetically modified varieties NK603 and MON 810. NK603 × MON 810 Maize transgenically expresses the protein CP4 EPSPS, obtained from *Agrobacterium sp.* strain CP4, which imparts tolerance to the herbicide Roundup^{®} (contains glyphosate), and also a Cry1Ab toxin obtained from *Bacillus thuringiensis subsp. kurstaki* which brings about tolerance to certain Lepidoptera, include the European corn borer.

Transgenic crops of insect-resistant plants are also described in BATS (Zentrum für Biosicherheit und Nachhaltigkeit, Zentrum BATS, Clarastrasse 13, 4058 Basel, Switzerland) Report 2003, (http://bats.ch).

The term "crops" is to be understood as including also crop plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818 and EP-A-0 353 191. The methods of producing such transgenic plants are generally known to the person skilled in the art and are described, for example, in the publications mentioned above.

Crops may also be modified for enhanced resistance to fungal (for example Fusarium, Anthracnose, or Phytophthora), bacterial (for example Pseudomonas) or viral (for example potato leafroll virus, tomato spotted wilt virus, cucumber mosaic virus) pathogens.

Crops also include those that have enhanced resistance to nematodes, such as the soybean cyst nematode.

Crops that are tolerance to abiotic stress include those that have enhanced tolerance to drought, high salt, high temperature, chill, frost, or light radiation, for example through expression of NF-YB or other proteins known in the art.

Antipathogenic substances which can be expressed by such transgenic plants include, for example, ion channel blockers, such as blockers for sodium and calcium channels, for example the viral KP1, KP4 or KP6 toxins; stilbene synthases; bibenzyl synthases; chitinases; glucanases; the so-called "pathogenesis-related proteins" (PRPs; see e.g. EP-A-0 392 225); antipathogenic substances produced by microorganisms, for example peptide antibiotics or heterocyclic antibiotics (see e.g. WO 95/33818) or protein or polypeptide factors involved in plant pathogen defence (so-called "plant disease resistance genes", as described in WO 03/000906).

Further areas of use of the compositions according to the invention are the protection of stored goods and store rooms and the protection of raw materials, such as wood, textiles, floor coverings or buildings, and also in the hygiene sector, especially the protection of humans, domestic animals and productive livestock against pests of the mentioned type.

The present invention also provides a method for controlling pests (such as mosquitoes and other disease vectors; see also http://www.who.int/malaria/vector_control/irs/en/). In one embodiment, the method for controlling pests comprises applying the compositions of the invention to the target pests, to their locus or to a surface or substrate by brushing, rolling, spraying, spreading or dipping. By way of example, an IRS (indoor residual spraying) application of a surface such as a wall, ceiling or floor surface is contemplated by the method of the invention. In another embodiment, it is contemplated to apply such compositions to a substrate such as non-woven or a fabric material in the form of (or which can be used in the manufacture of) netting, clothing, bedding, curtains and tents.

In one embodiment, the method for controlling such pests comprises applying a pesticidally effective amount of the compositions of the invention to the target pests, to their locus, or to a surface or substrate so as to provide effective residual pesticidal activity on the surface or substrate. Such application may be made by brushing, rolling, spraying, spreading or dipping the pesticidal composition of the invention. By way of example, an IRS application of a surface such as a wall, ceiling or floor surface is contemplated by the method of the invention so as to provide effective residual pesticidal activity on the surface. In another embodiment, it is contemplated to apply such compositions for residual control of pests on a substrate such as a fabric material in the form of (or which can be used in the manufacture of) netting, clothing, bedding, curtains and tents.

Substrates including non-woven, fabrics or netting to be treated may be made of natural fibres such as cotton, raffia, jute, flax, sisal, hessian, or wool, or synthetic fibres such as polyamide, polyester, polypropylene, polyacrylonitrile or the like. The polyesters are particularly suitable. The methods of textile treatment are known, e.g. WO 2008/151984, WO 2003/034823, US 5631072, WO 2005/64072, WO2006/128870, EP 1724392, WO 2005113886 or WO 2007/090739.

Further areas of use of the compositions according to the invention are the field of tree injection/trunk treatment for all ornamental trees as well all sort of fruit and nut trees.

In the field of tree injection/trunk treatment, the compounds according to the present invention are especially suitable against wood-boring insects from the order *Lepidoptera* as mentioned above and from the order *Coleoptera,* especially against woodborers listed in the following tables A and B:

**Table A. Examples of exotic woodborers of economic importance.**

| Family | Species | Host or Crop Infested |
|---|---|---|
| Buprestidae | *Agrilus planipennis* | Ash |
| Cerambycidae | *Anoplura glabripennis* | Hardwoods |
| Scolytidae | *Xylosandrus crassiusculus* | Hardwoods |
| | *X. mutilatus* | Hardwoods |
| | *Tomicus piniperda* | Conifers |

**Table B. Examples of native woodborers of economic importance.**

| Family | Species | Host or Crop Infested |
|---|---|---|
| Buprestidae | *Agrilus anxius* | Birch |
| | *Agrilus politus* | Willow, Maple |
| | *Agrilus sayi* | Bayberry, Sweetfern |
| | *Agrilus vittaticolllis* | Apple, Pear, Cranberry, Serviceberry, Hawthorn |
| | *Chrysobothris femorata* | Apple, Apricot, Beech, Boxelder, Cherry, Chestnut, Currant, Elm, Hawthorn, Hackberry, Hickory, Horsechestnut, Linden, Maple, Mountain-ash, Oak, Pecan, Pear, Peach, Persimmon, Plum, Poplar, Quince, Redbud, Serviceberry, Sycamore, Walnut, Willow |
| | *Texania campestris* | Basswood, Beech, Maple, Oak, Sycamore, Willow, Yellow-poplar |
| Cerambycidae | *Goes pulverulentus* | Beech, Elm, Nuttall, Willow, Black oak, Cherrybark oak, Water oak, Sycamore |
| | *Goes tigrinus* | Oak |
| | *Neoclytus acuminatus* | Ash, Hickory, Oak, Walnut, Birch, Beech, Maple, Eastern hophornbeam, Dogwood, Persimmon, Redbud, Holly, Hackberry, Black locust, Honeylocust, Yellow-poplar, Chestnut, Osage-orange, Sassafras, Lilac, Mountain-mahogany, Pear, Cherry, Plum, Peach, Apple, Elm, Basswood, Sweetgum |
| | *Neoptychodes trilineatus* | Fig, Alder, Mulberry, Willow, Netleaf hackberry |
| | *Oberea ocellata* | Sumac, Apple, Peach, Plum, Pear, Currant, Blackberry |
| | *Oberea tripunctata* | Dogwood, Viburnum, Elm, Sourwood, Blueberry, Rhododendron, Azalea, Laurel, Poplar, Willow, Mulberry |
| | *Oncideres cingulata* | Hickory, Pecan, Persimmon, Elm, Sourwood, Basswood, Honeylocust, Dogwood, Eucalyptus, Oak, Hackberry, Maple, Fruit trees |
| | *Saperda calcarata* | Poplar |
| | *Strophiona nitens* | Chestnut, Oak, Hickory, Walnut, Beech, Maple |
| Scolytidae | *Corthylus columbianus* | Maple, Oak, Yellow-poplar, Beech, Boxelder, Sycamore, Birch, Basswood, Chestnut, Elm |
| | *Dendroctonus frontalis* | Pine |
| | *Dryocoetes betulae* | Birch, Sweetgum, Wild cherry, Beech, Pear |
| | *Monarthrum fasciatum* | Oak, Maple, Birch, Chestnut, Sweetgum, Blackgum, Poplar, Hickory, Mimosa, Apple, Peach, Pine |
| | *Phloeotribus liminaris* | Peach, Cherry, Plum, Black cherry, Elm, Mulberry, Mountain-ash |
| | *Pseudopityophthorus pruinosus* | Oak, American beech, Black cherry, Chickasaw plum, Chestnut, Maple, Hickory, Hornbeam, Hophornbeam |
| Sesiidae | *Paranthrene simulans* | Oak, American chestnut |
| | *Sannina uroceriformis* | Persimmon |
| | *Synanthedon exitiosa* | Peach, Plum, Nectarine, Cherry, Apricot, Almond, Black cherry |
| | *Synanthedon pictipes* | Peach, Plum, Cherry, Beach, Black Cherry |
| | *Synanthedon rubrofascia* | Tupelo |
| | *Synanthedon scitula* | Dogwood, Pecan, Hickory, Oak, Chestnut, Beech, Birch, Black cherry, Elm, Mountain-ash, Viburnum, Willow, Apple, Loquat, Ninebark, Bayberry |
| | *Vitacea polistiformis* | Grape |

The present invention may be also used to control any insect pests that may be present in turfgrass, including for example beetles, caterpillars, fire ants, ground pearls, millipedes, sow bugs, mites, mole crickets, scales, mealybugs ticks, spittlebugs, southern chinch bugs and white grubs. The present invention may be used to control insect pests at various stages of their life cycle, including eggs, larvae, nymphs and adults.

In particular, the present invention may be used to control insect pests that feed on the roots of turfgrass including white grubs (such as *Cyclocephala spp.* (e.g. masked chafer, *C. lurida), Rhizotrogus spp.* (e.g. European chafer, *R. majalis), Cotinus spp.* (e.g. Green June beetle, *C. nitida), Popillia spp.* (e.g. Japanese beetle, *P. japonica), Phyllophaga spp.* (e.g. May/June beetle), *Ataenius spp.* (e.g. Black turfgrass ataenius, *A. spretulus), Maladera spp.* (e.g. Asiatic garden beetle, *M*. *castanea)* and *Tomarus spp.),* ground pearls *(Margarodes* spp.), mole crickets (tawny, southern, and short-winged; *Scapteriscus* spp., *Gryllotalpa africana*) and leatherjackets (European crane fly, *Tipula spp.).*

The present invention may also be used to control insect pests of turfgrass that are thatch dwelling, including armyworms (such as fall armyworm *Spodoptera frugiperda,* and common armyworm *Pseudaletia unipuncta),* cutworms, billbugs *(Sphenophorus spp.,* such as *S. venatus verstitus* and *S*. *parvulus),* and sod webworms (such as *Crambus spp.* and the tropical sod webworm, *Herpetogramma phaeopteralis).*

The present invention may also be used to control insect pests of turfgrass that live above the ground and feed on the turfgrass leaves, including chinch bugs (such as southern chinch bugs, *Blissus insularis),* Bermudagrass mite *(Eriophyes cynodoniensis),* rhodesgrass mealybug *(Antonina graminis),* two-lined spittlebug *(Propsapia bicincta),* leafhoppers, cutworms *(Noctuidae* family), and greenbugs. The present invention may also be used to control other pests of turfgrass such as red imported fire ants *(Solenopsis invicta)* that create ant mounds in turf.

In the hygiene sector, the compositions according to the invention are active against ectoparasites such as hard ticks, soft ticks, mange mites, harvest mites, flies (biting and licking), parasitic fly larvae, lice, hair lice, bird lice and fleas.

Examples of such parasites are:
Of the order Anoplurida: Haematopinus spp., Linognathus spp., Pediculus spp. and Phtirus spp., Solenopotes spp..

Of the order Mallophagida: Trimenopon spp., Menopon spp., Trinoton spp., Bovicola spp., Werneckiella spp., Lepikentron spp., Damalina spp., Trichodectes spp. and Felicola spp..

Of the order Diptera and the suborders Nematocerina and Brachycerina, for example Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp. and Melophagus spp..

Of the order Siphonapterida, for example Pulex spp., Ctenocephalides spp., Xenopsylla spp., Ceratophyllus spp..

Of the order Heteropterida, for example Cimex spp., Triatoma spp., Rhodnius spp., Panstrongylus spp..

Of the order Blattarida, for example Blatta orientalis, Periplaneta americana, Blattelagermanica and Supella spp..

Of the subclass Acaria (Acarida) and the orders Meta- and Meso-stigmata, for example Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp. and Varroa spp..

Of the orders Actinedida (Prostigmata) and Acaridida (Astigmata), for example Acarapis spp., Cheyletiella spp., Ornithocheyletia spp., Myobia spp., Psorergatesspp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp. and Laminosioptes spp..

The compositions according to the invention are also suitable for protecting against insect infestation in the case of materials such as wood, textiles, plastics, adhesives, glues, paints, paper and card, leather, floor coverings and buildings.

The compositions according to the invention can be used, for example, against the following pests: beetles such as Hylotrupes bajulus, Chlorophorus pilosis, Anobium punctatum, Xestobium rufovillosum, Ptilinuspecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthesrugicollis, Xyleborus spec.,Tryptodendron spec., Apate monachus, Bostrychus capucins, Heterobostrychus brunneus, Sinoxylon spec. and Dinoderus minutus, and also hymenopterans such as Sirex juvencus, Urocerus gigas, Urocerus gigas taignus and Urocerus augur, and termites such as Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis and Coptotermes formosanus, and bristletails such as Lepisma saccharina.

The compounds according to the invention can be used as pesticidal agents in unmodified form, but they are generally formulated into compositions in various ways using formulation adjuvants, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, microemulsifiable concentrates, oil-in-water emulsions, oil-flowables, aqueous dispersions, oily dispersions, suspo-emulsions, capsule suspensions, emulsifiable granules, soluble liquids, water-soluble concentrates (with water or a water-miscible organic solvent as carrier), impregnated polymer films or in other forms known e.g. from the Manual on Development and Use of FAO and WHO Specifications for Pesticides, United Nations, First Edition, Second Revision (2010). Such formulations can either be used directly or diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof.

The active ingredients can also be contained in very fine microcapsules. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes can comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, *N,N*-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, *n*-hexane, *n*-octylamine, octadecanoic acid, octylamine acetate, oleic acid, oleylamine, o-xylene, phenol, polyethylene glycol, propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, *N*-methyl-2-pyrrolidone and the like.

Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances. A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; and also further substances described e.g. in McCutcheon's Detergents and Emulsifiers Annual, MC Publishing Corp., Ridgewood New Jersey (1981).

Further adjuvants that can be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and liquid and solid fertilisers.

The compositions according to the invention can include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the mixture to be applied. For example, the oil additive can be added to a spray tank in the desired concentration after a spray mixture has been prepared. Preferred oil additives comprise mineral oils or an oil of vegetable origin, for example rapeseed oil, olive oil or sunflower oil, emulsified vegetable oil, alkyl esters of oils of vegetable origin, for example the methyl derivatives, or an oil of animal origin, such as fish oil or beef tallow. Preferred oil additives comprise alkyl esters of C₈-C₂₂ fatty acids, especially the methyl derivatives of C₁₂-C₁₈ fatty acids, for example the methyl esters of lauric acid, palmitic acid and oleic acid (methyl laurate, methyl palmitate and methyl oleate, respectively). Many oil derivatives are known from the Compendium of Herbicide Adjuvants, 10th Edition, Southern Illinois University, 2010.

The inventive compositions generally comprise from 0.1 to 99 % by weight, especially from 0.1 to 95 % by weight, of compounds of the present invention and from 1 to 99.9 % by weight of a formulation adjuvant which preferably includes from 0 to 25 % by weight of a surface-active substance. Whereas commercial products may preferably be formulated as concentrates, the end user will normally employ dilute formulations.

The rates of application vary within wide limits and depend on the nature of the soil, the method of application, the crop plant, the pest to be controlled, the prevailing climatic conditions, and other factors governed by the method of application, the time of application and the target crop. As a general guideline compounds may be applied at a rate of from 1 to 2000 I/ha, especially from 10 to 1000 I/ha.

Preferred formulations can have the following compositions (weight %):

### Emulsifiable concentrates:

| | |
|---|---|
| active ingredient: | 1 to 95 %, preferably 60 to 90 % |
| surface-active agent: | 1 to 30 %, preferably 5 to 20 % |
| liquid carrier: | 1 to 80 %, preferably 1 to 35 % |

### Dusts:

| | |
|---|---|
| active ingredient: | 0.1 to 10 %, preferably 0.1 to 5 % |
| solid carrier: | 99.9 to 90 %, preferably 99.9 to 99 % |

### Suspension concentrates:

| | |
|---|---|
| active ingredient: | 5 to 75 %, preferably 10 to 50 % |
| water: | 94 to 24 %, preferably 88 to 30 % |
| surface-active agent: | 1 to 40 %, preferably 2 to 30 % |

### Wettable powders:

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

### Granules:

| | |
|---|---|
| active ingredient: | 0.1 to 30 %, preferably 0.1 to 15 % |
| solid carrier: | 99.5 to 70 %, preferably 97 to 85 % |

The following Examples further illustrate, but do not limit, the invention.

| Wettable powders | a) | b) | c) |
|---|---|---|---|
| active ingredients | 25% | 50 % | 75% |
| sodium lignosulfonate | 5% | 5% | - |
| sodium lauryl sulfate | 3% | - | 5% |
| sodium diisobutylnaphthalenesulfonate | - | 6 % | 10 % |
| phenol polyethylene glycol ether (7-8 mol of ethylene oxide) | - | 2% | - |
| highly dispersed silicic acid | 5% | 10 % | 10 % |
| Kaolin | 62 % | 27% | - |

The combination is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredients | 25% | 50 % | 75% |
| light mineral oil | 5% | 5% | 5% |
| highly dispersed silicic acid | 5% | 5% | - |
| Kaolin | 65 % | 40 % | - |
| Talcum | - | | 20 % |

The combination is thoroughly mixed with the adjuvants and the mixture is thoroughly ground in a suitable mill, affording powders that can be used directly for seed treatment.

| Emulsifiable concentrate | |
|---|---|
| active ingredients | 10 % |
| octylphenol polyethylene glycol ether (4-5 mol of ethylene oxide) | 3 % |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredients | 5 % | 6 % | 4 % |
| Talcum | 95 % | - | - |
| Kaolin | - | 94 % | - |
| mineral filler | - | - | 96 % |

Ready-for-use dusts are obtained by mixing the combination with the carrier and grinding the mixture in a suitable mill. Such powders can also be used for dry dressings for seed.

| Extruder granules | |
|---|---|
| Active ingredients | 15 % |
| sodium lignosulfonate | 2 % |
| carboxymethylcellulose | 1 % |
| Kaolin | 82 % |

The combination is mixed and ground with the adjuvants, and the mixture is moistened with water. The mixture is extruded and then dried in a stream of air.

| Coated granules | |
|---|---|
| Active ingredients | 8 % |
| polyethylene glycol (mol. wt. 200) | 3 % |
| Kaolin | 89 % |

The finely ground combination is uniformly applied, in a mixer, to the kaolin moistened with polyethylene glycol. Non-dusty coated granules are obtained in this manner.

### Suspension concentrate

| | |
|---|---|
| active ingredients | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredients | 40 % |
| propylene glycol | 5 % |
| copolymer butanol PO/EO | 2 % |
| Tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground combination is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of the combination are mixed with 2 parts of an aromatic solvent and 7 parts of toluene diisocyanate/polymethylene-polyphenylisocyanate-mixture (8:1). This mixture is emulsified in a mixture of 1.2 parts of polyvinylalcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

Formulation types include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a wettable powder (WP), a soluble granule (SG) or any technically feasible formulation in combination with agriculturally acceptable adjuvants.

### Preparatory Examples:

"Mp" means melting point in °C. Free radicals represent methyl groups. ¹H NMR measurements were recorded on a Brucker 400 MHz spectrometer, chemical shifts are given in ppm relevant to a TMS standard. Spectra measured in deuterated solvents as indicated. Either one of the LCMS methods below was used to characterize the compounds. The characteristic LCMS values obtained for each compound were the retention time ("Rt", recorded in minutes) and the measured molecular ion (M+H)⁺ or (M-H)⁻.

### LCMS Methods:

### Method 1:

Spectra were recorded on a Mass Spectrometer from Waters (SQD Single quadrupole mass spectrometer) equipped with an electrospray source (Polarity: positive or negative ions, Full Scan, Capillary: 3.00 kV, Cone range: 41 V, Source Temperature: 150°C, Desolvation Temperature: 500°C, Cone Gas Flow: 50 L/Hr, Desolvation Gas Flow: 1000 L/Hr, Mass range: 110 to 800 Da) and a H-Class UPLC from Waters: quaternary pump, heated column compartment and diode-array detector. Column: Acquity UPLC HSS T3 C18, 1.8 µm, 30 x 2.1 mm, Temp: 40 °C, DAD Wavelength range (nm): 200 to 400, Solvent Gradient: A = water + 5% Acetonitrile + 0.1 % HCOOH, B= Acetonitrile + 0.05 % HCOOH: gradient: 0 min 10% B; 0.-0.2 min 10-50% B; 0.2-0.7 min 50-100% B; 0.7-1.3 min 100% B; 1.3-1.4 min 100-10% B; 1.4-1.6 min 10% B; Flow (mL/min) 0.6.

### Method 2:

Spectra were recorded on a Mass Spectrometer from Agilent Technologies (6410 Triple Quadrupole mass spectrometer) equipped with an equipped with an electrospray source (Polarity: positive or negative ions, MS2 Scan, Capillary: 4.00 kV, Fragmentor: 100 V, Desolvatation Temperature: 350°C, Gas Flow: 11 L/min, Nebulizer Gas: 45 psi, Mass range: 110 to 1000 Da) and a 1200 Series HPLC from Agilent: quaternary pump, heated column compartment and diode-array detector. Column: KINETEX EVO C18, 2.6 µm, 50 x 4.6 mm, Temp: 40 °C, DAD Wavelength range (nm): 210 to 400, Solvent Gradient: A = water + 5% Acetonitrile + 0.1 % HCOOH, B= Acetonitrile + 0.1 % HCOOH: gradient: 0 min 10% B, 90%A; 0.9-1.8 min 100% B; 1.8-2.2 min 100-10% B; 2.2-2.5 min 10%B; Flow (mL/min) 1.8.

### Method 3:

Spectra were recorded on a Mass Spectrometer from Waters (Acquity QDa Mass Spectrometer) equipped with an electrospray source (Polarity: Positive and Negative Polarity Switch), Capillary: 0.8 kV, Cone range: 25 V, Extractor: V (No extractor voltage for QDa detector) Source Temperature: 120°C, Desolvation Temperature: 600°C, Cone Gas Flow: 50 L/h, Desolvation Gas Flow: 1000 L/h, Mass range: 110 to 850 Da) and an Acquity UPLC from Waters: Quaternary solvent manager, heated column compartment , diode-array detector. Column: Waters UPLC HSS T3, 1.8 µm, 30 x 2.1 mm, Temp: 40 °C, PDA Wavelength range (nm): 230 to 400, Solvent Gradient: A = Water with 0.1% formic acid: Acetonitrile: 95: 5 v/v, B= Acetonitrile with 0.05% formic acid, : Gradient: 0 min-1.0min ,10% B-90%A; 1.0min-4.50min 10% -100% B; 4.51 min-5.30min ,100% B, 0 %A; 5.31 min-5.50min 100% -10% B; 5.51 min-6.00 min ,10% B, 90%A; Flow (ml/min) 0.6.

### Method 4:

Spectra were recorded on a Mass Spectrometer from Agilent Technologies (6410 Triple Quadrupole mass spectrometer) equipped with an electrospray source (Polarity: positive or negative ions, MS2 Scan, Capillary: 7.00 kV, Fragmentor: 120 V, Desolvatation Temperature: 350°C, Gas Flow: 11 L/min, Nebulizer Gas: 40 psi, Mass range: 110 to 1000 Da) and a 1200 Series HPLC from Agilent: quaternary pump, heated column compartment and diode-array detector. Column: KINETEX EVO C18, 2.6 µm, 50 x 4.6 mm, Temp: 40 °C, Detector VWD Wavelength: 254 nm, Solvent Gradient: A = water + 5% Acetonitrile + 0.1 % HCOOH, B= Acetonitrile + 0.1 % HCOOH: gradient: 0 min 10% B, 90%A; 0.9-1.8 min 100% B; 1.8-2.2 min 100-10% B; 2.2-2.5 min 10%B; Flow (mL/min) 1.8.

### Example P1: Preparation of 6-[3-ethylsulfonyl-6-(trifluoromethyl)imidazo[1,2-a]pyridin-2-yl]-2,2-difluoro-5H-[1,3]dioxolo[4,5-f]isoindol-7-one (compound P1)

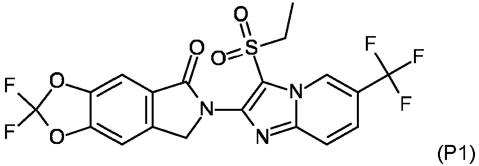

### Step A1: Preparation of (2,2-difluoro-1,3-benzodioxol-5-yl)methanol (Intermediate I-1)

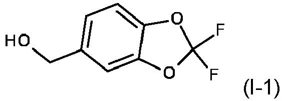

To 0 °C cooled solution of 2,2-difluoro-1,3-benzodioxole-5-carbaldehyde (CAS 656-42-8) (15 g, 76.56 mmol) in methanol (75 mL) was added sodium borohydride (4.57 g, 114.84 mmol) slowly. The reaction mixture was stirred at room temperature for overnight. After completion, the reaction mass was concentrated *in vacuo,* quenched with an aqueous ammonium chloride solution and extracted with ethyl acetate. The organic layer was dried over magnesium sulfate and concentrated *in vacuo* to afford (2,2-difluoro-1,3-benzodioxol-5-yl)methanol as a colourless liquid. ¹H NMR (400 MHz, CDCl₃) δ ppm: 1.91 (br s, 1 H), 4.69 (br s, 2 H), 7.03-7.09 (m, 2 H), 7.14 (s, 1 H).

### Step A2: Preparation of 6-chloro-2,2-difluoro-1,3-benzodioxole-5-carbaldehyde (Intermediate I-2)

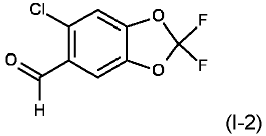

To a solution of (2,2-difluoro-1,3-benzodioxol-5-yl)methanol (Intermediate I-1 prepared as described above) (10 g, 50.49 mmol) in acetonitrile (60 mL) was added N-chlorosuccinimide (17.20 g, 126.24 mmol). The reaction mixture was stirred at room temperature for overnight. After completion, the reaction mass was concentrated *in vacuo,* triturated with cyclohexane, filtered through a Buchner funnel and filtrate was concentrated *in vacuo.* The crude compound was purified by combiflash (silica gel, 0-30% ethyl acetate in cyclohexane) to afford pure 6-chloro-2,2-difluoro-1,3-benzodioxole-5-carbaldehyde as a colourless liquid. ¹H NMR (400 MHz, CDCl₃) δ ppm: 7.22 (s, 1 H), 7.66 (s, 1 H), 10.41 (s, 1 H).

### Step A3: Preparation of 2,2-difluoro-6-methyl-1 ,3-benzodioxole-5-carbaldehyde (Intermediate I-3)

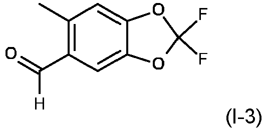

To a solution of 6-chloro-2,2-difluoro-1,3-benzodioxole-5-carbaldehyde (Intermediate I-2 prepared as described above) (1 g, 4.30 mmol) in toluene (10 mL) were added methylboronic acid (1.14 g, 17.22 mmol) followed by a solution of potassium carbonate (1.78 g, 12.92 mmol) in water (3 mL) while purging with nitrogen for 10 minutes. 1,1'-bis(diphenylphosphino)ferrocene-palladium(II)dichloride dichloromethane complex (0.18 g, 0.21 mmol) was added and the reaction mixture heated at 90 °C for 15 hours. The reaction mixture was allowed to cool to room temperature, concentrated *in vacuo.* The reaction mass was diluted with water and extracted with ethyl acetate. The organic layer was washed with water followed by brine, dried over sodium sulfate, filtered and concentrated *in vacuo.* The crude was purified by combiflash (silica gel, 0 to 30% ethyl acetate in cyclohexane) to afford 2,2-difluoro-6-methyl-1,3-benzodioxole-5-carbaldehyde as a brown oil. ¹H NMR (400 MHz, CDCl₃) δ ppm: 2.72 (s, 3 H), 6.99 (s, 1 H), 7.56 (s, 1 H), 10.27 (s, 1 H).

### Step A4: Preparation of 2,2-difluoro-6-methyl-1,3-benzodioxole-5-carboxylic acid (Intermediate I-4)

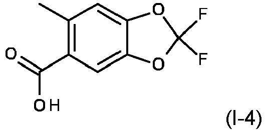

To a solution of silver nitrate (0.57 g, 3.22 mmol) in water (6.8 mL) was added a solution of sodium hydroxide (0.33 g, 8.06 mmol) in water (6.8 mL) dropwise at room temperature. To this reaction mixture, 2,2-difluoro-6-methyl-1,3-benzodioxole-5-carbaldehyde (Intermediate I-3 prepared as described above) (0.34 g, 1.61 mmol) was added portion wise over a period of 20 minutes. The reaction mixture was stirred at room temperature for 2 hours. After completion, the reaction mixture was filtered through celite and the filtrate acidified with an aqueous 2N hydrochloric acid solution. The formed solid was filtered, washed with cold water and dried *in vacuo* to afford 2,2-difluoro-6-methyl-1,3-benzodioxole-5-carboxylic acid as a white solid. LCMS (method 2): Rt= 1.42 min, m/z= 215 (M-H)⁻. ¹H NMR (400 MHz, DMSO-*d*6) δ ppm: 2.54 (s, 3 H), 7.41 (s, 1 H), 7.77 (s, 1 H).

### Step A5: Preparation of ethyl 2,2-difluoro-6-methyl-1,3-benzodioxole-5-carboxylate (Intermediate I-5)

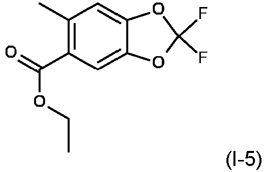

A solution of 2,2-difluoro-6-methyl-1,3-benzodioxole-5-carboxylic acid (Intermediate I-4 prepared as described above) (0.23 g, 1.01 mmol) in ethanol (10 mL) was stirred at room temperature for 15 minutes. To this reaction mass, sulfuric acid (0.02 mL, 0.40 mmol) was added dropwise (exotherm was observed). The reaction mixture was heated at 60 °C for 12 hours. After completion, the reaction mass was concentrated *in vacuo,* neutralized with an aqueous sodium bicarbonate solution, and the product extracted twice with ethyl acetate. The combined organic layers were dried over sodium sulfate, filtered and concentrated *in vacuo* to afford ethyl 2,2-difluoro-6-methyl-1,3-benzodioxole-5-carboxylate. The crude was used as such for next step. ¹H NMR (400 MHz, CDCl₃) δ ppm: 1.41 (t, 3 H), 2.64 (s, 3 H), 4.37 (q, 2 H), 6.97 (s, 1 H), 7.67 (s, 1 H).

### Step A6: Preparation of ethyl 6-(bromomethyl)-2,2-difluoro-1,3-benzodioxole-5-carboxylate (Intermediate I-6)

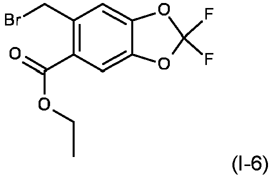

To a solution of ethyl 2,2-difluoro-6-methyl-1,3-benzodioxole-5-carboxylate (Intermediate I-5 prepared as described above) (0.25 g, 0.97 mmol) in benzotrifluoride (3 mL) were added N-bromosuccinimide (0.196 g, 1.06 mmol) and azobisisobutyronitrile (0.016 g, 0.097 mmol) at room temperature. The reaction mixture was heated at 90 °C for 3 hours. The reaction mixture was cooled to room temperature, and concentrated *in vacuo.* The crude was purified by combiflash (silica gel, 0 to 30% ethyl acetate in cyclohexane) to afford ethyl 6-(bromomethyl)-2,2-difluoro-1,3-benzodioxole-5-carboxylate as a gummy mass. ¹H NMR (400 MHz, CDCl₃) δ ppm: 1.39 -1.47 (m, 3 H), 4.38 - 4.45 (m, 2 H), 4.97 (s, 2 H), 7.20 (s, 1 H), 7.71 (s, 1 H).

### Step B1: Preparation of ethyl 6-(trifluoromethyl)imidazo[1,2-a]pyridine-2-carboxylate (Intermediate I-7)

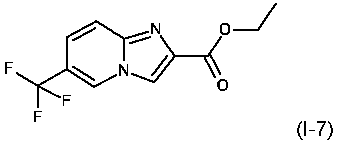

To a solution of 5-(trifluoromethyl)pyridin-2-amine (4.0 g, 25 mmol) in ethanol (40 mL) were added ethyl 3-bromo-2-oxo-propanoate (3.7 mL, 30 mmol) and sodium bicarbonate (4.1 g, 49 mmol) at room temperature. The reaction mass was heated at 85 °C for 4 hours. After completion, the reaction mass was added to ice cold water and the formed solid filtered through a Buchner funnel to afford ethyl 6-(trifluoromethyl)imidazo[1,2-a]pyridine-2-carboxylate. This material was used as such in the next step. LCMS (method 2): Rt = 1.35 min, m/z= 259 (M+H)⁺.

### Step B2: Preparation of ethyl 3-chloro-6-(trifluoromethyl)imidazo[1,2-a]pyridine-2-carboxylate (Intermediate I-8)

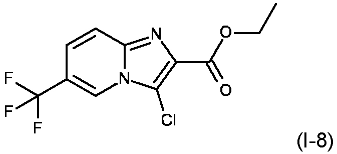

To a solution of ethyl 6-(trifluoromethyl)imidazo[1,2-a]pyridine-2-carboxylate (Intermediate I-7 prepared as described above) (8.5 g, 33 mmol) in N,N-dimethylformamide (85 mL) was added 1-chloropyrrolidine-2,5-dione (5.3 g, 40 mmol) at room temperature. The reaction mass was heated at 40 °C for 4 hours. After completion, the reaction mass was quenched with ice cold water, the formed solid filtered through a Buchner funnel and dried *in vacuo* to afford ethyl 3-chloro-6-(trifluoromethyl)imidazo[1,2-a]pyridine-2-carboxylate as a solid. LCMS (method 2): Rt= 1.45 min, m/z=293 (M+H)⁺.

### Step B3: Preparation of ethyl 3-ethylsulfanyl-6-(trifluoromethyl)imidazo[1,2-a]pyridine-2-carboxylate (Intermediate I-9)

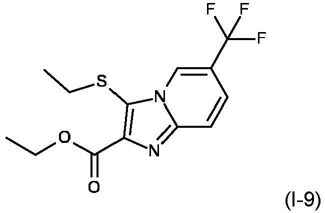

To a 0 °C cooled solution of ethyl 3-chloro-6-(trifluoromethyl)imidazo[1,2-a]pyridine-2-carboxylate (Intermediate I-8 prepared as described above) (6.0 g, 21 mmol) in N,N-dimethylformamide (60 mL) was added sodium ethanethiolate (2.1 g, 25 mmol) at room temperature. The reaction mass was stirred at room temperature for 5 hours. After completion, the reaction mass was added to ice cold water, the formed solid filtered through a Buchner funnel and dried *in vacuo* to afford ethyl 3-ethylsulfanyl-6-(trifluoromethyl)imidazo[1,2-a]pyridine-2-carboxylate. This material was used as such in the next step. LCMS (method 2): Rt= 1.49 min, m/z=319 (M+H)⁺.

### Step B4: Preparation of ethyl 3-ethylsulfonyl-6-(trifluoromethyl)imidazo[1,2-a]pyridine-2-carboxylate (Intermediate I-10)

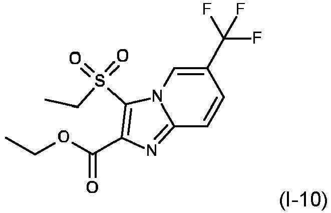

To 0 °C cooled solution of ethyl 3-ethylsulfanyl-6-(trifluoromethyl)imidazo[1,2-a]pyridine-2-carboxylate (Intermediate I-9 prepared as described above) (6.5 g, 20 mmol) in ethanol (65 mL) was added 3-chlorobenzenecarboperoxoic acid (11 g, 45 mmol, 70 mass%). The reaction mixture was stirred at 0 °C for 4 hours. The reaction mass was diluted with water (50 mL) and basified with an aqueous 2N sodium hydroxide solution. The aqueous phase was extracted with ethyl acetate (2x 50 mL). The combined organic layers were washed with brine (20 mL), dried over sodium sulfate and concentrated *in vacuo.* The crude was purified by combiflash (silica gel, 30-40% ethyl acetate in cyclohexane) to afford ethyl 3-ethylsulfonyl-6-(trifluoromethyl)imidazo[1,2-a]pyridine-2-carboxylate. LCMS (method 2): Rt =1.43 min, m/z=351 (M+H)⁺.

### Step B5: Preparation of 3-ethylsulfonyl-6-(trifluoromethyl)imidazo[1,2-a]pyridine-2-carboxylic acid (Intermediate I-11)

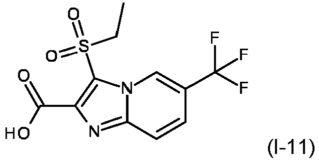

To a solution of ethyl 3-ethylsulfonyl-6-(trifluoromethyl)imidazo[1,2-a]pyridine-2-carboxylate (Intermediate I-10 prepared as described above) (4.2 g, 12 mmol) in tetrahydrofuran (42 mL) was added a solution of lithium hydroxide monohydrate (1.00 g, 24 mmol) in water (17 mL) at 0-5°C. The reaction mixture was stirred at room temperature for 4 hours. The reaction mixture was acidified with an aqueous 2N hydrochloric acid solution, the formed solid filtered through a Buchner funnel and dried *in vacuo* to afford 3-ethylsulfonyl-6-(trifluoromethyl)imidazo[1,2-a]pyridine-2-carboxylic acid. This material was used as such in the next step. LCMS (method 2): Rt= 0.37 min, m/z=323 (M+H)⁺. ¹H NMR (400 MHz, DMSO-*d*6) δ ppm: 1.27 (t, 3 H), 3.77 (q, 2 H), 7.95 (dd, 1 H), 8.10 (d, 1 H), 9.34 (s, 1 H).

### Step B6: Preparation of tert-butyl N-[3-ethylsulfonyl-6-(trifluoromethyl)imidazo[1,2-a]pyridin-2-yllcarbamate (Intermediate I-12) and 3-ethylsulfonyl-6-(trifluoromethyl)imidazo[1,2-a]pyridin-2-amine (Intermediate I-13)

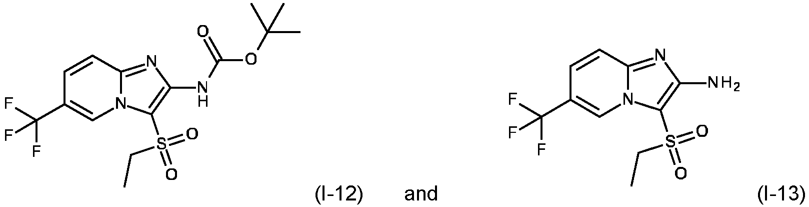

To a solution of 3-ethylsulfonyl-6-(trifluoromethyl)imidazo[1,2-a]pyridine-2-carboxylic acid (Intermediate I-11 prepared as described above) (4.1 g, 13 mmol) in tert-butanol (8.2 mL) was added triethylamine (2.1 g, 120 mmol) at room temperature and the reaction mass was heated at 90 °C for 10 minutes. To this was then added dropwise diphenylphosphoryl azide (5.7 g, 20 mmol) over 15 minutes and the reaction mixture further stirred at 90 °C for 40 minutes. The reaction mass was allowed to cool to room temperature, quenched with ice cold water (100 mL) and brine (50 mL) and the product extracted with ethyl acetate (3x 100 mL). The combined organic layers were dried over sodium sulfate, filtered and concentrated *in vacuo.* The crude was purified by combiflash (silica gel, ethyl acetate in cyclohexane) to afford each pure tert-butyl N-[3-ethylsulfonyl-6-(trifluoromethyl)imidazo[1,2-a]pyridin-2-yl]carbamate and 3-ethylsulfonyl-6-(trifluoromethyl)imidazo[1,2-a]pyridin-2-amine.

For tert-butyl N-[3-ethylsulfonyl-6-(trifluoromethyl)imidazo[1,2-a]pyridin-2-yl]carbamate (Intermediate I-12): LCMS (method 2): Rt=1.49 min, m/z=392 (M-H)⁻. ¹H NMR (400 MHz, DMSO-*d*6) δ ppm: 1.21 - 1.34 (m, 3 H), 1.48 (s, 9 H), 3.74 (d, 2 H), 7.85 - 7.94 (m, 2 H), 9.05 (s, 1 H), 9.60 (s, 1 H).

For 3-ethylsulfonyl-6-(trifluoromethyl)imidazo[1,2-a]pyridin-2-amine (Intermediate I-13): LCMS (method 2): Rt=1.22 min, m/z=294 (M+H)⁺. ¹H NMR (400 MHz, DMSO-*d*6) δ ppm: 1.17 (t, 3 H), 3.42 (q, 2 H), 6.37 (s, 2 H), 7.56 (d, 1 H), 7.71 (dd, 1 H), 8.77 (s, 1 H).

### Step B7: Preparation of tert-butyl N-[3-ethylsulfonyl-6-(trifluoromethyl)imidazo[1,2-a]pyridin-2-yllcarbamate (Intermediate I-12)

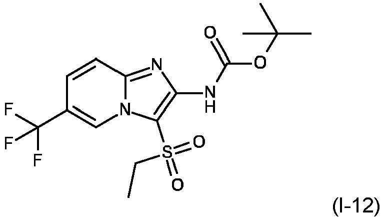

To a 0°C cooled solution of 3-ethylsulfonyl-6-(trifluoromethyl)imidazo[1,2-a]pyridin-2-amine (Intermediate I-13 prepared as described above) (1 g, 3.23 mmol) in N,N-dimethylformamide (10 mL) was added sodium hydride (0.310 g, 7.12 mmol) and the reaction mixture was stirred at 0°C for 30 minutes. A solution of tert-butoxycarbonyl tert-butyl carbonate (0.94 mL, 3.88 mmol) in N,N-dimethylformamide (3 mL) was added at 0°C and the mixture stirred at room temperature overnight. After completion, the reaction mixture was quenched with ice water, the product extracted with ethyl acetate (3x 50 mL), the combined organic layers washed with water and brine, dried over sodium sulfate, filtered and concentrated *in vacuo.* The crude was purified by purified by combiflash (silica gel, 10-70% ethyl acetate in cyclohexane) to afford tert-butyl N-[3-ethylsulfonyl-6-(trifluoromethyl)imidazo[1,2-a]pyridin-2-yl]carbamate as a white solid. LCMS (method 2): Rt=1.51 min, m/z = 392 (M-H)-.

### Step C-1: Preparation of ethyl 6-[[tert-butoxycarbonyl-[3-ethylsulfonyl-6-(trifluoromethyl)imidazol[1,2-a]pyridin-2-yl]aminolmethyl]-2,2-difluoro-1,3-benzodioxole-5-carboxylate (Intermediate I-14)

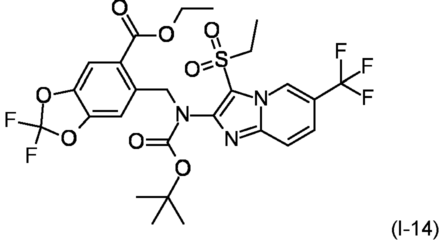

To solution of tert-butyl N-[3-ethylsulfonyl-6-(trifluoromethyl)imidazo[1,2-a]pyridin-2-yl]carbamate (Intermediate I-12 prepared as described above) (0.3 g, 0.724 mmol) in acetonitrile (10 mL) were added ethyl 6-(bromomethyl)-2,2-difluoro-1,3-benzodioxole-5-carboxylate (Intermediate I-6 prepared as described above) (0.295 g, 0.869 mmol) and cesium carbonate (0.472 g, 1.44 mmol) at room temperature. The reaction mixture was heated at 50 °C for 3 hours, then diluted with water and the product extracted with ethyl acetate. The organic layer was washed twice with water, then brine, dried over sodium sulfate, filtered and concentrated *in vacuo.* The crude was purified by combiflash (silica gel, 10 to 50% ethyl acetate in cyclohexane) to afford ethyl 6-[[tert-butoxycarbonyl-[3-ethylsulfonyl-6-(trifluoromethyl)imidazo[1,2-a]pyridin-2-yl]amino]methyl]-2,2-difluoro-1,3-benzodioxole-5-carboxylate as a gum. LCMS (method 1): Rt = 1.34 min, m/z= 658.41 (M+Na)⁺.

### Step C-2: Preparation of ethyl 6-[[[3-ethylsulfonyl-6-(trifluoromethyl)imidazo[1,2-a]pyridin-2-yl]aminolmethyl]-2,2-difluoro-1,3-benzodioxole-5-carboxylate (Intermediate I-15)

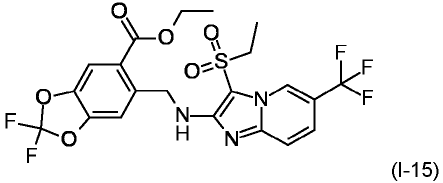

A solution of ethyl 6-[[tert-butoxycarbonyl-[3-ethylsulfonyl-6-(trifluoromethyl)imidazo[1,2-a]pyridin-2-yl]amino]methyl]-2,2-difluoro-1,3-benzodioxole-5-carboxylate (Intermediate I-14 prepared as described above) (0.431 g, 0.6442 mmol) in trifluoroacetic acid (3 mL, 38.6 mmol) was stirred for 3 hours at room temperature. After completion, the reaction mass was neutralized with an aqueous sodium bicarbonate solution. The aqueous layer was extracted with ethyl acetate (2x), the combined organic layers dried over sodium sulfate, filtered and concentrated *in vacuo* to afford ethyl 6-[[[3-ethylsulfonyl-6-(trifluoromethyl)imidazo[1,2-a]pyridin-2-yl]amino]methyl]-2,2-difluoro-1,3-benzodioxole-5-carboxylate. The crude was used as such for next step. LCMS (method 1): Rt = 1.25 min, m/z= 536 (M+H)⁺.

### Step C-3: Preparation of 6-[[[3-ethylsulfonyl-6-(trifluoromethyl)imidazo[1,2-a]pyridin-2-yl]aminolmethyl]-2,2-difluoro-1,3-benzodioxole-5-carboxylic acid (intermediate I-16)

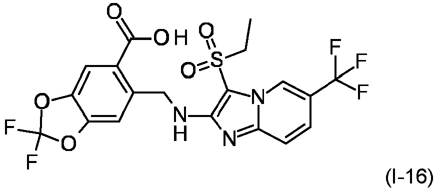

To a solution of ethyl 6-[[[3-ethylsulfonyl-6-(trifluoromethyl)imidazo[1,2-a]pyridin-2-yl]amino]methyl]-2,2-difluoro-1,3-benzodioxole-5-carboxylate (Intermediate I-15 prepared as described above) (0.3 g, 0.532 mmol) in tetrahydrofuran (6 mL) was added a solution of lithium hydroxide monohydrate (0.094 g, 2.129 mmol) in water (2 mL) at 10 °C. The reaction mixture was stirred at room temperature for 12 hours. After completion, the reaction mass was concentrated *in vacuo,* acidified with an aqueous 1N hydrochloric acid solution and the product extracted with ethyl acetate. The organic layer was washed with water (2x) followed by brine, dried over sodium sulfate, filtered and concentrated *in vacuo* to afford 6-[[[3-ethylsulfonyl-6-(trifluoromethyl)imidazo[1,2-a]pyridin-2-yl]amino]methyl]-2,2-difluoro-1,3-benzodioxole-5-carboxylic acid. The crude was used as such for next step. LCMS (method 1): Rt = 1.13 min, m/z= 508 (M+H)⁺.

### Step C-4: Preparation of 6-[3-ethylsulfonyl-6-(trifluoromethyl)imidazo[1 ,2-a]pyridin-2-yl]-2,2-difluoro-5H-[1,3]dioxolo[4,5-f]isoindol-7-one (compound P1)

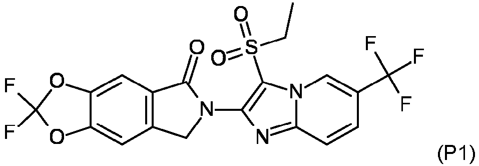

To a 0°C cooled solution of 6-[[[3-ethylsulfonyl-6-(trifluoromethyl)imidazo[1,2-a]pyridin-2-yl]amino]methyl]-2,2-difluoro-1,3-benzodioxole-5-carboxylic acid (Intermediate I-16 prepared as described above) (0.39 g, 0.7302 mmol) in pyridine (2 mL) was added phosphorus oxychloride (0.1375 mL, 1.460 mmol). The mixture was allowed to come at room temperature and stirred for 2 hours under nitrogen atmosphere. The reaction mass was acidified with an aqueous 2N hydrochloric acid (15 mL) solution and the product extracted with ethyl acetate (2x 30 mL). The combined organic layers were dried over sodium sulfate, filtered and concentrated *in vacuo.* The crude was purified by combiflash (silica gel, 50% ethyl acetate in cyclohexane) to afford 6-[3-ethylsulfonyl-6-(trifluoromethyl)imidazo[1,2-a]pyridin-2-yl]-2,2-difluoro-5H-[1,3]dioxolo[4,5-f]isoindol-7-one as a white solid. LCMS (method 1): Rt= 1.13 min, m/z= 490 (M+H)⁺. ¹H NMR (400 MHz, CDCl₃) δ ppm: 1.55 (t, 3 H), 3.89 (q, 2 H), 5.10 (s, 2 H), 7.29 (s, 1 H), 7.62 (s, 1 H), 7.68 (dd, 1 H), 7.82 (d, 1 H), 9.27 (s, 1 H).

### Example P2: Preparation of 6-(3-ethylsulfonyl-2-quinolyl)-2,2-difluoro-5H-[1,3]dioxolo[4,5-f]isoindol-7-one (compound P2)

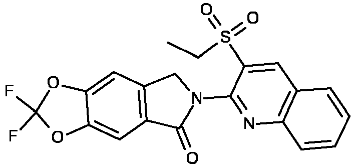

### Step-1: Preparation of 3-ethylsulfanylquinoline (Intermediate I-17)

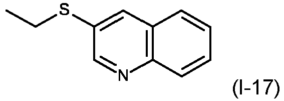

To a solution of 3-bromoquinoline (5.2 g, 25 mmol) in toluene (52 mL) degassed with nitrogen for 10 minutes were added sodium ethanethiolate (2.5 g, 30 mmol), N,N-diisopropylethylamine (15 mL, 87 mmol), tris(dibenzylideneacetone)dipalladium(0) (1.5 g, 1.6 mmol) and 4,5-bis(diphenylphosphino)-9,9-dimethylxanthene (1.6 g, 2.7 mmol). The reaction mixture was again degassed with nitrogen for another 5 minutes, then heated at 100 °C for 3 hours and further at 80 °C overnight. The reaction mass was quenched with water and the product extracted with ethyl acetate (2x). The combined organic layers were washed with brine, dried over sodium sulfate, filtered and concentrated *in vacuo.* The crude compound was purified by combiflash (silica gel, 0-15% ethyl acetate in cyclohexane) to afford 3-ethylsulfanylquinoline as an oil. LCMS (method 1): Rt= 1.03 min, m/z= 190 (M+H)⁺.

### Step-2: Preparation of 3-ethylsulfonyl-1-oxido-quinolin-1-ium (Intermediate I-18)

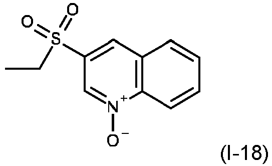

To a solution of 3-ethylsulfanylquinoline (Intermediate I-17 prepared as described above) (2.8 g, 15 mmol) in dichloromethane (40 mL) was added 3-chlorobenzenecarboperoxoic acid (12 g, 47 mmol, 70 mass%) and the reaction mass was stirred at room temperature overnight. The reaction mass was diluted with water and then basified with a saturated sodium bicarbonate solution. The aqueous phase was extracted with dichloromethane (3x 50 mL), the combined organic layers washed with brine (30 mL), dried over sodium sulfate, filtered and concentrated *in vacuo.* The crude was purified by combiflash (silica gel, 40% ethyl acetate in cyclohexane) to afford 3-ethylsulfonyl-1-oxido-quinolin-1-ium. LCMS (method 1): Rt= 0.55 min, m/z= 238 (M+H)⁺.

### Step-3: Preparation of 2-chloro-3-ethylsulfonyl-quinoline (Intermediate I-19)

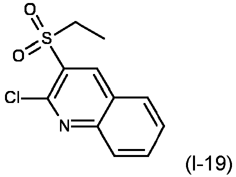

To 3-ethylsulfonyl-1-oxido-quinolin-1-ium (Intermediate I-18 prepared as described above) (2.59 g, 10.93 mmol) cooled to 0°C was added phosphoryl chloride (25.93 mL) dropwise under a nitrogen atmosphere. The reaction mixture was allowed to warm to room temperature and stirred for 2 hours under nitrogen. The reaction mixture was poured in ice cold water and the product extracted with ethyl acetate (3x 60 mL). The combined organic layers were washed with brine, dried over sodium sulfate, filtered and concentrated *in vacuo.* The crude was purified by combiflash (silica gel, 0-40% ethyl acetate in cyclohexane) to afford 2-chloro-3-ethylsulfonyl-quinoline. LCMS (method 1): Rt= 1.01 min, m/z= 256/258 (M+H)⁺.

### Step-4: Preparation of 3-ethylsulfonylquinolin-2-amine (Intermediate I-20)

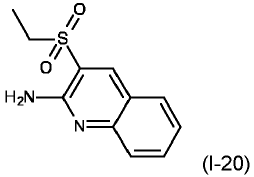

To a solution of 2-chloro-3-ethylsulfonyl-quinoline (Intermediate I-19 prepared as described above) (2 g, 7.82 mmol) in tetrahydrofuran (12 mL) in an Eyela vessel were added copper(II) sulfate (0.249 g, 1.56 mmol), copper (0.099 g, 1.56 mmol) and ammonium hydroxide (13.71 g, 117.3 mmol) at room temperature. The Eyela vessel was closed and the mixture stirred at 105°C for 6 hours. Upon completion, the apparatus was cooled to room temperature and the pressure carefully released. The reaction mass was diluted with water and the product extracted with ethyl acetate. The combined organic layers were washed with brine, dried over anhydrous sodium sulfate, filtered and concentrated *in vacuo.* The crude was purified by combiflash (silica gel, 50% ethyl acetate in cyclohexane) to afford 3-ethylsulfonylquinolin-2-amine as a solid. LCMS (method 2): Rt= 0.48 min, m/z= 237 (M+H)⁺.

### Step-5: Preparation of tert-butyl N-(3-ethylsulfonyl-2-quinolyl)carbamate (Intermediate I-21)

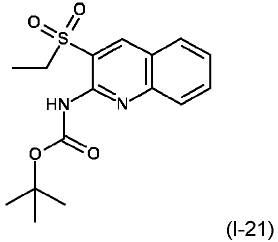

To a solution of 3-ethylsulfonylquinolin-2-amine (Intermediate I-20 prepared as described above) (1.20 g, 5.08 mmol) in N,N-dimethylformamide (20 mL) at 0°C was added sodium hydride (0.467 g, 11.7 mmol, 60 mass %). After stirring for 30 minutes at 0°C, a solution of tert-butoxycarbonyl tert-butyl carbonate (1.33 g, 6.09 mmol) in N,N-dimethylformamide (0.5 mL) was added at 0°C. The reaction mixture was allowed to warm to room temperature and stirred for 3 hours. Upon completion, the reaction mass was quenched with ice water and the product extracted with ethyl acetate. The combined organic layers were washed with brine, dried over sodium sulfate, filtered and concentrated *in vacuo.* The crude was purified by combiflash (silica gel, 40% ethyl acetate in cyclohexane) to afford tert-butyl N-(3-ethylsulfonyl-2-quinolyl)carbamate as a faint yellow solid. LCMS (method 1): Rt= 1.06 min, m/z= 281 [(M+H)⁺-56].

### Step-6: Preparation of ethyl 6-[[tert-butoxycarbonyl-(3-ethylsulfonyl-2-quinolyl)aminolmethyl]-2,2-difluoro-1,3-benzodioxole-5-carboxylate (Intermediate I-22)

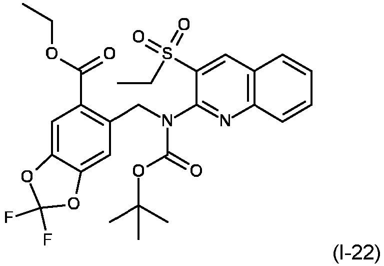

To a solution of tert-butyl N-(3-ethylsulfonyl-2-quinolyl)carbamate (Intermediate I-21 prepared as described above) (0.450 g, 1.338 mmol) in acetonitrile (20 mL) were added ethyl 6-(bromomethyl)-2,2-difluoro-1,3-benzodioxole-5-carboxylate (Intermediate I-6 prepared as described above) (0.54 g, 1.67 mmol) and cesium carbonate (0.65 g, 2.00 mmol) under a nitrogen atmosphere. The mixture was heated at 50 °C for 3.5 hours. The reaction mass was diluted with water (50 mL) and the product extracted with ethyl acetate (3x 50 mL). The combined organic layers were dried over sodium sulfate, filtered and concentrated *in vacuo* to afford ethyl 6-[[tert-butoxycarbonyl-(3-ethylsulfonyl-2-quinolyl)amino]methyl]-2,2-difluoro-1,3-benzodioxole-5-carboxylate as a gum. The crude was used as such for next step. LCMS (method 1): Rt = 1.37 min, m/z= 479 [(M+H)⁺-100].

### Step-7: Preparation of ethyl 6-[[(3-ethylsulfonyl-2-quinolyl)amino]lmethyl]-2,2-difluoro-1,3-benzodioxole-5-carboxylate (Intermediate I-23)

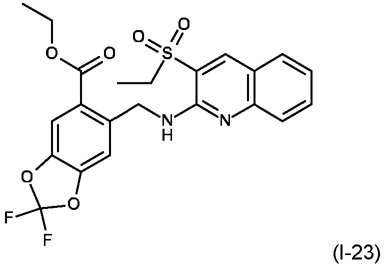

To a solution of ethyl 6-[[tert-butoxycarbonyl-(3-ethylsulfonyl-2-quinolyl)amino]methyl]-2,2-difluoro-1,3-benzodioxole-5-carboxylate (Intermediate I-22 prepared as described above) (0.855 g, 1.48 mmol) in trifluoromethylbenzene (10 mL) was added trifluoroacetic acid (1.79 mL, 22.2 mmol) at 0°C. The reaction mass was stirred for 10 hours at room temperature. After completion, the reaction mass was concentrated *in vacuo,* diluted with water (50 mL) and quenched with an aqueous sodium bicarbonate solution (30 mL). The aqueous layer was extracted with ethyl acetate (2x50 mL), the combined organic layers were washed with brine, dried on anhydrous sodium sulfate, filtered and concentrated *in vacuo.* The crude was purified by combiflash (silica gel, 0-30% ethyl acetate in cyclohexane) to afford ethyl 6-[[(3-ethylsulfonyl-2-quinolyl)amino]methyl]-2,2-difluoro-1,3-benzodioxole-5-carboxylate as a white solid. LCMS (method 1): Rt = 1.35 min, m/z= 479 (M+H)⁺.

### Step-8: Preparation of 6-[[(3-ethylsulfonyl-2-quinolyl)aminolmethyl]-2,2-difluoro-1,3-benzodioxole-5-carboxylic acid (Intermediate I-24)

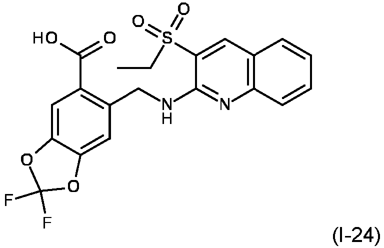

To solution of ethyl 6-[[(3-ethylsulfonyl-2-quinolyl)amino]methyl]-2,2-difluoro-1,3-benzodioxole-5-carboxylate (Intermediate I-23 prepared as described above) (0.49 g, 1.024 mmol) in tetrahydrofuran (10 mL) was added a solution of lithium hydroxide monohydrate (0.1809 g, 4.096 mmol) in water (3.5 mL) at room temperature. The reaction mixture was stirred at room temperature overnight. Additional lithium hydroxide monohydrate (0.042 g, 1.024 mmol) was added and stirring continued for another 18 hours. After completion, the reaction mass was concentrated *in vacuo,* acidified with an aqueous 1N hydrochloric acid solution and the product extracted with ethyl acetate (2 x 50 mL). The combined organic layers were washed with water (50 mL) followed by brine, dried over sodium sulfate, filtered and concentrated *in vacuo* to afford 6-[[(3-ethylsulfonyl-2-quinolyl)amino]methyl]-2,2-difluoro-1,3-benzodioxole-5-carboxylic acid as an off white solid. The crude was used as such for next step. LCMS (method 1): Rt = 1.10 min, m/z= 451 (M+H)⁺.

### Step-9: Preparation of 6-(3-ethylsulfonyl-2-quinolyl)-2,2-difluoro-5H-[1,3]dioxolo[4,5-f]isoindol-7-one (compound P2)

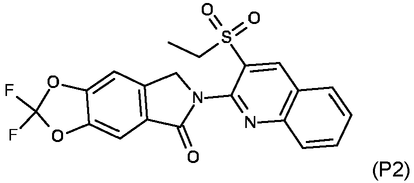

To a solution of 6-[[(3-ethylsulfonyl-2-quinolyl)amino]methyl]-2,2-difluoro-1,3-benzodioxole-5-carboxylic acid (Intermediate I-24 prepared as described above) (0.40 g, 0.894 mmol) in pyridine (2 mL) at 0°C was added phosphorus oxychloride (0.168 mL, 1.79 mmol). The reaction mixture was stirred at 0-10 °C for 40 minutes under a nitrogen atmosphere. The reaction mass was quenched with ice cold water (60 mL) and the product extracted with ethyl acetate (2x 25 mL). The combined organic layers were washed with brine (30 mL), dried over sodium sulfate, filtered and concentrated *in vacuo.* The crude compound was purified by combiflash (silica gel, 0-30% ethyl acetate in cyclohexane) to afford 6-(3-ethylsulfonyl-2-quinolyl)-2,2-difluoro-5H-[1,3]dioxolo[4,5-f]isoindol-7-one as a white solid. LCMS (method 1): Rt= 1.07 min, m/z= 433 (M+H)⁺. ¹H NMR (400 MHz, CDCl₃) δ ppm: 1.38 (t, 3 H), 3.55 (q, 2 H), 5.09 (s, 2 H), 7.25 (s, 1 H), 7.60 (s, 1 H), 7.78 (td, 1 H), 7.96 (ddd, 1 H), 8.09 (d, 1 H), 8.14 (d, 1 H), 9.02 (s, 1 H).

### Example P3: Preparation of 6-[3-ethylsulfonyl-6-(trifluoromethyl)pyrazolo[1,5-a]pyridin-2-yl]-2,2-difluoro-5H-[1,3]dioxolo[4,5-f]isoindol-7-one (compound P3)

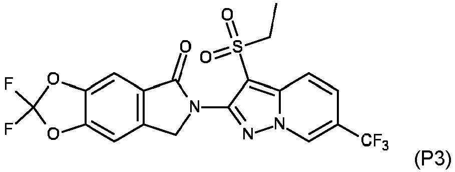

### Step-1: Preparation of tert-butyl 2-cyano-2-[5-(trifluoromethyl)-2-pyridyl]acetate (Intermediate I-25)

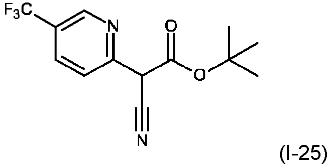

To a stirred solution of 2-chloro-5-(trifluoromethyl)pyridine (10.0 g, 55.1 mmol) in dimethyl sulfoxide (50 mL) was added potassium carbonate (11.4 g, 82.6 mmol) followed by tert-butyl 2-cyanoacetate (9.33 g, 66.1 mmol) at room temperature. The reaction mass was stirred at 100°C for 5 hours. After completion, the reaction mass was quenched with ice cold water and stirred for 10 minutes. The formed precipitate was filtered through a Buchner funnel, washed with n-pentane, dried *in vacuo* to afford tert-butyl 2-cyano-2-[5-(trifluoromethyl)-2-pyridyl]acetate as a yellow solid. LCMS (method 1): Rt= 1.09 min, m/z= 285 (M-H)⁻.

### Step-2: Preparation of 2-[5-(trifluoromethyl)-2-pyridyllacetonitrile (Intermediate 1-26)

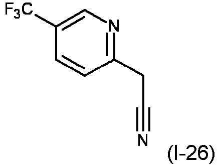

To a stirred solution of tert-butyl 2-cyano-2-[5-(trifluoromethyl)-2-pyridyl]acetate (Intermediate I-25 prepared as described above) (10.00 g, 34.94 mmol) in acetonitrile (100 mL) was added 4-methylbenzenesulfonic acid (3.04 g, 17.47 mmol) at room temperature. The reaction mass was stirred at 87°C for 1 hour. The mixture was quenched with ice cold water (100 mL) and the product extracted with ethyl acetate (2x 100 mL). The combined organic layers were dried over anhydrous sodium sulfate, filtered and concentrated *in vacuo.* The crude compound was purified by combiflash (silica gel, 20% ethyl acetate in cyclohexane) to afford 2-[5-(trifluoromethyl)-2-pyridyl]acetonitrile as yellow crystals. LCMS (method 1): Rt= 1.00 min, m/z= 187 (M+H)⁺.

### Step-3: Preparation of 2-[1-amino-5-(trifluoromethyl)pyridin-1-ium-2-yl]acetonitrile;2,4,6-trimethylbenzenesulfonate (Intermediate I-27)

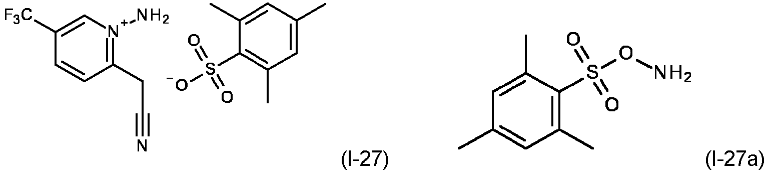

To a solution of ethyl N-(mesitylsulfonyl)oxyacetimidate (5.0 g, 17.52 mmol) in 1,4-dioxane (15 mL) at 0°C was added perchloric acid (2.147 mL, 21.34 mmol) dropwise and the reaction mixture stirred at 0 °C for 30 minutes. Ice cold water was added to the reaction mass which was then extracted with dichloromethane (2x 25 mL). The combined organic layers were dried over sodium sulfate and filtered to obtain a solution of amino 2,4,6-trimethylbenzenesulfonate (I-27a). To this freshly prepared solution of amino 2,4,6-trimethylbenzenesulfonate (I-27a) in dichloromethane (50 mL) was added 2-[5-(trifluoromethyl)-2-pyridyl]acetonitrile (Intermediate I-26 prepared as described above) (2.17 g, 11.66 mmol) dropwise at room temperature. The reaction mass was stirred at room temperature for 16 hours. After completion, the mixture was used as such for next step as a solution of 2-[1-amino-5-(trifluoromethyl)pyridin-1-ium-2-yl]acetonitrile;2,4,6-trimethylbenzenesulfonate in dichloromethane. LCMS (method 1): Rt= 0.99 min, m/z= 202 (M)⁺.

### Step-4: Preparation of 6-(trifluoromethyl)pyrazolo[1,5-a]pyridin-2-amine (Intermediate I-28)

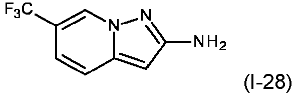

To a stirred solution of 2-[1-amino-5-(trifluoromethyl)pyridin-1-ium-2-yl]acetonitrile;2,4,6-trimethylbenzenesulfonate (Intermediate I-27 prepared as described above) (7 g, 17.44 mmol) in dichloromethane (50 mL) were added methanol (35 mL) and potassium carbonate (4.820 g, 34.88 mmol) at room temperature. The reaction mass was stirred at room temperature for 4 hours, then quenched with ice cold water and the organic layer was separated. The aqueous layer was extracted with ethyl acetate (2x 80 mL), the combined organic layers were dried over sodium sulfate, filtered and concentrated *in vacuo.* The crude compound was purified by combiflash (silica gel, 0-30% ethyl acetate in cyclohexane) to afford 6-(trifluoromethyl)pyrazolo[1,5-a]pyridin-2-amine as solid. LCMS (method 1): Rt= 0.97 min, m/z= 202 (M+H)⁺.

### Step-5: Preparation of N-[6-(trifluoromethyl)pyrazolo[1,5-a]pyridin-2-yl]acetamide (Intermediate I-29)

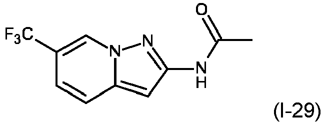

To a solution 6-(trifluoromethyl)pyrazolo[1,5-a]pyridin-2-amine (Intermediate I-28 prepared as described above) (2.58 g, 12.83 mmol) in pyridine (25 mL) at 0°C was added acetyl chloride (1.86 mL, 25.65 mmol) dropwise and the reaction mixture stirred at 0-10 °C for 1 hour under a nitrogen atmosphere. The reaction mass was diluted with water and ethyl acetate, then the organic layer was separated. The aqueous layer was extracted with ethyl acetate (2x 50 mL), the combined organic layers washed with water, dried over sodium sulfate, filtered and concentrated *in vacuo.* The crude compound was purified by combiflash (silica gel, 0-40% ethyl acetate in cyclohexane) to afford N-[6-(trifluoromethyl)pyrazolo[1,5-a]pyridin-2-yl]acetamide. LCMS (method 1): Rt= 0.99 min, m/z= 244 (M+H)⁺.

### Step-6: Preparation of N-[3-iodo-6-(trifluoromethyl)pyrazolo[1,5-a]pyridin-2-yl]acetamide (Intermediate I-30)

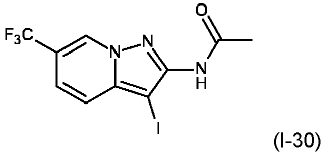

To a solution of N-[6-(trifluoromethyl)pyrazolo[1,5-a]pyridin-2-yl]acetamide (Intermediate I-29 prepared as described above) (2.63 g, 10.83 mmol) in acetonitrile (26.3 mL) was added 1-iodopyrrolidine-2,5-dione (2.92 g, 12.99 mmol) portionwise and the reaction mass was stirred at room temperature for 1.5 hours. After completion, the reaction mass was diluted with water and the product extracted with ethyl acetate (2x). The combined organic layers were washed with sodium thiosulfate solution followed by brine, dried over sodium sulfate, filtered and concentrated *in vacuo* to afford N-[3-iodo-6-(trifluoromethyl)pyrazolo[1,5-a]pyridin-2-yl]acetamide as solid. The crude was used as such for next step. LCMS (method 1): Rt= 1.01 min, m/z= 370 (M+H)⁺.

### Step-7: Preparation of tert-butyl N-acetyl-N-[3-iodo-6-(trifluoromethyl)pyrazolo[1,5-a]pyridin-2-yllcarbamate (Intermediate I-31)

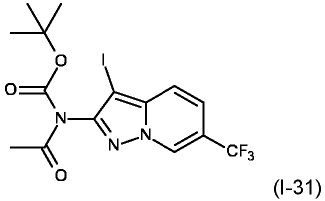

To a solution of N-[3-iodo-6-(trifluoromethyl)pyrazolo[1,5-a]pyridin-2-yl]acetamide (Intermediate I-30 prepared as described above) (3.77 g, 10.23 mmol) in acetonitrile (35 mL) at 0°C was added 4-dimethylaminopyridine (0.127 g. 1.02 mmol) followed by di-tert-butyl dicarbonate (2.76 g, 12.27 mmol). The mixture was stirred at room temperature for 2 hours. The reaction mass was concentrated *in vacuo,* quenched with ice cold water and the product extracted with ethyl acetate (3x 50 mL). The combined organic layers were washed with a solution of saturated bicarbonate solution, dried over sodium sulfate, filtered and concentrated *in vacuo.* The crude was purified by combiflash (silica gel, 0-30% ethyl acetate in cyclohexane) to afford tert-butyl N-acetyl-N-[3-iodo-6-(trifluoromethyl)pyrazolo[1,5-a]pyridin-2-yl]carbamate as a yellow solid. LCMS (method 1): Rt = 1.21 min, m/z= 370 [(M+H)⁺-100].

### Step-8: Preparation of tert-butyl N-[3-ethylsulfanyl-6-(trifluoromethyl)pyrazolo[1,5-a]pyridin-2-yllcarbamate (Intermediate I-32)

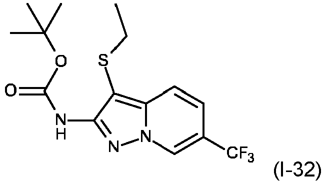

To a solution of tert-butyl N-acetyl-N-[3-iodo-6-(trifluoromethyl)pyrazolo[1,5-a]pyridin-2-yl]carbamate (Intermediate I-31 prepared as described above) (4.41 g, 9.40 mmol) in anhydrous 1,4-dioxane (40 mL) were added N-ethyl-N-isopropyl-propan-2-amine (4.18 mL, 24.44 mmol) and (5-diphenyl-phosphanyl-9,9-dimethyl-xanthen-4-yl)-diphenyl-phosphane (0.435 g, 0.752 mmol) at room temperature while purged with nitrogen for 5 minutes. Tris(dibenzylideneacetone)dipalladium(0) (0.602 g, 0.658 mmol) was added and the solution degassed with nitrogen for additional 5 minutes, then sodium ethanethiolate (1.63 g, 18.80 mmol) was added under nitrogen atmosphere and the reaction mixture stirred at 105 °C for 5 hours. The reaction mass was diluted with ethyl acetate, filtered over celite and the residue washed with ethyl acetate. The filtrate was washed with water, followed by brine and the organic layer was separated. The aqueous layer was extracted with ethyl acetate and the combined organic layers were dried over sodium sulfate, filtered and concentrated *in vacuo.* The crude compound was purified by combiflash (silica gel, 0-20% ethyl acetate in cyclohexane) to afford tert-butyl N-[3-ethylsulfanyl-6-(trifluoromethyl)pyrazolo[1,5-a]pyridin-2-yl]carbamate as gum. LCMS (method 1): Rt= 1.23 min, m/z= 306 [(M+H)⁺-56].

### Step-9: Preparation of tert-butyl N-[3-ethylsulfonyl-6-(trifluoromethyl)pyrazolo[1,5-a]pyridin-2-yllcarbamate (Intermediate I-33)

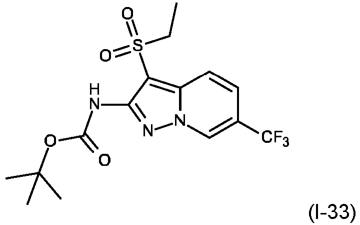

To a solution of tert-butyl N-[3-ethylsulfanyl-6-(trifluoromethyl)pyrazolo[1,5-a]pyridin-2-yl]carbamate (Intermediate I-32 prepared as described above) (3.21 g, 8.88 mmol) in acetonitrile (30 mL) at 0°C was added 3-chlorobenzenecarboperoxoic acid (4.82 g, 19.5 mmol, 70 mass%). The mixture was stirred at 0 to 10 °C for 1.5 hours. The reaction mass was concentrated *in vacuo* (water bath temperature was kept below 25 °C). The residue was diluted with water (60 mL) and basified with an aqueous 2N sodium hydroxide solution. The aqueous phase was extracted with ethyl acetate (2x 50 mL), the combined organic layers washed with brine (60 mL), dried over sodium sulfate and concentrated *in vacuo.* The crude compound was purified by combiflash (silica gel, 0-30% ethyl acetate in cyclohexane) to afford tert-butyl N-[3-ethylsulfonyl-6-(trifluoromethyl)pyrazolo[1,5-a]pyridin-2-yl] as an off white solid. The crude was used as such for next step. LCMS (method 1): Rt= 1.14 min, m/z= 338 [(M+H)⁺-56].

### Step-10: Preparation of ethyl 6-[[tert-butoxycarbonyl-[3-ethylsulfonyl-6-(trifluoromethyl)pyrazolo[1,5-a]pyridin-2-yl]aminolmethyl]-2,2-difluoro-1,3-benzodioxole-5-carboxylate (Intermediate I-34)

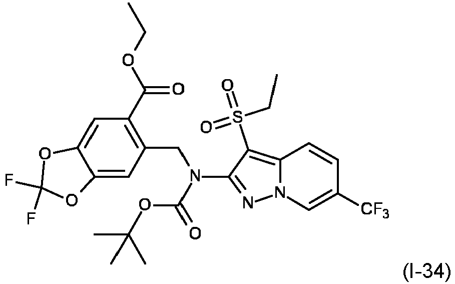

To a solution of tert-butyl N-[3-ethylsulfonyl-6-(trifluoromethyl)pyrazolo[1,5-a]pyridin-2-yl]carbamate (Intermediate I-33 prepared as described above) (0.60 g, 1.52 mmol) in acetonitrile (10 mL) were added cesium carbonate (0.746 g, 2.28 mmol) and ethyl 6-(bromomethyl)-2,2-difluoro-1,3-benzodioxole-5-carboxylate (Intermediate I-6 prepared as described above) (0.616 g, 1.90 mmol) at room temperature. The mixture was heated at 50 °C for 3.5 hours, then quenched with ice cold water (30 mL) and the product extracted with ethyl acetate (3x50 mL). The combined organic layers were dried over sodium sulfate, filtered and concentrated *in vacuo* to afford ethyl 6-[[tert-butoxycarbonyl-[3-ethylsulfonyl-6-(trifluoromethyl)pyrazolo[1,5-a]pyridin-2-yl]amino]methyl]-2,2-difluoro-1,3-benzodioxole-5-carboxylate as a gum. LCMS (method 2): Rt= 1.66 min, m/z= 536 [(M+H)⁺-100].

### Step-11: Preparation of ethyl 6-[[[3-ethylsulfonyl-6-(trifluoromethyl)pyrazolo[1,5-a]pyridin-2-yl]aminolmethyl]-2,2-difluoro-1,3-benzodioxole-5-carboxylate (Intermediate I-35)

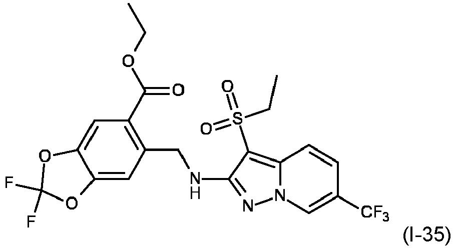

To a solution of ethyl 6-[[tert-butoxycarbonyl-[3-ethylsulfonyl-6-(trifluoromethyl)pyrazolo[1 ,5-a]pyridin-2-yl]amino]methyl]-2,2-difluoro-1,3-benzodioxole-5-carboxylate (Intermediate I-34 prepared as described above) (1.22 g, 1.92 mmol) in trifluoromethylbenzene (10 mL) at 0°C was added trifluoroacetic acid (2.32 mL, 28.8 mmol) and the mixture stirred at room temperature for 12 hours. The reaction mass was concentrated *in vacuo,* diluted with water (50 mL), and neutralized with an aqueous sodium bicarbonate solution (20 mL). The aqueous layer was extracted with ethyl acetate (2x 50 mL), the combined organic layers were washed with brine, dried over sodium sulfate, filtered and concentrated *in vacuo.* The crude was purified by combiflash (silica gel, 0 to 30% ethyl acetate in cyclohexane) to afford ethyl 6-[[[3-ethylsulfonyl-6-(trifluoromethyl)pyrazolo[1,5-a]pyridin-2-yl]amino]methyl]-2,2-difluoro-1,3-benzodioxole-5-carboxylate as a gum. LCMS (method 1): Rt= 1.22 min, m/z= 536 (M+H)⁺.

### Step-12: Preparation of 6-[[[3-ethylsulfonyl-6-(trifluoromethyl)pyrazolo[1,5-alpyridin-2-yl]aminolmethyl]-2,2-difluoro-1,3-benzodioxole-5-carboxylic acid (Intermediate I-36)

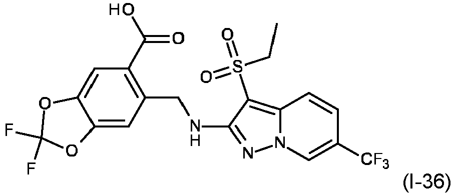

To solution of ethyl 6-[[[3-ethylsulfonyl-6-(trifluoromethyl)pyrazolo[1,5-a]pyridin-2-yl]amino]methyl]-2,2-difluoro-1,3-benzodioxole-5-carboxylate (Intermediate I-35 prepared as described above) (0.751 g, 1.403 mmol) in tetrahydrofuran (10 mL) was added a solution of lithium hydroxide monohydrate (0.123 g, 2.80 mmol) in water (3.5 mL) at room temperature overnight. Additional lithium hydroxide monohydrate (0.123 g, 2.80 mmol) was added and stirring continued at room temperature for another 6 hours. After completion, the reaction mass was concentrated *in vacuo,* acidified with an aqueous 1N hydrochloric acid solution and the product extracted with ethyl acetate (2x 30 mL). The combined organic layers were washed with water (20 mL) followed by brine, dried over sodium sulfate, filtered and concentrated *in vacuo.* The crude was purified by combiflash (silica gel, 80% ethyl acetate in cyclohexane) to afford 6-[[[3-ethylsulfonyl-6-(trifluoromethyl)pyrazolo[1,5-a]pyridin-2-yl]amino]methyl]-2,2-difluoro-1,3-benzodioxole-5-carboxylic acid. LCMS (method 1): Rt = 1.10 min, m/z= 508 (M+H)⁺.

### Step-13: Preparation of 6-[3-ethylsulfonyl-6-(trifluoromethyl)pyrazolo[1,5-a]pyridin-2-yl]-2,2-difluoro-5H-[1,3]dioxolo[4,5-f]isoindol-7-one (compound P3)

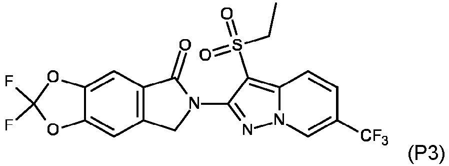

To a solution of 6-[[[3-ethylsulfonyl-6-(trifluoromethyl)pyrazolo[1,5-a]pyridin-2-yl]amino]methyl]-2,2-difluoro-1,3-benzodioxole-5-carboxylic acid (Intermediate I-36 prepared as described above) (0.332 g, 0.654 mmol) in pyridine (1.66 mL) at 0°C was added phosphorus oxychloride (0.12 mL, 1.309 mmol) and the reaction mass was stirred at 0-10 °C for 25 minutes under a nitrogen atmosphere. The mixture was quenched with ice cold water (60 mL) and the product extracted with ethyl acetate (3x 25 mL). The combined organic layers were washed with brine (30 mL), dried over sodium sulfate, filtered and concentrated *in vacuo.* The crude was purified by combiflash (silica gel, 0-30% ethyl acetate in cyclohexane) to afford 6-[3-ethylsulfonyl-6-(trifluoromethyl)pyrazolo[1,5-a]pyridin-2-yl]-2,2-difluoro-5H-[1,3]dioxolo[4,5-f]isoindol-7-one as an off white solid. LCMS (method 1): Rt= 1.23 min, m/z= 490 (M+H)⁺. ¹H NMR (400 MHz, CDCl₃) δ ppm: 1.43 (t, 3 H), 3.66 (q, 2 H), 5.01 (s, 2 H), 7.24 (s, 1 H), 7.61 (s, 1 H), 7.65 (dd, 1 H), 8.24 (d, 1 H), 8.83 (s, 1 H).

**Table P: Examples of compounds of formula (I)**

| No. | IUPAC name | Structures | LCMS | | | Mp (°C) |
|---|---|---|---|---|---|---|
| | | | Rₜ (min) | [M+H]⁺ (measured) | Method | |
| P1 | 6-[3-ethylsulfonyl-6-(trifluoromethyl)imidazo[1,2-a]pyridin-2-yl]-2,2-difluoro-5H-[1,3]dioxolo[4,5-f]isoindol-7-one | 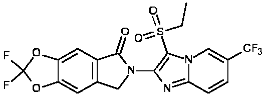 | 1.13 | 490 | 1 | 223-225 |
| P2 | 6-(3-ethylsulfonyl-2-quinolyl)-2,2-difluoro-5H-[1,3]dioxolo[4,5-f]isoindol-7-one | 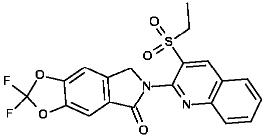 | 1.07 | 433 | 1 | 150-153 |
| P3 | 6-[3-ethylsulfonyl-6-(trifluoromethyl)pyrazolo[1,5-a]pyridin-2-yl]-2,2-difluoro-5H-[1,3]dioxolo[4,5-f]isoindol-7-one | 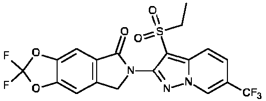 | 1.23 | 490 | 1 | 203-205 |
| P4 | 6-(6-cyclopropyl-3-ethylsulfonyl-pyrazolo[1,5-a]pyridin-2-yl)-2,2-difluoro-5H-[1,3]dioxolo[4,5-f]isoindol-7-one | 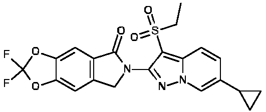 | 1.16 | 462 | 1 | 190-192 |
| P5 | 6-[3-ethylsulfonyl-7-(trifluoromethyl)imidazo[1,2-a]pyridin-2-yl]-2,2-difluoro-5H-[1,3]dioxolo[4,5-f]isoindol-7-one | 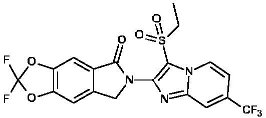 | 1.19 | 490 | 3 | 240 - 242 |
| P6 | 6-[3-ethylsulfonyl-5-(trifluoromethyl)pyrazolo[1,5-a]pyridin-2-yl]-2,2-difluoro-5H-[1,3]dioxolo[4,5-f]isoindol-7-one | 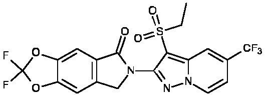 | 1.18 | 490 | 3 | 260 - 262 |
| P7 | 6-[6-(1,1-difluoroethyl)-3-ethylsulfonyl-imidazo[1,2-a]pyridin-2-yl]-2,2-difluoro-5H-[1,3]dioxolo[4,5-f]isoindol-7-one | 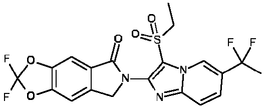 | 1.15 | 486 | 3 | 256 - 257 |
| P8 | 6-[7-(1,1-difluoroethyl)-3-ethylsulfonyl-imidazo[1,2-a]pyridin-2-yl]-2,2-difluoro-5H-[1,3]dioxolo[4,5-f]isoindol-7-one | 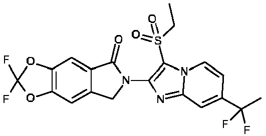 | 1.15 | 486 | 3 | 227 - 229 |
| P9 | 6-(7-bromo-3-ethylsulfonyl-imidazo[1,2-a]pyridin-2-yl)-2,2-difluoro-5H-[1,3]dioxolo[4,5-f]isoindol-7-one | 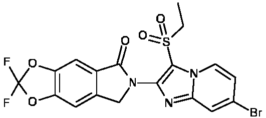 | 1.17 | 500 | 3 | 262 - 264 |
| P10 | 6-(6-bromo-3-ethylsulfonyl-imidazo[1,2-a]pyridin-2-yl)-2,2-difluoro-5H-[1,3]dioxolo[4,5-f]isoindol-7-one | 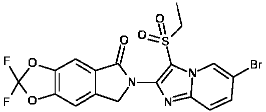 | 1.50 | 500 | 4 | 241 - 243 |
| P11 | 6-(6-chloro-3-ethylsulfonyl-imidazo[1,2-a]pyridin-2-yl)-2,2-difluoro-5H-[1,3]dioxolo[4,5-f]isoindol-7-one | 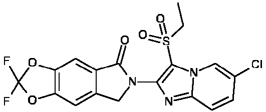 | 1.07 | 456 | 1 | 228 - 230 |
| P12 | 6-(6-bromo-3-ethylsulfonyl-pyrazolo[1,5-a]pyridin-2-yl)-2,2-difluoro-5H-[1,3]dioxolo[4,5-f]isoindol-7-one | 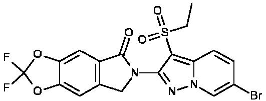 | 1.10 | 500 | 1 | |
| P13 | 6-(7-chloro-3-ethylsulfonyl-imidazo[1,2-a]pyridin-2-yl)-2,2-difluoro-5H-[1,3]dioxolo[4,5-f]isoindol-7-one | 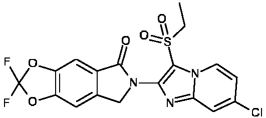 | 1.19 | 456 | 3 | 254 - 256 |
| P14 | 6-(6-chloro-3-ethylsulfonyl-pyrazolo[1,5-a]pyridin-2-yl)-2,2-difluoro-5H-[1,3]dioxolo[4,5-f]isoindol-7-one | 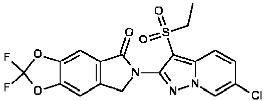 | 1.50 | 456 | 4 | 175 - 177 |
| P15 | 6-(5-chloro-3-ethylsulfonyl-pyrazolo[1,5-a]pyridin-2-yl)-2,2-difluoro-5H-[1,3]dioxolo[4,5-f]isoindol-7-one | 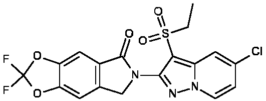 | 1.07 | 456 | 1 | 238 - 240 |
| P16 | 6-(3-ethylsulfonyl-7-iodo-imidazo[1,2-a]pyridin-2-yl)-2,2-difluoro-5H-[1,3]dioxolo[4,5-f]isoindol-7-one | 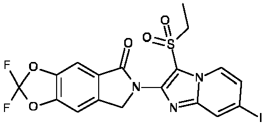 | 1.18 | 548 | 3 | 242 - 244 |
| P17 | 6-(3-ethylsulfonyl-6-iodo-imidazo[1,2-a]pyridin-2-yl)-2,2-difluoro-5H-[1,3]dioxolo[4,5-f]isoindol-7-one | 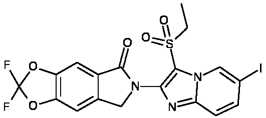 | 1.11 | 548 | 1 | 273 - 275 |
| P18 | 6-(3-ethylsulfonylimidazo[1,2-a]pyridin-2-yl)-2,2-difluoro-5H-[1,3]dioxolo[4,5-f]isoindol-7-one | 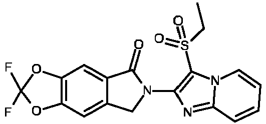 | 1.08 | 422 | 3 | 203 - 205 |

The activity of the compositions according to the invention can be broadened considerably, and adapted to prevailing circumstances, by adding other insecticidally, acaricidally and/or fungicidally active ingredients. The mixtures of the compounds of formula I with other insecticidally, acaricidally and/or fungicidally active ingredients may also have further surprising advantages which can also be described, in a wider sense, as synergistic activity. For example, better tolerance by plants, reduced phytotoxicity, insects can be controlled in their different development stages or better behaviour during their production, for example during grinding or mixing, during their storage or during their use.

Suitable additions to active ingredients here are, for example, representatives of the following classes of active ingredients: organophosphorus compounds, nitrophenol derivatives, thioureas, juvenile hormones, formamidines, benzophenone derivatives, ureas, pyrrole derivatives, carbamates, pyrethroids, chlorinated hydrocarbons, acylureas, pyridylmethyleneamino derivatives, macrolides, neonicotinoids and Bacillus thuringiensis preparations.

The following mixtures of the compounds of formula I with active ingredients are preferred (the abbreviation "TX" means "one compound selected from the group consisting of the compounds described in Tables A-1 to A-3, B-1 to B-3, C-1 to C-3, D-1 to D-3, E-1 to E-3, F-1 to F-3 and Table P of the present invention"):
an adjuvant selected from the group of substances consisting of petroleum oils (alternative name) (628) + TX;
abamectin + TX, acequinocyl + TX, acetamiprid + TX, acetoprole + TX, acrinathrin + TX, acynonapyr + TX, afidopyropen + TX, afoxolaner + TX, alanycarb + TX, allethrin + TX, alpha-cypermethrin + TX, alphamethrin + TX, amidoflumet + TX, aminocarb + TX, azocyclotin + TX, bensultap + TX, benzoximate + TX, benzpyrimoxan + TX, betacyfluthrin + TX, beta-cypermethrin + TX, bifenazate + TX, bifenthrin + TX, binapacryl + TX, bioallethrin + TX, S-bioallethrin + TX, bioresmethrin + TX, bistrifluron + TX, broflanilide + TX, brofluthrinate + TX, bromophos-ethyl + TX, buprofezine + TX, butocarboxim + TX, cadusafos + TX, carbaryl + TX, carbosulfan + TX, cartap + TX, CAS number: 1632218-00-8 + TX, CAS number: 1808115-49-2 + TX, CAS number: 2032403-97-5 + TX, CAS number: 2044701-44-0 + TX, CAS number: 2128706-05-6 + TX, CAS number: 2095470-94-1 + TX, CAS number: 2377084-09-6 + TX, CAS number: 1445683-71-5 + TX, CAS number: 2408220-94-8 + TX, CAS number: 2408220-91-5 + TX, CAS number: 1365070-72-9 + TX, CAS number: 2171099-09-3 + TX, CAS number: 2396747-83-2 + TX, CAS number: 2133042-31-4 + TX, CAS number: 2133042-44-9 + TX, CAS number: 1445684-82-1 + TX, CAS number: 1445684-82-1 + TX, CAS number: 1922957-45-6 + TX, CAS number: 1922957-46-7 + TX, CAS number: 1922957-47-8 + TX, CAS number: 1922957-48-9 + TX, CAS number: 2415706-16-8 + TX, CAS number: 1594624-87-9 + TX, CAS number: 1594637-65-6 + TX, CAS number: 1594626-19-3 + TX, CAS number: 1990457-52-7 + TX, CAS number: 1990457-55-0 + TX, CAS number: 1990457-57-2 + TX, CAS number: 1990457-77-6 + TX, CAS number: 1990457-66-3 + TX, CAS number: 1990457-85-6 + TX, CAS number: 2220132-55-6 + TX, CAS number: 1255091-74-7 + TX, CAS number: RNA (Leptinotarsa decemlineata-specific recombinant double-stranded interfering GS2) + TX, CAS number: 2719848-60-7 + TX, CAS number: 1956329-03-5 + TX, chlorantraniliprole + TX, chlordane + TX, chlorfenapyr + TX, chloroprallethrin + TX, chromafenozide + TX, clenpirin + TX, cloethocarb + TX, clothianidin + TX, 2-chlorophenyl N-methylcarbamate (CPMC) + TX, cyanofenphos + TX, cyantraniliprole + TX, cyclaniliprole + TX, cyclobutrifluram + TX, cycloprothrin + TX, cycloxaprid + TX, cyenopyrafen + TX, cyetpyrafen (or etpyrafen) + TX, cyflumetofen + TX, cyfluthrin + TX, cyhalodiamide + TX, cyhalothrin + TX, cypermethrin + TX, cyphenothrin + TX, cyproflanilide + TX, cyromazine + TX, deltamethrin + TX, diafenthiuron + TX, dialifos + TX, dibrom + TX, dicloromezotiaz + TX, diflovidazine + TX, diflubenzuron + TX, dimpropyridaz + TX, dinactin + TX, dinocap + TX, dinotefuran + TX, dioxabenzofos + TX, emamectin (or emamectin benzoate) + TX, empenthrin + TX, epsilon - momfluorothrin + TX, epsilon-metofluthrin + TX, esfenvalerate + TX, ethion + TX, ethiprole + TX, etofenprox + TX, etoxazole + TX, famphur + TX, fenazaquin + TX, fenfluthrin + TX, , fenmezoditiaz + TX, fenitrothion + TX, fenobucarb + TX, fenothiocarb + TX, fenoxycarb + TX, fenpropathrin + TX, fenpyroximate + TX, fensulfothion + TX, fenthion + TX, fentinacetate + TX, fenvalerate + TX, fipronil + TX, flometoquin + TX, flonicamid + TX, fluacrypyrim + TX, fluazaindolizine + TX, fluazuron + TX, flubendiamide + TX, flubenzimine + TX, fluchlordiniliprole + TX, flucitrinate + TX, flucycloxuron + TX, flucythrinate + TX, fluensulfone + TX, flufenerim + TX, flufenprox + TX, flufiprole + TX, fluhexafon + TX, flumethrin + TX, fluopyram + TX, flupentiofenox + TX, flupyradifurone + TX, flupyrimin + TX, fluralaner + TX, fluvalinate + TX, fluxametamide + TX, fosthiazate + TX, gamma-cyhalothrin + TX, guadipyr + TX, halofenozide + TX, halfenprox + TX, heptafluthrin + TX, hexythiazox + TX, hydramethylnon + TX, imicyafos + TX, imidacloprid + TX, imiprothrin + TX, indazapyroxamet + TX, indoxacarb + TX, iodomethane + TX, iprodione + TX, isocycloseram + TX, isothioate + TX, ivermectin + TX, kappa-bifenthrin + TX, kappa-tefluthrin + TX, lambda-Cyhalothrin + TX, lepimectin + TX, lotilaner + TX, lufenuron + TX, metaflumizone + TX, metaldehyde + TX, metam + TX, methomyl + TX, methoxyfenozide + TX, metofluthrin + TX, metolcarb + TX, mexacarbate + TX, milbemectin + TX, momfluorothrin + TX, niclosamide + TX, nicofluprole + TX; nitenpyram + TX, nithiazine + TX, omethoate + TX, oxamyl + TX, oxazosulfyl + TX, parathion-ethyl + TX, permethrin + TX, phenothrin + TX, phosphocarb + TX, piperonylbutoxide + TX, pirimicarb + TX, pirimiphos-ethyl + TX, pirimiphos-methyl + TX, Polyhedrosis virus + TX, prallethrin + TX, profenofos + TX, profluthrin + TX, propargite + TX, propetamphos + TX, propoxur + TX, prothiophos + TX, protrifenbute + TX, pyflubumide + TX, pymetrozine + TX, pyraclofos + TX, pyrafluprole + TX, pyridaben + TX, pyridalyl + TX, pyrifluquinazon + TX, pyrimidifen + TX, pyriminostrobin + TX, pyriprole + TX, pyriproxyfen + TX, resmethrin + TX, sarolaner + TX, selamectin + TX, silafluofen + TX, spinetoram + TX, spinosad + TX, spirobudifen + TX; spirodiclofen + TX, spiromesifen + TX, spiropidion + TX, spirotetramat + TX, spidoxamat + TX, sulfoxaflor + TX, tebufenozide + TX, tebufenpyrad + TX, tebupirimiphos + TX, tefluthrin + TX, temephos + TX, tetrachlorantraniliprole + TX, tetradiphon + TX, tetramethrin + TX, tetramethylfluthrin + TX, tetranactin + TX, tetraniliprole + TX, theta-cypermethrin + TX, thiacloprid + TX, thiamethoxam + TX, thiocyclam + TX, thiodicarb + TX, thiofanox + TX, thiometon + TX, thiosultap + TX, tigolaner + TX, tiorantraniliprole + TX; tioxazafen + TX, tolfenpyrad + TX, toxaphene + TX, tralomethrin + TX, transfluthrin + TX, triazamate + TX, triazophos + TX, trichlorfon + TX, trichloronate + TX, trichlorphon + TX, trifluenfuronate + TX, triflumezopyrim + TX, tyclopyrazoflor + TX, zeta-cypermethrin + TX, Extract of seaweed and fermentation product derived from melasse + TX, Extract of seaweed and fermentation product derived from melasse comprising urea + TX, amino acids + TX, potassium and molybdenum and EDTA-chelated manganese + TX, Extract of seaweed and fermented plant products + TX, Extract of seaweed and fermented plant products comprising phytohormones + TX, vitamins + TX, EDTA-chelated copper + TX, zinc + TX, and iron + TX, azadirachtin + TX, Bacillus aizawai + TX, Bacillus chitinosporus AQ746 (NRRL Accession No B-21 618) + TX, Bacillus firmus + TX, Bacillus kurstaki + TX, Bacillus mycoides AQ726 (NRRL Accession No. B-21664) + TX, Bacillus pumilus (NRRL Accession No B-30087) + TX, Bacillus pumilus AQ717 (NRRL Accession No. B-21662) + TX, Bacillus sp. AQ178 (ATCC Accession No. 53522) + TX, Bacillus sp. AQ175 (ATCC Accession No. 55608) + TX, Bacillus sp. AQ177 (ATCC Accession No. 55609) + TX, Bacillus subtilis unspecified + TX, Bacillus subtilis AQ153 (ATCC Accession No. 55614) + TX, Bacillus subtilis AQ30002 (NRRL Accession No. B-50421) + TX, Bacillus subtilis AQ30004 (NRRL Accession No. B- 50455) + TX, Bacillus subtilis AQ713 (NRRL Accession No. B-21661) + TX, Bacillus subtilis AQ743 (NRRL Accession No. B-21665) + TX, Bacillus thuringiensis AQ52 (NRRL Accession No. B-21619) + TX, Bacillus thuringiensis BD#32 (NRRL Accession No B-21530) + TX, Bacillus thuringiensis subspec. kurstaki BMP 123 + TX, Beauveria bassiana + TX, D-limonene + TX, Granulovirus + TX, Harpin + TX, Helicoverpa armigera Nucleopolyhedrovirus + TX, Helicoverpa zea Nucleopolyhedrovirus + TX, Heliothis virescens Nucleopolyhedrovirus + TX, Heliothis punctigera Nucleopolyhedrovirus + TX, Metarhizium spp. + TX, Muscodor albus 620 (NRRL Accession No. 30547) + TX, Muscodor roseus A3-5 (NRRL Accession No. 30548) + TX, Neem tree based products + TX, Paecilomyces fumosoroseus + TX, Paecilomyces lilacinus + TX, Pasteuria nishizawae + TX, Pasteuria penetrans + TX, Pasteuria ramosa + TX, Pasteuria thornei + TX, Pasteuria usgae + TX, P-cymene + TX, Plutella xylostella Granulosis virus + TX, Plutella xylostella Nucleopolyhedrovirus + TX, Polyhedrosis virus + TX, pyrethrum + TX, QRD 420 (a terpenoid blend) + TX, QRD 452 (a terpenoid blend) + TX, QRD 460 (a terpenoid blend) + TX, Quillaja saponaria + TX, Rhodococcus globerulus AQ719 (NRRL Accession No B-21663) + TX, Spodoptera frugiperda Nucleopolyhedrovirus + TX, Streptomyces galbus (NRRL Accession No. 30232) + TX, Streptomyces sp. (NRRL Accession No. B-30145) + TX, Terpenoid blend + TX, and Verticillium spp. + TX;
an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX;
an anthelmintic selected from the group of substances consisting of abamectin (1) + TX, crufomate (1011) + TX, cyclobutrifluram + TX, doramectin (alternative name) [CCN] + TX, emamectin (291) + TX, emamectin benzoate (291) + TX, eprinomectin (alternative name) [CCN] + TX, ivermectin (alternative name) [CCN] + TX, milbemycin oxime (alternative name) [CCN] + TX, moxidectin (alternative name) [CCN] + TX, piperazine [CCN] + TX, selamectin (alternative name) [CCN] + TX, spinosad (737) and thiophanate (1435) + TX;
an avicide selected from the group of substances consisting of chloralose (127) + TX, endrin (1122) + TX, fenthion (346) + TX, pyridin-4-amine (IUPAC name) (23) and strychnine (745) + TX;
a bactericide selected from the group of substances consisting of 1-hydroxy-1 H-pyridine-2-thione (IUPAC name) (1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen (alternative name) [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal (alternative name) [CCN] + TX;
a biological agent selected from the group of substances consisting of *Adoxophyes orana* GV (alternative name) (12) + TX, *Agrobacterium radiobacter* (alternative name) (13) + TX, *Amblyseius* spp. (alternative name) (19) + TX, *Anagrapha falcifera* NPV (alternative name) (28) + TX, *Anagrus atomus* (alternative name) (29) + TX, *Aphelinus abdominalis* (alternative name) (33) + TX, *Aphidius colemani* (alternative name) (34) + TX, *Aphidoletes aphidimyza* (alternative name) (35) + TX, *Autographa californica* NPV (alternative name) (38) + TX, *Bacillus firmus* (alternative name) (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis* subsp. *japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (alternative name) (53) + TX, *Beauveria brongniartii* (alternative name) (54) + TX, *Chrysoperla carnea* (alternative name) (151) + TX, *Cryptolaemus montrouzieri* (alternative name) (178) + TX, *Cydia pomonella* GV (alternative name) (191) + TX, *Dacnusa sibirica* (alternative name) (212) + TX, *Diglyphus isaea* (alternative name) (254) + TX, *Encarsia formosa* (scientific name) (293) + TX, *Eretmocerus eremicus* (alternative name) (300) + TX, *Helicoverpa zea* NPV (alternative name) (431) + TX, *Heterorhabditis bacteriophora* and *H. megidis* (alternative name) (433) + TX, *Hippodamia convergens* (alternative name) (442) + TX, *Leptomastix dactylopii* (alternative name) (488) + TX, *Macrolophus caliginosus* (alternative name) (491) + TX, *Mamestra brassicae* NPV (alternative name) (494) + TX, *Metaphycus helvolus* (alternative name) (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (alternative name) (575) + TX, *Orius* spp. (alternative name) (596) + TX, *Paecilomyces fumosoroseus* (alternative name) (613) + TX, *Phytoseiulus persimilis* (alternative name) (644) + TX, *Spodoptera exigua* multicapsid nuclear polyhedrosis virus (scientific name) (741) + TX, *Steinernema bibionis* (alternative name) (742) + TX, *Steinernema carpocapsae* (alternative name) (742) + TX, *Steinernema feltiae* (alternative name) (742) + TX, *Steinernema glaseri* (alternative name) (742) + TX, *Steinernema riobrave* (alternative name) (742) + TX, *Steinernema riobravis* (alternative name) (742) + TX, *Steinernema scapterisci* (alternative name) (742) + TX, *Steinernema* spp. (alternative name) (742) + TX, *Trichogramma* spp. (alternative name) (826) + TX, *Typhlodromus occidentalis* (alternative name) (844) and *Verticillium lecanii* (alternative name) (848) + TX;
a soil sterilant selected from the group of substances consisting of iodomethane (IUPAC name) (542) and methyl bromide (537) + TX;
a chemosterilant selected from the group of substances consisting of apholate [CCN] + TX, bisazir (alternative name) [CCN] + TX, busulfan (alternative name) [CCN] + TX, diflubenzuron (250) + TX, dimatif (alternative name) [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron (alternative name) [CCN] + TX, tepa [CCN] + TX, thiohempa (alternative name) [CCN] + TX, thiotepa (alternative name) [CCN] + TX, tretamine (alternative name) [CCN] and uredepa (alternative name) [CCN] + TX;
an insect pheromone selected from the group of substances consisting of (E)-dec-5-en-1-yl acetate with (E)-dec-5-en-1-ol (IUPAC name) (222) + TX, (E)-tridec-4-en-1-yl acetate (IUPAC name) (829) + TX, (E)-6-methylhept-2-en-4-ol (IUPAC name) (541) + TX, (*E,Z*)-tetradeca-4,10-dien-1-yl acetate (IUPAC name) (779) + TX, (Z)-dodec-7-en-1-yl acetate (IUPAC name) (285) + TX, (Z)-hexadec-11-enal (IUPAC name) (436) + TX, (Z)-hexadec-11-en-1-yl acetate (IUPAC name) (437) + TX, (Z)-hexadec-13-en-11-yn-1-yl acetate (IUPAC name) (438) + TX, (*Z*)-icos-13-en-10-one (IUPAC name) (448) + TX, (Z)-tetradec-7-en-1-al (IUPAC name) (782) + TX, (Z)-tetradec-9-en-1-ol (IUPAC name) (783) + TX, (Z)-tetradec-9-en-1-yl acetate (IUPAC name) (784) + TX, (7E,9Z)-dodeca-7,9-dien-1-yl acetate (IUPAC name) (283) + TX, (9Z,11E)-tetradeca-9,11-dien-1-yl acetate (IUPAC name) (780) + TX, (9Z,12E)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin (alternative name) [CCN] + TX, brevicomin (alternative name) [CCN] + TX, codlelure (alternative name) [CCN] + TX, codlemone (alternative name) (167) + TX, cuelure (alternative name) (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure (alternative name) [CCN] + TX, ethyl 4-methyloctanoate (IUPAC name) (317) + TX, eugenol (alternative name) [CCN] + TX, frontalin (alternative name) [CCN] + TX, Gossyplure^{®} (alternative name; 1:1 mixture of the (Z,E) and (Z,Z) isomers of hexadeca-7,11-dien-1-yl-acetate) (420) + TX, grandlure (421) + TX, grandlure I (alternative name) (421) + TX, grandlure II (alternative name) (421) + TX, grandlure III (alternative name) (421) + TX, grandlure IV (alternative name) (421) + TX, hexalure [CCN] + TX, ipsdienol (alternative name) [CCN] + TX, ipsenol (alternative name) [CCN] + TX, japonilure (alternative name) (481) + TX, lineatin (alternative name) [CCN] + TX, litlure (alternative name) [CCN] + TX, looplure (alternative name) [CCN] + TX, medlure [CCN] + TX, megatomoic acid (alternative name) [CCN] + TX, methyl eugenol (alternative name) (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure (alternative name) [CCN] + TX, oryctalure (alternative name) (317) + TX, ostramone (alternative name) [CCN] + TX, siglure [CCN] + TX, sordidin (alternative name) (736) + TX, sulcatol (alternative name) [CCN] + TX, tetradec-11-en-1-yl acetate (IUPAC name) (785) + TX, trimedlure (839) + TX, trimedlure A (alternative name) (839) + TX, trimedlure B₁ (alternative name) (839) + TX, trimedlure B₂ (alternative name) (839) + TX, trimedlure C (alternative name) (839) and trunc-call (alternative name) [CCN] + TX;
an insect repellent selected from the group of substances consisting of 2-(octylthio)ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX;
a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tributyltin oxide (913) + TX, trifenmorph (1454) + TX, trimethacarb (840) + TX, triphenyltin acetate (IUPAC name) (347) and triphenyltin hydroxide (IUPAC name) (347) + TX, pyriprole [394730-71-3] + TX;
a nematicide selected from the group of substances consisting of AKD-3088 (compound code) + TX, 1,2-dibromo-3-chloropropane (IUPAC/Chemical Abstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/Chemical Abstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (alternative name) (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben (alternative name) + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cyclobutrifluram + TX, cytokinins (alternative name) (210) + TX, dazomet (216) + TX, DBCP (1045) + TX, DCIP (218) + TX, diamidafos (1044) + TX, dichlofenthion (1051) + TX, dicliphos (alternative name) + TX, dimethoate (262) + TX, doramectin (alternative name) [CCN] + TX, emamectin (291) + TX, emamectin benzoate (291) + TX, eprinomectin (alternative name) [CCN] + TX, ethoprophos (312) + TX, ethylene dibromide (316) + TX, fenamiphos (326) + TX, fenpyrad (alternative name) + TX, fensulfothion (1158) + TX, fosthiazate (408) + TX, fosthietan (1196) + TX, furfural (alternative name) [CCN] + TX, GY-81 (development code) (423) + TX, heterophos [CCN] + TX, iodomethane (IUPAC name) (542) + TX, isamidofos (1230) + TX, isazofos (1231) + TX, ivermectin (alternative name) [CCN] + TX, kinetin (alternative name) (210) + TX, mecarphon (1258) + TX, metam (519) + TX, metam-potassium (alternative name) (519) + TX, metam-sodium (519) + TX, methyl bromide (537) + TX, methyl isothiocyanate (543) + TX, milbemycin oxime (alternative name) [CCN] + TX, moxidectin (alternative name) [CCN] + TX, *Myrothecium verrucaria* composition (alternative name) (565) + TX, NC-184 (compound code) + TX, oxamyl (602) + TX, phorate (636) + TX, phosphamidon (639) + TX, phosphocarb [CCN] + TX, sebufos (alternative name) + TX, selamectin (alternative name) [CCN] + TX, spinosad (737) + TX, terbam (alternative name) + TX, terbufos (773) + TX, tetrachlorothiophene (IUPAC/ Chemical Abstracts name) (1422) + TX, thiafenox (alternative name) + TX, thionazin (1434) + TX, triazophos (820) + TX, triazuron (alternative name) + TX, xylenols [CCN] + TX, YI-5302 (compound code) and zeatin (alternative name) (210) + TX, fluensulfone [318290-98-1] + TX, fluopyram + TX;
a nitrification inhibitor selected from the group of substances consisting of potassium ethylxanthate [CCN] and nitrapyrin (580) + TX;
a plant activator selected from the group of substances consisting of acibenzolar (6) + TX, acibenzolar-S-methyl (6) + TX, probenazole (658) and *Reynoutria sachalinensis* extract (alternative name) (720) + TX;
a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (including alpha-bromadiolone) + TX, bromethalin (92) + TX, calcium cyanide (444) + TX, chloralose (127) + TX, chlorophacinone (140) + TX, cholecalciferol (alternative name) (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX; a synergist selected from the group of substances consisting of 2-(2-butoxyethoxy)ethyl piperonylate (IUPAC name) (934) + TX, 5-(1,3-benzodioxol-5-yl)-3-hexylcyclohex-2-enone (IUPAC name) (903) + TX, farnesol with nerolidol (alternative name) (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX;
an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX;
a virucide selected from the group of substances consisting of imanin (alternative name) [CCN] and ribavirin (alternative name) [CCN] + TX;
a wound protectant selected from the group of substances consisting of mercuric oxide (512) + TX, octhilinone (590) and thiophanate-methyl (802) + TX;
a biologically active substance selected from 1,1-bis(4-chloro-phenyl)-2-ethoxyethanol + TX, 2,4-dichlorophenyl benzenesulfonate + TX, 2-fluoro-N-methyl-N-1-naphthylacetamide + TX, 4-chlorophenyl phenyl sulfone + TX, acetoprole + TX, aldoxycarb + TX, amidithion + TX, amidothioate + TX, amiton + TX, amiton hydrogen oxalate + TX, amitraz + TX, aramite + TX, arsenous oxide + TX, azobenzene + TX, azothoate + TX, benomyl + TX, benoxa-fos + TX, benzyl benzoate + TX, bixafen + TX, brofenvalerate + TX, bromo-cyclen + TX, bromophos + TX, bromopropylate + TX, buprofezin + TX, butocarboxim + TX, butoxycarboxim + TX, butylpyridaben + TX, calcium polysulfide + TX, camphechlor + TX, carbanolate + TX, carbophenothion + TX, cymiazole + TX, chino-methionat + TX, chlorbenside + TX, chlordimeform + TX, chlordimeform hydrochloride + TX, chlorfenethol + TX, chlorfenson + TX, chlorfensulfide + TX, chlorobenzilate + TX, chloromebuform + TX, chloromethiuron + TX, chloropropylate + TX, chlorthiophos + TX, cinerin I + TX, cinerin II + TX, cinerins + TX, closantel + TX, coumaphos + TX, crotamiton + TX, crotoxyphos + TX, cufraneb + TX, cyanthoate + TX, DCPM + TX, DDT + TX, demephion + TX, demephion-O + TX, demephion-S + TX, demeton-methyl + TX, demeton-O + TX, demeton-O-methyl + TX, demeton-S + TX, demeton-S-methyl + TX, demeton-S-methylsulfon + TX, dichlofluanid + TX, dichlorvos + TX, dicliphos + TX, dienochlor + TX, dimefox + TX, dinex + TX, dinex-diclexine + TX, dinocap-4 + TX, dinocap-6 + TX, dinocton + TX, dino-penton + TX, dinosulfon + TX, dinoterbon + TX, dioxathion + TX, diphenyl sulfone + TX, disulfiram + TX, DNOC + TX, dofenapyn + TX, doramectin + TX, endothion + TX, eprinomectin + TX, ethoate-methyl + TX, etrimfos + TX, fenazaflor + TX, fenbutatin oxide + TX, fenothiocarb + TX, fenpyrad + TX, fen-pyroximate + TX, fenpyrazamine + TX, fenson + TX, fentrifanil + TX, flubenzimine + TX, flucycloxuron + TX, fluenetil + TX, fluorbenside + TX, FMC 1137 + TX, formetanate + TX, formetanate hydrochloride + TX, formparanate + TX, gamma-HCH + TX, glyodin + TX, halfenprox + TX, hexadecyl cyclopropanecarboxylate + TX, isocarbophos + TX, jasmolin I + TX, jasmolin II + TX, jodfenphos + TX, lindane + TX, malonoben + TX, mecarbam + TX, mephosfolan + TX, mesulfen + TX, methacrifos + TX, methyl bromide + TX, metolcarb + TX, mexacarbate + TX, milbemycin oxime + TX, mipafox + TX, monocrotophos + TX, morphothion + TX, moxidectin + TX, naled + TX, 4-chloro-2-(2-chloro-2-methylpropyl)-5-[(6-iodo-3-pyridyl)methoxy]pyridazin-3-one + TX, nifluridide + TX, nikkomycins + TX, nitrilacarb + TX, nitrilacarb 1:1 zinc chloride complex + TX, omethoate + TX, oxydeprofos + TX, oxydisulfoton + TX, pp'-DDT + TX, parathion + TX, permethrin + TX, phenkapton + TX, phosalone + TX, phosfolan + TX, phosphamidon + TX, polychloroterpenes + TX, polynactins + TX, proclonol + TX, promacyl + TX, propoxur + TX, prothidathion + TX, prothoate + TX, pyrethrin I + TX, pyrethrin II + TX, pyrethrins + TX, pyridaphenthion + TX, pyrimitate + TX, quinalphos + TX, quintiofos + TX, R-1492 + TX, phosglycin + TX, rotenone + TX, schradan + TX, sebufos + TX, selamectin + TX, sophamide + TX, SSI-121 + TX, sulfiram + TX, sulfluramid + TX, sulfotep + TX, sulfur + TX, diflovidazin + TX, tau-fluvalinate + TX, TEPP + TX, terbam + TX, tetradifon + TX, tetrasul + TX, thiafenox + TX, thiocarboxime + TX, thiofanox + TX, thiometon + TX, thioquinox + TX, thuringiensin + TX, triamiphos + TX, triarathene + TX, triazophos + TX, triazuron + TX, trifenofos + TX, trinactin + TX, vamidothion + TX, vaniliprole + TX, bethoxazin + TX, copper dioctanoate + TX, copper sulfate + TX, cybutryne + TX, dichlone + TX, dichlorophen + TX, endothal + TX, fentin + TX, hydrated lime + TX, nabam + TX, quinoclamine + TX, quinonamid + TX, simazine + TX, triphenyltin acetate + TX, triphenyltin hydroxide + TX, crufomate + TX, piperazine + TX, thiophanate + TX, chloralose + TX, fenthion + TX, pyridin-4-amine + TX, strychnine + TX, 1-hydroxy-1H-pyridine-2-thione + TX, 4-(quinoxalin-2-ylamino)benzenesulfonamide + TX, 8-hydroxyquinoline sulfate + TX, bronopol + TX, copper hydroxide + TX, cresol + TX, dipyrithione + TX, dodicin + TX, fenaminosulf + TX, formaldehyde + TX, hydrargaphen + TX, kasugamycin + TX, kasugamycin hydrochloride hydrate + TX, nickel bis(dimethyldithiocarbamate) + TX, nitrapyrin + TX, octhilinone + TX, oxolinic acid + TX, oxytetracycline + TX, potassium hydroxyquinoline sulfate + TX, probenazole + TX, streptomycin + TX, streptomycin sesquisulfate + TX, tecloftalam + TX, thiomersal + TX, Adoxophyes orana GV + TX, Agrobacterium radiobacter + TX, Amblyseius spp. + TX, Anagrapha falcifera NPV + TX, Anagrus atomus + TX, Aphelinus abdominalis + TX, Aphidius colemani + TX, Aphidoletes aphidimyza + TX, Autographa californica NPV + TX, Bacillus sphaericus Neide + TX, Beauveria brongniartii + TX, Chrysoperla carnea + TX, Cryptolaemus montrouzieri + TX, Cydia pomonella GV + TX, Dacnusa sibirica + TX, Diglyphus isaea + TX, Encarsia formosa + TX, Eretmocerus eremicus + TX, Heterorhabditis bacteriophora and H. megidis + TX, Hippodamia convergens + TX, Leptomastix dactylopii + TX, Macrolophus caliginosus + TX, Mamestra brassicae NPV + TX, Metaphycus helvolus + TX, Metarhizium anisopliae var. acridum + TX, Metarhizium anisopliae var. anisopliae + TX, Neodiprion sertifer NPV and N. lecontei NPV + TX, Orius spp. + TX, Paecilomyces fumosoroseus + TX, Phytoseiulus persimilis + TX, Steinernema bibionis + TX, Steinernema carpocapsae + TX, Steinernema feltiae + TX, Steinernema glaseri + TX, Steinernema riobrave + TX, Steinernema riobravis + TX, Steinernema scapterisci + TX, Steinernema spp. + TX, Trichogramma spp. + TX, Typhlodromus occidentalis + TX , Verticillium lecanii + TX, apholate + TX, bisazir + TX, busulfan + TX, dimatif + TX, hemel + TX, hempa + TX, metepa + TX, methiotepa + TX, methyl apholate + TX, morzid + TX, penfluron + TX, tepa + TX, thiohempa + TX, thiotepa + TX, tretamine + TX, uredepa + TX, (E)-dec-5-en-1-yl acetate with (E)-dec-5-en-1-ol + TX, (E)-tridec-4-en-1-yl acetate + TX, (E)-6-methylhept-2-en-4-ol + TX, (E,Z)-tetradeca-4,10-dien-1-yl acetate + TX, (Z)-dodec-7-en-1-yl acetate + TX, (Z)-hexadec-11-enal + TX, (Z)-hexadec-11-en-1-yl acetate + TX, (Z)-hexadec-13-en-11-yn-1-yl acetate + TX, (Z)-icos-13-en-10-one + TX, (Z)-tetradec-7-en-1-al + TX, (Z)-tetradec-9-en-1-ol + TX, (Z)-tetradec-9-en-1-yl acetate + TX, (7E,9Z)-dodeca-7,9-dien-1-yl acetate + TX, (9Z,11E)-tetradeca-9,11-dien-1-yl acetate + TX, (9Z,12E)-tetradeca-9,12-dien-1-yl acetate + TX, 14-methyloctadec-1-ene + TX, 4-methylnonan-5-ol with 4-methylnonan-5-one + TX, alpha-multistriatin + TX, brevicomin + TX, codlelure + TX, codlemone + TX, cuelure + TX, disparlure + TX, dodec-8-en-1-yl acetate + TX, dodec-9-en-1-yl acetate + TX, dodeca-8 + TX, 10-dien-1-yl acetate + TX, dominicalure + TX, ethyl 4-methyloctanoate + TX, eugenol + TX, frontalin + TX, grandlure + TX, grandlure I + TX, grandlure II + TX, grandlure III + TX, grandlure IV + TX, hexalure + TX, ipsdienol + TX, ipsenol + TX, japonilure + TX, lineatin + TX, litlure + TX, looplure + TX, medlure + TX, megatomoic acid + TX, methyl eugenol + TX, muscalure + TX, octadeca-2,13-dien-1-yl acetate + TX, octadeca-3,13-dien-1-yl acetate + TX, orfralure + TX, oryctalure + TX, ostramone + TX, siglure + TX, sordidin + TX, sulcatol + TX, tetradec-11-en-1-yl acetate + TX, trimedlure + TX, trimedlure A + TX, trimedlure B₁ + TX, trimedlure B₂ + TX, trimedlure C + TX, trunc-call + TX, 2-(octylthio)-ethanol + TX, butopyronoxyl + TX, butoxy(polypropylene glycol) + TX, dibutyl adipate + TX, dibutyl phthalate + TX, dibutyl succinate + TX, diethyltoluamide + TX, dimethyl carbate + TX, dimethyl phthalate + TX, ethyl hexanediol + TX, hexamide + TX, methoquin-butyl + TX, methylneodecanamide + TX, oxamate + TX, picaridin + TX, 1-dichloro-1-nitroethane + TX, 1,1-dichloro-2,2-bis(4-ethylphenyl)-ethane + TX, 1,2-dichloropropane with 1,3-dichloropropene + TX, 1-bromo-2-chloroethane + TX, 2,2,2-trichloro-1-(3,4-dichloro-phenyl)ethyl acetate + TX, 2,2-dichlorovinyl 2-ethylsulfinylethyl methyl phosphate + TX, 2-(1,3-dithiolan-2-yl)phenyl dimethylcarbamate + TX, 2-(2-butoxyethoxy)ethyl thiocyanate + TX, 2-(4,5-dimethyl-1,3-dioxolan-2-yl)phenyl methylcarbamate + TX, 2-(4-chloro-3,5-xylyloxy)ethanol + TX, 2-chlorovinyl diethyl phosphate + TX, 2-imidazolidone + TX, 2-isovalerylindan-1,3-dione + TX, 2-methyl(prop-2-ynyl)aminophenyl methylcarbamate + TX, 2-thiocyanatoethyl laurate + TX, 3-bromo-1-chloroprop-1-ene + TX, 3-methyl-1-phenylpyrazol-5-yl dimethyl-carbamate + TX, 4-methyl(prop-2-ynyl)amino-3,5-xylyl methylcarbamate + TX, 5,5-dimethyl-3-oxocyclohex-1-enyl dimethylcarbamate + TX, acethion + TX, acrylonitrile + TX, aldrin + TX, allosamidin + TX, allyxycarb + TX, alpha-ecdysone + TX, aluminium phosphide + TX, aminocarb + TX, anabasine + TX, athidathion + TX, azamethiphos + TX, Bacillus thuringiensis delta endotoxins + TX, barium hexafluorosilicate + TX, barium polysulfide + TX, barthrin + TX, Bayer 22/190 + TX, Bayer 22408 + TX, beta-cyfluthrin + TX, beta-cypermethrin + TX, bioethanomethrin + TX, biopermethrin + TX, bis(2-chloroethyl) ether + TX, borax + TX, bromfenvinfos + TX, bromo-DDT + TX, bufencarb + TX, butacarb + TX, butathiofos + TX, butonate + TX, calcium arsenate + TX, calcium cyanide + TX, carbon disulfide + TX, carbon tetrachloride + TX, cartap hydrochloride + TX, cevadine + TX, chlorbicyclen + TX, chlordane + TX, chlordecone + TX, chloroform + TX, chloropicrin + TX, chlorphoxim + TX, chlorprazophos + TX, cis-resmethrin + TX, cismethrin + TX, clocythrin + TX, copper acetoarsenite + TX, copper arsenate + TX, copper oleate + TX, coumithoate + TX, cryolite + TX, CS 708 + TX, cyanofenphos + TX, cyanophos + TX, cyclethrin + TX, cythioate + TX, d-tetramethrin + TX, DAEP + TX, dazomet + TX, decarbofuran + TX, diamidafos + TX, dicapthon + TX, dichlofenthion + TX, dicresyl + TX, dicyclanil + TX, dieldrin + TX, diethyl 5-methylpyrazol-3-yl phosphate + TX, dilor + TX, dimefluthrin + TX, dimetan + TX, dimethrin + TX, dimethylvinphos + TX, dimetilan + TX, dinoprop + TX, dinosam + TX, dinoseb + TX, diofenolan + TX, dioxabenzofos + TX, dithicrofos + TX, DSP + TX, ecdysterone + TX, El 1642 + TX, EMPC + TX, EPBP + TX, etaphos + TX, ethiofencarb + TX, ethyl formate + TX, ethylene dibromide + TX, ethylene dichloride + TX, ethylene oxide + TX, EXD + TX, fenchlorphos + TX, fenethacarb + TX, fenitrothion + TX, fenoxacrim + TX, fenpirithrin + TX, fensulfothion + TX, fenthion-ethyl + TX, flucofuron + TX, fosmethilan + TX, fospirate + TX, fosthietan + TX, furathiocarb + TX, furethrin + TX, guazatine + TX, guazatine acetates + TX, sodium tetrathiocarbonate + TX, halfenprox + TX, HCH + TX, HEOD + TX, heptachlor + TX, heterophos + TX, HHDN + TX, hydrogen cyanide + TX, hyquincarb + TX, IPSP + TX, isazofos + TX, isobenzan + TX, isodrin + TX, isofenphos + TX, isolane + TX, isoprothiolane + TX, isoxathion + TX, juvenile hormone I + TX, juvenile hormone II + TX, juvenile hormone III + TX, kelevan + TX, kinoprene + TX, lead arsenate + TX, leptophos + TX, lirimfos + TX, lythidathion + TX, m-cumenyl methylcarbamate + TX, magnesium phosphide + TX, mazidox + TX, mecarphon + TX, menazon + TX, mercurous chloride + TX, mesulfenfos + TX, metam + TX, metam-potassium + TX, metam-sodium + TX, methanesulfonyl fluoride + TX, methocrotophos + TX, methoprene + TX, methothrin + TX, methoxychlor + TX, methyl isothiocyanate + TX, methylchloroform + TX, methylene chloride + TX, metoxadiazone + TX, mirex + TX, naftalofos + TX, naphthalene + TX, NC-170 + TX, nicotine + TX, nicotine sulfate + TX, nithiazine + TX, nornicotine + TX, O-5-dichloro-4-iodophenyl O-ethyl ethylphosphonothioate + TX, O,O-diethyl O-4-methyl-2-oxo-2H-chromen-7-yl phosphorothioate + TX, O,O-diethyl O-6-methyl-2-propylpyrimidin-4-yl phosphorothioate + TX, O,O,O',O'-tetrapropyl dithiopyrophosphate + TX, oleic acid + TX, para-dichlorobenzene + TX, parathion-methyl + TX, pentachlorophenol + TX, pentachlorophenyl laurate + TX, PH 60-38 + TX, phenkapton + TX, phosnichlor + TX, phosphine + TX, phoxim-methyl + TX, pirimetaphos + TX, polychlorodicyclopentadiene isomers + TX, potassium arsenite + TX, potassium thiocyanate + TX, precocene I + TX, precocene II + TX, precocene III + TX, primidophos + TX, profluthrin + TX, promecarb + TX, prothiofos + TX, pyrazophos + TX, pyresmethrin + TX, quassia + TX, quinalphos-methyl + TX, quinothion + TX, rafoxanide + TX, resmethrin + TX, rotenone + TX, kadethrin + TX, ryania + TX, ryanodine + TX, sabadilla + TX, schradan + TX, sebufos + TX, SI-0009 + TX, thiapronil + TX, sodium arsenite + TX, sodium cyanide + TX, sodium fluoride + TX, sodium hexafluorosilicate + TX, sodium pentachlorophenoxide + TX, sodium selenate + TX, sodium thiocyanate + TX, sulcofuron + TX, sulcofuron-sodium + TX, sulfuryl fluoride + TX, sulprofos + TX, tar oils + TX, tazimcarb + TX, TDE + TX, tebupirimfos + TX, temephos + TX, terallethrin + TX, tetrachloroethane + TX, thicrofos + TX, thiocyclam + TX, thiocyclam hydrogen oxalate + TX, thionazin + TX, thiosultap + TX, thiosultap-sodium + TX, tralomethrin + TX, transpermethrin + TX, triazamate + TX, trichlormetaphos-3 + TX, trichloronat + TX, trimethacarb + TX, tolprocarb + TX, triclopyricarb + TX, triprene + TX, veratridine + TX, veratrine + TX, XMC + TX, zetamethrin + TX, zinc phosphide + TX, zolaprofos + TX, meperfluthrin + TX, tetramethylfluthrin + TX, bis(tributyltin) oxide + TX, bromoacetamide + TX, ferric phosphate + TX, niclosamide-olamine + TX, tributyltin oxide + TX, pyrimorph + TX, trifenmorph + TX, 1,2-dibromo-3-chloropropane + TX, 1,3-dichloropropene + TX, 3,4-dichlorotetrahydrothio-phene 1,1-dioxide + TX, 3-(4-chlorophenyl)-5-methylrhodanine + TX, 5-methyl-6-thioxo-1,3,5-thiadiazinan-3-ylacetic acid + TX, 6-isopentenylaminopurine + TX, anisiflupurin + TX, benclothiaz + TX, cytokinins + TX, DCIP + TX, furfural + TX, isamidofos + TX, kinetin + TX, Myrothecium verrucaria composition + TX, tetrachlorothiophene + TX, xylenols + TX, zeatin + TX, potassium ethylxanthate + TX acibenzolar + TX, acibenzolar-S-methyl + TX, Reynoutria sachalinensis extract + TX, alpha-chlorohydrin + TX, antu + TX, barium carbonate + TX, bisthiosemi + TX, brodifacoum + TX, bromadiolone + TX, bromethalin + TX, chlorophacinone + TX, cholecalciferol + TX, coumachlor + TX, coumafuryl + TX, coumatetralyl + TX, crimidine + TX, difenacoum + TX, difethialone + TX, diphacinone + TX, ergocalciferol + TX, flocoumafen + TX, fluoroacetamide + TX, flupropadine + TX, flupropadine hydrochloride + TX, norbormide + TX, phosacetim + TX, phosphorus + TX, pindone + TX, pyrinuron + TX, scilliroside + TX, -sodium fluoroacetate + TX, thallium sulfate + TX, warfarin + TX, -2-(2-butoxyethoxy)ethyl piperonylate + TX, 5-(1,3-benzodioxol-5-yl)-3-hexylcyclohex-2-enone + TX, farnesol with nerolidol + TX, verbutin + TX, MGK 264 + TX, piperonyl butoxide + TX, piprotal + TX, propyl isomer + TX, S421 + TX, sesamex + TX, sesasmolin + TX, sulfoxide + TX, anthraquinone + TX, copper naphthenate + TX, copper oxychloride + TX, dicyclopentadiene + TX, thiram + TX, zinc naphthenate + TX, ziram + TX, imanin + TX, ribavirin + TX, chloroinconazide + TX, mercuric oxide + TX, thiophanate-methyl + TX, azaconazole + TX, bitertanol + TX, bromuconazole + TX, cyproconazole + TX, difenoconazole + TX, diniconazole -+ TX, epoxiconazole + TX, fenbuconazole + TX, fluquinconazole + TX, flusilazole + TX, flutriafol + TX, furametpyr + TX, hexaconazole + TX, imazalil- + TX, imiben-conazole + TX, ipconazole + TX, metconazole + TX, myclobutanil + TX, paclobutrazole + TX, pefurazoate + TX, penconazole + TX, prothioconazole + TX, pyrifenox + TX, prochloraz + TX, propiconazole + TX, pyrisoxazole + TX, -simeconazole + TX, tebucon-azole + TX, tetraconazole + TX, triadimefon + TX, triadimenol + TX, triflumizole + TX, triticonazole + TX, ancymidol + TX, fenarimol + TX, nuarimol + TX, bupirimate + TX, dimethirimol + TX, ethirimol + TX, dodemorph + TX, fenpropidin + TX, fenpropimorph + TX, spiroxamine + TX, tridemorph + TX, cyprodinil + TX, mepanipyrim + TX, pyrimethanil + TX, fenpiclonil + TX, fludioxonil + TX, benalaxyl + TX, furalaxyl + TX, metalaxyl + TX, R-metalaxyl + TX, ofurace + TX, oxadixyl + TX, carbendazim + TX, debacarb + TX, fuberidazole -+ TX, thiabendazole + TX, chlozolinate + TX, dichlozoline + TX, myclozoline- + TX, procymidone + TX, vinclozoline + TX, boscalid + TX, carboxin + TX, fenfuram + TX, flutolanil + TX, mepronil + TX, oxycarboxin + TX, penthiopyrad + TX, thifluzamide + TX, dodine + TX, iminoctadine + TX, azoxystrobin + TX, dimoxystrobin + TX, enestroburin + TX, fenaminstrobin + TX, flufenoxystrobin + TX, fluoxastrobin + TX, kresoxim--methyl + TX, metominostrobin + TX, trifloxystrobin + TX, orysastrobin + TX, picoxystrobin + TX, pyraclostrobin + TX, pyrametostrobin + TX, pyraoxystrobin + TX, ferbam + TX, mancozeb + TX, maneb + TX, metiram + TX, propineb + TX, zineb + TX, captafol + TX, captan + TX, fluoroimide + TX, folpet + TX, tolylfluanid + TX, bordeaux mixture + TX, copper oxide + TX, mancopper + TX, oxine-copper + TX, nitrothal-isopropyl + TX, edifenphos + TX, iprobenphos + TX, phosdiphen + TX, tolclofos-methyl + TX, anilazine + TX, benthiavalicarb + TX, blasticidin-S + TX, chloroneb -+ TX, chloro-tha-lonil + TX, cyflufenamid + TX, cymoxanil + TX, cyclobutrifluram + TX, diclocymet + TX, diclomezine -+ TX, dicloran + TX, diethofencarb + TX, dimethomorph -+ TX, flumorph + TX, dithianon + TX, ethaboxam + TX, etridiazole + TX, famoxadone + TX, fenamidone + TX, fenoxanil + TX, ferimzone + TX, fluazinam + TX, flumetylsulforim + TX, fluopicolide + TX, fluoxytioconazole + TX, flusulfamide + TX, fluxapyroxad + TX, -fenhexamid + TX, fosetyl-aluminium -+ TX, hymexazol + TX, iprovalicarb + TX, cyazofamid + TX, methasulfocarb + TX, metrafenone + TX, pencycuron + TX, phthalide + TX, polyoxins + TX, propamocarb + TX, pyribencarb + TX, proquinazid + TX, pyroquilon + TX, pyriofenone + TX, quinoxyfen + TX, quintozene + TX, tiadinil + TX, triazoxide + TX, tricyclazole + TX, triforine + TX, validamycin + TX, valifenalate + TX, zoxamide + TX, mandipropamid + TX, flubeneteram + TX, isopyrazam + TX, sedaxane + TX, benzovindiflupyr + TX, pydiflumetofen + TX, 3-difluoromethyl-1-methyl-1H-pyrazole-4-carboxylic acid (3',4',5'-trifluoro-biphenyl-2-yl)-amide + TX, isoflucypram + TX, isotianil + TX, dipymetitrone + TX, 6-ethyl-5,7-dioxo-pyrrolo[4,5][1,4]dithiino[1,2-c]isothiazole-3-carbonitrile + TX, 2-(difluoromethyl)-N-[3-ethyl-1,1-dimethyl-indan-4-yl]pyridine-3-carboxamide + TX, 4-(2,6-difluorophenyl)-6-methyl-5-phenyl-pyridazine-3-carbonitrile + TX, (R)-3-(difluoromethyl)-1-methyl-N-[1,1,3-trimethylindan-4-yl]pyrazole-4-carboxamide + TX, 4-(2-bromo-4-fluoro-phenyl)-N-(2-chloro-6-fluoro-phenyl)-2,5-dimethyl-pyrazol-3-amine + TX, 4- (2- bromo- 4- fluorophenyl) - N- (2- chloro- 6-fluorophenyl) - 1, 3- dimethyl- 1H- pyrazol- 5- amine + TX, fluindapyr + TX, coumethoxystrobin (jiaxiangjunzhi) + TX, Ivbenmixianan + TX, dichlobentiazox + TX, mandestrobin + TX, 3-(4,4-difluoro-3,4-dihydro-3,3-dimethylisoquinolin-1-yl)quinolone + TX, 2-[2-fluoro-6-[(8-fluoro-2-methyl-3-quinolyl)oxy]phenyl]propan-2-ol + TX, oxathiapiprolin + TX, tert-butyl N-[6-[[[(1-methyltetrazol-5-yl)-phenyl-methylene]amino]oxymethyl]-2-pyridyl]carbamate + TX, pyraziflumid + TX, inpyrfluxam + TX, trolprocarb + TX, mefentrifluconazole + TX, ipfentrifluconazole+ TX, 2-(difluoromethyl)-N-[(3R)-3-ethyl-1,1-dimethyl-indan-4-yl]pyridine-3-carboxamide + TX, N'-(2,5-dimethyl-4-phenoxy-phenyl)-N-ethyl-N-methyl-formamidine + TX, N'-[4-(4,5-dichlorothiazol-2-yl)oxy-2,5-dimethyl-phenyl]-N-ethyl-N-methyl-formamidine + TX, [2-[3-[2-[1-[2-[3,5-bis(difluoromethyl)pyrazol-1-yl]acetyl]-4-piperidyl]thiazol-4-yl]-4,5-dihydroisoxazol-5-yl]-3-chloro-phenyl] methanesulfonate + TX, but-3-ynyl N-[6-[[(Z)-[(1-methyltetrazol-5-yl)-phenyl-methylene]amino]oxymethyl]-2-pyridyl]carbamate + TX, methyl N-[[5-[4-(2,4-dimethylphenyl)triazol-2-yl]-2-methyl-phenyl]methyl]carbamate + TX, 3-chloro-6-methyl-5-phenyl-4-(2,4,6-trifluorophenyl)pyridazine + TX, pyridachlometyl + TX, 3-(difluoromethyl)-1-methyl-N-[1,1,3-trimethylindan-4-yl]pyrazole-4-carboxamide + TX, 1-[2-[[1-(4-chlorophenyl)pyrazol-3-yl]oxymethyl]-3-methyl-phenyl]-4-methyl-tetrazol-5-one + TX, 1-methyl-4-[3-methyl-2-[[2-methyl-4-(3,4,5-trimethylpyrazol-1-yl)phenoxy]methyl]phenyl]tetrazol-5-one + TX, aminopyrifen + TX, ametoctradin + TX, amisulbrom + TX, penflufen + TX, (Z,2E)-5-[1-(4-chlorophenyl)pyrazol-3-yl]oxy-2-methoxyimino-N,3-dimethyl-pent-3-enamide + TX, florylpicoxamid + TX, fenpicoxamid + TX, metarylpicoxamid + TX, tebufloquin + TX, ipflufenoquin + TX, quinofumelin + TX, isofetamid + TX, ethyl 1-[[4-[[2-(trifluoromethyl)-1,3-dioxolan-2-yl]methoxy]phenyl]methyl]pyrazole-3-carboxylate + TX (may be prepared from the methods described in WO 2020/056090), ethyl 1-[[4-[(Z)-2-ethoxy-3,3,3-trifluoro-prop-1-enoxy]phenyl]methyl]pyrazole-3-carboxylate + TX (may be prepared from the methods described in WO 2020/056090), methyl N-[[4-[1-(4-cyclopropyl-2,6-difluoro-phenyl) pyrazol-4-yl]-2-methylphenyl]methyl]carbamate + TX (may be prepared from the methods described in WO 2020/097012), methyl N-[[4-[1-(2,6-difluoro-4-isopropyl-phenyl)pyrazol-4-yl]-2-methyl-phenyl]methyl]carbamate + TX (may be prepared from the methods described in WO 2020/097012), 6-chloro-3-(3-cyclopropyl-2-fluoro-phenoxy)-N-[2-(2,4-dimethylphenyl)-2,2-difluoro-ethyl]-5-methyl-pyridazine-4-carboxamide + TX (may be prepared from the methods described in WO 2020/109391), 6-chloro-N-[2-(2-chloro-4-methylphenyl)-2,2-difluoro-ethyl]-3-(3-cyclopropyl-2-fluoro-phenoxy)-5-methyl-pyridazine-4-carboxamide + TX (may be prepared from the methods described in WO 2020/109391), 6-chloro-3-(3-cyclopropyl-2-fluoro-phenoxy)-N-[2-(3,4-dimethylphenyl)-2,2-difluoro-ethyl]-5-methyl-pyridazine-4-carboxamide + TX (may be prepared from the methods described in WO 2020/109391), N-[2-[2,4-dichloro-phenoxy]phenyl]-3-(difluoromethyl)-1-methyl-pyrazole-4-carboxamide + TX, N-[2-[2-chloro-4-(trifluoromethyl)phenoxy]phenyl]-3-(difluoromethyl)-1-methyl-pyrazole-4-carboxamide + TX, benzothiostrobin + TX, phenamacril + TX, 5-amino-1,3,4-thiadiazole-2-thiol zinc salt (2:1) + TX, fluopyram + TX, flufenoxadiazam + TX, flutianil + TX, fluopimomide + TX, pyrapropoyne + TX, picarbutrazox + TX, 2-(difluoromethyl)-N-(3-ethyl-1,1-dimethyl-indan-4-yl)pyridine-3-carboxamide + TX, 2- (difluoromethyl) - N- ((3R) - 1, 1, 3- trimethylindan- 4- yl) pyridine- 3- carboxamide + TX, 4-[[6-[2-(2,4-difluorophenyl)-1,1-difluoro-2-hydroxy-3-(1,2,4-triazol-1-yl)propyl]-3-pyridyl]oxy]benzonitrile + TX, metyltetraprole + TX, 2- (difluoromethyl) - N- ((3R) - 1, 1, 3- trimethylindan- 4- yl) pyridine- 3-carboxamide + TX, α- (1, 1- dimethylethyl) - α- [4'- (trifluoromethoxy) [1, 1'- biphenyl] - 4- yl] -5-pyrimidinemethanol + TX, fluoxapiprolin + TX, enoxastrobin + TX, methyl (Z)-3-methoxy-2-[2-methyl-5-[4-(trifluoromethyl)triazol-2-yl]phenoxy]prop-2-enoate + TX, methyl (Z)-3-methoxy-2-[2-methyl-5-(4-propyltriazol-2-yl)phenoxy]prop-2-enoate + TX, methyl (Z)-2-[5-(3-isopropylpyrazol-1-yl)-2-methyl-phenoxy]-3-methoxy-prop-2-enoate + TX, methyl (Z)-3-methoxy-2-[2-methyl-5-(3-propylpyrazol-1-yl)phenoxy]prop-2-enoate + TX, methyl (Z)-3-methoxy-2-[2-methyl-5-[3-(trifluoromethyl)pyrazol-1-yl]phenoxy]prop-2-enoate + TX (these compounds may be prepared from the methods described in WO2020/079111), methyl (Z)-2-(5-cyclohexyl-2-methyl-phenoxy)-3-methoxy-prop-2-enoate + TX, methyl (Z)-2-(5-cyclopentyl-2-methyl-phenoxy)-3-methoxy-prop-2-enoate + TX (these compounds may be prepared from the methods described in WO2020/193387), 4-[[6-[2-(2,4-difluorophenyl)-1,1-difluoro-2-hydroxy-3-(1,2,4-triazol-1-yl)propyl]-3-pyridyl]oxy] benzonitrile + TX, 4-[[6-[2-(2,4-difluorophenyl)-1,1-difluoro-2-hydroxy-3-(5-sulfanyl-1,2,4-triazol-1-yl)propyl]-3-pyridyl]oxy] benzonitrile + TX, 4-[[6-[2-(2,4-difluorophenyl)-1,1-difluoro-2-hydroxy-3-(5-thioxo-4H-1,2,4-triazol-1-yl)propyl]-3-pyridyl]oxy]benzonitrile + TX, trinexapac + TX, coumoxystrobin + TX, zhongshengmycin + TX, thiodiazole copper + TX, zinc thiazole + TX, amectotractin + TX, iprodione + TX, seboctylamine + TX; N'-[5-bromo-2-methyl-6-[(1S)-1-methyl-2-propoxy-ethoxy]-3-pyridyl]-N-ethyl-N-methyl-formamidine + TX, N'-[5-bromo-2-methyl-6-[(1R)-1-methyl-2-propoxy-ethoxy]-3-pyridyl]-N-ethyl-N-methyl-formamidine + TX, N'-[5-bromo-2-methyl-6-(1-methyl-2-propoxy-ethoxy)-3-pyridyl]-N-ethyl-N-methyl-formamidine + TX, N'-[5-chloro-2-methyl-6-(1-methyl-2-propoxy-ethoxy)-3-pyridyl]-N-ethyl-N-methyl-formamidine + TX, N'-[5-bromo-2-methyl-6-(1-methyl-2-propoxy-ethoxy)-3-pyridyl]-N-isopropyl-N-methyl-formamidine + TX (these compounds may be prepared from the methods described in WO2015/155075); N'-[5-bromo-2-methyl-6-(2-propoxypropoxy)-3-pyridyl]-N-ethyl-N-methyl-formamidine + TX (this compound may be prepared from the methods described in IPCOM000249876D); N-isopropyl-N'-[5-methoxy-2-methyl-4-(2,2,2-trifluoro-1-hydroxy-1-phenyl-ethyl)phenyl]-N-methyl-formamidine+ TX, N'-[4-(1-cyclopropyl-2,2,2-trifluoro-1-hydroxy-ethyl)-5-methoxy-2-methyl-phenyl]-N-isopropyl-N-methyl-formamidine + TX (these compounds may be prepared from the methods described in WO2018/228896); N-ethyl-N'-[5-methoxy-2-methyl-4-[(2-trifluoromethyl)oxetan-2-yl]phenyl]-N-methyl-formamidine + TX, N-ethyl-N'-[5-methoxy-2-methyl-4-[(2-trifuoromethyl)tetrahydrofuran-2-yl]phenyl]-N-methyl-formamidine + TX (these compounds may be prepared from the methods described in WO2019/110427); N-[(1R)-1-benzyl-3-chloro-1-methyl-but-3-enyl]-8-fluoro-quinoline-3-carboxamide + TX, N-[(1S)-1-benzyl-3-chloro-1-methyl-but-3-enyl]-8-fluoro-quinoline-3-carboxamide + TX, N-[(1R)-1-benzyl-3,3,3-trifluoro-1-methyl-propyl]-8-fluoro-quinoline-3-carboxamide + TX, N-[(1S)-1-benzyl-3,3,3-trifluoro-1-methyl-propyl]-8-fluoro-quinoline-3-carboxamide + TX, N-[(1R)-1-benzyl-1,3-dimethyl-butyl]-7,8-difluoro-quinoline-3-carboxamide + TX, N-[(1S)-1-benzyl-1,3-dimethyl-butyl]-7,8-difluoro-quinoline-3-carboxamide + TX, 8-fluoro-N-[(1R)-1-[(3-fluorophenyl)methyl]-1,3-dimethyl-butyl]quinoline-3-carboxamide + TX, 8-fluoro-N-[(1S)-1-[(3-fluorophenyl)methyl]-1,3-dimethyl-butyl]quinoline-3-carboxamide + TX, N-[(1R)-1-benzyl-1,3-dimethyl-butyl]-8-fluoro-quinoline-3-carboxamide + TX, N-[(1S)-1-benzyl-1,3-dimethyl-butyl]-8-fluoro-quinoline-3-carboxamide + TX, N-((1R)-1-benzyl-3-chloro-1-methyl-but-3-enyl)-8-fluoro-quinoline-3-carboxamide + TX, N-((1S)-1-benzyl-3-chloro-1-methyl-but-3-enyl)-8-fluoro-quinoline-3-carboxamide + TX (these compounds may be prepared from the methods described in WO2017/153380); 1-(6,7-dimethylpyrazolo[1,5-a]pyridin-3-yl)-4,4,5-trifluoro-3,3-dimethyl-isoquinoline + TX, 1-(6,7-dimethylpyrazolo[1,5-a]pyridin-3-yl)-4,4,6-trifluoro-3,3-dimethyl-isoquinoline + TX, 4,4-difluoro-3,3-dimethyl-1-(6-methylpyrazolo[1,5-a]pyridin-3-yl)isoquinoline + TX, 4,4-difluoro-3,3-dimethyl-1-(7-methylpyrazolo[1,5-a]pyridin-3-yl)isoquinoline + TX, 1-(6-chloro-7-methyl-pyrazolo[1,5-a]pyridin-3-yl)-4,4-difluoro-3,3-dimethyl-isoquinoline + TX (these compounds may be prepared from the methods described in WO2017/025510); 1-(4,5-dimethylbenzimidazol-1-yl)-4,4,5-trifluoro-3,3-dimethyl-isoquinoline + TX, 1-(4,5-dimethylbenzimidazol-1-yl)-4,4-difluoro-3,3-dimethyl-isoquinoline + TX, 6-chloro-4,4-difluoro-3,3-dimethyl-1-(4-methylbenzimidazol-1-yl)isoquinoline + TX, 4,4-difluoro-1-(5-fluoro-4-methyl-benzimidazol-1-yl)-3,3-dimethyl-isoquinoline + TX, 3-(4,4-difluoro-3,3-dimethyl-1-isoquinolyl)-7,8-dihydro-6H-cyclopenta[e]benzimidazole + TX (these compounds may be prepared from the methods described in WO2016/156085); N-methoxy-N-[[4-[5-(trifluoromethyl)-1,2,4-oxadiazol-3-yl]phenyl]methyl]cyclopropanecarboxamide + TX, N,2-dimethoxy-N-[[4-[5-(trifluoromethyl)-1,2,4-oxadiazol-3-yl]phenyl]methyl]propanamide + TX, N-ethyl-2-methyl-N-[[4-[5-(trifluoromethyl)-1,2,4-oxadiazol-3-yl]phenyl]methyl]propanamide + TX, 1-methoxy-3-methyl-1-[[4-[5-(trifluoromethyl)-1,2,4-oxadiazol-3-yl]phenyl]methyl]urea + TX, 1,3-dimethoxy-1-[[4-[5-(trifluoromethyl)-1,2,4-oxadiazol-3-yl]phenyl]methyl]urea + TX, 3-ethyl-1-methoxy-1-[[4-[5-(trifluoromethyl)-1,2,4-oxadiazol-3-yl]phenyl]methyl]urea + TX, N-[[4-[5-(trifluoromethyl)-1,2,4-oxadiazol-3-yl]phenyl]methyl]propanamide + TX, 4,4-dimethyl-2-[[4-[5-(trifluoromethyl)-1,2,4-oxadiazol-3-yl]phenyl]methyl]isoxazolidin-3-one + TX, 5,5-dimethyl-2-[[4-[5-(trifluoromethyl)-1,2,4-oxadiazol-3-yl]phenyl]methyl]isoxazolidin-3-one + TX, ethyl 1-[[4-[5-(trifluoromethyl)-1,2,4-oxadiazol-3-yl]phenyl]methyl]pyrazole-4-carboxylate + TX, N,N-dimethyl-1-[[4-[5-(trifluoromethyl)-1,2,4-oxadiazol-3-yl]phenyl]methyl]-1,2,4-triazol-3-amine + TX. The compounds in this paragraph may be prepared from the methods described in WO 2017/055473, WO 2017/055469, WO 2017/093348 and WO 2017/118689; 2-[6-(4-chlorophenoxy)-2-(trifluoromethyl)-3-pyridyl]-1-(1,2,4-triazol-1-yl)propan-2-ol + TX (this compound may be prepared from the methods described in WO 2017/029179); 2-[6-(4-bromophenoxy)-2-(trifluoromethyl)-3-pyridyl]-1-(1,2,4-triazol-1-yl)propan-2-ol + TX (this compound may be prepared from the methods described in WO 2017/029179); 3-[2-(1-chlorocyclopropyl)-3-(2-fluorophenyl)-2-hydroxy-propyl]imidazole-4-carbonitrile + TX (this compound may be prepared from the methods described in WO 2016/156290); 3-[2-(1-chlorocyclopropyl)-3-(3-chloro-2-fluoro-phenyl)-2-hydroxy-propyl]imidazole-4-carbonitrile + TX (this compound may be prepared from the methods described in WO 2016/156290); (4-phenoxyphenyl)methyl 2-amino-6-methyl-pyridine-3-carboxylate + TX (this compound may be prepared from the methods described in WO 2014/006945); 2,6-Dimethyl-1H,5H-[1,4]dithiino[2,3-c:5,6-c']dipyrrole-1,3,5,7(2H,6H)-tetrone + TX (this compound may be prepared from the methods described in WO 2011/138281); N-methyl-4-[5-(trifluoromethyl)-1,2,4-oxadiazol-3-yl]benzenecarbothioamide + TX; N-methyl-4-[5-(trifluoromethyl)-1,2,4-oxadiazol-3-yl]benzamide + TX; (Z,2E)-5-[1-(2,4-dichlorophenyl)pyrazol-3-yl]oxy-2-methoxyimino-N,3-dimethyl-pent-3-enamide + TX (this compound may be prepared from the methods described in WO 2018/153707); N'-(2-chloro-5-methyl-4-phenoxyphenyl)-N-ethyl-N-methyl-formamidine + TX; N'-[2-chloro-4-(2-fluorophenoxy)-5-methyl-phenyl]-N-ethyl-N-methyl-formamidine + TX (this compound may be prepared from the methods described in WO 2016/202742); 2-(difluoromethyl)-N-[(3S)-3-ethyl-1,1-dimethyl-indan-4-yl]pyridine-3-carboxamide + TX (this compound may be prepared from the methods described in WO 2014/095675); (5-methyl-2-pyridyl)-[4-[5-(trifluoromethyl)-1,2,4-oxadiazol-3-yl]phenyl]methanone + TX, (3-methylisoxazol-5-yl)-[4-[5-(trifluoromethyl)-1,2,4-oxadiazol-3-yl]phenyl]methanone + TX (these compounds may be prepared from the methods described in WO 2017/220485); 2-oxo-N-propyl-2-[4-[5-(trifluoromethyl)-1,2,4-oxadiazol-3-yl]phenyl]acetamide + TX (this compound may be prepared from the methods described in WO 2018/065414); ethyl 1-[[5-[5-(trifluoromethyl)-1,2,4-oxadiazol-3-yl]-2-thienyl]methyl]pyrazole-4-carboxylate + TX (this compound may be prepared from the methods described in WO 2018/158365); 2,2-difluoro-N-methyl-2-[4-[5-(trifluoromethyl)-1,2,4-oxadiazol-3-yl]phenyl]acetamide + TX, N-[(E)-methoxyiminomethyl]-4-[5-(trifluoromethyl)-1,2,4-oxadiazol-3-yl]benzamide + TX, N-[(Z)-methoxyiminomethyl]-4-[5-(trifluoromethyl)-1,2,4-oxadiazol-3-yl]benzamide + TX, N-[N-methoxy-C-methyl-carbonimidoyl]-4-[5-(trifluoromethyl)-1,2,4-oxadiazol-3-yl]benzamide + TX (these compounds may be prepared from the methods described in WO 2018/202428);
microbials including: *Acinetobacter Iwoffii +* TX, *Acremonium alternatum* + TX + TX, *Acremonium* cephalosporium + TX + TX, *Acremonium diospyri* + TX, *Acremonium obclavatum* + TX, *Adoxophyes orana granulovirus* (AdoxGV) (Capex^{®}) + TX, *Agrobacterium radiobacter strain* K84 (Galltrol-A^{®}) + TX, *Alternaria alternate +* TX, *Alternaria cassia +* TX, *Alternaria destruens* (Smolder^{®}) + TX, *Ampelomyces quisqualis* (AQ10^{®}) + TX, *Aspergillus flavus* AF36 (AF36^{®}) + TX, *Aspergillus flavus* NRRL 21882 (Aflaguard^{®}) + TX, *Aspergillus* spp. + TX, *Aureobasidium pullulans +* TX, *Azospirillum* + TX, (MicroAZ^{®} + TX, TAZO B^{®}) + TX, *Azotobacter +* TX, *Azotobacter chroocuccum* (Azotomeal^{®}) + TX, *Azotobacter* cysts (Bionatural Blooming Blossoms^{®}) + TX, *Bacillus amyloliquefaciens* + TX, *Bacillus cereus* + TX, *Bacillus chitinosporus* strain CM-1 + TX, *Bacillus chitinosporus* strain AQ746 + TX, *Bacillus licheniformis* strain HB-2 (Biostart^{™} Rhizoboost^{®}) + TX, *Bacillus licheniformis* strain 3086 (EcoGuard^{®} + TX, Green Releaf^{®}) + TX, *Bacillus circulans +* TX, *Bacillus firmus* (BioSafe^{®} + TX, BioNem-WP^{®} + TX, VOTiVO^{®}) + TX, *Bacillus firmus* strain I-1582 + TX, *Bacillus macerans +* TX, *Bacillus marismortui +* TX, *Bacillus megaterium* + TX, *Bacillus mycoides* strain AQ726 + TX, *Bacillus papillae* (Milky Spore Powder^{®}) + TX, *Bacillus pumilus* spp. + TX, *Bacillus pumilus* strain GB34 (Yield Shield^{®}) + TX, *Bacillus pumilus* strain AQ717 + TX, *Bacillus pumilus* strain QST 2808 (Sonata^{®} + TX, Ballad Plus^{®}) + TX, *Bacillus spahericus* (VectoLex^{®}) + TX, *Bacillus* spp. + TX, *Bacillus* spp. strain AQ175 + TX, *Bacillus* spp. strain AQ177 + TX, *Bacillus* spp. strain AQ178 + TX, *Bacillus subtilis* strain QST 713 (CEASE^{®} + TX, Serenade^{®} + TX, Rhapsody^{®}) + TX, *Bacillus subtilis* strain QST 714 (JAZZ^{®}) + TX, *Bacillus subtilis* strain AQ153 + TX, *Bacillus subtilis* strain AQ743 + TX, *Bacillus subtilis* strain QST3002 + TX, *Bacillus subtilis* strain QST3004 + TX, *Bacillus subtilis var. amyloliquefaciens* strain FZB24 (Taegro^{®} + TX, Rhizopro^{®}) + TX, *Bacillus thuringiensis* Cry 2Ae + TX, *Bacillus thuringiensis* Cry1Ab + TX, *Bacillus thuringiensis aizawai* GC 91 (Agree^{®}) + TX, *Bacillus thuringiensis israelensis* (BMP123^{®} + TX, Aquabac^{®} + TX, VectoBac^{®}) + TX, *Bacillus thuringiensis kurstaki* (Javelin^{®} + TX, Deliver^{®} + TX, CryMax^{®} + TX, Bonide^{®} + TX, Scutella WP^{®} + TX, Turilav WP ^{®} + TX, Astuto^{®} + TX, Dipel WP^{®} + TX, Biobit^{®} + TX, Foray^{®}) + TX, *Bacillus thuringiensis kurstaki* BMP 123 (Baritone^{®}) + TX, *Bacillus thuringiensis kurstaki* HD-1 (Bioprotec-CAF / 3P^{®}) + TX, *Bacillus thuringiensis* strain BD#32 + TX, *Bacillus thuringiensis* strain AQ52 + TX, *Bacillus thuringiensis var. aizawai* (XenTari^{®} + TX, DiPel^{®}) + TX, bacteria spp. (GROWMEND^{®} + TX, GROWSWEET^{®} + TX, Shootup^{®}) + TX, bacteriophage of *Clavipacter michiganensis* (AgriPhage^{®}) + TX, Bakflor^{®} + TX, *Beauveria bassiana* (Beaugenic^{®} + TX, Brocaril WP^{®}) + TX, *Beauveria bassiana* GHA (Mycotrol ES^{®} + TX, Mycotrol O^{®} + TX, BotaniGuard^{®}) + TX, *Beauveria brongniartii* (Engerlingspilz^{®} + TX, Schweizer Beauveria^{®} + TX, Melocont^{®}) + TX, *Beauveria* spp. + TX, *Botrytis cineria* + TX, *Bradyrhizobium japonicum* (TerraMax^{®}) + TX, *Brevibacillus brevis +* TX, *Bacillus thuringiensis tenebrionis* (Novodor^{®}) + TX, BtBooster + TX, *Burkholderia cepacia* (Deny^{®} + TX, Intercept^{®} + TX, Blue Circle^{®}) + TX, *Burkholderia gladii +* TX, *Burkholderia gladioli +* TX, *Burkholderia* spp. + TX, Canadian thistle fungus (CBH Canadian Bioherbicide^{®}) + TX, *Candida butyri +* TX, *Candida famata +* TX, *Candida fructus +* TX, *Candida glabrata* + TX, *Candida guilliermondii +* TX, *Candida melibiosica* + TX, *Candida oleophila* strain O + TX, *Candida parapsilosis +* TX, *Candida pelliculosa +* TX, *Candida pulcherrima* + TX, *Candida reukaufii* + TX, *Candida saitoana* (Bio-Coat^{®} + TX, Biocure^{®}) + TX, *Candida sake* + TX, *Candida* spp. + TX, *Candida tenius* + TX, *Cedecea dravisae* + TX, *Cellulomonas flavigena* + TX, *Chaetomium cochliodes* (Nova-Cide^{®}) + TX, *Chaetomium globosum* (Nova-Cide^{®}) + TX, *Chromobacterium subtsugae* strain PRAA4-1T (Grandevo^{®}) + TX, *Cladosporium cladosporioides +* TX, *Cladosporium oxysporum* + TX, *Cladosporium chlorocephalum +* TX, *Cladosporium* spp. + TX, *Cladosporium tenuissimum* + TX, *Clonostachys rosea* (EndoFine^{®}) + TX, *Colletotrichum acutatum* + TX, *Coniothyrium minitans* (Cotans WG^{®}) + TX, *Coniothyrium* spp. + TX, *Cryptococcus albidus* (YIELDPLUS^{®}) + TX, *Cryptococcus humicola* + TX, *Cryptococcus infirmo-miniatus +* TX, *Cryptococcus laurentii +* TX, *Cryptophlebia leucotreta granulovirus* (Cryptex^{®}) + TX, *Cupriavidus campinensis +* TX, *Cydia pomonella granulovirus* (CYD-X^{®}) + TX, *Cydia pomonella granulovirus* (Madex^{®} + TX, Madex Plus^{®} + TX, Madex Max/ Carpovirusine^{®}) + TX, *Cylindrobasidium laeve* (Stumpout^{®}) + TX, *Cylindrocladium* + TX, *Debaryomyces hansenii +* TX, *Drechslera hawaiinensis* + TX, *Enterobacter cloacae +* TX, *Enterobacteriaceae* + TX, *Entomophtora virulenta* (Vektor^{®}) + TX, *Epicoccum nigrum* + TX, *Epicoccum purpurascens* + TX, *Epicoccum* spp. + TX, *Filobasidium floriforme* + TX, *Fusarium acuminatum* + TX, *Fusarium chlamydosporum* + TX, *Fusarium oxysporum* (Fusaclean^{®} / Biofox C^{®}) + TX, *Fusarium proliferatum* + TX, *Fusarium* spp. + TX, *Galactomyces geotrichum* + TX, *Gliocladium catenulatum* (Primastop^{®} + TX, Prestop^{®}) + TX, *Gliocladium roseum* + TX, *Gliocladium* spp. (SoilGard^{®}) + TX, *Gliocladium virens* (Soilgard^{®}) + TX, *Granulovirus* (Granupom^{®}) + TX, *Halobacillus halophilus* + TX, *Halobacillus litoralis* + TX, *Halobacillus trueperi +* TX, *Halomonas* spp. + TX, *Halomonas subglaciescola +* TX, *Halovibrio variabilis +* TX, *Hanseniaspora uvarum* + TX, *Helicoverpa armigera nucleopolyhedrovirus* (Helicovex^{®}) + TX, *Helicoverpa zea nuclear polyhedrosis virus* (Gemstar^{®}) + TX, Isoflavone - formononetin (Myconate^{®}) + TX, *Kloeckera apiculata +* TX, *Kloeckera* spp. + TX, *Lagenidium giganteum* (Laginex^{®}) + TX, *Lecanicillium longisporum* (Vertiblast^{®}) + TX, *Lecanicillium muscarium* (Vertikil^{®}) + TX, *Lymantria Dispar nucleopolyhedrosis virus* (Disparvirus^{®}) + TX, *Marinococcus halophilus* + TX, *Meira geulakonigii* + TX, *Metarhizium anisopliae* (Met52^{®}) + TX, *Metarhizium anisopliae* (Destruxin WP^{®}) + TX, *Metschnikowia fruticola* (Shemer^{®}) + TX, *Metschnikowia pulcherrima +* TX, *Microdochium dimerum* (Antibot^{®}) + TX, *Micromonospora coerulea* + TX, *Microsphaeropsis ochracea +* TX, *Muscodor albus* 620 (Muscudor^{®}) + TX, *Muscodor roseus* strain A3-5 + TX, *Mycorrhizae* spp. (AMykor^{®} + TX, Root Maximizer^{®}) + TX, *Myrothecium verrucaria* strain AARC-0255 (DiTera^{®}) + TX, BROS PLUS^{®} + TX, *Ophiostoma piliferum* strain D97 (Sylvanex^{®}) + TX, *Paecilomyces farinosus +* TX, *Paecilomyces fumosoroseus* (PFR-97^{®} + TX, PreFeRal^{®}) + TX, *Paecilomyces linacinus* (Biostat WP^{®}) + TX, *Paecilomyces lilacinus* strain 251 (MeloCon WG^{®}) + TX, *Paenibacillus polymyxa +* TX, *Pantoea agglomerans* (BlightBan C9-1^{®}) + TX, *Pantoea* spp. + TX, *Pasteuria* spp. (Econem^{®}) + TX, *Pasteuria nishizawae +* TX, *Penicillium aurantiogriseum* + TX, *Penicillium billai* (Jumpstart^{®} + TX, TagTeam^{®}) + TX, *Penicillium brevicompactum* + TX, *Penicillium frequentans +* TX, *Penicillium griseofulvum* + TX, *Penicillium purpurogenum* + TX, *Penicillium* spp. + TX, *Penicillium viridicatum* + TX, *Phlebiopsis gigantean* (Rotstop^{®}) + TX, phosphate solubilizing bacteria (Phosphomeal^{®}) + TX, *Phytophthora cryptogea +* TX, *Phytophthora palmivora* (Devine^{®}) + TX, *Pichia anomala +* TX, *Pichia guilermondii +* TX, *Pichia membranaefaciens* + TX, *Pichia onychis +* TX, *Pichia stipites +* TX, *Pseudomonas aeruginosa +* TX, *Pseudomonas aureofasciens* (Spot-Less Biofungicide^{®}) + TX, *Pseudomonas cepacia +* TX, *Pseudomonas chlororaphis* (AtEze^{®}) + TX, *Pseudomonas corrugate +* TX, *Pseudomonas fluorescens* strain A506 (BlightBan A506^{®}) + TX, *Pseudomonas putida +* TX, *Pseudomonas reactans +* TX, *Pseudomonas* spp. + TX, *Pseudomonas syringae* (Bio-Save^{®}) + TX, *Pseudomonas viridiflava +* TX, *Pseudomons fluorescens* (Zequanox^{®}) + TX, *Pseudozyma flocculosa* strain PF-A22 UL (Sporodex L^{®}) + TX, *Puccinia canaliculata* + TX, *Puccinia thlaspeos* (Wood Warrior^{®}) + TX, *Pythium paroecandrum +* TX, *Pythium oligandrum* (Polygandron^{®} + TX, Polyversum^{®}) + TX, *Pythium periplocum* + TX, *Rhanella aquatilis +* TX, *Rhanella* spp. + TX, *Rhizobia* (Dormal^{®} + TX, Vault^{®}) + TX, *Rhizoctonia +* TX, *Rhodococcus globerulus* strain AQ719 + TX, *Rhodosporidium diobovatum* + TX, *Rhodosporidium toruloides +* TX, *Rhodotorula* spp. + TX, *Rhodotorula glutinis +* TX, *Rhodotorula graminis +* TX, *Rhodotorula mucilagnosa +* TX, *Rhodotorula rubra +* TX, *Saccharomyces cerevisiae* + TX, *Salinococcus roseus* + TX, *Sclerotinia minor +* TX, *Sclerotinia minor* (SARRITOR^{®}) + TX, *Scytalidium* spp. + TX, *Scytalidium uredinicola* + TX, *Spodoptera exigua nuclear polyhedrosis virus* (Spod-X^{®} + TX, Spexit^{®}) + TX, *Serratia marcescens* + TX, *Serratia plymuthica* + TX, *Serratia* spp. + TX, *Sordaria fimicola +* TX, *Spodoptera littoralis nucleopolyhedrovirus* (Littovir^{®}) + TX, *Sporobolomyces roseus* + TX, *Stenotrophomonas maltophilia* + TX, *Streptomyces ahygroscopicus* + TX, *Streptomyces albaduncus +* TX, *Streptomyces exfoliates +* TX, *Streptomyces galbus +* TX, *Streptomyces griseoplanus +* TX, *Streptomyces griseoviridis* (Mycostop^{®}) + TX, *Streptomyces lydicus* (Actinovate^{®}) + TX, *Streptomyces lydicus* WYEC-108 (ActinoGrow^{®}) + TX, *Streptomyces violaceus +* TX, *Tilletiopsis minor +* TX, *Tilletiopsis* spp. + TX, *Trichoderma asperellum* (T34 Biocontrol^{®}) + TX, *Trichoderma gamsii* (Tenet^{®}) + TX, *Trichoderma atroviride* (Plantmate^{®}) + TX, *Trichoderma hamatum* TH 382 + TX, *Trichoderma harzianum rifai* (Mycostar^{®}) + TX, *Trichoderma harzianum* T-22 (Trianum-P^{®} + TX, PlantShield HC^{®} + TX, RootShield^{®} + TX, Trianum-G^{®}) + TX, *Trichoderma harzianum* T-39 (Trichodex^{®}) + TX, *Trichoderma inhamatum* + TX, *Trichoderma koningii +* TX, *Trichoderma* spp. LC 52 (Sentinel^{®}) + TX, *Trichoderma lignorum* + TX, *Trichoderma longibrachiatum* + TX, *Trichoderma polysporum* (Binab T^{®}) + TX, *Trichoderma taxi +* TX, *Trichoderma virens +* TX, *Trichoderma virens* (formerly Gliocladium virens GL-21) (SoilGuard^{®}) + TX, *Trichoderma viride* + TX, *Trichoderma viride* strain ICC 080 (Remedier^{®}) + TX, *Trichosporon pullulans +* TX, *Trichosporon* spp. + TX, *Trichothecium* spp. + TX, *Trichothecium roseum* + TX, *Typhula phacorrhiza* strain 94670 + TX, *Typhula phacorrhiza* strain 94671 + TX, *Ulocladium atrum* + TX, *Ulocladium oudemansii* (Botry-Zen^{®}) + TX, *Ustilago maydis +* TX, various bacteria and supplementary micronutrients (Natural II^{®}) + TX, various fungi (Millennium Microbes^{®}) + TX, *Verticillium chlamydosporium* + TX, *Verticillium lecanii* (Mycotal^{®} + TX, Vertalec^{®}) + TX, Vip3Aa20 (VIPtera^{®}) + TX, *Virgibaclillus marismortui +* TX, *Xanthomonas campestris pv. Poae* (Camperico^{®}) + TX, *Xenorhabdus bovienii +* TX, *Xenorhabdus nematophilus;*
Plant extracts including: pine oil (Retenol^{®}) + TX, azadirachtin (Plasma Neem Oil^{®} + TX, AzaGuard^{®} + TX, MeemAzal^{®} + TX, Molt-X^{®} + TX, Botanical IGR (Neemazad^{®} + TX, Neemix^{®}) + TX, canola oil (Lilly Miller Vegol^{®}) + TX, *Chenopodium ambrosioides near ambrosioides* (Requiem^{®}) + TX, *Chrysanthemum* extract (Crisant^{®}) + TX, extract of neem oil (Trilogy^{®}) + TX, essentials oils of *Labiatae* (Botania^{®}) + TX, extracts of clove rosemary peppermint and thyme oil (Garden insect killer^{®}) + TX, Glycinebetaine (Greenstim^{®}) + TX, garlic + TX, lemongrass oil (GreenMatch^{®}) + TX, neem oil + TX, *Nepeta cataria* (Catnip oil) + TX, *Nepeta catarina +* TX, nicotine + TX, oregano oil (MossBuster^{®}) + TX, *Pedaliaceae* oil (Nematon^{®}) + TX, pyrethrum + TX, *Quillaja saponaria* (NemaQ^{®}) + TX, *Reynoutria sachalinensis* (Regalia^{®} + TX, Sakalia^{®}) + TX, rotenone (Eco Roten^{®}) + TX, *Rutaceae* plant extract (Soleo^{®}) + TX, soybean oil (Ortho ecosense^{®}) + TX, tea tree oil (Timorex Gold^{®}) + TX, thymus oil + TX, AGNIQUE^{®} MMF + TX, BugOil^{®} + TX, mixture of rosemary sesame pepermint thyme and cinnamon extracts (EF 300^{®}) + TX, mixture of clove rosemary and peppermint extract (EF 400^{®}) + TX, mixture of clove pepermint garlic oil and mint (Soil Shot^{®}) + TX, kaolin (Screen^{®}) + TX, storage glucam of brown algae (Laminarin^{®});
pheromones including: blackheaded fireworm pheromone (3M Sprayable Blackheaded Fireworm Pheromone^{®}) + TX, Codling Moth Pheromone (Paramount dispenser-(CM)/ Isomate C-Plus^{®}) + TX, Grape Berry Moth Pheromone (3M MEC-GBM Sprayable Pheromone^{®}) + TX, Leafroller pheromone (3M MEC - LR Sprayable Pheromone^{®}) + TX, Muscamone (Snip7 Fly Bait^{®} + TX, Starbar Premium Fly Bait^{®}) + TX, Oriental Fruit Moth Pheromone (3M oriental fruit moth sprayable pheromone^{®}) + TX, Peachtree Borer Pheromone (Isomate-P^{®}) + TX, Tomato Pinworm Pheromone (3M Sprayable pheromone^{®}) + TX, Entostat powder (extract from palm tree) (Exosex CM^{®}) + TX, (E + TX,Z + TX,Z)-3 + TX,8 + TX,11 Tetradecatrienyl acetate + TX, (Z + TX,Z + TX,E)-7 + TX,11 + TX,13-Hexadecatrienal + TX, (E + TX,Z)-7 + TX,9-Dodecadien-1-yl acetate + TX, 2-Methyl-1-butanol + TX, Calcium acetate + TX, Scenturion^{®} + TX, Biolure^{®} + TX, Check-Mate^{®} + TX, Lavandulyl senecioate; Macrobials including: *Aphelinus abdominalis +* TX, *Aphidius ervi* (Aphelinus-System^{®}) + TX, *Acerophagus papaya +* TX, *Adalia bipunctata* (Adalia-System^{®}) + TX, *Adalia bipunctata* (Adaline^{®}) + TX, *Adalia bipunctata* (Aphidalia^{®}) + TX, *Ageniaspis citricola +* TX, *Ageniaspis fuscicollis +* TX, *Amblyseius andersoni* (Anderline^{®} + TX, Andersoni-System^{®}) + TX, *Amblyseius californicus* (Amblyline^{®} + TX, Spical^{®}) + TX, *Amblyseius cucumeris* (Thripex^{®} + TX, Bugline cucumeris^{®}) + TX, *Amblyseius fallacis* (Fallacis^{®}) + TX, *Amblyseius swirskii* (Bugline swirskii^{®} + TX, Swirskii-Mite^{®}) + TX, *Amblyseius womersleyi* (WomerMite^{®}) + TX, *Amitus hesperidum +* TX, *Anagrus atomus +* TX, *Anagyrus fusciventris +* TX, *Anagyrus kamali +* TX, *Anagyrus loecki +* TX, *Anagyrus pseudococci* (Citripar^{®}) + TX, *Anicetus benefices +* TX, *Anisopteromalus calandrae +* TX, *Anthocoris nemoralis* (Anthocoris-System^{®}) + TX, *Aphelinus abdominalis* (Apheline^{®} + TX, Aphiline^{®}) + TX, *Aphelinus asychis +* TX, *Aphidius colemani* (Aphipar^{®}) + TX, *Aphidius ervi* (Ervipar^{®}) + TX, *Aphidius gifuensis +* TX, *Aphidius matricariae* (Aphipar-M^{®}) + TX, *Aphidoletes aphidimyza* (Aphidend^{®}) + TX, *Aphidoletes aphidimyza* (Aphidoline^{®}) + TX, *Aphytis lingnanensis +* TX, *Aphytis melinus +* TX, *Aprostocetus hagenowii +* TX, *Atheta coriaria* (Staphyline^{®}) + TX, *Bombus* spp. + TX, *Bombus terrestris* (Natupol Beehive^{®}) + TX, *Bombus terrestris* (Beeline^{®} + TX, Tripol^{®}) + TX, *Cephalonomia stephanoderis +* TX, *Chilocorus nigritus +* TX, *Chrysoperla carnea* (Chrysoline^{®}) + TX, *Chrysoperla carnea* (Chrysopa^{®}) + TX, *Chrysoperla rufilabris +* TX, *Cirrospilus ingenuus +* TX, *Cirrospilus quadristriatus +* TX, *Citrostichus phyllocnistoides +* TX, *Closterocerus chamaeleon +* TX, *Closterocerus* spp. + TX, *Coccidoxenoides perminutus* (Planopar^{®}) + TX, *Coccophagus cowperi +* TX, *Coccophagus lycimnia +* TX, *Cotesia flavipes +* TX, *Cotesia plutellae +* TX, *Cryptolaemus montrouzieri* (Cryptobug^{®} + TX, Cryptoline^{®}) + TX, *Cybocephalus nipponicus +* TX, *Dacnusa sibirica +* TX, *Dacnusa sibirica* (Minusa^{®}) + TX, *Diglyphus isaea* (Diminex^{®}) + TX, *Delphastus catalinae* (Delphastus^{®}) + TX, *Delphastus pusillus +* TX, *Diachasmimorpha krausii +* TX, *Diachasmimorpha longicaudata +* TX, *Diaparsis jucunda +* TX, *Diaphorencyrtus aligarhensis* + TX, *Diglyphus isaea +* TX, *Diglyphus isaea* (Miglyphus^{®} + TX, Digline^{®}) + TX, *Dacnusa sibirica* (DacDigline^{®} + TX, Minex^{®}) + TX, *Diversinervus* spp. + TX, *Encarsia citrina* + TX, *Encarsia formosa* (Encarsia max^{®} + TX, Encarline^{®} + TX, En-Strip^{®}) + TX, *Eretmocerus eremicus* (Enermix^{®}) + TX, *Encarsia guadeloupae +* TX, *Encarsia haitiensis +* TX, *Episyrphus balteatus* (Syrphidend^{®}) + TX, *Eretmoceris siphonini +* TX, *Eretmocerus californicus +* TX, *Eretmocerus eremicus* (Ercal^{®} + TX, Eretline e^{®}) + TX, *Eretmocerus eremicus* (Bemimix^{®}) + TX, *Eretmocerus hayati +* TX, *Eretmocerus mundus* (Bemipar^{®} + TX, Eretline m^{®}) + TX, *Eretmocerus siphonini +* TX, *Exochomus quadripustulatus +* TX, *Feltiella acarisuga* (Spidend^{®}) + TX, *Feltiella acarisuga* (Feltiline^{®}) + TX, *Fopius arisanus +* TX, *Fopius ceratitivorus +* TX, Formononetin (Wirless Beehome^{®}) + TX, *Franklinothrips vespiformis* (Vespop^{®}) + TX, *Galendromus occidentalis +* TX, *Goniozus legneri +* TX, *Habrobracon hebetor +* TX, *Harmonia axyridis* (HarmoBeetle^{®}) + TX, *Heterorhabditis* spp. (Lawn Patrol^{®}) + TX, *Heterorhabditis bacteriophora* (NemaShield HB^{®} + TX, Nemaseek^{®} + TX, Terranem-Nam^{®} + TX, Terranem^{®} + TX, Larvanem^{®} + TX, B-Green^{®} + TX, NemAttack ^{®} + TX, Nematop^{®}) + TX, *Heterorhabditis megidis* (Nemasys H^{®} + TX, BioNem H^{®} + TX, Exhibitline hm^{®} + TX, Larvanem-M^{®}) + TX, *Hippodamia convergens +* TX, *Hypoaspis aculeifer* (Aculeifer-System^{®} + TX, Entomite-A^{®}) + TX, *Hypoaspis miles* (Hypoline m^{®} + TX, Entomite-M^{®}) + TX, *Lbalia leucospoides +* TX, *Lecanoideus floccissimus +* TX, *Lemophagus errabundus +* TX, *Leptomastidea abnormis +* TX, *Leptomastix dactylopii* (Leptopar^{®}) + TX, *Leptomastix epona +* TX, *Lindorus lophanthae* + TX, *Lipolexis oregmae* + TX, *Lucilia caesar* (Natufly^{®}) + TX, *Lysiphlebus testaceipes +* TX, *Macrolophus caliginosus* (Mirical-N^{®} + TX, Macroline c^{®} + TX, Mirical^{®}) + TX, *Mesoseiulus longipes +* TX, *Metaphycus flavus +* TX, *Metaphycus lounsburyi* + TX, *Micromus angulatus* (Milacewing^{®}) + TX, *Microterys flavus +* TX, *Muscidifurax raptorellus* and *Spalangia cameroni* (Biopar^{®}) + TX, *Neodryinus typhlocybae +* TX, *Neoseiulus californicus +* TX, *Neoseiulus cucumeris* (THRYPEX^{®}) + TX, *Neoseiulus fallacis +* TX, *Nesideocoris tenuis* (NesidioBug^{®} + TX, Nesibug^{®}) + TX, *Ophyra aenescens* (Biofly^{®}) + TX, *Orius insidiosus* (Thripor-l^{®} + TX, Oriline i^{®}) + TX, *Orius laevigatus* (Thripor-L^{®} + TX, Oriline I^{®}) + TX, *Orius majusculus* (Oriline m^{®}) + TX, *Orius strigicollis* (Thripor-S^{®}) + TX, *Pauesia juniperorum* + TX, *Pediobius foveolatus +* TX, *Phasmarhabditis hermaphrodita* (Nemaslug^{®}) + TX, *Phymastichus coffea* + TX, *Phytoseiulus macropilus +* TX, *Phytoseiulus persimilis* (Spidex^{®} + TX, Phytoline p^{®}) + TX, *Podisus maculiventris* (Podisus^{®}) + TX, *Pseudacteon curvatus +* TX, *Pseudacteon obtusus +* TX, *Pseudacteon tricuspis +* TX, *Pseudaphycus maculipennis +* TX, *Pseudleptomastix mexicana +* TX, *Psyllaephagus pilosus +* TX, *Psyttalia concolor* (complex) + TX, *Quadrastichus* spp. + TX, *Rhyzobius lophanthae +* TX, *Rodolia cardinalis +* TX, *Rumina decollate +* TX, *Semielacher petiolatus +* TX, *Sitobion avenae* (Ervibank^{®}) + TX, *Steinernema carpocapsae* (Nematac C^{®} + TX, Millenium^{®} + TX, BioNem C^{®} + TX, NemAttack^{®} + TX, Nemastar^{®} + TX, Capsanem^{®}) + TX, *Steinernema feltiae* (NemaShield^{®} + TX, Nemasys F^{®} + TX, BioNem F^{®} + TX, Steinernema-System^{®} + TX, NemAttack^{®} + TX, Nemaplus^{®} + TX, Exhibitline sf^{®} + TX, Scia-rid^{®} + TX, Entonem^{®}) + TX, *Steinernema kraussei* (Nemasys L^{®} + TX, BioNem L^{®} + TX, Exhibitline srb^{®}) + TX, *Steinernema riobrave* (BioVector^{®} + TX, BioVektor^{®}) + TX, *Steinernema scapterisci* (Nematac S^{®}) + TX, *Steinernema* spp. + TX, *Steinernematid* spp. (Guardian Nematodes^{®}) + TX, *Stethorus punctillum* (Stethorus^{®}) + TX, *Tamarixia radiate +* TX, *Tetrastichus setifer +* TX, *Thripobius semiluteus +* TX, *Torymus sinensis +* TX, *Trichogramma brassicae* (Tricholine b^{®}) + TX, *Trichogramma brassicae* (Tricho-Strip^{®}) + TX, *Trichogramma evanescens +* TX, *Trichogramma minutum* + TX, *Trichogramma ostriniae +* TX, *Trichogramma platneri +* TX, *Trichogramma pretiosum +* TX, *Xanthopimpla stemmator;*
other biologicals including: abscisic acid + TX, bioSea^{®} + TX, *Chondrostereum purpureum* (Chontrol Paste^{®}) + TX, *Colletotrichum gloeosporioides* (Collego^{®}) + TX, Copper Octanoate (Cueva^{®}) + TX, Delta traps (Trapline d^{®}) + TX, Erwinia amylovora (Harpin) (ProAct^{®} + TX, Ni-HIBIT Gold CST^{®}) + TX, fatty acids derived from a natural by-product of extra virgin olive oil (FLIPPER^{®}) + TX, Ferri-phosphate (Ferramol^{®}) + TX, Funnel traps (Trapline y^{®}) + TX, Gallex^{®} + TX, Grower's Secret^{®} + TX, Homo-brassonolide + TX, Iron Phosphate (Lilly Miller Worry Free Ferramol Slug & Snail Bait^{®}) + TX, MCP hail trap (Trapline f^{®}) + TX, *Microctonus hyperodae +* TX, *Mycoleptodiscus terrestris* (Des-X^{®}) + TX, BioGain^{®} + TX, Aminomite^{®} + TX, Zenox^{®} + TX, Pheromone trap (Thripline ams^{®}) + TX, potassium bicarbonate (MilStop^{®}) + TX, potassium salts of fatty acids (Sanova^{®}) + TX, potassium silicate solution (Sil-Matrix^{®}) + TX, potassium iodide + potassiumthiocyanate (Enzicur^{®}) + TX, SuffOil-X^{®} + TX, Spider venom + TX, *Nosema locustae* (Semaspore Organic Grasshopper Control^{®}) + TX, Sticky traps (Trapline YF^{®} + TX, Rebell Amarillo^{®}) + TX and Traps (Takitrapline y + b^{®}) + TX;
   (1) antibacterial agents selected from the group of:
      (1.1) bacteria, examples of which are *Bacillus mojavensis* strain R3B (Accession No. NCAIM (P) B001389) (WO 2013/034938) from Certis USA LLC, a subsidiary of Mitsui & Co. + TX; *Bacillus pumilus,* in particular strain BU F-33, having NRRL Accession No. 50185 (available as part of the CARTISSA^{®} product from BASF, EPA Reg. No. 71840-19) + TX; *Bacillus subtilis,* in particular strain QST713/AQ713 (available as SERENADE OPTI or SERENADE ASO from Bayer CropScience LP, US, having NRRL Accession No. B21661, U.S. Patent No. 6,060,051) + TX; *Bacillus subtilis* strain BU1814, (available as VELONDIS^{®} PLUS, VELONDIS^{®} FLEX and VELONDIS^{®} EXTRA from BASF SE) + TX; *Bacillus subtilis* var. *amyloliquefaciens* strain FZB24 having Accession No. DSM 10271 (available from Novozymes as TAEGRO^{®} or TAEGRO^{®} ECO (EPA Registration No. 70127-5)) + TX; *Bacillus subtilis* CX-9060 from Certis USA LLC, a subsidiary of Mitsui & Co. + TX; *Bacillus* sp., in particular strain D747 (available as DOUBLE NICKEL^{®} from Kumiai Chemical Industry Co., Ltd.), having Accession No. FERM BP-8234, U.S. Patent No. 7,094,592 + TX; *Paenibacillus* sp. strain having Accession No. NRRL B-50972 or Accession No. NRRL B-67129, WO 2016/154297 + TX; *Paenibacillus polymyxa,* in particular strain AC-1 (e.g. TOPSEED^{®} from Green Biotech Company Ltd.) + TX; *Pantoea agglomerans,* in particular strain E325 (Accession No. NRRL B-21856) (available as BLOOMTIME BIOLOGICAL^{™} FD BIOPESTICIDE from Northwest Agri Products) + TX; *Pseudomonas proradix* (e.g. PRORADIX^{®} from Sourcon Padena) + TX; and
      (1.2) fungi, examples of which are *Aureobasidium pullulans,* in particular blastospores of strain DSM14940, blastospores of strain DSM 14941 or mixtures of blastospores of strains DSM14940 and DSM14941 (e.g., BOTECTOR^{®} and BLOSSOM PROTECT^{®} from bio-ferm, CH) + TX; *Pseudozyma aphidis* (as disclosed in WO2011/151819 by Yissum Research Development Company of the Hebrew University of Jerusalem) + TX; Saccharomyces cerevisiae, in particular strains CNCM No. 1-3936, CNCM No. 1-3937, CNCM No. 1-3938 or CNCM No. 1-3939 (WO 2010/086790) from Lesaffre et Compagnie, FR;
   (2) biological fungicides selected from the group of:
      (2.1) bacteria, examples of which are *Agrobacterium radiobacter* strain K84 (e.g. GALLTROL-A^{®} from AgBioChem, CA) + TX; *Agrobacterium radiobacter* strain K1026 (e.g. NOGALL^{™} from BASF SE) + TX; *Bacillus subtilis* var. *amyloliquefaciens* strain FZB24 having Accession No. DSM 10271 (available from Novozymes as TAEGRO^{®} or TAEGRO^{®} ECO (EPA Registration No. 70127-5)) + TX; *Bacillus amyloliquefaciens,* in particular strain D747 (available as Double Nickel^{™} from Kumiai Chemical Industry Co., Ltd., having accession number FERM BP-8234, US Patent No. 7,094,592) + TX; *Bacillus amyloliquefaciens* strain F727 (also known as strain MBI110) (NRRL Accession No. B-50768, WO 2014/028521) (STARGUS^{®} from Marrone Bio Innovations) + TX; *Bacillus amyloliquefaciens* strain FZB42, Accession No. DSM 23117 (available as RHIZOVITAL^{®} from ABiTEP, DE) + TX; *Bacillus amyloliquefaciens* isolate B246 (e.g. AVOGREEN^{™} from University of Pretoria) + TX; *Bacillus licheniformis,* in particular strain SB3086, having Accession No. ATCC 55406, WO 2003/000051 (available as ECOGUARD^{®} Biofungicide and GREEN RELEAF^{™} from Novozymes) + TX + TX; *Bacillus licheniformis* FMCH001 and *Bacillus subtilis* FMCH002 (QUARTZO^{®} (WG) and PRESENCE^{®} (WP) from FMC Corporation) + TX; *Bacillus methylotrophicus* strain BAC-9912 (from Chinese Academy of Sciences' Institute of Applied Ecology) + TX; *Bacillus mojavensis* strain R3B (Accession No. NCAIM (P) B001389) (WO 2013/034938) from Certis USA LLC, a subsidiary of Mitsui & Co. + TX; *Bacillus mycoides,* isolate, having Accession No. B-30890 (available as BMJ TGAI^{®} or WG and LifeGard^{™} from Certis USA LLC, a subsidiary of Mitsui & Co.) + TX; *Bacillus pumilus,* in particular strain QST2808 (available as SONATA^{®} from Bayer CropScience LP, US, having Accession No. NRRL B-30087 and described in U.S. Patent No. 6,245,551) + TX; *Bacillus pumilus,* in particular strain GB34 (available as Yield Shield^{®} from Bayer AG, DE) + TX; *Bacillus pumilus,* in particular strain BU F-33, having NRRL Accession No. 50185 (available as part of the CARTISSA product from BASF, EPA Reg. No. 71840-19) + TX; *Bacillus subtilis,* in particular strain QST713/AQ713 (available as SERENADE OPTI or SERENADE ASO from Bayer CropScience LP, US, having NRRL Accession No. B21661 and described in U.S. Patent No. 6,060,051) + TX; *Bacillus subtilis* Y1336 (available as BIOBAC^{®} WP from Bion-Tech, Taiwan, registered as a biological fungicide in Taiwan under Registration Nos. 4764, 5454, 5096 and 5277) + TX; *Bacillus subtilis* strain MBI 600 (available as SUBTILEX from BASF SE), having Accession Number NRRL B-50595, U.S. Patent No. 5,061,495 + TX; *Bacillus subtilis* strain GB03 (available as Kodiak^{®} from Bayer AG, DE) + TX; *Bacillus subtilis* strain BU1814, (available as VELONDIS^{®} PLUS, VELONDIS^{®} FLEX and VELONDIS^{®} EXTRA from BASF SE) + TX; *Bacillus subtilis* CX-9060 from Certis USA LLC, a subsidiary of Mitsui & Co. + TX; *Bacillus subtilis* KTSB strain (FOLIACTIVE^{®} from Donaghys) + TX; *Bacillus subtilis* IAB/BS03 (AVIV^{™} from STK Bio-Ag Technologies, PORTENTO^{®} from Idai Nature) + TX; *Bacillus subtilis* strain Y1336 (available as BIOBAC^{®} WP from Bion-Tech, Taiwan, registered as a biological fungicide in Taiwan under Registration Nos. 4764, 5454, 5096 and 5277) + TX; *Paenibacillus epiphyticus* (WO 2016/020371) from BASF SE + TX; *Paenibacillus polymyxa* ssp. plantarum (WO 2016/020371) from BASF SE + TX; *Paenibacillus* sp. strain having Accession No. NRRL B-50972 or Accession No. NRRL B-67129, WO 2016/154297 + TX; *Pseudomonas chlororaphis* strain AFS009, having Accession No. NRRL B-50897, WO 2017/019448 (e.g., HOWLER^{™} and ZIO^{®} from AgBiome Innovations, US) + TX; *Pseudomonas chlororaphis,* in particular strain MA342 (e.g. CEDOMON^{®}, CERALL^{®}, and CEDRESS^{®} by Bioagri and Koppert) + TX; *Pseudomonas fluorescens* strain A506 (e.g. BLIGHTBAN^{®} A506 by NuFarm) + TX; *Pseudomonas proradix* (e.g. PRORADIX^{®} from Sourcon Padena) + TX; *Streptomyces griseoviridis* strain K61 (also known as *Streptomyces galbus* strain K61) (Accession No. DSM 7206) (MYCOSTOP^{®} from Verdera, PREFENCE^{®} from BioWorks, cf. Crop Protection 2006, 25, 468-475) + TX; *Streptomyces lydicus* strain WYEC108 (also known as *Streptomyces lydicus* strain WYCD108US) (ACTINO-IRON^{®} and ACTINOVATE^{®} from Novozymes) + TX; and
      (2.2) fungi, examples of which are *Ampelomyces quisqualis,* in particular strain AQ 10 (e.g. AQ 10^{®} by IntrachemBio Italia) + TX; *Ampelomyces quisqualis* strain AQ10, having Accession No. CNCM 1-807 (e.g., AQ 10^{®} by IntrachemBio Italia) + TX; *Aspergillus flavus* strain NRRL 21882 (products known as AFLA-GUARD^{®} from Syngenta/ChemChina) + TX; *Aureobasidium pullulans,* in particular blastospores of strain DSM14940 + TX; *Aureobasidium pullulans,* in particular blastospores of strain DSM 14941 + TX; *Aureobasidium pullulans,* in particular mixtures of blastospores of strains DSM14940 and DSM 14941 (e.g. Botector^{®} by bio-ferm, CH) + TX; *Chaetomium cupreum* (Accession No. CABI 353812) (e.g. BIOKUPRUM^{™} by AgriLife) + TX; *Chaetomium globosum* (available as RIVADIOM^{®} by Rivale) + TX; *Cladosporium cladosporioides,* strain H39, having Accession No. CBS122244, US 2010/0291039 (by Stichting Dienst Landbouwkundig Onderzoek) + TX; *Coniothyrium minitans,* in particular strain CON/M/91-8 (Accession No. DSM9660, e.g. Contans ^{®} from Bayer CropScience Biologics GmbH) + TX; *Cryptococcus flavescens,* strain 3C (NRRL Y-50378), (B2.2.99) + TX; *Dactylaria candida +* TX; Dilophosphora alopecuri (available as TWIST FUNGUS^{®}) + TX; Fusarium oxysporum, strain Fo47 (available as FUSACLEAN^{®} by Natural Plant Protection) + TX; Gliocladium catenulatum (Synonym: Clonostachys rosea f. catenulate) strain J1446 (e.g. Prestop ^{®} by Lallemand) + TX; Gliocladium roseum (also known as Clonostachys rosea f rosea), in particular strain 321U from Adjuvants Plus, strain ACM941 as disclosed in Xue (Efficacy of Clonostachys rosea strain ACM941 and fungicide seed treatments for controlling the root tot complex of field pea, Can Jour Plant Sci 83(3): 519-524), or strain IK726 (Jensen DF, et al. Development of a biocontrol agent for plant disease control with special emphasis on the near commercial fungal antagonist Clonostachys rosea strain 'IK726', Australas Plant Pathol. 2007,36:95-101) + TX; Lecanicillium lecanii (formerly known as Verticillium lecanii) conidia of strain KV01 (e.g. Vertalec^{®} by Koppert/Arysta) + TX; Metschnikowia fructicola, in particular strain NRRL Y-30752, (B2.2.3) + TX; Microsphaeropsis ochracea + TX; Muscodor roseus, in particular strain A3-5 (Accession No. NRRL 30548) + TX; Penicillium steckii (DSM 27859, WO 2015/067800) from BASF SE + TX; Penicillium vermiculatum + TX; Phlebiopsis gigantea strain VRA 1992 (ROTSTOP^{®} C from Danstar Ferment) + TX; Pichia anomala, strain WRL-076 (NRRL Y-30842), U.S. Patent No. 7,579,183 + TX; Pseudozyma flocculosa, strain PF-A22 UL (available as SPORODEX^{®} L by Plant Products Co., CA) + TX; Saccharomyces cerevisiae, in particular strain LASO2 (from Agro-Levures et Dérivés), strain LAS117 cell walls (CEREVISANE^{®} from Lesaffre, ROMEO^{®} from BASF SE), strains CNCM No. 1-3936, CNCM No. 1-3937, CNCM No. 1-3938, CNCM No. 1-3939 (WO 2010/086790) from Lesaffre et Compagnie, FR + TX; Simplicillium lanosoniveum + TX; Talaromyces flavus, strain V117b + TX; Trichoderma asperelloides JM41R (Accession No. NRRL B-50759) (TRICHO PLUS^{®} from BASF SE) + TX; Trichoderma asperellum, in particular, strain kd (e.g. T-Gro from Andermatt Biocontrol) + TX; Trichoderma asperellum, in particular strain SKT-1, having Accession No. FERM P-16510 (e.g. ECO-HOPE^{®} from Kumiai Chemical Industry), strain T34 (e.g. T34 Biocontrol by Biocontrol Technologies S.L., ES) or strain ICC 012 from Isagro + TX; Trichoderma atroviride, in particular strain SC1 (having Accession No. CBS 122089, WO 2009/116106 and U.S. Patent No. 8,431,120 (from Bi-PA)), strain 77B (T77 from Andermatt Biocontrol) or strain LU132 (e.g. Sentinel from Agrimm Technologies Limited) + TX; Trichoderma atroviride, strain CNCM 1-1237 (e.g. Esquive^{®} WP from Agrauxine, FR) + TX; Trichoderma atroviride, strain no. V08/002387 + TX; Trichoderma atroviride, strain NMI no. V08/002388 + TX; Trichoderma atroviride, strain NMI no. V08/002389 + TX; Trichoderma atroviride, strain NMI no. V08/002390 + TX; Trichoderma atroviride, strain LC52 (e.g. Tenet by Agrimm Technologies Limited) + TX; Trichoderma atroviride, strain ATCC 20476 (IMI 206040) + TX; Trichoderma atroviride, strain T11 (IMI352941/ CECT20498) + TX; Trichoderma atroviride, strain SKT-1 (FERM P-16510), JP Patent Publication (Kokai) 11-253151 A + TX; Trichoderma atroviride, strain SKT-2 (FERM P-16511), JP Patent Publication (Kokai) 11-253151 A + TX; Trichoderma atroviride, strain SKT-3 (FERM P-17021), JP Patent Publication (Kokai) 11-253151 A + TX; Trichoderma fertile (e.g. product TrichoPlus from BASF) + TX; Trichoderma gamsii (formerly T. viride), strain ICC080 (IMI CC 392151 CABI, e.g. BioDerma by AGROBIOSOL DE MEXICO, S.A. DE C.V.) + TX; Trichoderma gamsii (formerly T. viride), strain ICC 080 (IMI CC 392151 CABI) (available as BIODERMA^{®} by AGROBIOSOL DE MEXICO, S.A. DE C.V.) + TX; Trichoderma harmatum + TX; Trichoderma harmatum, having Accession No. ATCC 28012 + TX; Trichoderma harzianum strain T-22 (e.g. Trianum-P from Andermatt Biocontrol or Koppert) or strain Cepa SimbT5 (from Simbiose Agro) + TX; Trichoderma harzianum + TX; Trichoderma harzianum rifai T39 (e.g. Trichodex^{®} from Makhteshim, US) + TX; Trichoderma harzianum, strain ITEM 908 (e.g. Trianum-P from Koppert) + TX; Trichoderma harzianum, strain TH35 (e.g. Root-Pro by Mycontrol) + TX; Trichoderma harzianum, strain DB 103 (available as T-GRO^{®} 7456 by Dagutat Biolab) + TX; Trichoderma polysporum, strain IMI 206039 (e.g. Binab TF WP by BINAB Bio-Innovation AB, Sweden) + TX; Trichoderma stromaticum, having Accession No. Ts3550 (e.g. Tricovab by CEPLAC, Brazil) + TX; Trichoderma virens (also known as Gliocladium virens), in particular strain GL-21 (e.g. SoilGard by Certis, US) + TX; Trichoderma virens strain G-41, formerly known as Gliocladium virens (Accession No. ATCC 20906) (e.g., ROOTSHIELD^{®} PLUS WP and TURFSHIELD^{®} PLUS WP from BioWorks, US) + TX; Trichoderma viride, strain TV1 (e.g. Trianum-P by Koppert) + TX; Trichoderma viride, in particular strain B35 (Pietr et al., 1993, Zesz. Nauk. A R w Szczecinie 161: 125-137) + TX; mixtures of Trichoderma asperellum strain ICC 012 (also known as Trichoderma harzianum ICC012), having Accession No. CABI CC IMI 392716 and Trichoderma gamsii (formerly T. viride) strain ICC 080, having Accession No. IMI 392151 (e.g., BIO-TAM^{™} from Isagro USA, Inc. and BIODERMA^{®} by Agrobiosol de Mexico, S.A. de C.V.) + TX; Ulocladium oudemansii strain U3, having Accession No. NM 99/06216 (e.g., BOTRY-ZEN^{®} by Botry-Zen Ltd, New Zealand and BOTRYSTOP^{®} from BioWorks, Inc.) + TX; Verticillium albo-atrum (formerly V. dahliae), strain WCS850 having Accession No. WCS850, deposited at the Central Bureau for Fungi Cultures (e.g., DUTCH TRIG^{®} by Tree Care Innovations) + TX; Verticillium chlamydosporium + TX;
   (3) biological control agents having an effect for improving plant growth and/or plant health selected from the group of:
      (3.1) bacteria, examples of which are *Azospirillum brasilense* (e.g., VIGOR^{®} from KALO, Inc.) + TX; *Azospirillum lipoferum* (e.g., VERTEX-IF^{™} from TerraMax, Inc.) + TX; *Azorhizobium caulinodans,* in particular strain ZB-SK-5 + TX; *Azotobacter chroococcum,* in particular strain H23 + TX; *Azotobacter vinelandii,* in particular strain ATCC 12837 + TX; a mixture of *Azotobacter vinelandii* and *Clostridium pasteurianum* (available as INVIGORATE^{®} from Agrinos) + TX; *Bacillus amyloliquefaciens* pm414 (LOLI-PEPTA^{®} from Biofilm Crop Protection) + TX; *Bacillus amyloliquefaciens* SB3281 (ATCC # PTA-7542, WO 2017/205258) + TX; *Bacillus amyloliquefaciens* TJ1000 (available as QUIKROOTS^{®} from Novozymes) + TX; *Bacillus amyloliquefaciens,* in particular strain IN937a + TX; *Bacillus amyloliquefaciens,* in particular strain FZB42 (e.g. RHIZOVITAL^{®} from ABiTEP, DE) + TX; *Bacillus amyloliquefaciens* BS27 (Accession No. NRRL B-5015) + TX; *Bacillus cereus* family member EE128 (NRRL No. B-50917) + TX; *Bacillus cereus* family member EE349 (NRRL No. B-50928) + TX; *Bacillus cereus,* in particular strain BP01 (ATCC 55675, e.g. MEPICHLOR^{®} from Arysta Lifescience, US) + TX; *Bacillus firmus,* in particular strain CNMC 1-1582 (e.g. VOTIVO^{®} from BASF SE) + TX; *Bacillus mycoides* BT155 (NRRL No. B-50921) + TX; *Bacillus mycoides* EE118 (NRRL No. B-50918) + TX; *Bacillus mycoides* EE141 (NRRL No. B-50916) + TX; *Bacillus mycoides* BT46-3 (NRRL No. B-50922) + TX; *Bacillus pumilus,* in particular strain QST2808 (having Accession No. NRRL No. B-30087) + TX; *Bacillus pumilus,* in particular strain GB34 (e.g. YIELD SHIELD^{®} from Bayer Crop Science, DE) + TX; *Bacillus siamensis,* in particular strain KCTC 13613T + TX; *Bacillus subtilis,* in particular strain QST713/AQ713 (having NRRL Accession No. B-21661 and described in U.S. Patent No. 6,060,051, available as SERENADE^{®} OPTI or SERENADE^{®} ASO from Bayer CropScience LP, US) + TX; *Bacillus subtilis,* in particular strain AQ30002 (having Accession Nos. NRRL B-50421 and described in U.S. Patent Application No. 13/330,576) + TX; *Bacillus subtilis,* in particular strain AQ30004 (and NRRL B-50455 and described in U.S. Patent Application No. 13/330,576) + TX; *Bacillus subtilis* strain BU1814, (available as TEQUALIS^{®} from BASF SE), *Bacillus* subtilis rm303 (RHIZOMAX^{®} from Biofilm Crop Protection) + TX; *Bacillus thuringiensis* BT013A (NRRL No. B-50924) also known as *Bacillus thuringiensis 4Q7* + TX; a mixture of *Bacillus licheniformis* FMCH001 and *Bacillus subtilis* FMCH002 (available as QUARTZO^{®} (WG), PRESENCE^{®} (WP) from FMC Corporation) + TX; *Bacillus subtilis,* in particular strain MBI 600 (e.g. SUBTILEX^{®} from BASF SE) + TX; *Bacillus tequilensis,* in particular strain NII-0943 + TX; *Bradyrhizobium japonicum* (e.g. OPTIMIZE^{®} from Novozymes) + TX; *Delftia acidovorans,* in particular strain RAY209 (e.g. BIOBOOST^{®} from Brett Young Seeds) + TX; *Mesorhizobium cicer* (e.g., NODULATOR from BASF SE) + TX; *Lactobacillus* sp. (e.g. LACTOPLANT^{®} from LactoPAFI) + TX; *Rhizobium leguminosarium biovar viciae* (e.g., NODULATOR from BASF SE) + TX; Pseudomonas proradix (e.g. PRORADIX^{®} from Sourcon Padena) + TX; Pseudomonas aeruginosa, in particular strain PN1 + TX; Rhizobium leguminosarum, in particular bv. viceae strain Z25 (Accession No. CECT 4585) + TX; Paenibacillus polymyxa, in particular strain AC-1 (e.g. TOPSEED^{®} from Green Biotech Company Ltd.) + TX; Serratia marcescens, in particular strain SRM (Accession No. MTCC 8708) + TX; Sinorhizobium meliloti strain NRG-185-1 (NITRAGIN^{®} GOLD from Bayer CropScience) + TX; Thiobacillus sp. (e.g. CROPAID^{®} from Cropaid Ltd UK) + TX; and
      (3.2) fungi, examples of which are *Purpureocillium lilacinum* (previously known as *Paecilomyces lilacinus)* strain 251 (AGAL 89/030550, e.g. BioAct from Bayer CropScience Biologics GmbH) + TX; *Penicillium bilaii,* strain ATCC 22348 (e.g. JumpStart^{®} from Acceleron BioAg), *Talaromyces flavus,* strain V117b + TX; Trichoderma atroviride strain CNCM 1-1237 (e.g. Esquive^{®} WP from Agrauxine, FR), Trichoderma viride, e.g. strain B35 (Pietr et al., 1993, Zesz. Nauk. A R w Szczecinie 161: 125-137) + TX; Trichoderma atroviride strain LC52 (also known as Trichoderma atroviride strain LU132, e.g. Sentinel from Agrimm Technologies Limited) + TX; Trichoderma atroviride strain SC1 described in International Application No. PCT/IT2008/000196) + TX;Trichoderma asperellum strain kd (e.g. T-Gro from Andermatt Biocontrol) + TX; Trichoderma asperellum strain Eco-T (Plant Health Products, ZA), Trichoderma harzianum strain T-22 (e.g. Trianum-P from Andermatt Biocontrol or Koppert) + TX; Myrothecium verrucaria strain AARC-0255 (e.g. DiTera^{™} from Valent Biosciences) + TX; Penicillium bilaii strain ATCC ATCC20851 + TX; Pythium oligandrum strain M1 (ATCC 38472, e.g. Polyversum from Bioprepraty, CZ) + TX; Trichoderma virens strain GL-21 (e.g. SoilGard^{®} from Certis, USA) + TX; Verticillium albo-atrum (formerly V. dahliae) strain WCS850 (CBS 276.92, e.g. Dutch Trig from Tree Care Innovations) + TX; Trichoderma atroviride, in particular strain no. V08/002387, strain no. NMI No. V08/002388, strain no. NMI No. V08/002389, strain no. NMI No. V08/002390 + TX; Trichoderma harzianum strain ITEM 908, Trichoderma harzianum, strain TSTh20 + TX; Trichoderma harzianum strain 1295-22 + TX; Pythium oligandrum strain DV74 + TX; Rhizopogon amylopogon (e.g. comprised in Myco-Sol from Helena Chemical Company) + TX; Rhizopogon fulvigleba (e.g. comprised in Myco-Sol from Helena Chemical Company) + TX;Trichoderma virens strain GI-3 + TX;
   (4) insecticidally active biological control agents selected from
      (4.1) bacteria, examples of which are *Agrobacterium radiobacter* strain K84 (Galltrol from AgBiochem Inc.) + TX; *Bacillus amyloliquefaciens,* in particular strain PTS-4838 (e.g. AVEO from Valent Biosciences, US) + TX; *Bacillus firmus,* in particular strain CNMC 1-1582 (e.g. VOTIVO^{®} from BASF SE) + TX; *Bacillus mycoides,* isolate J. (e.g. BmJ from Certis USA LLC, a subsidiary of Mitsui & Co.) + TX; *Bacillus sphaericus,* in particular Serotype H5a5b strain 2362 (strain ABTS-1743) (e.g. VECTOLEX^{®} from Valent BioSciences, US) + TX; *Bacillus thuringiensis* subsp. aizawai, in particular strain ABTS-1857 (SD-1372, e.g. XENTARI^{®} from Valent BioSciences) + TX; *Bacillus thuringiensis* subsp. aizawai, in particular serotype H-7 (e.g. FLORBAC^{®} WG from Valent BioSciences, US) + TX; *Bacillus thuringiensis israelensis* strain BMP 144 (e.g. AQUABAC^{®} by Becker Microbial Products IL) + TX; *Bacillus thuringiensis* subsp. *israelensis* (serotype H-14) strain AM65-52 (Accession No. ATCC 1276) (e.g. VECTOBAC^{®} by Valent BioSciences, US) + TX; *Bacillus thuringiensis* subsp. *aizawai* strain GC-91 + TX; Bacillus thuringiensis var. Colmeri (e.g. TIANBAOBTC by Changzhou Jianghai Chemical Factory) + TX; Bacillus thuringiensis var. japonensis strain Buibui + TX; Bacillus thuringiensis subsp. kurstaki strain BMP 123 from Becker Microbial Products, IL + TX; Bacillus thuringiensis subsp. kurstaki strain BMP 123 by Becker Microbial Products, IL, e.g. BARITONE from Bayer CropScience + TX; Bacillus thuringiensis subsp. kurstaki strain HD-1 (e.g. DIPEL^{®} ES from Valent BioSciences, US) + TX; Bacillus thuringiensis var. kurstaki strain EVB-113-19 (e.g., BIOPROTEC^{®} from AEF Global) + TX; Bacillus thuringiensis subsp. kurstaki strain ABTS 351 + TX; Bacillus thuringiensis subsp. kurstaki strain PB 54 + TX; Bacillus thuringiensis subsp. kurstaki strain SA 11, (JAVELIN from Certis, US) + TX; Bacillus thuringiensis subsp. kurstaki strain SA 12 (THURICIDE from Certis, US) + TX; Bacillus thuringiensis subsp. kurstaki strain EG 2348 (LEPINOX from Certis, US) + TX; Bacillus thuringiensis subsp. kurstaki strain EG 7841 (CRYMAX from Certis, US) + TX; Bacillus thuringiensis subsp. tenebrionis strain NB 176 (SD-5428, e.g. NOVODOR^{®} FC from BioFa DE) + TX; Brevibacillus laterosporus (LATERAL from Ecolibrium Biologicals) + TX; Burkholderia spp., in particular Burkholderia rinojensis strain A396 (also known as Burkholderia rinojensis strain MBI 305) (Accession No. NRRL B-50319 + TX; WO 2011/106491 and WO 2013/032693 + TX; e.g. MBI206 TGAI and ZELTO^{®} from Marrone Bio Innovations) + TX; Chromobacterium subtsugae, in particular strain PRAA4-1T (MBI-203 + TX; e.g. GRANDEVO^{®} from Marrone Bio Innovations) + TX; Lecanicillium muscarium Ve6 (MYCOTAL from Koppert) + TX; Paenibacillus popilliae (formerly Bacillus popilliae + TX; e.g. MILKY SPORE POWDER^{™} and MILKY SPORE GRANULAR^{™} from St. Gabriel Laboratories) + TX; Pasteuria nishizawae strain Pn1 (CLARIVA from Syngenta/ChemChina) + TX;Serratia entomophila (e.g. INVADE^{®} by Wrightson Seeds) + TX; Serratia marcescens, in particular strain SRM (Accession No. MTCC 8708) + TX;Trichoderma asperellum (TRICHODERMAX from Novozymes) + TX; Wolbachia pipientis ZAP strain (e.g., ZAP MALES^{®} from MosquitoMate) + TX; and
      (4.2) fungi, examples of which are *Beauveria bassiana* strain ATCC 74040 (e.g. NATURALIS^{®} from Intrachem Bio Italia) + TX; *Beauveria bassiana* strain GHA (Accession No. ATCC74250, e.g. BOTANIGUARD^{®} ES and MYCONTROL-O^{®} from Laverlam International Corporation) + TX; *Beauveria bassiana* strain ATP02 (Accession No. DSM 24665) + TX;*Isaria fumosorosea* (previously known as *Paecilomyces fumosoroseus)* strain Apopka 97) PREFERAL from SePRO + TX; *Metarhizium anisopliae* 3213-1 (deposited under NRRL accession number 67074) (WO 2017/066094 + TX; Pioneer Hi-Bred International) + TX; *Metarhizium robertsii* 15013-1 (deposited under NRRL accession number 67073) + TX; *Metarhizium robertsii* 23013-3 (deposited under NRRL accession number 67075) + TX; *Paecilomyces lilacinus* strain 251 (MELOCON from Certis, US) + TX; *Zoophtora radicans +* TX;
   (5) Viruses selected from the group consisting of *Adoxophyes orana* (summer fruit tortrix) granulosis virus (GV) + TX; *Cydia pomonella* (codling moth) granulosis virus (GV) + TX; Helicoverpa armigera (cotton bollworm) nuclear polyhedrosis virus (NPV) + TX; *Spodoptera exigua* (beet armyworm) mNPV + TX; *Spodoptera frugiperda* (fall armyworm) mNPV + TX; *Spodoptera littoralis* (African cotton leafworm) NPV + TX;
   (6) Bacteria and fungi which can be added as 'inoculant' to plants or plant parts or plant organs and which, by virtue of their particular properties, promote plant growth and plant health selected from *Agrobacterium spp. + TX;* Azorhizobium caulinodans + TX; Azospirillum spp. + TX; Azotobacter spp. + TX; Bradyrhizobium spp. + TX; Burkholderia spp., in particular Burkholderia cepacia (formerly known as Pseudomonas cepacia) + TX; Gigaspora spp., or Gigaspora monosporum + TX; Glomus spp. + TX; Laccaria spp. + TX; LactoBacillus buchneri + TX; Paraglomus spp. + TX; Pisolithus tinctorus + TX; Pseudomonas spp. + TX; Rhizobium spp., in particular Rhizobium trifolii + TX; Rhizopogon spp. + TX; Scleroderma spp. + TX; Suillus spp. + TX; Streptomyces spp. + TX;
   (7) Plant extracts and products formed by microorganisms including proteins and secondary metabolites which can be used as biological control agents, selected from *Allium sativum* (NEMGUARD from Eco-Spray + TX; BRALIC from ADAMA) + TX; Armour-Zen + TX; *Artemisia absinthium +* TX; Azadirachtin (e.g. AZATIN XL from Certis, US) + TX; Biokeeper WP + TX; Brassicaceae extract, in particular oilseed rape powder or mustard powder + TX; Cassia nigricans + TX; Celastrus angulatus + TX; Chenopodium anthelminticum + TX; Chitin + TX; Dryopteris filix-mas + TX; Equisetum arvense + TX; Fortune Aza + TX; Fungastop + TX; Heads Up (Chenopodium quinoa saponin extract) + TX; PROBLAD (naturally occurring Blad polypeptide from Lupin seeds), Certis EU + TX; FRACTURE (naturally occurring Blad polypeptide from Lupin seeds), FMC + TX; Pyrethrum/Pyrethrins + TX; Quassia amara + TX; Quercus + TX; Quillaja extract (QL AGRI 35 from BASF) + TX; Reynoutria sachalinensis extract (REGALLIA / REGALIA MAXX from Marrone Bio) + TX; "Requiem ^{™} Insecticide" + TX; Rotenone + TX; ryania/ryanodine + TX; Symphytum officinale + TX; Tanacetum vulgare + TX; Thymol + TX; Thymol mixed with Geraniol (CEDROZ from Eden Research) + TX; Thymol mixed with Geraniol and Eugenol (MEVALONE from Eden Research) + TX; Triact 70 + TX; TriCon + TX; Tropaeulum majus + TX; Melaleuca alternifolia extract (TIMOREX GOLD from STK) + TX; Urtica dioica + TX; Veratrin + TX; and Viscum album + TX; and a safener, such as benoxacor + TX, cloquintocet (including cloquintocet-mexyl) + TX, cyprosulfamide + TX, dichlormid + TX, fenchlorazole (including fenchlorazole-ethyl) + TX, fenclorim + TX, fluxofenim + TX, furilazole + TX, isoxadifen (including isoxadifen-ethyl) + TX, mefenpyr (including mefenpyr-diethyl) + TX, metcamifen + TX and oxabetrinil + TX.

The mixtures as described above can be used in a method for controlling pests, which comprises applying a composition comprising a mixture as described above to the pests or their environment, with the exception of a method for treatment of the human or animal body by surgery or therapy and diagnostic methods practised on the human or animal body.

The mixtures comprising a compound of formula I selected from Tables A-1 to A-3, B-1 to B-3, C-1 to C-3, D-1 to D-3, E-1 to E-3, and F-1 to F-3 and Table P and one or more active ingredients as described above can be applied, for example, in a single "ready-mix" form, in a combined spray mixture composed from separate formulations of the single active ingredient components, such as a "tank-mix", and in a combined use of the single active ingredients when applied in a sequential manner, i.e. one after the other with a reasonably short period, such as a few hours or days. The order of applying the compounds of formula I selected from Tables A-1 to A-3, B-1 to B-3, C-1 to C-3, D-1 to D-3, E-1 to E-3, and F-1 to F-3 and Table P and the active ingredients as described above is not essential for working the present invention.

The compositions according to the invention can also comprise further solid or liquid auxiliaries, such as stabilizers, for example unepoxidized or epoxidized vegetable oils (for example epoxidized coconut oil, rapeseed oil or soya oil), antifoams, for example silicone oil, preservatives, viscosity regulators, binders and/or tackifiers, fertilizers or other active ingredients for achieving specific effects, for example bactericides, fungicides, nematocides, plant activators, molluscicides or herbicides.

The compositions according to the invention are prepared in a manner known per se, in the absence of auxiliaries for example by grinding, screening and/or compressing a solid active ingredient and in the presence of at least one auxiliary for example by intimately mixing and/or grinding the active ingredient with the auxiliary (auxiliaries). These processes for the preparation of the compositions and the use of the compounds I for the preparation of these compositions are also a subject of the invention.

The application methods for the compositions, that is the methods of controlling pests of the abovementioned type, such as spraying, atomizing, dusting, brushing on, dressing, scattering or pouring - which are to be selected to suit the intended aims of the prevailing circumstances - and the use of the compositions for controlling pests of the abovementioned type are other subjects of the invention. Typical rates of concentration are between 0.1 and 1000 ppm, preferably between 0.1 and 500 ppm, of active ingredient. The rate of application per hectare is generally 1 to 2000 g of active ingredient per hectare, in particular 10 to 1000 g/ha, preferably 10 to 600 g/ha.

A preferred method of application in the field of crop protection is application to the foliage of the plants (foliar application), it being possible to select frequency and rate of application to match the danger of infestation with the pest in question. Alternatively, the active ingredient can reach the plants via the root system (systemic action), by drenching the locus of the plants with a liquid composition or by incorporating the active ingredient in solid form into the locus of the plants, for example into the soil, for example in the form of granules (soil application). In the case of paddy rice crops, such granules can be metered into the flooded paddy-field.

The compounds of the invention and compositions thereof are also be suitable for the protection of plant propagation material, for example seeds, such as fruit, tubers or kernels, or nursery plants, against pests of the abovementioned type. The propagation material can be treated with the compound prior to planting, for example seed can be treated prior to sowing. Alternatively, the compound can be applied to seed kernels (coating), either by soaking the kernels in a liquid composition or by applying a layer of a solid composition. It is also possible to apply the compositions when the propagation material is planted to the site of application, for example into the seed furrow during drilling. These treatment methods for plant propagation material and the plant propagation material thus treated are further subjects of the invention. Typical treatment rates would depend on the plant and pest/fungi to be controlled and are generally between 1 to 200 grams per 100 kg of seeds, preferably between 5 to 150 grams per 100 kg of seeds, such as between 10 to 100 grams per 100 kg of seeds.

The term seed embraces seeds and plant propagules of all kinds including but not limited to true seeds, seed pieces, suckers, corns, bulbs, fruit, tubers, grains, rhizomes, cuttings, cut shoots and the like and means in a preferred embodiment true seeds.

The present invention also comprises seeds coated or treated with or containing a compound of formula I. The term "coated or treated with and/or containing" generally signifies that the active ingredient is for the most part on the surface of the seed at the time of application, although a greater or lesser part of the ingredient may penetrate into the seed material, depending on the method of application. When the said seed product is (re)planted, it may absorb the active ingredient. In an embodiment, the present invention makes available a plant propagation material adhered thereto with a compound of formula (I). Further, it is hereby made available, a composition comprising a plant propagation material treated with a compound of formula (I).

Seed treatment comprises all suitable seed treatment techniques known in the art, such as seed dressing, seed coating, seed dusting, seed soaking and seed pelleting. The seed treatment application of the compound formula (I) can be carried out by any known methods, such as spraying or by dusting the seeds before sowing or during the sowing/planting of the seeds.

### Biological Examples:

The Examples which follow serve to illustrate the invention. Certain compounds of the invention can be distinguished from known compounds by virtue of greater efficacy at low application rates, which can be verified by the person skilled in the art using the experimental procedures outlined in the Examples, using lower application rates if necessary, for example 50 ppm, 24 ppm, 12.5 ppm, 6 ppm, 3 ppm, 1.5 ppm, 0.8 ppm or 0.2 ppm.

### Example B1: Activity against Chilo suppressalis (Striped rice stemborer)

24-well microtiter plates with artificial diet were treated with aqueous test solutions prepared from 10'000 ppm DMSO stock solutions by pipetting. After drying, the plates were infested with L2 larvae (6-8 per well). The samples were assessed for mortality, anti-feeding effect, and growth inhibition in comparison to untreated samples 6 days after infestation. Control of Chilo suppressalis by a test sample is given when at least one of the categories mortality, anti-feedant effect, and growth inhibition is higher than the untreated sample.

The following compounds resulted in at least 80% control at an application rate of 200 ppm: P1, P3, P4, P5, P6, P7, P8, P9, P10, P11, P12, P13, P14, P15, P16, P17.

### Example B2: Activity against Diabrotica balteata (Corn root worm)

Maize sprouts placed onto an agar layer in 24-well microtiter plates were treated with aqueous test solutions prepared from 10'000 ppm DMSO stock solutions by spraying. After drying, the plates were infested with L2 larvae (6 to 10 per well). The samples were assessed for mortality and growth inhibition in comparison to untreated samples 4 days after infestation.

The following compounds gave an effect of at least 80% in at least one of the two categories (mortality or growth inhibition) at an application rate of 200 ppm: P1, P2, P3, P4, P5, P6, P7, P8, P9, P11, P12, P13, P14, P15, P16, P17.

### Example B3: Activity against Euschistus heros (Neotropical Brown Stink Bug)

Soybean leaves on agar in 24-well microtiter plates were sprayed with aqueous test solutions prepared from 10'000 ppm DMSO stock solutions. After drying the leaves were infested with N2 nymphs. The samples were assessed for mortality and growth inhibition in comparison to untreated samples 5 days after infestation.

The following compounds gave an effect of at least 80% in at least one of the two categories (mortality or growth inhibition) at an application rate of 200 ppm: P2, P4, P11, P15.

### Example B4: Activity against Myzus persicae (Green peach aphid) Feeding/Contact activity

Sunflower leaf discs were placed onto agar in a 24-well microtiter plate and sprayed with aqueous test solutions prepared from 10'000 ppm DMSO stock solutions. After drying, the leaf discs were infested with an aphid population of mixed ages. The samples were assessed for mortality 6 days after infestation.

The following compounds resulted in at least 80% mortality at an application rate of 200 ppm: P1, P2, P3, P4, P12, P14.

### Example B5: Activity against Plutella xylostella (Diamond back moth)

24-well microtiter plates with artificial diet were treated with aqueous test solutions prepared from 10'000 ppm DMSO stock solutions by pipetting. After drying, Plutella eggs were pipetted through a plastic stencil onto a gel blotting paper and the plate was closed with it. The samples were assessed for mortality and growth inhibition in comparison to untreated samples 8 days after infestation.

The following compounds gave an effect of at least 80% in at least one of the two categories (mortality or growth inhibition) at an application rate of 200 ppm: P1, P2, P3, P4, P5, P6, P7, P8, P9, P11, P12, P13, P14, P15, P16, P17.

### Example B6: Activity against Spodoptera littoralis (Egyptian cotton leaf worm)

Cotton leaf discs were placed onto agar in 24-well microtiter plates and sprayed with aqueous test solutions prepared from 10'000 ppm DMSO stock solutions. After drying the leaf discs were infested with five L1 larvae. The samples were assessed for mortality, anti-feeding effect, and growth inhibition in comparison to untreated samples 3 days after infestation. Control of Spodoptera littoralis by a test sample is given when at least one of the categories mortality, anti-feedant effect, and growth inhibition is higher than the untreated sample.

The following compounds resulted in at least 80% control at an application rate of 200 ppm: P1, P2, P3, P4, P5, P6, P7, P8, P9, P11, P12, P13, P14, P15, P16, P17.

## Claims

1. A compound of formula (I): 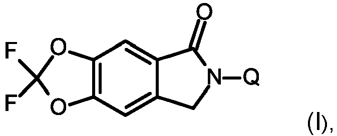 wherein
Q is a radical selected from the group consisting of formula Qa, Qb and Qc, 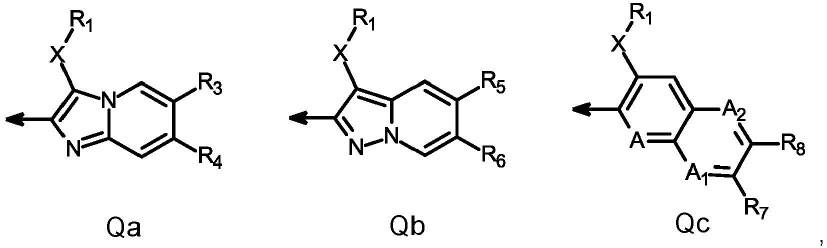
wherein the arrow denotes the point of attachment to the nitrogen atom of the tricyclic ring;
and wherein
A, A₁ and A₂ are, independently from each other, CH or N;
X is S, SO, or SO₂;
R₁ is C₁-C₄alkyl or C₃-C₆cycloalkyl-C₁-C₄alkyl;
R₃, R₄, R₅ and R₆ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, -N(R₉R₁₀), or -N(R₉)C(=O)R₁₀;
R₉ and R₁₀ are, independently from each other, hydrogen, C₁-C₄alkyl, C₁-C₆haloalkyl, or C₃-C₆cycloalkyl;
R₇ and R₈ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy or -N(R₁₁)C(=O)R₁₂; and
R₁₁ and R₁₂ are, independently from each other, hydrogen, C₁-C₄alkyl, C₁-C₆haloalkyl, or C₃-C₆cycloalkyl.

2. A compound of formula I according to claim 1, represented by the compounds of formula I-1: 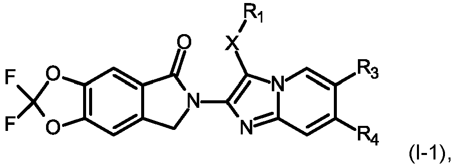 wherein X, R₁, R₃, R₄, R₉ and R₁₀ are as defined under formula I in claim 1, or an agrochemically acceptable salt, stereoisomer, enantiomer, tautomer or N-oxide of a compound of formula I-1.

3. A compound according to any one of claim 1 or claim 2, wherein R₃ and R₄ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, or -N(R₉)C(=O)R₁₀; and R₉ and R₁₀ are, independently from each other, hydrogen or C₁-C₄alkyl; preferably R₉ and R₁₀ are, independently from each other, hydrogen or methyl; more preferably R₉ is hydrogen or methyl, and R₁₀ is methyl.

4. A compound according to any one of claims 1 - 3, wherein R₄ is hydrogen and R₃ is hydrogen, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃; or the compounds of formula I-1 wherein R₃ is hydrogen and R₄ is trifluoromethyl, 1,1-difluoroethyl, - OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.

5. A compound of formula I according to claim 1, represented by the compounds of formula I-2: 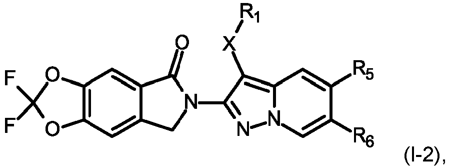 wherein X, R₁, R₅, R₆, R₉ and R₁₀ are as defined under formula I in claim 1, or an agrochemically acceptable salt, stereoisomer, enantiomer, tautomer or N-oxide of a compound of formula I-2.

6. A compound according to any one of claim 1 or claim 5, wherein R₅ and R₆ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, or -N(R₉)C(=O)R₁₀; and R₉ and R₁₀ are, independently from each other, hydrogen or C₁-C₄alkyl; preferably R₉ and R₁₀ are, independently from each other, hydrogen or methyl, more preferably R₉ is hydrogen or methyl, and R₁₀ is methyl.

7. A compound according to any one of claim 1, claim 5 or claim 6, wherein R₆ is hydrogen and R₅ is hydrogen, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or - NCH₃C(O)CH₃; or the compounds of formula I-2 wherein R₅ is hydrogen and R₆ is trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.

8. A compound of formula I according to claim 1, represented by the compounds of formula I-3: 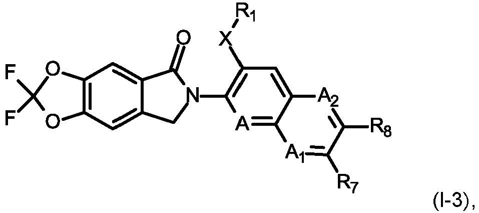 wherein A, A₁, A₂, X, R₁, R₇, R₈, R₁₁ and R₁₂ are as defined under formula I in claim 1, or an agrochemically acceptable salt, stereoisomer, enantiomer, tautomer or N-oxide of a compound of formula I-3.

9. A compound according to any one of claim 1 or claim 8, wherein R₇ and R₈ are, independently from each other, hydrogen, halogen, C₁-C₄alkyl, C₁-C₆haloalkyl, C₃-C₆cycloalkyl, C₃-C₆cycloalkyl monosubstituted by cyano, C₁-C₆cyanoalkyl, C₁-C₆cyanoalkoxy, cyano, C₁-C₄alkoxy, C₁-C₆haloalkoxy, or -N(R₁₁)C(=O)R₁₂; and R₁₁ and R₁₂ are, independently from each other, hydrogen or C₁-C₄alkyl; preferably R₁₁ and R₁₂ are, independently from each other, hydrogen or methyl; more preferably R₁₁ is hydrogen or methyl, and R₁₂ is methyl.

10. A compound according to any one of claim 1, claim 8 or claim 9, wherein R₈ is hydrogen and R₇ is hydrogen, trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or - NCH₃C(O)CH₃; or the compounds of formula I-3 wherein R₇ is hydrogen and R₈ is trifluoromethyl, 1,1-difluoroethyl, -OCHF₂, -OCH₂CHF₂, -OCH₂CF₃, cyclopropyl, cyanocyclopropyl, cyanoisopropyl, trifluoromethoxy, -CHF₂, -OC(CH₃)₂CN, -NHC(O)CH₃ or -NCH₃C(O)CH₃.

11. A compound according to any one of claim 1, claim 8, claim 9 or claim 10, wherein A is N.

12. A compound according to any one of claim 1, claim 8, claim 9, claim 10, or claim 11 wherein A₁ and A₂ are both CH.

13. A compound according to any one of claim 1, claim 8, claim 9, claim 10, or claim 11 wherein A₁ and A₂ are both N.

14. A compound according to any one of claim 1, claim 8, claim 9, claim 10, or claim 11 wherein A₁ is N and and A₂ is CH; or wherein A₁ is CH and and A₂ is N.

15. A compound according to any one of the previous claims, wherein: X is S or SO₂; preferably X is SO₂; and wherein R₁ is ethyl or cyclopropylmethyl; preferably R₁ is ethyl.

16. A compound of formula I according to claim 1, represented by the compounds of formula I-4: 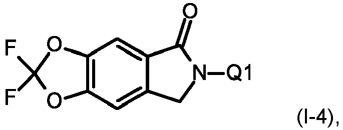 wherein
Q1 is a radical selected from the group consisting of formula Q1a, Q1b and Q1c, 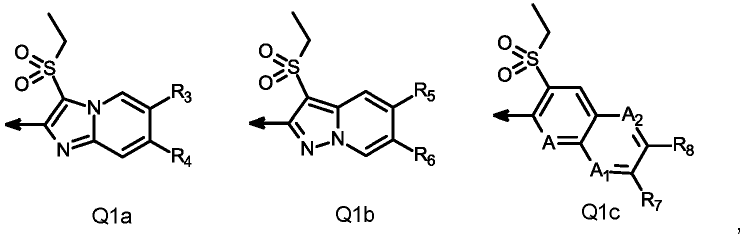
wherein the arrow denotes the point of attachment to the nitrogen atom of the tricyclic ring;
and wherein A, A₁, A₂, R₃, R₄, R₅, R₆, R₇, R₈, R₉, R₁₀, R₁₁ and R₁₂ are as defined under formula I in claim 1, or an agrochemically acceptable salt, stereoisomer, enantiomer, tautomer or N-oxide of a compound of formula I-4.

17. A compound of formula I according to claim 1, selected from the group consisting of:
6-[3-ethylsulfonyl-6-(trifluoromethyl)imidazo[1,2-a]pyridin-2-yl]-2,2-difluoro-5H-[1,3]dioxolo[4,5-f]isoindol-7-one (compound P1);
6-(3-ethylsulfonyl-2-quinolyl)-2,2-difluoro-5H-[1,3]dioxolo[4,5-f]isoindol-7-one (compound P2);
6-[3-ethylsulfonyl-6-(trifluoromethyl)pyrazolo[1,5-a]pyridin-2-yl]-2,2-difluoro-5H-[1,3]dioxolo[4,5-f]isoindol-7-one (compound P3);
6-(6-cyclopropyl-3-ethylsulfonyl-pyrazolo[1,5-a]pyridin-2-yl)-2,2-difluoro-5H-[1,3]dioxolo[4,5-f]isoindol-7-one (compound P4);
6-[3-ethylsulfonyl-7-(trifluoromethyl)imidazo[1,2-a]pyridin-2-yl]-2,2-difluoro-5H-[1,3]dioxolo[4,5-f]isoindol-7-one (compound P5);
6-[3-ethylsulfonyl-5-(trifluoromethyl)pyrazolo[1,5-a]pyridin-2-yl]-2,2-difluoro-5H-[1,3]dioxolo[4,5-f]isoindol-7-one (compound P6);
6-[6-(1,1-difluoroethyl)-3-ethylsulfonyl-imidazo[1,2-a]pyridin-2-yl]-2,2-difluoro-5H-[1,3]dioxolo[4,5-f]isoindol-7-one (compound P7);
6-[7-(1,1-difluoroethyl)-3-ethylsulfonyl-imidazo[1,2-a]pyridin-2-yl]-2,2-difluoro-5H-[1,3]dioxolo[4,5-f]isoindol-7-one (compound P8);
6-(7-bromo-3-ethylsulfonyl-imidazo[1,2-a]pyridin-2-yl)-2,2-difluoro-5H-[1,3]dioxolo[4,5-f]isoindol-7-one (compound P9);
6-(6-bromo-3-ethylsulfonyl-imidazo[1,2-a]pyridin-2-yl)-2,2-difluoro-5H-[1,3]dioxolo[4,5-f]isoindol-7-one (compound P10);
6-(6-chloro-3-ethylsulfonyl-imidazo[1,2-a]pyridin-2-yl)-2,2-difluoro-5H-[1,3]dioxolo[4,5-f]isoindol-7-one (compound P11);
6-(6-bromo-3-ethylsulfonyl-pyrazolo[1,5-a]pyridin-2-yl)-2,2-difluoro-5H-[1,3]dioxolo[4,5-f]isoindol-7-one (compound P12);
6-(7-chloro-3-ethylsulfonyl-imidazo[1,2-a]pyridin-2-yl)-2,2-difluoro-5H-[1,3]dioxolo[4,5-f]isoindol-7-one (compound P13);
6-(6-chloro-3-ethylsulfonyl-pyrazolo[1,5-a]pyridin-2-yl)-2,2-difluoro-5H-[1,3]dioxolo[4,5-f]isoindol-7-one (compound P14);
6-(5-chloro-3-ethylsulfonyl-pyrazolo[1,5-a]pyridin-2-yl)-2,2-difluoro-5H-[1,3]dioxolo[4,5-f]isoindol-7-one (compound P15);
6-(3-ethylsulfonyl-7-iodo-imidazo[1,2-a]pyridin-2-yl)-2,2-difluoro-5H-[1,3]dioxolo[4,5-f]isoindol-7-one (compound P16);
6-(3-ethylsulfonyl-6-iodo-imidazo[1,2-a]pyridin-2-yl)-2,2-difluoro-5H-[1,3]dioxolo[4,5-f]isoindol-7-one (compound P17); and
6-(3-ethylsulfonylimidazo[1,2-a]pyridin-2-yl)-2,2-difluoro-5H-[1,3]dioxolo[4,5-f]isoindol-7-one (compound P18).

18. A composition comprising an insecticidally, acaricidally, nematicidally or molluscicidally effective amount of a compound of formula (I), or an agrochemically acceptable salt, stereoisomer, enantiomer, tautomer or N-oxide thereof, as defined in any of claims 1 - 17 and, optionally, an auxiliary or diluent.

19. A method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound of formula (I), or an agrochemically acceptable salt, stereoisomer, enantiomer, tautomer or N-oxide thereof, as defined in any of claims 1 - 17 or a composition as defined claim 8, with the exception of a method for treatment of the human or animal body by surgery or therapy and diagnostic methods practised on the human or animal body.

20. A method for the protection of plant propagation material from the attack by insects, acarines, nematodes or molluscs, which comprises treating the propagation material or the site, where the propagation material is planted, with a composition according to claim 18.

## Patentansprüche

1. Verbindung der Formel (I): 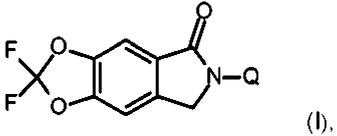 wobei
Q für einen Rest aus der Gruppe bestehend aus Formel Qa, Qb und Qc 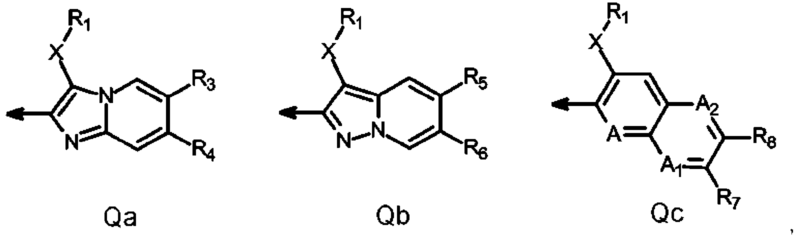
steht, wobei der Pfeil den Verknüpfungspunkt mit dem Stickstoffatom des tricyclischen Rings markiert;
und wobei
A, A₁ und A₂ unabhängig voneinander für CH oder N stehen; X für S, SO oder SO₂ steht;
R₁ für C₁-C₄-Alkyl oder C₃-C₆-Cycloalkyl-C₁-C₄-alkyl steht; R₃, R₄, R₅ und R₆ unabhängig voneinander für Wasserstoff, Halogen, C₁-C₄-Alkyl, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Cyanoalkoxy, Cyano, C₁-C₄-Alkoxy, C₁-C₆-Halogenalkoxy, -N(R₉R₁₀) oder - N(R₉)C(=O)R₁₀ stehen;
R₉ und R₁₀ unabhängig voneinander für Wasserstoff, C₁-C₄-Alkyl, C₁-C₆-Halogenalkyl oder C₃-C₆-Cycloalkyl stehen; R₇ und R₈ unabhängig voneinander für Wasserstoff, Halogen, C₁-C₄-Alkyl, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Cyanoalkoxy, Cyano, C₁-C₄-Alkoxy, C₁-C₆-Halogenalkoxy oder -N(R₁₁)C(=O)R₁₂ stehen; und
R₁₁ und R₁₂ unabhängig voneinander für Wasserstoff, Halogen, C₁-C₄-Alkyl, C₁-C₆-Halogenalkyl oder C₃-C₆-Cycloalkyl stehen.

2. Verbindung der Formel I nach Anspruch 1, wiedergegeben durch die Verbindungen der Formel I-1: 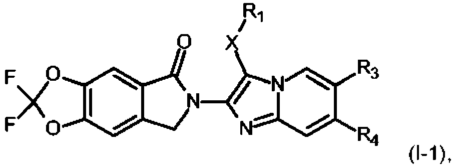 wobei X, R₁, R₃, R₄, R₉ und R₁₀ wie unter Formel I in Anspruch 1 definiert sind, oder ein agrochemisch unbedenkliches Salz, Stereoisomer, Enantiomer, Tautomer oder N-Oxid einer Verbindung der Formel I-1.

3. Verbindung nach Anspruch 1 oder Anspruch 2, wobei R₃ und R₄ unabhängig voneinander für Wasserstoff, Halogen, C₁-C₄-Alkyl, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Cyanoalkoxy, Cyano, C₁-C₄-Alkoxy, C₁-C₆-Halogenalkoxy oder -N(R₉)C(=O)R₁₀ stehen und R₉ und R₁₀ unabhängig voneinander für Wasserstoff oder C₁-C₄-Alkyl stehen; vorzugsweise R₉ und R₁₀ unabhängig voneinander für Wasserstoff oder Methyl stehen; weiter bevorzugt R₉ für Wasserstoff oder Methyl steht und R₁₀ für Methyl steht.

4. Verbindung nach einem der Ansprüche 1 - 3, wobei R₄ für Wasserstoff steht und R₃ für Wasserstoff, Trifluormethyl, 1,1-Difluorethyl, -OCHF₂, -OCH₂CHF₂, - OCH₂CF₃, Cyclopropyl, Cyanocyclopropyl, Cyanoisopropyl, Trifluormethoxy, -CHF₂, -OC(CH₃)₂CN, -NHC(O)CH₃ oder - NCH₃C(O)CH₃ steht; oder die Verbindungen der Formel I-1, wobei R₃ für Wasserstoff steht und R₄ für Trifluormethyl, 1,1-Difluorethyl, -OCHF₂, -OCH₂CHF₂, -OCH₂CF₃, Cyclopropyl, Cyanocyclopropyl, Cyanoisopropyl, Trifluormethoxy, -CHF₂, -OC(CH₃)₂CN, -NHC(O)CH₃ oder - NCH₃C(O)CH₃ steht.

5. Verbindung der Formel I nach Anspruch 1, wiedergegeben durch die Verbindungen der Formel 1-2: 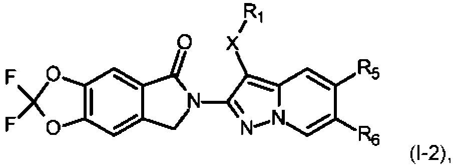 wobei X, R₁, R₅, R₆, R₉ und R₁₀ wie unter Formel I in Anspruch 1 definiert sind, oder ein agrochemisch unbedenkliches Salz, Stereoisomer, Enantiomer, Tautomer oder N-Oxid einer Verbindung der Formel 1-2.

6. Verbindung nach Anspruch 1 oder Anspruch 5, wobei R₅ und R₆ unabhängig voneinander für Wasserstoff, Halogen, C₁-C₄-Alkyl, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Cyanoalkoxy, Cyano, C₁-C₄-Alkoxy, C₁-C₆-Halogenalkoxy oder -N(R₉)C(=O)R₁₀ stehen und R₉ und R₁₀ unabhängig voneinander für Wasserstoff oder C₁-C₄-Alkyl stehen; vorzugsweise R₉ und R₁₀ unabhängig voneinander für Wasserstoff oder Methyl stehen, weiter bevorzugt R₉ für Wasserstoff oder Methyl steht und R₁₀ für Methyl steht.

7. Verbindung nach einem von Anspruch 1, Anspruch 5 oder Anspruch 6, wobei R₆ für Wasserstoff steht und R₅ für Wasserstoff, Trifluormethyl, 1,1-Difluorethyl, - OCHF₂, -OCH₂CHF₂, -OCH₂CF₃, Cyclopropyl, Cyanocyclopropyl, Cyanoisopropyl, Trifluormethoxy, -CHF₂, -OC(CH₃)₂CN, - NHC(O)CH₃ oder -NCH₃C(O)CH₃ steht; oder die Verbindungen der Formel I-2, wobei R₅ für Wasserstoff steht und R₆ für Trifluormethyl, 1,1-Difluorethyl, -OCHF₂, -OCH₂CHF₂, - OCH₂CF₃, Cyclopropyl, Cyanocyclopropyl, Cyanoisopropyl, Trifluormethoxy, -CHF₂, -OC(CH₃)₂CN, -NHC(O)CH₃ oder - NCH₃C(O)CH₃ steht.

8. Verbindung der Formel I nach Anspruch 1, wiedergegeben durch die Verbindungen der Formel 1-3: 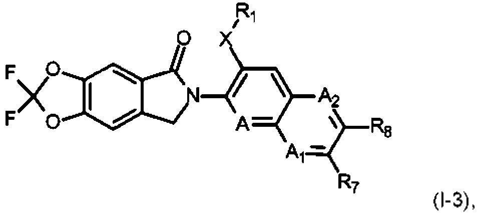 wobei A, A₁, A₂, X, R₁, R₇, R₈, R₁₁ und R₁₂ wie unter Formel I in Anspruch 1 definiert sind, oder ein agrochemisch unbedenkliches Salz, Stereoisomer, Enantiomer, Tautomer oder N-Oxid einer Verbindung der Formel 1-3.

9. Verbindung nach Anspruch 1 oder Anspruch 8, wobei R₇ und R₈ unabhängig voneinander für Wasserstoff, Halogen, C₁-C₄-Alkyl, C₁-C₆-Halogenalkyl, C₃-C₆-Cycloalkyl, C₃-C₆-Cycloalkyl, das einfach durch Cyano substituiert ist, C₁-C₆-Cyanoalkyl, C₁-C₆-Cyanoalkoxy, Cyano, C₁-C₄-Alkoxy, C₁-C₆-Halogenalkoxy oder -N(R₁₁)C(=O)R₁₂ stehen und R₁₁ und R₁₂ unabhängig voneinander für Wasserstoff oder C₁-C₄-Alkyl stehen; vorzugsweise R₁₁ und R₁₂ unabhängig voneinander für Wasserstoff oder Methyl stehen; weiter bevorzugt R₁₁ für Wasserstoff oder Methyl steht und R₁₂ für Methyl steht.

10. Verbindung nach einem von Anspruch 1, Anspruch 8 oder Anspruch 9, wobei R₈ für Wasserstoff steht und R₇ für Wasserstoff, Trifluormethyl, 1,1-Difluorethyl, - OCHF₂, -OCH₂CHF₂, -OCH₂CF₃, Cyclopropyl, Cyanocyclopropyl, Cyanoisopropyl, Trifluormethoxy, -CHF₂, -OC(CH₃)₂CN, - NHC(O)CH₃ oder -NCH₃C(O)CH₃ steht; oder die Verbindungen der Formel I-3, wobei R₇ für Wasserstoff steht und R₈ für Trifluormethyl, 1,1-Difluorethyl, -OCHF₂, -OCH₂CHF₂, - OCH₂CF₃, Cyclopropyl, Cyanocyclopropyl, Cyanoisopropyl, Trifluormethoxy, -CHF₂, -OC(CH₃)₂CN, -NHC(O)CH₃ oder - NCH₃C(O)CH₃ steht.

11. Verbindung nach einem von Anspruch 1, Anspruch 8, Anspruch 9 oder Anspruch 10, wobei A für N steht.

12. Verbindung nach einem von Anspruch 1, Anspruch 8, Anspruch 9, Anspruch 10 oder Anspruch 11, wobei A₁ und A₂ beide für CH stehen.

13. Verbindung nach einem von Anspruch 1, Anspruch 8, Anspruch 9, Anspruch 10 oder Anspruch 11, wobei A₁ und A₂ beide für N stehen.

14. Verbindung nach einem von Anspruch 1, Anspruch 8, Anspruch 9, Anspruch 10 oder Anspruch 11, wobei A₁ für N steht und A₂ für CH steht oder wobei A₁ für CH steht und A₂ für N steht.

15. Verbindung nach einem der vorhergehenden Ansprüche, wobei: X für S oder SO₂ steht; vorzugsweise X für SO₂ steht; und wobei R₁ für Ethyl oder Cyclopropylmethyl steht; vorzugsweise R₁ für Ethyl steht.

16. Verbindung der Formel I nach Anspruch 1, wiedergegeben durch die Verbindungen der Formel 1-4: 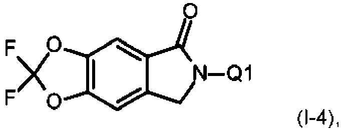 wobei
Q1 für einen Rest aus der Gruppe bestehend aus Formel Q1a, Q1b und Q1c 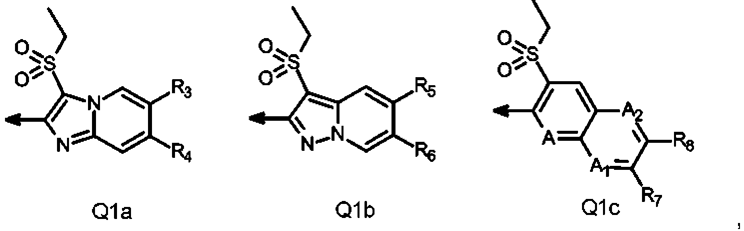
steht, wobei der Pfeil den Verknüpfungspunkt mit dem Stickstoffatom des tricyclischen Rings markiert;
und wobei A, A₁, A₂, R₃, R₄, R₅, R₆, R₇, R₈, R₉, R₁₀, R₁₁ und R₁₂ wie unter Formel I in Anspruch 1 definiert sind, oder ein agrochemisch unbedenkliches Salz, Stereoisomer, Enantiomer, Tautomer oder N-Oxid einer Verbindung der Formel I-4.

17. Verbindung der Formel I nach Anspruch 1, ausgewählt aus der Gruppe bestehend aus:
6-[3-Ethylsulfonyl-6-(trifluormethyl)imidazo[1,2-a]pyridin-2-yl]-2,2-difluor-5H-[1,3]-dioxolo[4,5-f]isoindol-7-on (Verbindung P1);
6-(3-Ethylsulfonyl-2-chinolyl)-2,2-difluor-5H-[1,3]-dioxolo[4,5-f]isoindol-7-on (Verbindung P2);
6-[3-Ethylsulfonyl-6-(trifluormethyl)pyrazolo[1,5-a]pyridin-2-yl]-2,2-difluor-5H-[1,3]-dioxolo[4,5-f]isoindol-7-on (Verbindung P3);
6-(6-Cyclopropyl-3-ethylsulfonylpyrazolo[1,5-a]pyridin-2-yl)-2,2-difluor-5H-[1,3]-dioxolo[4,5-f]isoindol-7-on (Verbindung P4);
6-[3-Ethylsulfonyl-7-(trifluormethyl)imidazo[1,2-a]pyridin-2-yl]-2,2-difluor-5H-[1,3]-dioxolo[4,5-f]isoindol-7-on (Verbindung P5);
6-[3-Ethylsulfonyl-5-(trifluormethyl)pyrazolo[1,5-a]pyridin-2-yl]-2,2-difluor-5H-[1,3]-dioxolo[4,5-f]isoindol-7-on (Verbindung P6);
6-[6-(1,1-Difluorethyl)-3-ethylsulfonylimidazo[1,2-a]pyridin-2-yl]-2,2-difluor-5H-[1,3]-dioxolo[4,5-f]isoindol-7-on (Verbindung P7);
6-[7-(1,1-Difluorethyl)-3-ethylsulfonylimidazo[1,2-a]pyridin-2-yl]-2,2-difluor-5H-[1,3]-dioxolo[4,5-f]isoindol-7-on (Verbindung P8);
6-(7-Brom-3-ethylsulfonylimidazo[1,2-a]pyridin-2-yl)-2,2-difluor-5H-[1,3]-dioxolo[4,5-f]isoindol-7-on (Verbindung P9);
6-(6-Brom-3-ethylsulfonylimidazo[1,2-a]pyridin-2-yl)-2,2-difluor-5H-[1,3]-dioxolo[4,5-f]isoindol-7-on (Verbindung P10);
6-(6-Chlor-3-ethylsulfonylimidazo[1,2-a]pyridin-2-yl)-2,2-difluor-5H-[1,3]-dioxolo[4,5-f]isoindol-7-on (Verbindung P11);
6-(6-Brom-3-ethylsulfonylpyrazolo[1,5-a]pyridin-2-yl)-2,2-difluor-5H-[1,3]-dioxolo[4,5-f]isoindol-7-on (Verbindung P12);
6-(7-Chlor-3-ethylsulfonylimidazo[1,2-a]pyridin-2-yl)-2,2-difluor-5H-[1,3]-dioxolo[4,5-f]isoindol-7-on (Verbindung P13);
6-(6-Chlor-3-ethylsulfonylpyrazolo[1,5-a]pyridin-2-yl)-2,2-difluor-5H-[1,3]-dioxolo[4,5-f]isoindol-7-on (Verbindung P14);
6-(5-Chlor-3-ethylsulfonylpyrazolo[1,5-a]pyridin-2-yl)-2,2-difluor-5H-[1,3]-dioxolo[4,5-f]isoindol-7-on (Verbindung P15);
6-(3-Ethylsulfonyl-7-iodimidazo[1,2-a]pyridin-2-yl)-2,2-difluor-5H-[1,3]-dioxolo[4,5-f]isoindol-7-on (Verbindung P16);
6-(3-Ethylsulfonyl-6-iodimidazo[1,2-a]pyridin-2-yl)-2,2-difluor-5H-[1,3]-dioxolo[4,5-f]isoindol-7-on (Verbindung P17); und
6-(3-Ethylsulfonylimidazo[1,2-a]pyridin-2-yl)-2,2-difluor-5H-[1,3]-dioxolo[4,5-f]isoindol-7-on (Verbindung P18).

18. Zusammensetzung, umfassend eine insektizid, akarizid, nematizid oder molluszizid wirksame Menge einer Verbindung der Formel (I) oder eines agrochemisch unbedenklichen Salzes, Stereoisomers, Enantiomers, Tautomers oder N-Oxids davon, gemäß einem der Ansprüche 1-17 und gegebenenfalls einen Hilfsstoff oder ein Verdünnungsmittel.

19. Verfahren zur Bekämpfung und Kontrolle von Insekten, Akarina, Nematoden oder Mollusken, bei dem man auf einen Schädling, den Standort eines Schädling oder eine Pflanze, die gegenüber Befall durch einen Schädling empfindlich ist, eine insektizid, akarizid, nematizid bzw. molluskizid wirksame Menge einer Verbindung der Formel (I) oder eines agrochemisch unbedenklichen Salzes, Stereoisomers, Enantiomers, Tautomers oder N-Oxids davon, gemäß einem der Ansprüche 1-17 oder einer Zusammensetzung gemäß Anspruch 8 ausbringt, mit Ausnahme von einem Verfahren zur chirurgischen oder therapeutischen Behandlung des menschlichen oder tierischen Körpers und Diagnostizierverfahren, die am menschlichen oder tierischen Körper vorgenommen werden.

20. Verfahren zum Schutz von Pflanzenfortpflanzungsmaterial gegen Befall durch Insekten, Akarina, Nematoden oder Mollusken, bei dem man das Fortpflanzungsmaterial oder die Stelle, an der das Fortpflanzungsmaterial angepflanzt ist, mit einer Zusammensetzung nach Anspruch 18 behandelt.

## Revendications

1. Composé de formule (I) : 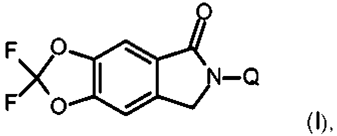
Q étant un radical choisi dans le groupe constitué par la formule Qa, Qb et Qc, 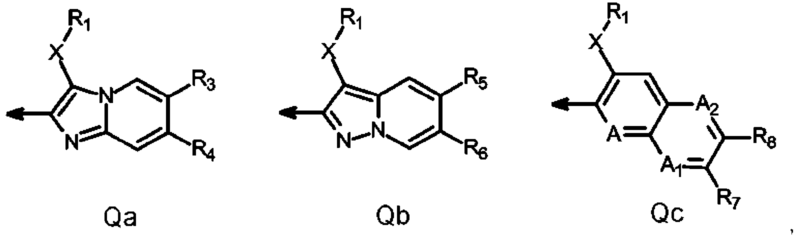
la flèche désignant le point de fixation à l'atome d'azote du cycle tricyclique ;
et
A, A₁ et A₂ étant, indépendamment l'un de l'autre, CH ou N ;
X étant S, SO ou SO₂ ;
R₁ étant C₁-C₄alkyle ou C₃-C₆cycloalkyl-C₁-C₄alkyle ;
R₃, R₄, R₅ et R₆ étant, indépendamment les uns des autres, hydrogène, halogène, C₁-C₄alkyle, C₁-C₆halogénoalkyle, C₃-C₆cycloalkyle, C₃-C₆cycloalkyle monosubstitué par cyano, C₁-C₆cyanoalkyle, C₁-C₆cyanoalcoxy, cyano, C₁-C₄alcoxy, C₁-C₆halogénoalcoxy, -N(R₉R₁₀), ou -N(R₉)C(=O)R₁₀ ;
R₉ et R₁₀ étant, indépendamment l'un de l'autre, hydrogène, C₁-C₄alkyle, C₁-C₆halogénoalkyle, ou C₃-C₆cycloalkyle ;
R₇ et R₈ étant, indépendamment l'un de l'autre, hydrogène, halogène, C₁-C₄alkyle, C₁-C₆halogénoalkyle, C₃-C₆cycloalkyle, C₃-C₆cycloalkyle monosubstitué par cyano, C₁-C₆cyanoalkyle, C₁-C₆cyanoalcoxy, cyano, C₁-C₄alcoxy, C₁-C₆halogénoalcoxy ou -N(R₁₁)C(=O)R₁₂ ; et
R₁₁ et R₁₂ étant, indépendamment l'un de l'autre, hydrogène, C₁-C₄alkyle, C₁-C₆halogénoalkyle, ou C₃-C₆cycloalkyle.

2. Composé de formule I selon la revendication 1, représenté par les composés de formule I-1 : 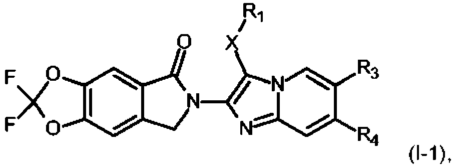 X, R₁, R₃, R₄, R₉ et R₁₀ étant tels que définis pour la formule I dans la revendication 1, ou sel, stéréoisomère, énantiomère, tautomère ou N-oxyde acceptable sur le plan agrochimique d'un composé de formule I-1.

3. Composé selon l'une quelconque parmi la revendication 1 ou la revendication 2, R₃ et R₄ étant, indépendamment l'un de l'autre, hydrogène, halogène, C₁-C₄alkyle, C₁-C₆halogénoalkyle, C₃-C₆cycloalkyle, C₃-C₆cycloalkyle monosubstitué par cyano, C₁-C₆cyanoalkyle, C₁-C₆cyanoalcoxy, cyano, C₁-C₄alcoxy, C₁-C₆halogénoalcoxy, ou -N(R₉)C(=O)R₁₀ ; et R₉ et R₁₀ étant, indépendamment l'un de l'autre, hydrogène ou C₁-C₄alkyle ; de préférence R₉ et R₁₀ étant, indépendamment l'un de l'autre, hydrogène ou méthyle ; plus préférablement R₉ étant hydrogène ou méthyle, et R₁₀ étant méthyle.

4. Composé selon l'une quelconque des revendications 1 à 3, R₄ étant hydrogène et R₃ étant hydrogène, trifluorométhyle, 1,1-difluoroéthyle, -OCHF₂, -OCH₂CHF₂, -OCH₂CF₃, cyclopropyle, cyanocyclopropyle, cyanoisopropyle, trifluorométhoxy, -CHF₂, -OC(CH₃)₂CN, - NHC(O)CH₃ ou -NCH₃C(O)CH₃ ; ou les composés de formule 1-1, R₃ étant hydrogène et R₄ étant trifluorométhyle, 1,1-difluoroéthyle, -OCHF₂, -OCH₂CHF₂, -OCH₂CF₃, cyclopropyle, cyanocyclopropyle, cyanoisopropyle, trifluorométhoxy, - CHF₂, -OC(CH₃)₂CN, -NHC(O)CH₃ ou -NCH₃C(O)CH₃.

5. Composé de formule I selon la revendication 1, représenté par les composés de formule I-2 : 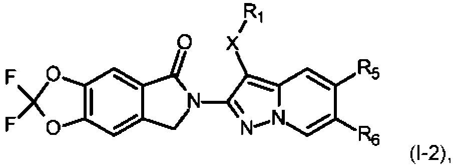 X, R₁, R₅, R₆, R₉ et R₁₀ étant tels que définis pour la formule I dans la revendication 1, ou sel, stéréoisomère, énantiomère, tautomère ou N-oxyde acceptable sur le plan agrochimique d'un composé de formule I-2.

6. Composé selon l'une quelconque parmi la revendication 1 ou la revendication 5, R₅ et R₆ étant, indépendamment l'un de l'autre, hydrogène, halogène, C₁-C₄alkyle, C₁-C₆halogénoalkyle, C₃-C₆cycloalkyle, C₃-C₆cycloalkyle monosubstitué par cyano, C₁-C₆cyanoalkyle, C₁-C₆cyanoalcoxy, cyano, C₁-C₄alcoxy, C₁-C₆halogénoalcoxy, ou -N(R₉)C(=O)R₁₀ ; et R₉ et R₁₀ étant, indépendamment l'un de l'autre, hydrogène ou C₁-C₄alkyle ; de préférence R₉ et R₁₀ étant, indépendamment l'un de l'autre, hydrogène ou méthyle ; plus préférablement R₉ étant hydrogène ou méthyle, et R₁₀ étant méthyle.

7. Composé selon l'une quelconque parmi la revendication 1, la revendication 5 ou la revendication 6, R₆ étant hydrogène et R₅ étant hydrogène, trifluorométhyle, 1,1-difluoroéthyle, -OCHF₂, -OCH₂CHF₂, -OCH₂CF₃, cyclopropyle, cyanocyclopropyle, cyanoisopropyle, trifluorométhoxy, -CHF₂, -OC(CH₃)₂CN, - NHC(O)CH₃ ou -NCH₃C(O)CH₃ ; ou les composés de formule I-2, R₅ étant hydrogène et R₆ étant trifluorométhyle, 1,1-difluoroéthyle, -OCHF₂, -OCH₂CHF₂, -OCH₂CF₃, cyclopropyle, cyanocyclopropyle, cyanoisopropyle, trifluorométhoxy, - CHF₂, -OC(CH₃)₂CN, -NHC(O)CH₃ ou -NCH₃C (O)CH₃.

8. Composé de formule I selon la revendication 1, représenté par les composés de formule I-3 : 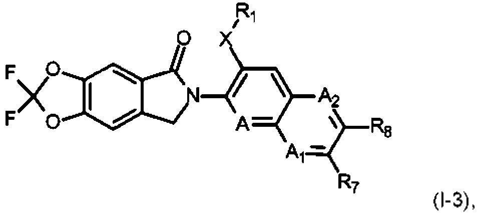 A, A₁, A₂, X, R₁, R₇, R₈, R₁₁ et R₁₂ étant tels que définis pour la formule I dans la revendication 1, ou sel, stéréoisomère, énantiomère, tautomère ou N-oxyde acceptable sur le plan agrochimique d'un composé de formule I-3.

9. Composé selon l'une quelconque parmi la revendication 1 ou la revendication 8, R₇ et R₈ étant, indépendamment l'un de l'autre, hydrogène, halogène, C₁-C₄alkyle, C₁-C₆halogénoalkyle, C₃-C₆cycloalkyle, C₃-C₆cycloalkyle monosubstitué par cyano, C₁-C₆cyanoalkyle, C₁-C₆cyanoalcoxy, cyano, C₁-C₄alcoxy, C₁-C₆halogénoalcoxy, ou -N(R₁₁)C(=O)R₁₂ ; et R₁₁ et R₁₂ étant, indépendamment l'un de l'autre, hydrogène ou C₁-C₄alkyle ; de préférence R₁₁ et R₁₂ étant, indépendamment l'un de l'autre, hydrogène ou méthyle ; plus préférablement R₁₁ étant hydrogène ou méthyle, et R₁₂ étant méthyle.

10. Composé selon l'une quelconque parmi la revendication 1, la revendication 8 ou la revendication 9, RS étant hydrogène et R₇ étant hydrogène, trifluorométhyle, 1,1-difluoroéthyle, -OCHF₂, -OCH₂CHF₂, -OCH₂CF₃, cyclopropyle, cyanocyclopropyle, cyanoisopropyle, trifluorométhoxy, -CHF₂, -OC(CH₃)₂CN, - NHC(O)CH₃ ou -NCH₃C(O)CH₃ ; ou les composés de formule I-3, R₇ étant hydrogène et R₈ étant trifluorométhyle, 1,1-difluoroéthyle, -OCHF₂, -OCH₂CHF₂, -OCH₂CF₃, cyclopropyle, cyanocyclopropyle, cyanoisopropyle, trifluorométhoxy, - CHF₂, -OC(CH₃)₂CN, -NHC(O)CH₃ ou -NCH₃C (O)CH₃.

11. Composé selon l'une quelconque parmi la revendication 1, la revendication 8, la revendication 9 ou la revendication 10, A étant N.

12. Composé selon l'une quelconque parmi la revendication 1, la revendication 8, la revendication 9, la revendication 10 ou la revendication 11, A₁ et A₂ étant tous deux CH.

13. Composé selon l'une quelconque parmi la revendication 1, la revendication 8, la revendication 9, la revendication 10 ou la revendication 11, A₁ et A₂ étant tous deux N.

14. Composé selon l'une quelconque parmi la revendication 1, la revendication 8, la revendication 9, la revendication 10 ou la revendication 11, A₁ étant N et A₂ étant CH ; ou A₁ étant CH et A₂ étant N.

15. Composé selon l'une quelconque des revendications précédentes, X étant S ou SO₂ ; de préférence X étant SO₂ ; et R₁ étant éthyle ou cyclopropylméthyle ; de préférence R₁ étant éthyle.

16. Composé de formule I selon la revendication 1, représenté par les composés de formule I-4 : 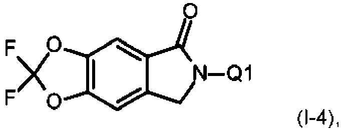
Q1 étant un radical choisi dans le groupe constitué par la formule Q1a, Q1b et Q1c, 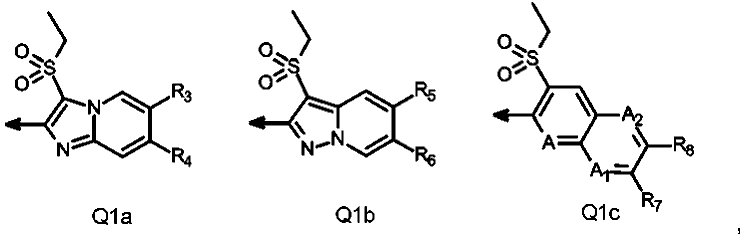
la flèche désignant le point de fixation à l'atome d'azote du cycle tricyclique ;
et A, A₁, A₂, R₃, R₄, R₅, R₆, R₇, R₈, R₉, R₁₀, R₁₁ et R₁₂ étant tels que définis pour la formule I dans la revendication 1, ou sel, stéréoisomère, énantiomère, tautomère ou N-oxyde acceptable sur le plan agrochimique d'un composé de formule I-4.

17. Composé de formule I selon la revendication 1, choisi dans le groupe constitué par :
6-[3-éthylsulfonyl-6-(trifluorométhyl)imidazo[1,2-a]pyridin-2-yl]-2,2-difluoro-5H-[1,3]dioxolo[4,5-f]isoindol-7-one (composé P1) ;
6-(3-éthylsulfonyl-2-quinoléinyl)-2,2-difluoro-5H-[1,3]dioxolo[4,5-f]isoindol-7-one (composé P2) ;
6-[3-éthylsulfonyl-6-(trifluorométhyl)pyrazolo[1,5-a]pyridin-2-yl]-2,2-difluoro-5H-[1,3]dioxolo[4,5-f]isoindol-7-one (composé P3) ;
6-(6-cyclopropyl-3-éthylsulfonyl-pyrazolo[1,5-a]pyridin-2-yl)-2,2-difluoro-5H-[1,3]dioxolo[4,5-f]isoindol-7-one (composé P4) ;
6-[3-éthylsulfonyl-7-(trifluorométhyl)imidazo[1,2-a]pyridin-2-yl]-2,2-difluoro-5H-[1,3]dioxolo[4,5-f]isoindol-7-one (composé P5) ;
6-[3-éthylsulfonyl-5-(trifluorométhyl)pyrazolo[1,5-a]pyridin-2-yl]-2,2-difluoro-5H-[1,3]dioxolo[4,5-f]isoindol-7-one (composé P6) ;
6-[6-(1,1-difluoroéthyl)-3-éthylsulfonyl-imidazo[1,2-a]pyridin-2-yl]-2,2-difluoro-5H-[1,3]dioxolo[4,5-f]isoindol-7-one (composé P7) ;
6-[7-(1,1-difluoroéthyl)-3-éthylsulfonyl-imidazo[1,2-a]pyridin-2-yl]-2,2-difluoro-5H-[1,3]dioxolo[4,5-f]isoindol-7-one (composé P8) ;
6-(7-bromo-3-éthylsulfonyl-imidazo[1,2-a]pyridin-2-yl)-2,2-difluoro-5H-[1,3]dioxolo[4,5-f]isoindol-7-one (composé P9) ;
6-(6-bromo-3-éthylsulfonyl-imidazo[1,2-a]pyridin-2-yl)-2,2-difluoro-5H-[1,3]dioxolo[4,5-f]isoindol-7-one (composé P10) ;
6-(6-chloro-3-éthylsulfonyl-imidazo[1,2-a]pyridin-2-yl)-2,2-difluoro-5H-[1,3]dioxolo[4,5-f]isoindol-7-one (composé P11) ;
6-(6-bromo-3-éthylsulfonyl-pyrazolo[1,5-a]pyridin-2-yl)-2,2-difluoro-5H-[1,3]dioxolo[4,5-f]isoindol-7-one (composé P12) ;
6-(7-chloro-3-éthylsulfonyl-imidazo[1,2-a]pyridin-2-yl)-2,2-difluoro-5H-[1,3]dioxolo[4,5-f]isoindol-7-one (composé P13) ;
6-(6-chloro-3-éthylsulfonyl-pyrazolo[1,5-a]pyridin-2-yl)-2,2-difluoro-5H-[1,3]dioxolo[4,5-f]isoindol-7-one (composé P14) ;
6-(5-chloro-3-éthylsulfonyl-pyrazolo[1,5-a]pyridin-2-yl)-2,2-difluoro-5H-[1,3]dioxolo[4,5-f]isoindol-7-one (composé P15) ;
6-(3-éthylsulfonyl-7-iodo-imidazo[1,2-a]pyridin-2-yl)-2,2-difluoro-5H-[1,3]dioxolo[4,5-f]isoindol-7-one (composé P16) ;
6-(3-éthylsulfonyl-6-iodo-imidazo[1,2-a]pyridin-2-yl)-2,2-difluoro-5H-[1,3]dioxolo[4,5-f]isoindol-7-one (composé P17) ; et
6-(3-éthylsulfonylimidazo[1,2-a]pyridin-2-yl)-2,2-difluoro-5H-[1,3]dioxolo[4,5-f]isoindol-7-one (composé P18).

18. Composition comprenant une quantité efficace sur le plan insecticide, acaricide, nématicide ou molluscicide d'un composé de formule (I), ou un sel, stéréoisomère, énantiomère, tautomère ou N-oxyde acceptable sur le plan agrochimique de celui-ci, tel que défini selon l'une quelconque des revendications 1 à 17 et, éventuellement, un auxiliaire ou diluant.

19. Procédé de combat et de lutte contre les insectes, les acariens, les nématodes ou les mollusques, qui comprend l'application à un nuisible, à un lieu où se développe un nuisible, ou à une plante susceptible d'être attaquée par un nuisible, d'une quantité efficace sur le plan insecticide, acaricide, nématicide ou molluscicide d'un composé de formule (1), ou un sel, stéréoisomère, énantiomère, tautomère ou N-oxyde acceptable sur le plan agrochimique de celui-ci, tel que défini dans l'une quelconque des revendications 1 à 17 ou d'une composition telle que définie dans la revendication 8, à l'exception d'un procédé pour le traitement du corps humain ou animal par chirurgie ou thérapie et de procédés de diagnostic pratiqués sur le corps humain ou animal.

20. Procédé pour la protection d'un matériel de propagation végétale contre l'attaque par des insectes, des acariens, des nématodes ou des mollusques, qui comprend un traitement du matériel de propagation ou du site où le matériel de propagation est planté, par une composition selon la revendication 18.
